(19) **Europäisches Patentamt European Patent Office Office européen des brevets**

(11) **EP 4 436 558 B1**

(12) **EUROPÄISCHE PATENTSCHRIFT**

(45) Veröffentlichungstag und Bekanntmachung des Hinweises auf die Patenterteilung:
**24.09.2025  Patentblatt 2025/39**

(21) Anmeldenummer: **23703715.5**

(22) Anmeldetag: **02.02.2023**

(51) Internationale Patentklassifikation (IPC):
**A61K 9/51** (2006.01)   **A61K 9/127** (2025.01)
**A61K 47/69** (2017.01)   **A61K 31/7105** (2006.01)
**A61K 47/28** (2006.01)   **A61K 48/00** (2006.01)
**A61P 35/00** (2006.01)   **A61K 9/00** (2006.01)
**A61K 47/10** (2017.01)   **A61K 47/12** (2006.01)
**A61K 47/14** (2017.01)   **A61K 47/24** (2006.01)
**A61K 47/26** (2006.01)

(52) Gemeinsame Patentklassifikation (CPC):
**A61K 9/5123; A61K 9/0014; A61K 9/0019;
A61K 9/0053; A61K 9/1272; A61K 39/12;
A61K 47/10; A61K 47/12; A61K 47/14;
A61K 47/24; A61K 47/26; A61K 47/28;
A61P 35/00; C12N 15/88;** C12N 2770/20034

(86) Internationale Anmeldenummer:
**PCT/EP2023/052618**

(87) Internationale Veröffentlichungsnummer:
**WO 2023/148303 (10.08.2023 Gazette 2023/32)**

(54) **VERFAHREN ZUR MEDIKAMENT- UND IMPFSTOFFHERSTELLUNG**

METHOD FOR PRODUCING MEDICATIONS AND VACCINES

PROCÉDÉ DE PRODUCTION DE MÉDICAMENTS ET DE VACCINS

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität:  **02.02.2022  DE 102022102399
08.04.2022  DE 102022108643
23.06.2022  DE 102022115653**

(43) Veröffentlichungstag der Anmeldung:
**02.10.2024  Patentblatt 2024/40**

(73) Patentinhaber: **MSLsolutions GmbH
46047 Oberhausen (DE)**

(72) Erfinder: **DAHMS, Gerd Herbert
47138 Duisburg (DE)**

(74) Vertreter: **Von Rohr Patentanwälte Partnerschaft mbB
Rüttenscheider Straße 62
45130 Essen (DE)**

(56) Entgegenhaltungen:
WO-A1-2014/136086   WO-A1-2017/194454
WO-A1-2018/006052   WO-A1-2018/078053
WO-A1-2019/051289   WO-A1-2021/022173

• **KIMURA N. ET AL.: "Development of a Microfluidic-Based Post-Treatment Process for Size-Controlled Lipid Nanoparticles and Application to siRNA Delivery", APPLIED MATERIALS & INTERFACES, vol. 12, no. 30, 15 July 2020 (2020-07-15), US, pages 34011 - 34020, XP055978520, ISSN: 1944-8244, DOI: 10.1021/ acsami.0c05489**

- UMERSKA A. ET AL.: "Antibacterial action of lipid nanocapsules containing fatty acids or monoglycerides as co-surfactants", EUROPEAN JOURNAL OF PHARMACEUTICS AND BIOPHARMACEUTICS : OFFICIAL JOURNAL OF ARBEITSGEMEINSCHAFT FÜR PHARMAZEUTISCHE VERFAHRENSTECHNIK E.V OCT 2012,, vol. 108, 3 September 2016 (2016-09-03), pages 100 - 110, XP002791470, ISSN: 1873-3441, DOI: 10.1016/ J.EJPB.2016.09.001

**Beschreibung**

Die vorliegende Erfindung betrifft das technische Gebiet der lipidbasierten Systeme, welche beispielsweise im medizinischen Bereich und insbesondere zur Verabreichung bzw. Applikation von insbesondere pharmazeutischen Wirkstoffen eingesetzt werden können, sowie in diesem Zusammenhang stehende insbesondere pharmazeutische Zusammensetzungen sowie deren Anwendungen insbesondere zu Zwecken der Verabreichung bzw. Applikation von prophylaktisch und/oder therapeutisch wirksamen Substanzen, wie Vakzin- bzw. Impfwirkstoffen, antibiotischen Wirkstoffen oder dergleichen.

Insbesondere betrifft die vorliegende Erfindung ein Verfahren zur Herstellung eines lipidbasierten Trägersystems, welches in partikulärer Form, bevorzugt in Form von Lipidnanopartikeln (LNP), vorliegt und welches insbesondere in einem flüssigen Medium (d. h. insbesondere ein bei einer Temperatur im Bereich von 20 °C bis 40 °C, vorzugsweise Raumtemperatur bzw. 20 °C, und Umgebungsdruck bzw. 1.013,25 hPa flüssiges Medium) zur Ausbildung einer diesbezüglichen Zusammensetzung eingebracht bzw. hergestellt wird bzw. vorliegt, wobei das lipidbasierte Trägersystem mit einem pharmazeutischen Wirkstoff beladen bzw. ausgerüstet werden kann. In diesem Zusammenhang betrifft die vorliegende Erfindung auch das nach dem erfindungsgemäßen Verfahren erhältliche lipidbasierte Trägersystem sowie ein diesbezügliches Trägersystem als solches.

Des Weiteren betrifft die vorliegende Erfindung auch eine Zusammensetzung, welche insbesondere als Dispersion ausgebildet ist und welche das erfindungsgemäße lipidbasierte Trägersystem enthält, wobei das Trägersystem insbesondere in einem flüssigen Medium aufgenommen ist bzw. hierin eingebracht vorliegt. Die vorliegende Erfindung betrifft auch ein Arzneimittel bzw. Medikament, welches das erfindungsgemäße Trägersystem bzw. die Zusammensetzung nach der Erfindung insbesondere zusammen mit mindestens einem pharmazeutisch akzeptablen bzw. verträglichen Exzipienten aufweist. In diesem Zusammenhang betrifft die vorliegende Erfindung auch die Anwendungen bzw. Verwendungen des Arzneimittels bzw. Medikaments nach der Erfindung, beispielsweise im Rahmen einer Ausbildung bzw. eines Einsatzes als Impfstoff oder antimikrobielle Zusammensetzung.

Bei der Verabreichung von therapeutischen (z. B. antibiotisch wirksamen Substanzen) oder prophylaktischen (z. B. als Vakzin bzw. Impfstoff wirkende Substanzen) Wirkstoffen besteht aus pharmakologischer Sicht im Allgemeinen die Notwendigkeit, dass die Wirkstoffe in pharmakologisch wirksamer Menge am Wirkort vorliegen, wie beispielsweise speziellen Organen, Geweben oder Zellen bzw. Zellsystemen, und dies auch vor dem Hintergrund, dass entsprechende Wirkstoffe oftmals instabil bzw. sensitiv gegenüber äußeren Einflüssen, wie hohen oder niedrigen Temperaturen oder dergleichen, sind. Folglich sind im Hinblick auf entsprechende Zusammensetzungen, welche derartige Wirkstoffe aufweisen, entsprechende Maßnahmen erforderlich, die einerseits zu einer Stabilisierung des Wirkstoffs und andererseits zu einer effizienten Freisetzung bzw. Bioverfügbarkeit des Wirkstoffs am Wirkort, beispielsweise einer (Körper-) Zelle, führen, wobei derartige wirkstoffhaltige Zusammensetzungen auch insgesamt als solche eine gute Verträglichkeit bzw. ein geringes Nebenwirkungsprofil aufweisen sollen, und dies bei insgesamt guter Handhabbarkeit, Herstellbarkeit und (Lager-)Stabilität. Insbesondere ist es in diesem Zusammenhang auch erforderlich, dass die in einer Zusammensetzung eingebrachten Wirkstoffe, wie zuvor angeführt, weiterführend stabilisiert werden bzw. vor äußeren Einwirkungen, wie hohen Temperaturen oder UV-Strahlung, geschützt werden, so dass beispielsweise ein entsprechend breites Temperaturprofil vorliegt.

Vor diesem Hintergrund werden im Stand der Technik Wirkstoffe oftmals in geeignete Trägersysteme eingebracht bzw. hiermit gekoppelt, wie es beispielsweise unter anderem für mizell-, vesikel- und lipisomenbasierten oder anderen Verkapselungen der Fall ist.

Hierzu sind im Stand der Technik beispielsweise auf Polynukleotiden, wie insbesondere Ribonukleinsäuren (RNA) (*ribonucleic acid*), insbesondere Messenger-RNA (mRNA), basierende Wirkstoffe bzw. Zusammensetzungen bekannt, welche in jüngster Zeit insbesondere als Impfstoffe eingesetzt werden, und zwar insbesondere im Hinblick auf eine Immunisierung gegenüber dem zur Familie der Coronaviren gehörenden Virus SARS-CoV-2 (*severe acute respiratory syndrome corona virus type 2*). Dabei codiert der Stoff in Form einer speziellen mRNA für ein Antigen des Virus, nämlich im Hinblick auf das sogenannte Spike-Protein des Coronavirus ("Stachelprotein"), wobei dieses Protein an der Oberfläche des Virus positioniert ist und eine Rolle bei der Zellinfektion spielt, nämlich insbesondere auf Basis einer Interaktion mit speziellen Proteinen auf bzw. an der Zellmembran von (Körper-)Zellen (ACE-2, angiotensin-converting-enzyme 2). In Bezug auf die zugrundeliegenden mRNA-basierten Impfstoffe wird die empfindliche mRNA zu ihrem Schutz und zur Ermöglichung der Aufnahme in (Körper-)Zellen in ein Trägersystem eingebracht, welches insbesondere auf Lipiden bzw. lipidbasierten Partikeln basiert. Nach Aufnahme in die (Körper-)Zellen wird der von der mRNA codierte Bauplan von der (Körper-)Zelle sozusagen abgelesen und nachfolgend das *Spike*-Protein (als Antigen) produziert und auf der Oberfläche der (Körper-)Zelle präsentiert, so dass dieses von Immunzellen des Körpers erkannt werden kann, wodurch wiederum das Immunsystem aktiviert und unter anderem Antikörper gegen das als Antigen fungierende *Spike*-Protein gebildet werden, wobei zudem auch eine "immunologische Erinnerung" hervorgerufen wird, einhergehend mit einem gewissen Schutz für nachfolgende Infektionen.

Grundsätzlich eignen sich nanostrukturierte Zusammensetzungen, wie beispielweise auf Basis von Liposomen bzw. Vesikeln bzw. Lipid(nano)partikeln als Träger bzw. Verkapselungen entsprechender Wirkstoffe, wie insbesondere auch mRNA, wie zuvor angeführt.

Derartige Zusammensetzungen bzw. Systeme sind jedoch im Allgemeinen hinsichtlich ihrer Herstellung problematisch, da sie oftmals nur sehr aufwendig und unter Heranziehung eines umfangreichen Prozess-know-hows hergestellt werden können. In diesem Zusammenhang ist es im Stand der Technik oftmals von Nachteil, dass entsprechende Zusammensetzungen inhomogen ausgebildet sind, insbesondere was die Größenausbildung der zugrundeliegenden Liposomen bzw. Lipid(nano)partikel anbelangt, wobei auch eine über den Lagerzeitraum gleichbleibende Produktqualität mitunter nicht zufriedenstellend gegeben ist. Dabei verhält es sich insbesondere derart, dass oftmals übermäßig große Partikel auf Basis von Lipiden oder dergleichen bzw. übermäßig großen Liposomen bzw. übermäßig große Lipid(nano)partikel in der Zusammensetzung resultieren, oftmals einhergehend mit einer inhomogenen Partikelgrößenverteilung sowie mit einer hohen Dispersität bzw. großen Varianz, was insbesondere im Hinblick auf die Produktqualität, Lagerstabilität, Verpackung des Wirkstoffs und Anwendung im Körper sowie damit verbundener Nebenwirkungen problematisch sein kann. Dabei können die Ursachen für mitunter vorliegende Inhomogenitäten auch dadurch zurückgeführt werden, dass es im Stand der Technik oftmals schwierig ist, die hydrophilen bzw. lipophilen Hauptkomponenten, welche die Grundlage für die Liposomen bzw. Lipid(nano)partikel bilden, in optimaler Weise in ein wässriges Milieu einzubringen bzw. zu lösen. Zudem werden für entsprechende Zusammensetzungen des Standes der Technik oftmals Komponenten eingesetzt, welche beispielsweise beim Einfrieren der Zusammensetzungen zu Zwecken der Lagerung bzw. dem sich anschließenden Auftauen nachteilig auf die in den Zusammensetzungen vorliegenden Teilchenstrukturen sind.

Darüber hinaus ist es im Stand der Technik oftmals erforderlich, einen hohen Energieeintrag zur Ausbildung entsprechender partikulärer Strukturen, beispielsweise auf Basis von Liposomen bzw. Lipid(nano)partikeln, in eine die Bestandteile zur Ausbildung der partikulären Strukturen enthaltende Zusammensetzung einzubringen, beispielsweise in Form von Ultraschall oder dergleichen. Der diesbezüglich erforderliche hohe Energieeintrag kann aber zu einer Zerstörung bzw. Inaktivierung des Wirkstoffs führen, zumal es sich beispielsweise bei mRNA um eine nicht nur gegenüber Temperatureinflüssen, sondern auch gegenüber Krafteinwirkung empfindliche Substanz handelt. Dabei kann es auch problematisch sein, dass ein entsprechend hoher Energieeintrag mit einer mitunter übermäßigen Temperaturerhöhung des zugrundeliegenden Systems bzw. der Zusammensetzung einhergeht, was zu einer weiteren Beeinträchtigung des Wirkstoffs führen kann, zumal im Stand der Technik insbesondere aufgrund der mitunter schlechten Löslichkeit der Partikel bereits von vornherein im Stand der Technik zur Herstellung der Zusammensetzungen hohe Temperaturen erforderlich sind. Auch in diesem Zusammenhang sind insgesamt mitunter aufwendige Erwärmungs- bzw. Abkühlprozesse bzw. -schritte erforderlich, was aber neben einem hohen energetischen Aufwand auch mit einer komplizierten Verfahrensführung bzw. Prozesskontrolle und aufwendigen Verfahrensapparaturen bzw. -vorrichtungen verbunden ist.

Insgesamt ist somit die Herstellung entsprechender wirkstoffhaltiger Zusammensetzungen im Stand der Technik, welche Lipid(nano)partikel-Strukturen bzw. Vesikel als Träger des Wirkstoffs, wie mRNA, aufweisen, relativ aufwendig, wobei keine zufriedenstellenden homogenen Vesikelstrukturen bzw. -größen erhalten werden.

Darüber hinaus ist es im Stand der Technik mitunter erforderlich, die für die eingesetzten Zusammensetzungen verwendeten Komponenten in entsprechenden Vormischungen bzw. -lösungen einzusetzen, wobei oftmals Alkohol in Form von Ethanol bzw. Chloroform als Lösemittel eingesetzt werden, welche aber im weiteren Herstellungsprozess mitunter aufwendig entfernt werden müssen. Die vorgenannten Substanzen können dabei auch zu Inkompatibilitäten führen, und zwar insbesondere auch im Hinblick auf einzusetzende Wirkstoffe, was die Anzahl einzusetzender Wirkstoffarten bzw. -typen beschränken oder aber deren Wirksamkeit beeinflussen kann.

Aufgrund der im Stand der Technik vorliegenden Verfahrensführung zur Herstellung derartiger Substanzen resultieren in den Zusammensetzungen auch oftmals unerwünscht große mehrlagige Vesikel (*multilayer vesicles*) (MLV), welche unter Einsatz von Hochdruckhomogenisatoren oder dergleichen nachfolgend zu kleineren und insbesondere unilamellaren Vesikeln umgewandelt bzw. verarbeitet werden, was aber gleichermaßen verfahrenstechnisch aufwendig und zu einer zusätzlich physikalischen Belastung der zugrundeliegenden Zusammensetzung bzw. des Wirkstoffsystems führt.

Insbesondere ist es im Stand der Technik erforderlich, das zur Lösung der Komponenten insbesondere in entsprechenden Vormischungen eingesetzte Ethanol bzw. Chloroform aus der resultierenden Zusammensetzung zu entfernen, beispielsweise mittels Abdampfen bzw. Entzug oder dergleichen.

Bei den bislang bekannten Verfahren zur Herstellung entsprechender Vesikelbasierender bzw. Lipid(nano)partikel-basierender und wirkstoffhaltiger Zusammensetzungen kommt es insgesamt oftmals zu gewissen Schwankungen in der Produktqualität, und zwar insbesondere auch im Hinblick auf die Partikelgrößen und Partikelgrößenverteilungen der zugrundeliegenden Vesikel- bzw. Lipid(nano)partikel, wobei oftmals auch Partikel in hohem Mikrometer-Bereich resultieren bzw. vorliegen. Mitunter kann es auch zu nicht unerheblichen Schwankungen, insbesondere *batch-to-batch*-Schwankungen, bezüglich der Qualität bei der Produktion kommen.

Im Allgemeinen wird die Struktur der zugrundeliegenden Partikel, insbesondere in Form von Vesikeln- bzw. Lipid(nano)partikel, auf Basis spezieller Lipide gewährleistet, wie sie grundsätzlich auch in biologischen Membranen vorkommen können. Zudem wird oftmals Cholesterin eingesetzt. Die zugrundeliegenden Vesikel- bzw. Lipid(nano) partikel dienen hinsichtlich ihres Aufbaus bzw. ihrer Struktur im Allgemeinen den sogenannten Liposomen, welche als Träger für Arzneistoffe bzw. Wirkstoffe im Bereich der Medizin schon seit längerer Zeit eingesetzt werden. Im Allgemeinen wird insbesondere im Hinblick auf die Verwendung von messenger-RNA (mRNA) insbesondere als Impfwirkstoff durch die zugrundeliegende partikuläre Struktur bzw. durch die Vesikel- bzw. Lipid(nano)partikel ein Transport des Wirkstoffs zu den (Körper-)Zellen ermöglicht und eine diesbezügliche Aufnahme ermöglicht, wobei der Wirkstoff dann seine Wirkung entfalten kann.

Die WO 2018/078053 A1 bzw. die zu derselben Patentfamilie gehörende EP 3 532 094 A1 bzw. US 2020/0163878 A1 bzw. US 2021/251898 A1 betrifft Lipid-Nanopartikel umfassend mRNA und deren medizinische Verwendungen sowie ein diesbezügliches Verfahren zur Herstellung des Lipidnanopartikels, wobei ein Teil der diesbezüglichen Bestandteile zunächst in Ethanol gelöst werden und der Ethanol nachfolgend insbesondere mittels Dialyse oder Diafiltration entfernt wird.

Die WO 2018/006052 A1 betrifft ein Lipid-Nanopartikel, umfassend ein kationisches Lipid, ein nicht-kationisches Lipid, ein Kortikosteroid und eine Nukleinsäure, wobei die Nukleinsäure und das Kortikosteroid im Lipid-Nanopartikel eingekapselt sind, sowie ein Verfahren zur Herstellung eines Lipid-Nanopartikels, wobei das Verfahren das Kombinieren eines kationischen Lipids, eines nicht-kationischen Lipids und gereinigter mRNA umfasst, um ein Lipid-Nanopartikel zu bilden, wobei die mRNA im Lipid-Nanopartikel eingekapselt ist und wobei das Lipid-Nanopartikel eine spezielle IFIT-Reaktion aufweisen soll.

Darüber hinaus betrifft die WO 2019/051289 A1 ein Lipid-Partikel, welches ein ionisierbares Lipid und einen nicht-viralen Kapsid-freien DNA-Vektor mit kovalent geschlossenen Enden umfasst, wobei der Vektor mindestens eine heterologe Nukleotidsequenz umfasst, welche funktionsfähig zwischen asymmetrischen invertierten terminalen Wiederholungssequenzen bzw. asymmetrischen ITRs positioniert ist, wobei mindestens einer der asymmetrischen ITRs eine funktionelle terminale Auflösungsstelle und eine Rep-Bindungsstelle umfasst.

Die WO 2017/194454 A1 betrifft ein entzündungshemmendes Lipid-Nanopartikel, welches aus einer Lipid-Phase und mindestens einem lipophilen entzündungshemmenden Wirkstoff besteht.

Die WO 2021/022173 A1 betrifft ein Lipid-Nanopartikel zur Immunzellenabgabe, bestehend aus einem ionisierbaren Lipid; einem Sterol oder einem anderen Strukturlipid; einem nicht-kationischen Helferlipid oder Phospholipid; einem RNA-Interferenz-mittel sowie optional einem PEG-Lipid, wobei das ionisierbare Lipid bzw. das Sterol bzw. das Strukturlipid ein Lipid zur Potenzierung der Immunzellenabgabe in einer Menge umfasst, welche wirksam ist, um die Abgabe des Lipid-Nanopartikels an eine Immunzelle zu verstärken.

Weiterhin betrifft die WO 2014/136086 A1 eine Lipid-Zusammensetzung, welche eine spezielle Verbindung bzw. ein pharmazeutisch verträgliches Salz hiervon umfasst.

Kimura, N. et al., "Development of a Microfluidic-Based Post-Treatment Process for Size-Controled Lipid Nanoparticles and Application to siRNA Delivery", Applied Materials and Interfaces, Bd. 12, Nr. 30, 15. Juli 2020, Seiten 34011 bis 34020, betrifft Ausführungen zur Entwicklung eines mikrofluidischen Nachbehandlungsverfahrens für größenkontrollierte Lipid-Nanopartikel und Anwendung auf die Verabreichung von siRNA.

Umerska, A. et al., "Antibacterial action of lipid nanocapsules containing fatty acids or monoglycerides as co-surfactants", European Journal of Pharmaceutics and Biopharmaceutics, Official Journal of Arbeitsgemeinschaft für Pharmazeutische Verfahrenstechnik e.V. Oktober 2012, Bd. 108, 3. September 2016, Seiten 100 bis 110, betrifft Untersuchungen zur antibakteriellen Wirkung von Lipid-Nanokapseln, welche Fettsäuren oder Monoglyceride als Co-Tenside enthalten.

Insgesamt besteht im Stand der Technik daher ein großer Bedarf an entsprechenden trägerbasierten Zusammensetzungen auf Basis von partikulären lipidbasierten Strukturen, insbesondere Lipidnanopartikeln, wobei sich diese Systeme zur Aufnahme bzw. Verabreichung entsprechender Wirkstoffe eignen sollen, und zwar auch vor dem großen Potenzial hierauf basierender Arzneimittel bzw. Medikamente im Hinblick auf die prophylaktische bzw. therapeutische Behandlung zugrundeliegender Erkrankungen bzw. im Hinblick auf die Verwendung als Impfstoff oder dergleichen.

Vor diesem Hintergrund liegt daher der vorliegenden Erfindung die Aufgabe zugrunde, ein effizientes Konzept zur Bereitstellung eines vorzugsweise lipidbasierten und in partikulärer Form vorliegenden Trägersystems bereitzustellen, welches sich für eine Ausrüstung bzw. Beladung mit einem pharmazeutischen Wirkstoff eignet, wobei die zuvor geschilderten Nachteile des Standes der Technik zumindest weitgehend vermieden oder aber wenigstens abgeschwächt werden sollen.

Insbesondere ist eine Aufgabe der vorliegenden Erfindung darin zu sehen, ein diesbezügliches Verfahren zur Herstellung eines derartigen und vorzugsweise lipidbasierten Trägersystems bzw. diesbezügliche Trägersysteme als solche bereitzustellen, welches insbesondere im Hinblick auf die Ausbildung der Partikel in Form von Lipidnanopartikeln (LNP) zu Partikeln mit geringen Größen insbesondere im Nanometer-Bereich bei gleichzeitig homogener Partikelgrößenverteilung führt, wobei diesbezüglich auch eine geringe Dispersität bzw. geringe Varianz vorliegen soll.

Gleichermaßen besteht eine wiederum weitere Aufgabe der vorliegenden Erfindung darin, ein diesbezügliches Verfahren zur Herstellung von vorzugsweise lipidbasierten Trägersystemen bzw. diesbezügliche Trägersysteme als solche bereitzustellen, wobei sich die bereitgestellten Trägersysteme in hervorragender Weise zur Ausrüstung bzw. Beladung mit pharmazeutischen Wirkstoffen eignen, und zwar auch im Hinblick auf eine Vielzahl verschiedener Wirkstoffarten bzw. -typen, so dass erfindungsgemäß insgesamt ein universelles Trägersystem bereitgestellt werden soll.

In diesem Zusammenhang ist eine nochmals weitere Aufgabe der vorliegenden Erfindung auch darin zu sehen, dass erfindungsgemäß ein diesbezügliches Verfahren bzw. ein Trägersystem als solches bereitgestellt wird, wobei das erfindungsgemäß hergestellte Trägersystem in hierauf basierenden Zusammensetzungen nach der Erfindung vorliegen soll, wobei die diesbezüglichen Zusammensetzungen mit den darin befindlichem Trägersystem bei gleichzeitig hoher (Lager-)Stabilität eine optimale Anwendung bzw. Verwendung im Rahmen der prophylaktischen bzw. therapeutischen Behandlung von Erkrankungen auf Grundlage entsprechender Arzneimittel bzw. Medikamente bei gleichzeitig hoher Verträglichkeit aufweisen sollen.

In diesem Zusammenhang ist eine wiederum weitere Aufgabe der vorliegenden Erfindung auch darin zu sehen, eine hohe Kompatibilität des Trägersystems bzw. diesbezüglicher Zusammensetzungen gegenüber den einzusetzenden Wirkstoffen zu gewährleisten.

Zudem soll erfindungsgemäß insbesondere auch ein solches Verfahren zur Herstellung entsprechender vorzugsweise lipidbasierter Trägersysteme bzw. erfindungsgemäße Trägersysteme als solche, welche vorzugsweise lipidbasiert ausgebildet sind, bereitgestellt werden, wobei das diesbezüglich zugrundeliegende Herstellungsverfahren insgesamt gut kontrollierbar bzw. steuerbar und zu gleichbleibenden hohen Produktqualitäten führen soll, und zwar auch im Hinblick auf die Gewährleistung der Ausbildung definierter Partikelstrukturen insbesondere in Form von Lipidnanopartikeln. Dabei soll zudem auch gewährleistet sein, dass das zugrundeliegende Verfahren zur Herstellung des vorzugsweise lipidbasierten Trägersystems auf insgesamt weniger komplexen Vorrichtungen und unter Ausübung einer möglichst geringen Anzahl an Verfahrensschritten eingesetzt werden kann.

Vor diesem Hintergrund sollen im Rahmen der vorliegenden Erfindung gemäß einer weiteren Aufgabe insgesamt auch verbesserte Arzneimittel bzw. Medikamente auf Basis der insbesondere lipidbasierten Trägersysteme bereitgestellt werden.

Zur Lösung der geschilderten Aufgabe schlägt die vorliegende Erfindung - gemäß einem **ersten** Aspekt der vorliegenden Erfindung - ein Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen bzw. ausgerüsteten lipidbasierten Trägersystems in partikulärer Form, bevorzugt in Form Lipidnanopartikeln (LNP) gemäß Anspruch 1 vor; jeweils vorteilhafte Weiterbildungen und Ausgestaltungen dieses Erfindungsaspekts sind Gegenstand der entsprechenden und das erfindungsgemäße Verfahren betreffenden Nebenansprüche sowie der diesbezüglichen Unteransprüche.

Weiterhin betrifft die vorliegende Erfindung - gemäß einem **zweiten** Aspekt der vorliegenden Erfindung - ein nach dem erfindungsgemäßen Verfahren erhältliches lipidbasiertes Trägersystem, welches in partikulärer Form, bevorzugt in Form von Lipidnanopartikeln, vorliegt und welches insbesondere mit einem pharmazeutischen Wirkstoff beladen bzw. ausgerüstet ist, gemäß dem diesbezüglichen Anspruch bzw. ein entsprechendes vorzugsweise lipidbasiertes Trägersystem als solches, wie in dem diesbezüglichen Anspruch definiert; weitere, insbesondere vorteilhafte Weiterbildungen und Ausgestaltungen dieses Erfindungsaspekts sind Gegenstand der das vorzugsweise lipidbasierte Trägersystem nach der Erfindung betreffenden Neben- bzw. Unteransprüche.

Zudem betrifft die vorliegende Erfindung - gemäß einem **dritten** Aspekt der vorliegenden Erfindung - auch eine Zusammensetzung, welche das erfindungsgemäße lipidbasierte Trägersystem in einem flüssigen Medium enthält, wie in dem diesbezüglichen und die erfindungsgemäße Zusammensetzung betreffenden Anspruch definiert; jeweils vorteilhafte Weiterbildungen und Ausgestaltungen dieses Erfindungsaspekts sind Gegenstand der entsprechenden und die Zusammensetzung betreffenden Unteransprüche.

Nochmals weiterer Gegenstand der vorliegenden Erfindung - gemäß einem **vierten** Aspekt der vorliegenden Erfindung - ist auch das erfindungsgemäße Arzneimittel bzw. Medikament, insbesondere zur Verwendung bei der prophylaktischen und/oder therapeutischen Behandlung von Erkrankungen des menschlichen oder tierischen Körpers, welches das erfindungsgemäße lipidbasierte Trägersystem, welches insbesondere mit einem pharmazeutischen Wirkstoff beladen bzw. ausgerüstet ist, oder eine diesbezügliche Zusammensetzung, insbesondere zusammen mit mindestens einem pharmazeutischen Exzipienten, enthält, wie in dem diesbezüglichen und das Arzneimittel bzw. Medikament nach der Erfindung betreffenden Anspruch definiert; jeweils weitere vorteilhafte Weiterbildungen und Ausgestaltungen dieses Erfindungsaspekts sind Gegenstand der diesbezüglichen und das erfindungsgemäße Arzneimittel bzw. Medikament betreffenden Unteransprüche.

In diesem Zusammenhang betrifft die vorliegende Erfindung insbesondere auch die Verwendung des Arzneimittels bzw. Trägersystems bzw. der Zusammensetzung nach der Erfindung zur prophylaktischen und/oder therapeutischen Behandlung von Erkrankungen des menschlichen und/oder tierischen Körpers bzw. zur Herstellung eines Arzneimittels zur prophylaktischen und/oder therapeutischen Behandlungen von Erkrankungen des menschlichen oder tierischen Körpers, wie in den diesbezüglichen unabhängigen und Ansprüchen definiert. In diesem Zusammenhang betrifft die vorliegende Erfindung weiterhin auch ein Verfahren zur prophylaktischen bzw. therapeutischen Behandlung von Erkrankungen des menschlichen bzw. tierischen Körpers. Hierzu kann auch Bezug genommen werden auf die entsprechenden Neben- bzw. Unteransprüche. Die vorliegende Erfindung betrifft auch die Verwendung mindestens eines Prozessparameters zur Steuerung, insbesondere zur Verfahrens- und/oder Prozesskontrolle, des vorliegend beschriebenen Verfahrens.

Es versteht sich im Zusammenhang mit den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt angeführt sind, selbstverständlich auch in Bezug auf die übrigen Erfindungsaspekt entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbezug sämtlicher Komponenten bzw. Inhaltsstoffe, insbesondere wie nachfolgend definiert, stets zu 100 % bzw. 100 Gew.-% ergänzen bzw. addieren; dies versteht sich für den Fachmann aber von selbst.

Weiterhin gilt, dass der Fachmann - anwendungsbezogen oder einzelfallbedingt - von den nachfolgend angeführten Konzentrations-, Gewichts-, Mengen- und Bereichsangaben abweichen kann, ohne dass er den Rahmen der vorliegenden Erfindung verlässt.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten lipidbasierten Trägersystems in partikulärer Form, bevorzugt in Form von Lipidnanopartikeln (LNP), wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird, wobei die Bestandteile des Trägersystems die folgenden Substanzen umfassen:

(i) mindestens ein Phospholipid, vorzugsweise Phosphatidylcholin (Lecithin); wobei das Phosholipid einen HLB-Wert nach Griffin (*hydrophilic-lipophilicbalance-Wert*) von höchstens 11, insbesondere höchstens 8, aufweist und/oder wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern; wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D); wobei das Steroid und/oder Secosteroid, insbesondere das Sterol, einen HLB-Wert nach Griffin von höchstens 1,5 aufweist und/oder wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins; wobei das kationische Lipid einen HLB-Wert nach Griffin von höchstens 4 aufweist und/oder wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin; wobei der Mono-, Di- oder Tricarbonsäureester von Glycerin einen HLB-Wert nach Griffin von höchstens 8 aufweist und/oder wobei der Mono-, Di- oder Tricarbonsäureester von Glycerin einen Log P-Wert von mindestens 0,80 aufweist;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

wobei die Zusammenlagerung, insbesondere Aggregation, der Bestandteile zur Ausbildung des Trägersystems unter Eintrag von Energie, bevorzugt Eintrag von Misch- und/oder Rührenergie, in das zumindest einen Teil der Bestandteile und/oder Substanzen des Trägersystems aufweisende flüssige Medium und/oder in eine zumindest einen Teil der Bestandteile und/oder Substanzen des Trägersystems aufweisenden Mischung, durchgeführt wird, wobei die eingetragene Energie, insbesondere Misch- und/oder Rührenergie, eingestellt wird derart, dass eine zumindest im Wesentlichen laminare Strömung, in dem zumindest einen Teil der Bestandteile und/oder Substanzen des Trägersystems aufweisenden flüssigen Medium und/oder in der zumindest einen Teil der Bestandteile und/oder Substanzen des Trägersystems aufweisenden Mischung, vorliegt, insbesondere unter zumindest im Wesentlichen vollständiger Vermeidung der Ausbildung einer nichtlaminaren und/oder turbulenten Strömung und/oder insbesondere unter zumindest im Wesentlichen vollständiger Vermeidung der Ausbildung nichtlaminarer und/oder turbulenter Strömungsanteile; und

wobei eine kontinuierliche Verfahrens- und/oder Prozesskontrolle (Produktionskontrolle), insbesondere *online*- und/oder *inline*-Prozesskontrolle, zur Überwachung des Verfahrensstatus und/oder des Verfahrensfortschritts, vorzugsweise zur Überwachung und/oder Einstellung der Ausbildung der Partikel des Trägersystems, insbesondere Lipidnanopartikel (LNP), und/oder insbesondere zur Überwachung und/oder Einstellung von Verfahrensparametern, ausgewählt aus Eintrag von Energie, bevorzugt Eintrag von Misch- und/oder Rührenergie, Eintrag und/oder Abfuhr von Wärmenergie, Strömungsverhalten, Massendurchsatz, Volumenströmen oder dergleichen, durchgeführt wird; wobei die Verfahrens- und/oder Prozesskontrolle über die Erfassung von mindestens einem Prozessparameter, insbesondere mindestens zwei Prozessparametern, erfolgt,

wobei der Prozessparameter ausgewählt wird aus der Gruppe von Temperatur, Leitfähigkeit, Viskosität und pH-Wert jeweils des flüssigen Mediums mitsamt den darin eingebrachten Bestandteilen und/oder Substanzen, und/oder der Zusammensetzung;

wobei das Verfahren in zumindest im Wesentlichen vollständiger Abwesenheit von Ethanol durchgeführt wird.

Erfindungsgemäß wird somit ein spezielles Verfahren zur Herstellung von vorzugsweise lipidbasierten Trägersystemen bereitgestellt, wobei im Rahmen der vorliegenden Erfindung spezielle Bestandteile des Trägersystems in spezieller Auswahl und Abstimmung zur Ausbildung definierter partikulärer Formen, insbesondere zur Ausbildung von Lipidnanopartikeln (LNP), in Kontakt bzw. in Wechselwirkung gebracht werden. Daraus resultiert erfindungsgemäß ein entsprechendes Trägersystem in partikulärer Form mit definierten Eigenschaften. Erfindungsgemäß verhält es sich dabei insbesondere derart, dass das Trägersystem mit mindestens einem pharmazeutischen Wirkstoff beladen bzw. ausgerüstet ist bzw. werden kann. Insbesondere kann das Trägersystem somit mindestens einen pharmazeutischen Wirkstoff aufweisen, insbesondere als (weiteren) Bestandteil bzw. Substanz des Trägersystems (vgl. auch nachfolgende Ausführungen).

Die Anmelderin hat in überraschender Weise herausgefunden, dass auch dem sogenannten HLB-Wert nach Griffin, welcher den jeweiligen Bestandteilen bzw. Substanzen zugeordnet ist, eine signifikante Bedeutung insbesondere im Hinblick auf die Ausbildung homogener und nanopartikulärer Strukturen sowie in Form von Lipidnanopartikeln mit geringer Dispersität bzw. kleiner Varianz zukommt, wobei auf Basis der speziellen Verfahrensführung diesbezüglich auch (lager-)stabile Zusammensetzungen bereitgestellt und die diesbezüglichen Trägersysteme mit einer Vielzahl verschiedener pharmazeutischer Wirkstoffe beladen bzw. ausgerüstet werden können, wie nachfolgend noch im Detail angeführt.

Im Rahmen der vorliegenden Erfindung ist es dabei völlig überraschend, dass die Verwendung einer Substanz (i) in Form eines Phospholipids, welches insbesondere einen HLB-Wert nach Griffin von höchstens 11, insbesondere höchstens 8, aufweist, in Kombination mit der Substanz (ii), nämlich in Form der nichtionogenen amphiphilen bzw. tensidischen Substanz, wie tensidischen PEGylierten Substanzen, in entsprechenden Zusammensetzungen bzw. fluiden Medien zur Ausbildung besonders definierter partikulärer Formen, bevorzugt in Form von Lipidnanopartikelnm, führt. Auch auf dieser Basis können hinsichtlich ihrer Größenausbildung homogene partikuläre Formen ausgebildet bzw. bereitgestellt werden.

Was den erfindungsgemäß angeführten HLB-Wert nach Griffin anbelangt, so beschreibt dieser insbesondere den hydrophilen und lipophilen Anteil von entsprechenden Substanzen. Der HLB-Wert nach Griffin kann insbesondere gemäß der Formel $20 \cdot M_{hydrophil} / M_{gesamt}$ berechnet werden, wobei $M_{hydrophil}$ das Molekulargewicht des hydrophilen Anteils bzw. der Kopfgruppe der zugrundeliegenden Substanz bzw. des Moleküls betrifft und wobei $M_{gesamt}$ sich auf das Molekulargewicht bzw. die Molmasse der Gesamtsubstanz bzw. des gesamten Moleküls bezieht. Der Faktor 20 stellt dabei einen definierten Skalierungsfaktor dar. Auf Basis des HLB-Werts nach Griffin resultiert somit eine Skala von 0 bis 20, wobei ein HLB-Wert nach Griffin von 1 eine insgesamt lipophile Substanz beschreibt, während ein HLB-Wert nach Griffin von 20 eine insgesamt hydrophile Substanz beschreibt. Alternativ kann der HLB-Wert nach Griffin auch gemäß der Formel $HLB = 20 \cdot (1 - M_{lipophil} / M_{gesamt})$ berechnet werden, wobei $M_{lipophil}$ sich auf den lipophilen Anteil einer Substanz bzw. eines Moleküls bezieht.

Anhand des HLB-Werts nach Griffin können somit die lipophilen bzw. hydrophilen Eigenschaften der erfindungsgemäß eingesetzten Substanzen charakterisiert werden, wobei die im Rahmen des erfindungsgemäßen Verfahrens als Bestandteile des Trägersystems eingesetzten Substanzen, wie vorliegend definiert, zur Ausbildung von Trägersystemen in partikulärer Form, insbesondere in Form von Lipidnanopartikeln, mit insgesamt verbesserten Eigenschaften führen. Dabei hat die Anmelderin auch überraschend gefunden, dass im Rahmen des erfindungsgemäßen Verfahrens in Bezug auf das Trägersystem nach der Erfindung und den diesem zugrundeliegenden Partikeln, insbesondere Lipidnanopartikeln, besonders definierte Eigenschaften resultieren, wenn die für das Trägersystem eingesetzten insbesondere amphiphilen Substanzen in ihrer Gesamtheit bzw. auf Basis spezieller Kombinationen der zugrundeliegenden Substanzen einen mittleren HLB-Wert nach Griffin in einem definierten Bereich aufweisen, und zwar insbesondere in einem Bereich von 3 bis 8. Dies ist umso überraschender, als ein derartiger Wert üblicherweise W/O-Emulgatoren (Wasser-in-Öl-Emulgatoren) zugeordnet wird.

Für weitere Ausführungen zu dem HLB-Wert nach Griffin kann insbesondere auch verwiesen werden auf Griffin "W.C. Classification of Surface Active Agent by HLB", J. SOC. Cosmet. Chem. 1, 1949. Zudem kann verwiesen werden auf *"Heusch, eine experimentelle Methode zur Bestimmung des HLB-Werts von Tensiden"*, Cholloid-Zeitschrift und Zeitschrift für Polymere, Bd. 236, Heft 1.

Für zwitterionische Substanzen bzw. für die Phospholipide bezieht sich der angeführte HLB-Wert nach Griffin insbesondere auf denjenigen, welcher am isoelektrischen Punkt vorliegt.

Die Anmelderin hat darüber hinaus in überraschender Weise gefunden, dass weiterführend auch dem sogenannten Log P-Wert, welcher den jeweiligen Bestandteilen bzw. Substanzen zugeordnet ist, eine weiterführende Bedeutung insbesondere im Hinblick auf die Ausbildung homogener und nanopartikulärer Strukturen auf Basis definierter Trägersysteme zukommt.

Der Log P-Wert (synonym auch $\log_{10} P$ bzw. lg P) ist der dekadische Logarithmus des Verteilungskoeffizienten P für eine bestimmte Substanz. Im Allgemeinen kann der P-Wert als Verhältnis der Konzentrationen der Substanz in einer n-Octanol-Phase und einer Wasser-Phase bestimmt werden. Der Log P-Wert ist ein Maß für die Lipophilie einer Substanz bzw. eines pharmazeutischen Wirkstoffs. Insbesondere kann der Log P-Wert auch zur Abschätzung von Permeabilität, Absorption und Bioverfügbarkeit verwendet werden. Im Allgemeinen gilt, dass mit zunehmenden Log P-Wert die Lipophilie der Substanz zunimmt. Umgekehrt bedeuten kleine, insbesondere negative, Log P-Werte eine hohe Hydrophilie.

Wie zuvor angeführt, handelt es sich bei dem Log P-Wert um den dekadischen Logarithmus des Verteilungskoeffizienten P bzw. des P-Wertes. Der P-Wert ist ein Verteilungskoeffizient, der mithilfe eines biphasischen Systems mit den beiden Lösungsmitteln n-Octanol und Wasser bestimmt wird. Dabei werden die Konzentrationen in den beiden Phasen bestimmt. Für die Berechnung des P-Werts wird die Konzentration der Substanz in n-Octanol durch die Konzentration der Substanz in Wasser geteilt: P = c (n-Octanol) / c (Wasser). Der Log P-Wert ist der dekadische Logarithmus von P, d. h. log P = log (c(n-Octanol) / c(Wasser)).

Somit ist der Log P-Wert eine stoffspezifische Kenngröße, welche auf Basis der zuvor beschriebenen Herangehensweise bestimmt werden kann. Die diesbezügliche Herangehensweise bzw. Bestimmung ist dem Fachmann dabei wohlbekannt. Zudem sind die entsprechenden Log P-Werte auch in Standardwerken angeführt.

Darüber hinaus können die Log P-Werte vorliegend insbesondere auch rechnerisch bzw. computerbasiert bestimmt werden, insbesondere mittels der Anwendung bzw. dem Tool XLogP3. Insbesondere kann die Bestimmung der Log P-Werte vorliegend somit mittels der XLogP3-Methode erfolgen. Hierzu kann auch verwiesen werden auf Cheng, T. et al., "Computation of Octanol-Water Partition Coefficients by Guiding an Additive Model with Knowledge", J. Chem. Inf. Model. 2007, 47, 2140-2148.

Insbesondere können aufgrund der rechenbasierten bzw. computergestützten Ermittling sehr genaue Werte erhalten werden.

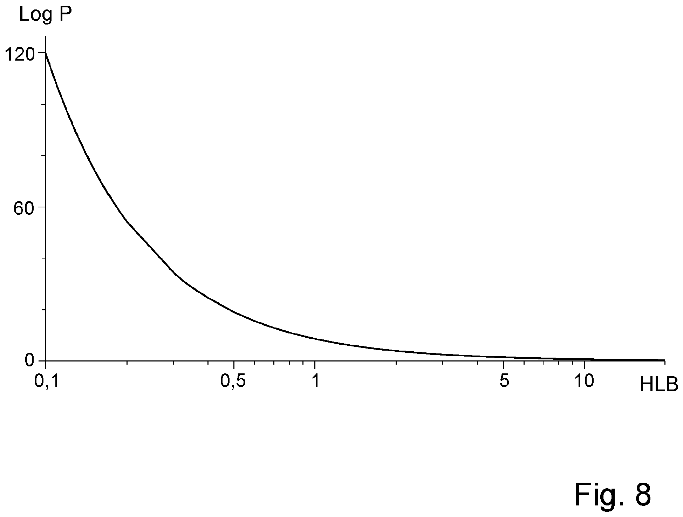

Auf Basis des Log P-Wertes liegt im Rahmen der vorliegenden Erfindung eine weiterführende Charakterisierung der eingesetzten Bestandteile bzw. Substanzen vor. In diesem Zusammenhang hat die Anmelderin in überraschender Weise einen funktionalen Zusammenhang zwischen Log P-Werten einerseits und HLB-Werten andererseits für die eingesetzten Bestandteile bzw. Substanzen bzw. erfindungsgemäßen Trägersysteme gefunden. Hierzu kann auch auf Fig. 8 und die diesbezüglich nachfolgenden Ausführungen verwiesen werden.

Insbesondere konnte die Anmelderin einen funktionalen Zusammenhang auf Basis der Formel HLB = 6,5469 · Log $P^{-0,874}$ aufzeigen (vgl. auch Fig. 8, welche den vorliegenden funktionalen Zusammenhang auf Basis der vorgenannten Formel (mit entsprechender Formelumstellung) grafisch veranschaulicht).

Im Rahmen der vorliegenden Erfindung können die Log P-Werte somit im Hinblick auf die HLB-Werte nach Griffin als alternatives und/oder kumulatives Maß für die Beschreibung der Lipophilie bzw. Hydrophilie der eingesetzten Substanzen bzw. Bestandteile herangezogen werden. Auch kann auf dieser Basis eine ergänzende und weiterführende Charakterisierung der erfindungsgemäß erhaltenen Trägersysteme vorgenommen werden. Auf Basis bzw. unter Heranziehung der entsprechenden Log P-Werte kann somit eine weiterführende Charakterisierung und Beschreibung auch der Lipophilie der zugrundeliegenden Komponenten durchgeführt werden. Insbesondere können auf dieser Basis auch die resultierenden Eigenschaften im Hinblick auf passive Diffusion, Absorption und Bioverfügbarkeit weiterführend charakterisiert werden.

Insgesamt liegt mit der Heranziehung der Log P-Werte auch eine zu den HLB-Werten nach Griffin ergänzende bzw. komplementäre sowie auch weiterführende Charakterisierung der zugrundeliegenden Bestandteile, Substanzen bzw. der erhaltenen Trägersysteme vor. Insbesondere verhält es sich in diesem Zusammenhang auch derart, dass im Hinblick auf die Bestimmung des Log P-Wertes, wie zuvor angeführt, n-Octanol im Vergleich zu Wasser als Verteilungssystem eingesetzt wird. In diesem Zusammenhang wird n-Octanol im Allgemeinen als langkettiger Alkohol auch als Modell für die Zellmembran, welche vorwiegend auf amphiphilen Phospholipiden aufgebaut ist, herangezogen. Auch von daher eignet sich der Log P-Wert in besonderem Maße zur Charakterisierung der eingesetzten Komponenten sowie der resultierenden Trägersysteme nach der Erfindung, und zwar auch im Hinblick auf deren Bioverfügbarkeit und Interaktion mit Wirkstoffen oder dergleichen.

Durch die ziel- und zweckgerichtete Verwendung spezieller Bestandteile bzw. Substanzen mit jeweils definiertem HLB-Wert nach Griffin einerseits bzw. Log P-Wert andererseits und mit der diesbezüglichen Abstimmung können insgesamt optimierte Trägersysteme bereitgestellt werden, und zwar auch im Hinblick auf deren Ausbildung bzw. Verhalten in dem flüssigen Medium, und zwar auch unter Einbezug beispielsweise von Polyol als ein weiterer Bestandteil des zugrundeliegenden flüssigen Mediums. Auf dieser Basis können insgesamt definierte, stabile sowie maßgeschneiderte Trägersysteme ausgebildet werden.

Was den HLB-Wert nach Griffin sowie den Log P-Wert weiterhin angelangt, so können diese jeweils insbesondere unter Standardbedingungen (d.h. bei Raumtemperatur (20 °C) und Umgebungsdruck (1.013,25 hPa)) ermittelt werden.

Zudem führt der erfindungsgemäß in spezieller Weise vorgesehene Eintrag von Energie, insbesondere von Misch- bzw. Rührenergie, in das zugrundeliegende System zur Ausbildung des Trägersystems nach der Erfindung bzw. der erfindungsgemäßen Zusammensetzung unter Bereitstellung einer im Wesentlichen laminaren Strömung bzw. unter Vermeidung der Ausbildung einer nichtlaminaren bzw. turbulenten Strömung in völlig überraschender Weise zu weiterführend verbesserten Trägersystemen bzw. diesbezüglichen Zusammensetzungen nach der Erfindung. Denn durch die Ausbildung einer laminaren Strömung liegt - ohne sich auf dieses Thema beschränken oder berufen zu wollen - ein besonders definierter Energieeintrag vor, welcher die zugrundeliegende Selbstaggregation der Bestandteile zur Ausbildung des Trägersystems nicht nachteilig beeinflusst. Vielmehr liegt auf Basis der erfindungsgemäßen Maßnahme ein besonders schonender Energieeintrag vor, was zur Ausbildung definierter Teilchensysteme mit enger Partikelgrößenverteilung bei gleichzeitig definierter Partikelgröße und definierter körperlicher Ausbildung (z. B. in Form von Disk-LNPs) führt. Insbesondere wird das Auftreten von Defektstrukturen oder dergleichen wirkungsvoll verhindert bzw. minimiert.

Weiterführend ermöglicht die im Rahmen der Erfindung gleichermaßen in gezielter Weise vorgesehene Prozesskontrolle unter Erfassung spezieller Prozessparameter eine nochmals weiterführend optimierte Verfahrensdurchführung. Insbesondere können auf dieser Basis noch während der Verfahrensführung entsprechende Verfahrensparameter angepasst bzw. adjustiert werden, so dass auch auf dieser Basis insgesamt einheitliche bzw. gezielt gesteuerte Verfahrensbedingungen zur Ausbildung optimaler Trägersysteme bzw. diesbezüglicher Zusammensetzungen nach der Erfindung vorliegen.

Dabei ergänzen sich die erfindungsgemäßen Maßnahmen des kontrollierten Energieeintrags unter Ausbildung einer laminaren Strömung und der speziellen Prozesskontrolle andererseits in über die Summe der einzelnen Maßnahmen hinausgehender und somit synergistischer Weise. Folglich können erfindungsgemäß auch von daher insgesamt optimierte bzw. maßgeschneiderte Trägersysteme mit definierter Partikelausbildung sowie hierauf basierende Zusammensetzungen bereitgestellt werden.

Im Hinblick auf Erfassung spezieller Prozessparameter kann beispielsweise eine sprunghafte (relative) Veränderung eines Prozessparameters (z. B. der Leitfähigkeit) auf eine gegebenenfalls unerwünschte Veränderung im strukturellen Gefüge des zugrundeliegenden Systems bzw. Mediums mit den diesbezüglichen Bestandteilen hinweisen. Bei entsprechender Veränderung der Prozessparameter können dann entsprechende Verfahrensparameter verändert bzw. nachjustiert werden, um einen optimalen Herstellungsablauf zu gewährleisten.

Auf Basis der erfindungsgemäßen Konzeption wird insgesamt ein vorzugsweise lipidbasiertes Trägersystem bereitgestellt, welches in ziel- und zweckgerichteter Weise mit Wirkstoffen ausgerüstet bzw. beladen werden kann. Durch Auswahl und Abstimmung der jeweiligen Substanzen können auch diesbezüglich maßgeschneiderte bzw. optimierte Trägersysteme bereitgestellt werden, um auf diese Weise eine optimale Ausrüstung mit dem Wirkstoff zu ermöglichen. Die erfindungsgemäßen Systeme zeichnen sich, wie zuvor angeführt, insbesondere durch geringe Partikelgrößen aus, wobei diesbezüglich insbesondere spezielle Lipidnanopartikel (LNP) ausgebildet werden bzw. vorliegen, und zwar insbesondere in Form von diskförmigen (scheibchenförmigen) Lipidnanopartikeln, wie nachfolgend noch ausgeführt. Dabei weisen die Partikel, insbesondere Lipidnanopartikel (LNP), relativ geringe Größen bzw. Partikeldurchmesser auf, und dies bei homogener Partikelgrößenverteilung bzw. geringerer Dispersität und/oder Varianz, so dass die insbesondere in einem flüssigen Medium vorliegenden Partikel des erfindungsgemäßen Trägersystems eine hohe Homogenität aufweisen. Dies geht mit definierten Eigenschaften zugrundeliegender Zusammensetzungen einher, und zwar auch, was deren (Lager-)Stabilität und deren definierte Abgabe von Wirksubstanzen bzw. Wirkstoffen am Wirkort (z. B. (Körper-) Zellen) anbelangt.

Zudem kann eine optimale Beladung bzw. Ausrüstung des Trägersystems mit Wirkstoffen als diesbezügliche Bestandteile realisiert werden.

Auch auf dieser Basis wird insgesamt im Rahmen der vorliegenden Erfindung ein universelles Trägersystem bereitgestellt, welches sich für eine Vielzahl von Wirkstofftypen bzw. -arten eignet und diesbezüglich individuell maßgeschneidert werden kann.

Zudem basiert das erfindungsgemäße Verfahren auf einer geringen Anzahl an Prozessschritten, wobei das erfindungsgemäße Verfahren unter Heranziehung relativ einfach aufgebauter Vorrichtungen und bei hervorragender Prozesskontrolle durchgeführt werden kann, was nicht zuletzt zu gleichbleibenden Produktqualitäten und einer Anwendbarkeit des erfindungsgemäßen Verfahrens führt. Diesbezüglich kann auch ein hoher Automatisierungsgrad realisiert werden. Zudem zeichnet sich das erfindungsgemäße Verfahren insofern aus, als hohe Durchsätze realisiert werden können, so dass auch ein kostenoptimiertes bzw. insgesamt ökonomisches Verfahren bereitgestellt wird.

Insbesondere kann im Rahmen des erfindungsgemäßen Verfahrens auf die Verwendung von mitunter problematischen bzw. physiologisch bedenklichen Substanzen, wie Ethanol und Chloroform, verzichtet werden, so dass diesbezüglich somit auch aufwändige Entfernungsschritte entfallen, was der Produktqualität und -ökonomie zuträglich ist. Insbesondere wird auch hierdurch die Verträglichkeit von verfahrensgemäß resultierenden Zusammensetzungen im Hinblick auf deren Verwendung als Arzneimittel bzw. Medikament verbessert.

Was die Zusammenlagerung bzw. Aggregation der Bestandteile des Trägersystems auf Basis der angeführten Substanzen im Allgemeinen anbelangt, so beruht diese - ohne sich auf diese Theorie beschränken oder berufen zu wollen - beispielsweise bzw. insbesondere auf entropiegetriebenen Prozessen. Zudem kann die Aggregation bzw. Aneinanderlagerung auf Basis von Van-der-Waals-Kräften, intermolekularen Wechselwirkungen, chemischen Bindungen oder dergleichen hervorgerufen sein. Wie nachfolgend noch im Detail angeführt, wird im Rahmen der vorliegenden Erfindung die Zusammenlagerung bzw. Aggregation insbesondere durch den Eintrag von Energie, insbesondere Misch- und/oder Rührenergie, in das flüssige Medium, in welchem die Bestandteile bzw. Substanzen eingebracht werden, hervorgerufen bzw. induziert, wobei auf dieser Basis auch die resultierenden Partikelgrößen bzw. Partikeldurchmesser gezielt eingestellt werden können. Dies erfolgt insbesondere im Hinblick auf die in diesem Zusammenhang stehende Dispersität bzw. Varianz der zugrundeliegenden Partikel des insbesondere lipidbasierten erfindungsgemäßen Trägersystems.

Wie zuvor angeführt, ist im Hinblick auf die Ausbildung der Partikel, insbesondere Lipidnanopartikel (LNP), des erfindungsgemäßen insbesondere lipidbasierten Trägersystems der jeweilige HLB-Wert nach Griffin von Bedeutung. Durch die gezielte Auswahl und Abstimmung der Komponenten im Hinblick auf deren jeweiligen HLB-Wert nach Griffin können die resultierenden Partikel, insbesondere Lipidnanopartikel (LNP), hinsichtlich ihres Aufbaus, Formgebung und Größe weiterführend eingestellt werden bzw. die diesbezüglichen Eigenschaften gezielt vorgegeben werden. Die Abstimmung und Auswahl der Substanzen in Abhängigkeit von deren HLB-Werten nach Griffin führt auch zu einer verbesserten Verarbeitbarkeit bzw. Handhabbarkeit bzw. Handhabung der Substanzen im Rahmen der erfindungsgemäßen Verfahrensführung, insbesondere was die Einarbeitung in das zugrundeliegende fluide Medium zur Ausbildung der Zusammensetzung mit dem darin befindlichen Trägersystem auf Basis der Partikel anbelangt, worauf nachfolgend gleichermaßen noch im Detail eingegangen wird.

Was den erfindungsgemäß vorgesehenen Energieeintrag anbelangt, so hat sich insbesondere die nachfolgende Vorgehensweise als vorteilhaft herausgestellt: So kann die eingetragene Menge an Energie, insbesondere die eingetragene Menge an Misch- und/oder Rührenergie, eingestellt werden derart, dass eine Reynolds-Zahl $R_e$, insbesondere eine auf eine Rührerströmung bezogene Reynolds-Zahl $R_e$, von höchstens 2.000, insbesondere höchstens 1.500, vorzugsweise höchstens 1.000, bevorzugt höchstens 500, vorliegt.

**In** Bezug auf die erfindungsgemäße Verfahrensführung hat es sich insbesondere als vorteilhaft erwiesen, wenn die zuvor angeführten Substanzen jeweils folgende Eigenschaften aufweisen:

- Insbesondere kann die Substanz (i) einen HLB-Wert nach Griffin im Bereich von 0,5 bis 8, vorzugsweise im Bereich von 2 bis 7, bevorzugt im Bereich von 3 bis 6, aufweisen, bzw. insbesondere kann die Substanz (i) einen Log P-Wert im Bereich von 0,80 bis 18,97, vorzugsweise im Bereich von 0,93 bis 3,88, bevorzugt im Bereich von 1,11 bis 2,44, aufweisen.

- Zudem kann die Substanz (ii) einen HLB-Wert nach Griffin im Bereich von 13 bis 19, insbesondere im Bereich von 13 bis 17,9, vorzugsweise im Bereich von 13,5 bis 17,5, bevorzugt im Bereich von 14 bis 17, aufweisen, bzw. zudem kann die Substanz (ii) einen Log P-Wert im Bereich von 0,30 bis 0,46, insbesondere im Bereich von 0,32 bis 0,46, vorzugsweise im Bereich von 0,33 bis 0,44, bevorzugt im Bereich von 0,34 bis 0,42, aufweisen.

- Weiterhin kann die Substanz (iii) einen HLB-Wert nach Griffin im Bereich von 0,5 bis 1,5, vorzugsweise im Bereich von 0,6 bis 1,4, bevorzugt im Bereich von 0,7 bis 1,3, aufweisen, bzw. weiterhin kann die Substanz (iii) einen Log P-Wert im Bereich von 5,40 bis 18,97, vorzugsweise im Bereich von 5,84 bis 15,40, bevorzugt im Bereich von 6,36 bis 12,91, aufweisen.

- Die Substanz (iv) kann einen HLB-Wert nach Griffin im Bereich von 1,5 bis 3,5, bevorzugt im Bereich von 2 bis 3, aufweisen, bzw. die Substanz (iv) kann einen Log P-Wert im Bereich von 2,05 bis 5,40, bevorzugt im Bereich von 2,44 bis 3,88, aufweisen.

- Die Substanz (v) kann einen HLB-Wert nach Griffin im Bereich von 1 bis 8, vorzugsweise im Bereich von 1,25 bis 7, bevorzugt im Bereich von 1,5 bis 6,5, aufweisen, bzw. die Substanz (v) kann einen Log P-Wert im Bereich von 0,80 bis 8,58, vorzugsweise im Bereich von 0,93 bis 6,65, bevorzugt im Bereich von 1,01 bis 5,40, aufweisen.

Die Auswahl der erfindungsgemäßen Substanzen im Hinblick auf den jeweils zugrundeliegenden HLB-Wert nach Griffin ermöglicht insbesondere die Steuerung der konkreten Ausbildung bzw. Formgebung der Partikel des erfindungsgemäßen vorzugsweise lipidbasierten Trägersystems, wobei durch die spezielle Auswahl und Abstimmung der Substanzen auf Basis des HLB-Wertes nach Griffin beispielsweise Lipidnanopartikel (LNP) in Form von diskförmigen (scheibchenförmigen) Lipidnanopartikeln erhalten werden können, welche besonders vorteilhafte Eigenschaften aufweisen.

Insbesondere kann es sich erfindungsgemäß derart verhalten, dass das Verfahren durch mindestens eine(s) der nachfolgenden Merkmale und/oder Maßnahmen (1a) bis (5a), insbesondere durch eine Kombination von mindestens zwei, vorzugsweise mindestens drei, bevorzugt mindestens vier, besonders bevorzugt sämtlichen fünf, der nachfolgenden Merkmale und/oder Maßnahmen (1a) bis (5a), gekennzeichnet ist:

(1a) die Substanz (i) weist einen HLB-Wert nach Griffin von höchstens 11, insbesondere höchstens 8, insbesondere im Bereich von 0,5 bis 8, vorzugsweise im Bereich von 2 bis 7, bevorzugt im Bereich von 3 bis 6, auf und/oder die Substanz (i) weist eine einen Log P-Wert von mindestens 0,55, insbesondere mindestens 0,80, insbesondere im Bereich von 0,80 bis 18,97, vorzugsweise im Bereich von 0,93 bis 3,88, bevorzugt im Bereich von 1,11 bis 2,44, auf;

(2a) die Substanz (ii) weist einen HLB-Wert nach Griffin von mindestens 13, insbesondere im Bereich von 13 bis 19, vorzugsweise im Bereich von 13 bis 17,9, bevorzugt im Bereich von 13,5 bis 17,5, besonders bevorzugt im Bereich von 14 bis 17, auf und/oder die Substanz (ii) weist einen Log P-Wert von höchstens 0,46, insbesondere im Bereich von 0,32 bis 0,46, vorzugsweise im Bereich von 0,33 bis 0,44, bevorzugt im Bereich von 0,34 bis 0,42, auf;

(3a) die Substanz (iii) weist einen HLB-Wert nach Griffin von höchstens 1,5, insbesondere im Bereich von 0,5 bis 1,5, vorzugsweise im Bereich von 0,6 bis 1,4, bevorzugt im Bereich von 0,7 bis 1,3, auf und/oder die Substanz (iii) weist einen Log P-Wert von mindestens 5,40, insbesondere im Bereich von 5,40 bis 18,97, vorzugsweise im Bereich von 5,84 bis 15,40, bevorzugt im Bereich von 6,36 bis 12,91, auf;

(4a) die Substanz (iv) weist einen HLB-Wert nach Griffin von höchstens 4, insbesondere im Bereich von 1 bis 4, vorzugsweise im Bereich von 1,5 bis 3,5, bevorzugt im Bereich von 2 bis 3, auf und/oder die Substanz (iv) weist einen Log P-Wert von mindestens 1,76, insbesondere im Bereich von 1,76 bis 8,58, vorzugsweise im Bereich von 2,05 bis 5,40, bevorzugt im Bereich von 2,44 bis 3,88, auf;

(5a) die Substanz (v) weist einen HLB-Wert nach Griffin von höchstens 8, insbesondere im Bereich von 1 bis 8, vorzugsweise im Bereich von 1,25 bis 7, bevorzugt im Bereich von 1,5 bis 6,5, auf und/oder die Substanz (v) weist einen Log P-Wert von mindestens 0,80, insbesondere im Bereich von 0,80 bis 8,58, vorzugsweise im Bereich von 0,93 bis 6,65, bevorzugt im Bereich von 1,01 bis 5,40, auf.

Vor diesem Hintergrund betrifft die vorliegende Erfindung gemäß dem vorliegenden ersten Aspekt auch ein Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten lipidbasierten Trägersystems in partikulärer Form, bevorzugt in Form von Lipidnanopartikeln (LNP), insbesondere wie zuvor definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten (polyoxyethylierten) Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern, insbesondere Polyolestern von PEGylierten Fettsäuren;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D);

(iv) ggf. mindestens ein kationisches Lipid, insbesondere aminbasiertes kationisches Lipid, vorzugsweise ein kationisches Lipid auf Basis eines tertiären Amins;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;

wobei das Verfahren durch mindestens eine(s) der nachfolgenden Merkmale und/oder Maßnahmen (1a) bis (5a), insbesondere durch eine Kombination von mindestens zwei, vorzugsweise mindestens drei, bevorzugt mindestens vier, besonders bevorzugt sämtlichen fünf, der nachfolgenden Merkmale und/oder Maßnahmen (1a) bis (5a), gekennzeichnet ist:

(1a) die Substanz (i) weist einen HLB-Wert nach Griffin von höchstens 11, insbesondere höchstens 8, insbesondere im Bereich von 0,5 bis 8, vorzugsweise im Bereich von 2 bis 7, bevorzugt im Bereich von 3 bis 6, auf und/oder die Substanz (i) weist eine einen Log P-Wert von mindestens 0,55, insbesondere mindestens 0,80, insbesondere im Bereich von 0,80 bis 18,97, vorzugsweise im Bereich von 0,93 bis 3,88, bevorzugt im Bereich von 1,11 bis 2,44, auf;

(2a) die Substanz (ii) weist einen HLB-Wert nach Griffin von mindestens 13, insbesondere im Bereich von 13 bis 19, vorzugsweise im Bereich von 13 bis 17,9, bevorzugt im Bereich von 13,5 bis 17,5, besonders bevorzugt im Bereich von 14 bis 17, auf und/oder die Substanz (ii) weist einen Log P-Wert von höchstens 0,46, insbesondere im Bereich von 0,32 bis 0,46, vorzugsweise im Bereich von 0,33 bis 0,44, bevorzugt im Bereich von 0,34 bis 0,42, auf;

(3a) die Substanz (iii) weist einen HLB-Wert nach Griffin von höchstens 1,5, insbesondere im Bereich von 0,5 bis 1,5, vorzugsweise im Bereich von 0,6 bis 1,4, bevorzugt im Bereich von 0,7 bis 1,3, auf und/oder die Substanz (iii) weist einen Log P-Wert von mindestens 5,40, insbesondere im Bereich von 5,40 bis 18,97, vorzugsweise im Bereich von 5,84 bis 15,40, bevorzugt im Bereich von 6,36 bis 12,91, auf;

(4a) die Substanz (iv) weist einen HLB-Wert nach Griffin von höchstens 4, insbesondere im Bereich von 1 bis 4, vorzugsweise im Bereich von 1,5 bis 3,5, bevorzugt im Bereich von 2 bis 3, auf und/oder die Substanz (iv) weist einen Log P-Wert von mindestens 1,76, insbesondere im Bereich von 1,76 bis 8,58, vorzugsweise im Bereich von 2,05 bis 5,40, bevorzugt im Bereich von 2,44 bis 3,88, auf;

(5a) die Substanz (v) weist einen HLB-Wert nach Griffin von höchstens 8, insbesondere im Bereich von 1 bis 8, vorzugsweise im Bereich von 1,25 bis 7, bevorzugt im Bereich von 1,5 bis 6,5, auf und/oder die Substanz (v) weist einen Log P-Wert von mindestens 0,80, insbesondere im Bereich von 0,80 bis 8,58, vorzugsweise im Bereich von 0,93 bis 6,65, bevorzugt im Bereich von 1,01 bis 5,40, auf;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

wobei die Zusammenlagerung, insbesondere Aggregation, der Bestandteile zur Ausbildung des Trägersystems unter Eintrag von Energie, bevorzugt Eintrag von Misch- und/oder Rührenergie, in das zumindest einen Teil der Bestandteile und/oder Substanzen des Trägersystems aufweisende flüssige Medium und/oder in eine zumindest einen Teil der Bestandteile und/oder Substanzen des Trägersystems aufweisenden Mischung, durchgeführt wird, wobei die eingetragene Energie, insbesondere Misch- und/oder Rührenergie, eingestellt wird derart, dass eine zumindest im Wesentlichen laminare Strömung, in dem zumindest einen Teil der Bestandteile und/oder Substanzen des Trägersystems aufweisenden flüssigen Medium und/oder in der zumindest einen Teil der Bestandteile und/oder Substanzen des Trägersystems aufweisenden Mischung, vorliegt, insbesondere unter zumindest im Wesentlichen vollständiger Vermeidung der Ausbildung einer nichtlaminaren und/oder turbulenten Strömung und/oder insbesondere unter zumindest im Wesentlichen vollständiger Vermeidung der Ausbildung nichtlaminarer und/oder turbulenter Strömungsanteile; und

wobei eine kontinuierliche Verfahrens- und/oder Prozesskontrolle (Produktionskontrolle), insbesondere *online*- und/oder *inline*-Prozesskontrolle, zur Überwachung des Verfahrensstatus und/oder des Verfahrensfortschritts, vorzugsweise zur Überwachung und/oder Einstellung der Ausbildung der Partikel des Trägersystems, insbesondere Lipidnanopartikel (LNP), und/oder insbesondere zur Überwachung und/oder Einstellung von Verfahrensparametern, ausgewählt aus Eintrag von Energie, bevorzugt Eintrag von Misch- und/oder Rührenergie, Eintrag und/oder Abfuhr von Wärmenergie, Strömungsverhalten, Massendurchsatz, Volumenströmen oder dergleichen durchgeführt wird; wobei die Verfahrens-

und/oder Prozesskontrolle über die Erfassung von mindestens einem Prozessparameter, insbesondere mindestens zwei Prozessparametern, erfolgt, wobei der Prozessparameter ausgewählt wird aus der Gruppe von Temperatur, Leitfähigkeit, Viskosität und pH-Wert jeweils des flüssigen Mediums mitsamt den darin eingebrachten Bestandteilen und/oder Substanzen, und/oder der Zusammensetzung;

wobei das Verfahren in zumindest im Wesentlichen vollständiger Abwesenheit von Ethanol durchgeführt wird.

Erfindungsgemäß hat es sich insbesondere bewährt, wenn im Hinblick auf die vorgenannten Merkmale bzw. Maßnahmen (1a) bis (5a) mit den diesbezüglichen HLB-Werten nach Griffin die nachfolgenden Kombinationen realisiert sind: So kann es erfindungsgemäß insbesondere vorgesehen sein, dass das Verfahren durch eine Kombination der Merkmale und/oder Maßnahmen (1a) und (2a), insbesondere durch eine Kombination der Merkmale und/oder Maßnahmen (1a), (2a) und (3a), vorzugsweise durch eine Kombination der Merkmale und/oder Maßnahmen (1a), (2a), (3a) und (4a) oder vorzugsweise durch eine Kombination der Merkmale und/oder Maßnahmen (1a), (2a), (3a) und (5a), bevorzugt durch eine Kombination der Merkmale und/oder Maßnahmen (1a), (2a), (3a), (4a) und (5a), gekennzeichnet ist.

Auf Basis der vorgenannten Merkmalskombinationen mit den definierten HLB-Werten kann das erfindungsgemäße Verfahren weiterführend optimiert werden, und zwar auch im Hinblick auf die Bereitstellung entsprechender Partikel des vorzugsweise lipidbasierten Trägersystems mit definierten Eigenschaften insbesondere im Hinblick auf relativ kleine Partikelgrößen mit kleiner Dispersität bzw. kleiner Varianz der zugrundeliegenden mittleren Partikeldurchmesser.

In diesem Zusammenhang sind insbesondere auch die nachfolgend angeführten Maßnahmen von Vorteil:

- Erfindungsgemäß können die Substanzen (i) und (iii) oder die Substanzen (i) und (iv) oder die Substanzen (i) und (v) einen mittleren HLB-Wert nach Griffin (jeweiliger arithmetischer Mittelwert der HLB-Werte nach Griffin der vorgenannten Substanzenkombinationen) im Bereich von 1 bis 10, insbesondere im Bereich von 2 bis 9, vorzugsweise im Bereich von 3 bis 8, aufweisen, bzw. erfindungsgemäß können die Substanzen (i) und (iii) oder die Substanzen (i) und (iv) oder die Substanzen (i) und (v) einen mittleren Log P-Wert (jeweiliger arithmetischer Mittelwert der Log P-Werte der vorgenannten Substanzenkombinationen) im Bereich von 0,62 bis 8,58, insbesondere im Bereich von 0,70 bis 3,88, vorzugsweise im Bereich von 0,80 bis 2,44, aufweisen.

- Weiterhin können die Substanzen (i), (iii) und (iv) oder die Substanzen (i), (iii) und (v) oder die Substanzen (i), (iv) und (v) einen mittleren HLB-Wert nach Griffin (jeweiliger arithmetischer Mittelwert der HLB-Werte nach Griffin der vorgenannten Substanzenkombinationen) im Bereich von 1 bis 10, insbesondere im Bereich von 2 bis 9, vorzugsweise im Bereich von 3 bis 8, aufweisen, bzw. weiterhin können die Substanzen (i), (iii) und (iv) oder die Substanzen (i), (iii) und (v) oder die Substanzen (i), (iv) und (v) einen mittleren Log P-Wert (jeweiliger arithmetischer Mittelwert der Log P-Werte der vorgenannten Substanzenkombinationen) der vorgenannten Substanzenkombinationen) im Bereich von 0,62 bis 8,58, insbesondere im Bereich von 0,70 bis 3,88, vorzugsweise im Bereich von 0,80 bis 2,44, aufweisen.

- Zudem können die Substanzen (i), (iii), (iv) und (v) einen mittleren HLB-Wert nach Griffin (jeweiliger arithmetischer Mittelwert der HLB-Werte nach Griffin der vorgenannten Substanzenkombination) im Bereich von 1 bis 10, insbesondere im Bereich von 2 bis 9, vorzugsweise im Bereich von 3 bis 8, aufweisen, bzw. zudem können die Substanzen (i), (iii), (iv) und (v) einen mittleren Log P-Wert (jeweiliger arithmetischer Mittelwert der Log P-Werte der vorgenannten Substanzenkombinationen) der vorgenannten Substanzenkombinationen) im Bereich von 0,62 bis 8,58, insbesondere im Bereich von 0,70 bis 3,88, vorzugsweise im Bereich von 0,80 bis 2,44, aufweisen.

stellen dabei insbesondere amphiphile bzw. hydrophobe Substanzen dar. Folglich ist es erfindungsgemäß insbesondere vorgesehen, dass die amphiphilen Substanzen, insbesondere auf Basis der Substanzen (i), (iii), (iv) und (v), einen mittleren HLB-Wert nach Griffin (jeweiliger arithmetischer Mittelwert erhalten gemäß Griffin in der vorgenannten Substanzkombination) im Bereich von 1 bis 10, insbesondere im Bereich von 2 bis 9, vorzugsweise im Bereich von 3 bis 8, aufweisen. Ein entsprechender Mittelwert nach Griffin gilt dabei insbesondere auch für den weiteren Einbezug von nachfolgend noch angeführten Substanzen, welche optionaler Bestandteil des erfindungsgemäßen Trägersystems sein können, wie beispielsweise die Substanz (vii) und/oder die Substanz (viii).

Weiterhin kann es erfindungsgemäß vorgesehen sein, dass die amphiphilen Substanzen, insbesondere auf Basis der Substanzen (i), (iii), (iv) und (v), einen mittleren Log P-Wert (jeweiliger arithmetischer Mittelwert in der vorgenannten Substanzkombination) im Bereich von 0,62 bis 8,58, insbesondere im Bereich von 0,70 bis 3,88, vorzugsweise im Bereich von 0,80 bis 2,44, aufweisen. Ein entsprechender Log P-Mittelwert gilt dabei insbesondere auch für den weiteren Einbezug von nachfolgend noch angeführten Substanzen, welche optionaler Bestandteil des erfindungsgemäßen Trägersystems sein können, wie beispielsweise die Substanz (vii) und/oder die Substanz (viii).

Im Hinblick auf die erfindungsgemäß im Rahmen des Verfahrens als Bestandteile des Trägersystems nach der Erfindung einsetzbaren Substanzen ist auch der diesbezüglich zugrundeliegende Packungsparameter bzw. kritische Packungsparameter $P_{kr}$ von Bedeutung.

Die Auswahl der Substanzen als Bestandteile des erfindungsgemäßen vorzugsweise lipidbasierten Trägersystems unter Heranziehung des kritischen Packungsparameters $P_{kr}$ ermöglicht gleichermaßen eine Steuerung der Ausbildung bzw. Formgebung der dem erfindungsgemäßen vorzugsweise lipidbasierten Trägersystem zugrundeliegenden Partikeln, beispielsweise in Form von besonders definierten Lipidnanopartikeln (LNP) bzw. insbesondere diskförmigen (scheibchenförmigen) Lipidnanopartikeln, wie nachfolgend noch ausgeführt.

Dem kritischen Packungsparameter $P_{kr}$ liegt gemäß dem Modell nach Israelachvili die Formel gemäß $P_{kr} = V / a_0 \cdot l_C$ zugrunde, wobei $l_C$ die Länge des hydrophoben Rests des der Substanz zugrundeliegenden Moleküls, V das Volumen des der Substanz zugrundeliegenden Moleküls und $a_0$ die Querschnittsoberfläche der hydrophilen Kopfgruppe des der Substanz zugrundeliegenden Moleküls darstellt. Der $P_{kr}$-Wert beschreibt somit ein Verhältnis hydrophiler Kopfgruppen zu lipophilen Gruppen des zugrundeliegenden Moleküls, und zwar mit Schwerpunkt auf geometrische bzw. raumfüllende Komponenten. Der $P_{kr}$-Wert ist dimensionslos und charakteristisch für die diesbezüglichen Moleküle. Wie zuvor angeführt, kann die Ausbildung bzw. Formgebung der Partikel des vorzugsweise lipidbasierten Trägersystems nach der Erfindung auch in Abhängigkeit des $P_{kr}$-Wertes erfolgen. In diesem Zusammenhang hat es sich insbesondere als vorteilhaft erwiesen, wenn die erfindungsgemäß eingesetzten Substanzen die folgenden $P_{kr}$-Werte aufweisen:

- So kann die Substanz (i) einen kritischen Packungsparameter $P_{kr}$ von mindestens 0,5, insbesondere mindestens 0,7, insbesondere im Bereich von 0,7 bis 1,225, vorzugsweise im Bereich von 0,77 bis 1,12, bevorzugt im Bereich von 0,84 bis 1,05, aufweisen.

- Zudem kann die Substanz (ii) einen kritischen Packungsparameter $P_{kr}$ von höchstens 0,35, insbesondere im Bereich von 0,007 bis 0,35, vorzugsweise im Bereich von 0,035 bis 0,315, bevorzugt im Bereich von 0,07 bis 0,28, aufweisen.

- Darüber hinaus kann die Substanz (iii) einen kritischen Packungsparameter $P_{kr}$ von mindestens 1,155, insbesondere im Bereich von 1,155 bis 1,225, vorzugsweise im Bereich von 1,162 bis 1,218, bevorzugt im Bereich von 1,169 bis 1,211, aufweisen.

- Zudem kann die Substanz (iv) einen kritischen Packungsparameter $P_{kr}$ von mindestens 0,98, insbesondere im Bereich von 0,98 bis 1,19, vorzugsweise im Bereich von 1,015 bis 1,155, bevorzugt im Bereich von 1,05 bis 1,12, aufweisen.

- Die Substanz (v) kann einen kritischen Packungsparameter $P_{kr}$ von mindestens 0,7, insbesondere im Bereich von 0,7 bis 1,19, vorzugsweise im Bereich von 0,77 bis 1,1725, bevorzugt im Bereich von 0,805 bis 1,155, aufweisen.

Vor diesem Hintergrund ist insbesondere auch Folgendes von Vorteil: So kann das Verfahren insbesondere durch mindestens eine(s) der nachfolgenden Merkmale und/oder Maßnahmen (1b) bis (5b), insbesondere durch eine Kombination von mindestens zwei, vorzugsweise mindestens drei, bevorzugt mindestens vier, besonders bevorzugt sämtlichen fünf, der nachfolgenden Merkmale und/oder Maßnahmen (1b) bis (5b), gekennzeichnet sein:

(1b) die Substanz (i) weist einen kritischen Packungsparameter $P_{kr}$ von mindestens 0,5, insbesondere mindestens 0,7, insbesondere im Bereich von 0,7 bis 1,225, vorzugsweise im Bereich von 0,77 bis 1,12, bevorzugt im Bereich von 0,84 bis 1,05, auf;

(2b) die Substanz (ii) weist einen kritischen Packungsparameter $P_{kr}$ von höchstens 0,35, insbesondere im Bereich von 0,007 bis 0,35, vorzugsweise im Bereich von 0,035 bis 0,315, bevorzugt im Bereich von 0,07 bis 0,28, auf;

(3b) wobei die Substanz (iii) weist einen kritischen Packungsparameter $P_{kr}$ von mindestens 1,155, insbesondere im Bereich von 1,155 bis 1,225, vorzugsweise im Bereich von 1,162 bis 1,218, bevorzugt im Bereich von 1,169 bis 1,211, auf;

(4b) die Substanz (iv) weist einen kritischen Packungsparameter $P_{kr}$ von mindestens 0,98, insbesondere im Bereich von 0,98 bis 1,19, vorzugsweise im Bereich von 1,015 bis 1,155, bevorzugt im Bereich von 1,05 bis 1,12, auf;

(5b) die Substanz (v) weist einen kritischen Packungsparameter $P_{kr}$ von mindestens 0,7, insbesondere im Bereich von 0,7 bis 1,19, vorzugsweise im Bereich von 0,77 bis 1,1725, bevorzugt im Bereich von 0,805 bis 1,155, auf.

Erfindungsgemäß haben sich insbesondere auch die folgenden Kombinationen als vorteilhaft erwiesen, und zwar auch im Hinblick auf die gezielte Steuerung der Ausbildung bzw. Formgebung der zugrundeliegenden Partikel: Insbesondere kann das Verfahren durch eine Kombination der Merkmale und/oder Maßnahmen (1b) und (2b), insbesondere durch eine Kombination der Merkmale und/oder Maßnahmen (1b), (2b) und (3b), vorzugsweise durch eine Kombination der Merkmale und/oder Maßnahmen (1b), (2b), (3b) und (4b) oder vorzugsweise durch eine Kombination der Merkmale und/oder Maßnahmen (1b), (2b), (3b) und (5b), bevorzugt durch eine Kombination der Merkmale und/oder Maßnahmen (1b), (2b), (3b), (4b) und (5b), gekennzeichnet sein.

Weiterhin kann das im Rahmen des erfindungsgemäßen Verfahrens bereitgestellte Trägersystem nach der Erfindung die angeführten Substanzen insbesondere in den nachfolgend angeführten Mengen aufweisen. Erfindungsgemäß kann es aber im Einzelfall vorgesehen sein, von den nachfolgend angeführten Werten abzuweichen, ohne dass die vorliegende Erfindung hierdurch verlassen wird.

Im Hinblick auf die Substanz (i) kann es sich insbesondere wie folgt verhalten:

- So kann das Trägersystem die Substanz (i) in einer Menge im Bereich von 0,5 Gew.-% bis 50 Gew.-%, insbesondere im Bereich von 1 Gew.-% bis 40 Gew.-%, vorzugsweise im Bereich von 1,25 Gew.-% bis 30 Gew.-%, bevorzugt im Bereich von 1,5 Gew.-% bis 25 Gew.-%, bezogen auf das Trägersystem und/oder bezogen auf die Gesamtheit der Bestandteile des Trägersystems, aufweisen.
- Gleichermaßen kann die Substanz (i) in einer Menge eingesetzt werden derart, dass das Trägersystem die Substanz (i) in einer Menge im Bereich von 0,1 Gew.-% bis 50 Gew.-%, insbesondere im Bereich von 0,25 Gew.-% bis 40 Gew.-%, vorzugsweise im Bereich von 0,5 Gew.-% bis 30 Gew.-%, bevorzugt im Bereich von 1 Gew.-% bis 25 Gew.-%, bezogen auf das Trägersystem und/oder bezogen auf die Gesamtheit der Bestandteile des Trägersystems, aufweist.

Darüber hinaus kann es sich für die Substanz (ii) wie folgt verhalten:

- So kann das Trägersystem die Substanz (ii) in einer Menge im Bereich von 1 Gew.-% bis 80 Gew.-%, insbesondere im Bereich von 2 Gew.-% bis 75 Gew.-%, vorzugsweise im Bereich von 3 Gew.-% bis 70 Gew.-%, bevorzugt im Bereich von 5 Gew.-% bis 65 Gew.-%, bezogen auf das Trägersystem und/oder bezogen auf die Gesamtheit der Bestandteile des Trägersystems, aufweisen.

- Gleichermaßen kann die Substanz (ii) in einer Menge eingesetzt werden derart, dass das Trägersystem die Substanz (ii) in einer Menge im Bereich von 1 Gew.-% bis 80 Gew.-%, insbesondere im Bereich von 2 Gew.-% bis 75 Gew.-%, vorzugsweise im Bereich von 3 Gew.-% bis 70 Gew.-%, bevorzugt im Bereich von 5 Gew.-% bis 65 Gew.-%, bezogen auf das Trägersystem und/oder bezogen auf die Gesamtheit der Bestandteile des Trägersystems, aufweist.

Darüber hinaus kann es sich für die Substanz (iii) wie folgt verhalten:

- So kann das Trägersystem die Substanz (iii) in einer Menge im Bereich von 0,2 Gew.-% bis 40 Gew.-%, insbesondere im Bereich von 0,5 Gew.-% bis 25 Gew.-%, vorzugsweise im Bereich von 1 Gew.-% bis 20 Gew.-%, bevorzugt im Bereich von 3 Gew.-% bis 15 Gew.-%, bezogen auf das Trägersystem und/oder bezogen auf die Gesamtheit der Bestandteile des Trägersystems, aufweisen.

- Gleichermaßen kann die Substanz (iii) in einer Menge eingesetzt werden derart, dass das Trägersystem die Substanz (iii) in einer Menge im Bereich von 0,2 Gew.-% bis 40 Gew.-%, insbesondere im Bereich von 0,5 Gew.-% bis 25 Gew.-%, vorzugsweise im Bereich von 1 Gew.-% bis 20 Gew.-%, bevorzugt im Bereich von 3 Gew.-% bis 15 Gew.-%, bezogen auf das Trägersystem und/oder bezogen auf die Gesamtheit der Bestandteile des Trägersystems, aufweist.

Darüber hinaus kann es sich für die Substanz (iv) wie folgt verhalten:

- So kann das Trägersystem die Substanz (iv) in einer Menge im Bereich von 1 Gew.-% bis 80 Gew.-%, insbesondere im Bereich von 2 Gew.-% bis 75 Gew.-%, vorzugsweise im Bereich von 3 Gew.-% bis 70 Gew.-%, bevorzugt im Bereich von 5 Gew.-% bis 65 Gew.-%, bezogen auf das Trägersystem und/oder bezogen auf die Gesamtheit der Bestandteile des Trägersystems, aufweisen.

- Gleichermaßen kann die Substanz (iv) in einer Menge eingesetzt werden derart, dass das Trägersystem die Substanz (iv) in einer Menge im Bereich von 1 Gew.-% bis 80 Gew.-%, insbesondere im Bereich von 2 Gew.-% bis 75 Gew.-%, vorzugsweise im Bereich von 3 Gew.-% bis 70 Gew.-%, bevorzugt im Bereich von 5 Gew.-% bis 65 Gew.-%, bezogen auf das Trägersystem und/oder bezogen auf die Gesamtheit der Bestandteile des Trägersystems, aufweist.

Darüber hinaus kann es sich für die Substanz (v) wie folgt verhalten:

- So kann das Trägersystem die Substanz (v) in einer Menge im Bereich von 2 Gew.-% bis 85 Gew.-%, insbesondere im Bereich von 4 Gew.-% bis 80 Gew.-%, vorzugsweise im Bereich von 6 Gew.-% bis 75 Gew.-%, bevorzugt im Bereich von 8 Gew.-% bis 70 Gew.-%, bezogen auf das Trägersystem und/oder bezogen auf die Gesamtheit der Bestandteile des Trägersystems, aufweisen.

- Gleichermaßen kann die Substanz (v) in einer Menge eingesetzt wird derart, dass das Trägersystem die Substanz (v) in einer Menge im Bereich von 2 Gew.-% bis 85 Gew.-%, insbesondere im Bereich von 4 Gew.-% bis 80 Gew.-%, vorzugsweise im Bereich von 6 Gew.-% bis 75 Gew.-%, bevorzugt im Bereich von 8 Gew.-% bis 70 Gew.-%, bezogen auf das Trägersystem und/oder bezogen auf die Gesamtheit der Bestandteile des Trägersystems, aufweist.

Weiterführend kommt auch dem Gewichtsverhältnis der erfindungsgemäß für das Trägersystem bzw. im Rahmen des erfindungsgemäßen Verfahrens eingesetzten Substanzen eine große Bedeutung zu: Auf dieser Basis können die Eigenschaften der resultierenden Partikel weiterführend vorgegeben bzw. eingestellt werden, und zwar auch im Hinblick auf eine verbesserte Kompatibilität beispielsweise gegenüber dem zugrundeliegenden flüssigen Medium bzw. gegenüber dem Wirkstoff, mit welchem die Partikel bzw. das Trägersystem beladen bzw. ausgerüstet werden bzw. wird.

Weiterhin kann erfindungsgemäß insbesondere Folgendes vorgesehen sein:

- So kann das gewichtsbezogene Mengenverhältnis von Substanz (i) zu Substanz (ii) [Substanz (i) : Substanz (ii)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, liegen bzw. auf die vorgenannten Werte eingestellt werden.

- Insbesondere können die Substanzen (i) und (ii) in einer Menge eingesetzt werden derart, dass das Trägersystem die Substanzen (i) und (ii) in einem gewichtsbezogenen Mengenverhältnis von Substanz (i) zu Substanz (ii) [Substanz (i) : Substanz (ii)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, aufweist.

Zudem kann erfindungsgemäß insbesondere Folgendes vorgesehen sein:

- So kann das gewichtsbezogene Mengenverhältnis von Substanz (i) zu Substanz (iii) [Substanz (i) : Substanz (iii)] im Bereich von 250 : 1 bis 1 : 80, insbesondere im Bereich von 80 : 1 bis 1 : 25, vorzugsweise im Bereich von 30 : 1 bis 1 : 16, bevorzugt im Bereich von 8 : 1 bis 1 : 10, liegen bzw. auf die vorgenannten Werte eingestellt werden.

- Insbesondere können die Substanzen (i) und (iii) in einer Menge eingesetzt werden derart, dass das Trägersystem die Substanzen (i) und (iii) in einem gewichtsbezogenen Mengenverhältnis von Substanz (i) zu Substanz (iii) [Substanz (i) : Substanz (iii)] im Bereich von 250 : 1 bis 1 : 80, insbesondere im Bereich von 80 : 1 bis 1 : 25, vorzugsweise im Bereich von 30 : 1 bis 1 : 16, bevorzugt im Bereich von 8 : 1 bis 1 : 10, aufweist.

Darüber hinaus kann erfindungsgemäß insbesondere Folgendes vorgesehen sein:

- So kann das gewichtsbezogene Mengenverhältnis von Substanz (i) zu Substanz (iv) [Substanz (i) : Substanz (iv)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, liegen bzw. auf die vorgenannten Werte eingestellt werden.

- Insbesondere können die Substanzen (i) und (iv) in einer Menge eingesetzt werden derart, dass das Trägersystem die Substanzen (i) und (iv) in einem gewichtsbezogenen Mengenverhältnis von Substanz (i) zu Substanz (iv) [Substanz (i) : Substanz (iv)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, aufweist.

Weiterführend kann erfindungsgemäß insbesondere Folgendes vorgesehen sein:

- So kann das gewichtsbezogene Mengenverhältnis von Substanz (i) zu Substanz (v) [Substanz (i) : Substanz (v)] im Bereich von 25 : 1 bis 1 : 170, insbesondere im Bereich von 10 : 1 bis 1 : 80, vorzugsweise im Bereich von 5 : 1 bis 1 : 60, bevorzugt im Bereich von 4 : 1 bis 1 : 46, liegen bzw. auf die vorgenannten Werte eingestellt werden.

- Insbesondere können die Substanzen (i) und (v) in einer Menge eingesetzt werden derart, dass das Trägersystem die Substanzen (i) und (v) in einem gewichtsbezogenen Mengenverhältnis von Substanz (i) zu Substanz (ii) [Substanz (i) : Substanz (v)] im Bereich von 25 : 1 bis 1 : 170, insbesondere im Bereich von 10 : 1 bis 1 : 80, vorzugsweise im Bereich von 5 : 1 bis 1 : 60, bevorzugt im Bereich von 4 : 1 bis 1 : 46, aufweist.

Zudem kann erfindungsgemäß insbesondere Folgendes vorgesehen sein:

- So kann das gewichtsbezogene Mengenverhältnis von Substanz (i) zu Substanz (ii) zu Substanz (iii) zu Substanz (iv) [Substanz (i) : Substanz (ii) : Substanz (iii) : Substanz (iv)] im Bereich von (0,5 bis 50) : (1 bis 80) : (0,2 bis 40) : (1 bis 80), insbesondere im Bereich von (1 bis 40) : (2 bis 75) : (0,5 bis 25) : (2 bis 75), vorzugsweise im Bereich von (1,25 bis 30) : (3 bis 70) :(1 bis 20) : (3 bis 70), bevorzugt im Bereich von (1,5 bis 25) : (5 bis 65) : (3 bis 15) : (5 bis 65), liegen bzw. auf die vorgenannten Werte eingestellt werden.

- Insbesondere können die Substanzen (i), (ii), (iii) und (iv) in einer Menge eingesetzt werden derart, dass das Trägersystem die Substanzen (i), (ii), (iii) und (iv) in einem gewichtsbezogenen Mengenverhältnis von Substanz (i) zu Substanz (ii) zu Substanz (iii) zu Substanz (iv) [Substanz (i) : Substanz (ii) : Substanz (iii) : Substanz (iv)] im Bereich von (0,5 bis 50) : (1 bis 80) : (0,2 bis 40) : (1 bis 80), insbesondere im Bereich von (1 bis 40) : (2 bis 75) : (0,5 bis 25) : (2 bis 75), vorzugsweise im Bereich von (1,25 bis 30) : (3 bis 70) :(1 bis 20) : (3 bis 70), bevorzugt im Bereich von (1,5 bis 25) : (5 bis 65) : (3 bis 15) : (5 bis 65), aufweist.

In diesem Zusammenhang kann erfindungsgemäß insbesondere Folgendes vorgesehen sein:

- So kann das gewichtsbezogene Mengenverhältnis von Substanz (i) zu Substanz (ii) zu Substanz (iii) zu Substanz (v) [Substanz (i) : Substanz (ii) : Substanz (iii) : Substanz (v)] im Bereich von (0,5 bis 50) : (1 bis 80) : (0,2 bis 40) : (2 bis 85), insbesondere im Bereich von (1 bis 40) : (2 bis 75) : (0,5 bis 25) : (4 bis 80), vorzugsweise im Bereich von (1,25 bis 30) : (3 bis 70) :(1 bis 20) : (6 bis 75), bevorzugt im Bereich von (1,5 bis 25) : (5 bis 65) : (3 bis 15) : (8 bis 70), liegen bzw. auf die vorgenannten Werte eingestellt werden.

- Insbesondere können die Substanzen (i), (ii), (iii) und (v) in einer Menge eingesetzt werden derart, dass das Trägersystem die Substanzen (i), (ii), (iii) und (v) in einem gewichtsbezogenen Mengenverhältnis von Substanz (i) zu Substanz (ii) zu Substanz (iii) zu Substanz (v) [Substanz (i) : Substanz (ii) : Substanz (iii) : Substanz (v)] im Bereich von (0,5 bis 50) : (1 bis 80) : (0,2 bis 40) : (2 bis 85), insbesondere im Bereich von (1 bis 40) : (2 bis 75) : (0,5 bis 25) : (4 bis 80), vorzugsweise im Bereich von (1,25 bis 30) : (3 bis 70) : (1 bis 20) : (6 bis 75), bevorzugt im Bereich von (1,5 bis 25) : (5 bis 65) : (3 bis 15) : (8 bis 70), aufweist.

Weiterhin kann erfindungsgemäß insbesondere Folgendes vorgesehen sein:

- So kann das gewichtsbezogene Mengenverhältnis von Substanz (i) zu Substanz (ii) zu Substanz (iii) zu Substanz (iv) zu Substanz (v) [Substanz (i) : Substanz (ii) : Substanz (iii) : Substanz (iv) : Substanz (v)] im Bereich von (0,5 bis 50) : (1 bis 80) : (0,2 bis 40) : (1 bis 80) : (2 bis 85), insbesondere (1 bis 40) : (2 bis 75) : (0,5 bis 25) : (2 bis 75) : (4 bis 80), vorzugsweise (1,25 bis 30) : (3 bis 70) :(1 bis 20) : (3 bis 70) : (6 bis 75), bevorzugt (1,5 bis 25) : (5 bis 65) : (3 bis 15) : (5 bis 65) : (8 bis 70), liegen bzw. auf die vorgenannten Werte eingestellt werden.

- Insbesondere können die Substanzen (i), (ii), (iii) und (iv) in einer Menge eingesetzt werden derart, dass das Trägersystem die Substanzen (i), (ii), (iii) und (iv) in einem gewichtsbezogenen Mengenverhältnis von Substanz (i) zu Substanz (ii) zu Substanz (iii) zu Substanz (iv) zu Substanz (v) [Substanz (i) : Substanz (ii) : Substanz (iii) : Substanz (iv)] im Bereich von (0,5 bis 50) : (1 bis 80) : (0,2 bis 40) : (1 bis 80), insbesondere (1 bis 40) : (2 bis 75) : (0,5 bis 25) : (2 bis 75), vorzugsweise (1,25 bis 30) : (3 bis 70) :(1 bis 20) : (3 bis 70), bevorzugt (1,5 bis 25) : (5 bis 65) : (3 bis 15) : (5 bis 65), aufweist.

Gemäß einer weiteren erfindungsgemäß bevorzugten Ausführungsform kann auch Folgendes vorgesehen sein:

- So kann das gewichtsbezogene Mengenverhältnis von Substanz (ii) zu den hydrophoben Substanzen, insbesondere hydrophoben amphiphilen Substanzen, vorzugsweise ausgewählt aus der Gruppe von Substanz (i), Substanz (iii), Substanz (iv), Substanz (v) und deren Kombinationen, vorzugsweise zu Substanz (i) und/oder Substanz (iii) und ggf. Substanz (iv) und ggf. Substanz (v) [Substanz (ii) : hydrophobe Substanzen] im Bereich von 10 : 1 bis 1 : 20, vorzugsweise im Bereich von 5 : 1 bis 1 : 15, bevorzugt im Bereich von 2 : 1 bis 1 : 10, liegen und/oder auf die vorgenannten Werte eingestellt werden.
Insbesondere können die Substanz (ii) und die hydrophoben Substanzen, insbesondere die hydrophoben amphiphilen Substanzen, vorzugsweise ausgewählt aus der Gruppe von Substanz (i), Substanz (iii), Substanz (iv), Substanz (v) und deren Kombinationen, in einer Menge eingesetzt werden derart, dass das Trägersystem die Substanzen in einem gewichtsbezogenen Mengenverhältnis von Substanz (ii) zu den hydrophoben Substanzen, insbesondere hydrophoben amphiphilen Substanzen, vorzugsweise ausgewählt aus der Gruppe von Substanz (i), Substanz (iii), Substanz (iv), Substanz (v) und deren Kombinationen, vorzugsweise zu Substanz (i) und/oder Substanz (iii) und ggf. Substanz (iv) und ggf. Substanz (v) [Substanz (ii) : hydrophobe Substanzen] im Bereich von 10 : 1 bis 1 : 20, vorzugsweise im Bereich von 5 : 1 bis 1 : 15, bevorzugt im Bereich von 2 : 1 bis 1 : 10, aufweist.

Was die erfindungsgemäß als Bestandteile für das vorzugsweise lipidbasierte Trägersystem anbelangt, so hat sich der Einsatz der nachfolgend angeführten Substanzen als besonders vorteilhaft herausgestellt:
So kann es erfindungsgemäß insbesondere vorgesehen sein, dass die Substanz (i) 1,2-Distearoyl-sn-glycero-3-phosphocholin (DSPC, Distearoylphosphatidylcholin, Colfoscerilstearat) ist oder umfasst. Grundsätzlich können als Substanz (i) Lecithin, insbesondere Sojalecithin, eingesetzt werden.

Zudem kann es erfindungsgemäß vorgesehen sein, dass die Substanz (ii) ein PEG-Stearat, insbesondere PEG 40-Stearat, bzw. ein Macrogol-Stearat, insbesondere Macrogol 40-Stearat, ist und/oder umfasst; bzw. dass die Substanz (ii) PEG 2000-DMG (1,2-Dimyristoyl-rac-glycero-3-methoxypolyethyleneglykol-2000) ist und/oder umfasst; bzw. dass die Substanz (ii) ein Cetromacrogol (Polyethylenglycolhexadecylether) ist und/oder umfasst; bzw. dass die Substanz (ii) 2-[(Polyethylenglycol)-2000]-N,N-ditetradecylacetamid (ALC-0159) ist und/oder umfasst; bzw. dass die Substanz (ii) ein Polaxamer ist und/oder umfasst; bzw. dass die Substanz (ii) ein Alkylpolyglykosid, insbesondere $C_8$- bis $C_{14}$-Alkylpolyglykosid, vorzugsweise $C_{10}$- bis $C_{12}$-Alkylpolyglykosid, ist oder umfasst; bzw. dass die Substanz (ii) ein ethoxylierter Sorbitanfettsäureester, insbesondere Polysorbat, vorzugsweise Polysorbat 80 und/oder Polysorbat 20, ist oder umfasst.

Insbesondere kann die Substanz (iii) Cholesterol oder Phytosterol oder deren Mischungen, insbesondere Phytosterol, sein oder umfassen. Neben einer Optimierung des Aufbaus und der Struktur der Partikel des lipidbasierten Trägersystems gehen Phytosterole sowie Cholesterol auch mit einem antioxidativen Schutzeffekt einher, welcher für Phytosterole besonders ausgeprägt ist. Dies geht mit einem Schutz gegenüber Oxidation insbesondere im Hinblick auf erfindungsgemäß eingesetzte Wirkstoffe bzw. andere oxidationsempfindliche Substanzen bzw. Bestandteile einher. Als Phytosterol kann beispielsweise Stigmasterin eingesetzt werden.

Was zudem die Verwendung von Calciferol (Vitamin D) insbesondere als Substanz (iii) anbelangt, so kann es sich erfindungsgemäß insbesondere derart verhalten, dass - ohne sich auf diese Theorie beschränken oder berufen zu wollen, diesbezüglich ein Einbau in die Lamellenstruktur der Partikel vorliegt, was auch aufgrund der antioxidativen Wirkung von Calciferol (Vitamin D) zu einer Stabilisierung bzw. einem Oxidationsschutz führt. Calciferol (Vitamin D) kann somit insbesondere als Lamellen- bzw. Membran-Antioxidans fungieren. Zudem wirkt Calciferol (Vitamin D) imunstimulierend bzw. imunmodulierend, so dass bei Verabreichung auch eine Stärkung des Imunsystems erfolgen kann.

Wie zuvor angeführt, wird im Rahmen des erfindungsgemäßen Verfahrens ein sozusagen universelles Trägersystem bereitgestellt, welches mit einer Vielzahl verschiedener Wirkstofftypen bzw. -arten beladen bzw. ausgerüstet werden kann, wobei zudem auf Basis der Abstimmung und Auswahl der Bestandteile bzw. Substanzen und der verfahrensgemäß der kontrollierten Ausgestaltung bzw. Formgebung der Partikel, insbesondere Lipidnanopartikel, des Trägersystems eine weiterführende Maßschneiderung erfolgen kann.

Im Hinblick auf die Substanz (iv) in Form des kationischen Lipids verhält es sich insbesondere derart, dass es sich hierbei um ein Lipid handelt, welches bei einem pH-Wert zumindest im Bereich von 6,5 bis 7,8, insbesondere im Bereich von 6,8 bis 7,6, vorzugsweise im Bereich von 7 bis 7,5, bevorzugt im Bereich von 7,35 bis 7,45 (physiologischer Bereich), positiv geladen bzw. als Kation ausgebildet ist.

Erfindungsgemäß ist es zudem für den Einsatz eines Wirkstoffs insbesondere vorgesehen, dass der Wirkstoff, insbesondere als Substanz (vi), Bestandteil des Trägersystems ist und/oder einen Bestandteil des Trägersystems ausbildet und/oder wobei das Trägersystem den Wirkstoff, insbesondere als Substanz (vi), aufweist.

Insbesondere kann der Wirkstoff, insbesondere als Substanz (vi), in entsprechender Weise Bestandteil der Partikel, bevorzugt Lipidnanopartikel, des vorzugsweise lipidbasierten Trägersystems ein. Diesbezüglich kann der Wirkstoff, insbesondere als Substanz (vi) in Bezug auf die Partikel, insbesondere Lipidnanopartikel, auf-, an- bzw. eingelagert bzw. integriert, vorzugsweise eingelagert bzw. integriert, sein bzw. werden. Dies führt zu einem weiteren Schutz des Wirkstoffs, zu einer verbesserten (Lager-)Stabilität der resultierenden Zusammensetzung und zu einer verbesserten Verabreichung des Wirkstoffs auch am Wirkort, insbesondere zu einer verbesserten Aufnahme in (Körper-) Zellen.

Für den Wirkstoff bzw. die als Bestandteil des Trägersystems vorliegende bzw. eingesetzte Substanz (vi) kann insbesondere Folgendes gelten:
Insbesondere kann der Wirkstoff ein therapeutischer und/oder prophylaktischer Wirkstoff, insbesondere therapeutischer und/oder prophylaktischer pharmazeutischer Wirkstoff, sein.

Erfindungsgemäß kann der Wirkstoff insbesondere ein zumindest im Wesentlichen wasserunlöslicher, vorzugsweise ein zumindest im Wesentlichen wasserunlöslicher amphiphiler Wirkstoff, sein. Diesbezüglich bezieht sich der Begriff "wasserunlöslich" insbesondere auf die diesbezügliche Eigenschaft des Wirkstoffs bei Raumtemperatur (20 °C) und Umgebungsdruck (1.013,25 hPa). Erfindungsgemäß können demgegenüber aber auch bei den vorgenannten Bedingungen wasserlösliche Wirkstoffe eingesetzt werden.

Erfindungsgemäß kann der Wirkstoff ausgewählt werden aus der Gruppe von Vakzinwirkstoffen (Impfwirkstoffen), antibiotischen Wirkstoffen, antimykotischen Wirkstoffen, antiviralen Wirkstoffen, antiphlogistischen Wirkstoffen, antipyretischen Wirkstoffen, antiallergischen Wirkstoffen, Antikrebswirkstoffen, Hormonwirkstoffen, antikoagulativen Wirkstoffen, Antitumorwirkstoffen (wie Zytostatika), Nahrungsergänzungsstoffen, Vitaminen, Mineralstoffen, UV-absorbierenden Stoffen und deren Mischungen sowie Kombinationen, vorzugsweise Vakzinwirkstoffen (Impfwirkstoffen).

Weiterhin kann der Wirkstoff ausgewählt werden aus der Gruppe von RNA, insbesondere messenger-RNA (mRNA); Retinol; Tocopherol; Kortikosteroiden, insbesondere Kortison; Antibiotika, insbesondere Tetracyklinen und Benzoylpenicillinen; Diclofenac; Estradiol, Estradiol-Hemihydrat, Salicylsäure, Acetylsalicylsäure; Indometacin; ätherischen Ölen; insbesondere amphiphilen UVA-Filtern und UVB-Filter; Nicotinamid; und deren Mischungen sowie Kombinationen.

Insbesondere im Hinblick auf die Verwendung von RNA bzw. mRNA, vorzugsweise einsträniger mRNA, hat es sich erfindungsgemäß als vorteilhaft erwiesen, wenn das Trägersystem, insbesondere weiterhin die Substanz (iv) in Form des kationischen Lipids aufweist, insbesondere wie zuvor definiert. Hierdurch kann eine weiterführende Optimierung der Ausrüstung des Trägersystems bzw. der diesbezüglichen Partikel, bevorzugt Lipidnanopartikel, mit dem Wirkstoff bereitgestellt werden. Insbesondere kann eine optimierte Interaktion im Hinblick auf die negativ geladene bzw. anionisch ausgebildete RNA, insbesondere mRNA, vorliegen bzw. gewährleistet werden.

Erfindungsgemäß kann es sich insbesondere derart verhalten, dass der Wirkstoff ein Vakzinwirkstoff (Impfwirkstoff), insbesondere ein Nukleinsäure-basierter Vakzinwirkstoff und/oder genbasierter Vakzinwirkstoff, vorzugsweise ein Antigen eines Erregers und/oder eines Pathogens, insbesondere ein Antigen eines Virus, codierender Vakzinwirkstoff ist.

In diesem Zusammenhang kann es insbesondere vorgesehen sein, dass der Vakzinwirkstoff ausgewählt wird aus der Gruppe von DNA; RNA, insbesondere messenger-RNA (mRNA); viralen Vektoren; Plasmiden, insbesondere Plasmid-DNA (pDNA); vorzugsweise RNA; bevorzugt mRNA; und deren Mischungen sowie Kombinationen.

Gleichermaßen kann es in diesem Zusammenhang vorgesehen sein, dass der Wirkstoff ein mRNA-basierter Vakzinwirkstoff und/oder mRNA ist, insbesondere wobei die mRNA einzelsträngig ausgebildet ist und/oder insbesondere wobei die mRNA eine 5'-Cap-Struktur aufweist. Diesbezüglich kann der mRNA-basierte Vakziinwirkstoff bzw. die mRNA gleichermaßen mindestens eine poly(A)-Sequenz und/oder mindestens eine poly(C)-Sequenz aufweisen.

Zudem kann es sich in diesem Zusammenhang im Rahmen der vorliegenden Erfindung derart verhalten, dass der Wirkstoff ein mRNA-basierter Vakzinwirkstoff und/oder mRNA ist, insbesondere wobei die mRNA ein Protein, insbesondere Antigen, eines Coronavirus, vorzugsweise SARS-CoV-2, codiert, insbesondere wobei das Protein, insbesondere Antigen, das insbesondere membranverankerte *Spike*-Protein (*Spike*-Glykoprotein bzw. S-Protein bzw. S-Glykoprotein) des Coronavirus, vorzugsweise SARS-CoV-2, zumindest teilweise, vorzugsweise zumindest im Wesentlichen vollständig, codiert. In diesem Zusammenhang kann es sich insbesondere um eine einzelsträngige, 5'-gekappte Boten-RNA (mRNA) handeln, welche für das membranverankerte virale *Spike*-(S)Protein bzw. *Spike*-(*S*-)Glykoprotein von SARS-CoV-2 codiert. Im Allgemeinen kann es sich bei dem Wirkstoff um einen genbasierten Wirkstoff bzw. genbasierten Vakzinwirkstoff handeln. Erfindungsgemäß kann es sich aber auch bei dem Wirkstoff bzw. Vakzinwirkstoff um ein Protein und/oder Antigen, insbesondere eines pathogenen Erregers, wie eines Bakteriums, Virus oder dergleichen, handeln.

Im Allgemeinen kann der Wirkstoff bzw. die Substanz (vi) einen HLB-Wert nach Griffin im Bereich von 0,5 bis 6, insbesondere im Bereich von 1 bis 5, aufweisen. Demgegenüber kann der Wirkstoff bzw. die Substanz (vi) aber auch einen HLB-Wert nach Griffin im Bereich von 6 bis 15, insbesondere im Bereich von 8 bis 14, vorzugsweise im Bereich von 10 bis 13, aufweisen. Für mRNA kann insbesondere ein HLB-Wert nach Griffin im Bereich von 8 bis 14, insbesondere 10 bis 13, vorliegen. Zudem kann der Wirkstoff bzw. die Substanz (vi) einen Log P-Wert im Bereich von 1,11 bis 18,97, insbesondere im Bereich von 1,36 bis 8,58, aufweisen. Demgegenüber kann der Wirkstoff bzw. die Substanz (vi) aber auch einen Log P-Wert im Bereich von 0,39 bis 1,11, insbesondere im Bereich von 0,42 bis 0,80, vorzugsweise im Bereich von 0,46 bis 0,62, aufweisen. Für mRNA kann insbesondere ein Log P-Wert im Bereich von 0,42 bis 0,80, insbesondere 0,46 bis 0,62, vorliegen.

Erfindungsgemäß kann das Trägersystem den Wirkstoff, insbesondere als Substanz (vi), bzw. die Substanz (vi) in einer Menge im Bereich von 0,01 Gew.-% bis 80 Gew.-%, insbesondere im Bereich von 0,1 Gew.-% bis 70 Gew.-%, vorzugsweise im Bereich von 0,5 Gew.-% bis 60 Gew.-%, bevorzugt im Bereich von 1 Gew.-% bis 50 Gew.-%, bezogen auf das Trägersystem und/oder bezogen auf die Gesamtheit der Bestandteile des Trägersystems, aufweisen.

In diesem Zusammenhang kommt auch dem Verhältnis des Wirkstoffes bzw. der Substanz (vi) zu den weiteren Bestandteilen bzw. Substanzen des Trägersystems eine Bedeutung zu:

So kann das gewichtsbezogene Mengenverhältnis von Substanz (i) zu Substanz (ii) zu Wirkstoff bzw. Substanz (vi) (d. h. dem Wirkstoff) [Substanz (i) : Substanz (ii) : Substanz (vi)] im Bereich von (0,5 bis 50) : (1 bis 80) : (0,01 bis 80), insbesondere im Bereich von (1 bis 40) : (2 bis 75) : (0,1 bis 70), vorzugsweise im Bereich von (1,25 bis 30) : (3 bis 70) : (0,5 bis 60), bevorzugt im Bereich von (1,5 bis 25) : (5 bis 65) : (1 bis 50) liegen und/oder auf die vorgenannten Werte eingestellt werden.

Weiterhin kann das gewichtsbezogene Mengenverhältnis von Substanz (i) zu Substanz (ii) zu Substanz (iii) zu Substanz (iv) zu Substanz (v) zu Wirkstoff bzw. Substanz (vi) [Substanz (i) : Substanz (ii) : Substanz (iii) : Substanz (iv) : Substanz (v) : Substanz (vi)] im Bereich von (0,5 bis 50) : (1 bis 80) : (0,2 bis 40) : (1 bis 80) : (2 bis 85) : (0,01 bis 80), insbesondere im Bereich von (1 bis 40) : (2 bis 75) : (0,5 bis 25) : (2 bis 75) : (4 bis 80) : (0,1 bis 70), vorzugsweise im Bereich von (1,25 bis 30) : (3 bis 70) : (1 bis 20) : (3 bis 70) : (6 bis 75) : (0,5 bis 60), bevorzugt im Bereich von (1,5 bis 25) : (5 bis 65) : (3 bis 15) : (5 bis 65) :(8 bis 70) : (1 : 50), liegen und/oder auf die vorgenannten Werte eingestellt werden.

Darüber hinaus kann das insbesondere lipidbasierte Trägersystem noch weitere Bestandteile bzw. Substanzen aufweisen, welche im Rahmen des erfindungsgemäßen Verfahrens eingesetzt werden können. Hierdurch können die Eigenschaften der resultierenden Partikel bzw. Lipidnanopartikel weiterführend eingestellt und die Ausbildung der Partikel im Rahmen der Verfahrensführung weiterführend optimiert werden:

Erfindungsgemäß kann das Trägersystem als weiteren Bestandteil, insbesondere als Substanz (vii), mindestens einen Fettalkohol, insbesondere $C_8$- bis $C_{25}$-Fettalkohol, vorzugsweise mindestens einen verzweigten Fettalkohol, bevorzugt Octyldodecanol, besonders bevorzugt 2-Octyldocen-1-ol, umfassen, insbesondere in einer Menge im Bereich von 1 Gew.-% bis 80 Gew.-%, vorzugsweise im Bereich von 5 Gew.-% bis 70 Gew.-%, bevorzugt im Bereich von 10 Gew.-% bis 60 Gew.-%, bezogen auf das Trägersystem und/oder bezogen auf die Gesamtheit der Bestandteile des Trägersystems.

In diesem Zusammenhang kann der Fettalkohol, bevorzugt Octyldodecanol, einen HLB-Wert nach Griffin im Bereich von 1 bis 5, insbesondere im Bereich von 2 bis 4, aufweisen und/oder kann der Fettalkohol, bevorzugt Octyldodecanol, einen Log P-Wert im Bereich von 1,36 bis 8,58, insbesondere im Bereich von 1,76 bis 3,88, aufweisen.

Gleichermaßen kann der Fettalkohol, bevorzugt Octyldodecanol, einen kritischen Packungsparameter $P_{kr}$ im Bereich von 0,91 bis 1,19, insbesondere im Bereich von 0,98 bis 1,12, aufweisen.

Insbesondere kann es sich bei dem Fettalkohol bzw. Octyldodecanol um 2-Octyldodecan-1-ol (Handelsname Eutanol G) handeln. Octyldecanol weist einen HLB-Wert nach Griffin von etwa 3 auf und ist von daher in seinen physikalischen Eigenschaften sehr ähnlich zu der zuvor angeführten Substanz ALC-0315. Durch die Verwendung eines Fettalkohols kann die Ausbildung des Trägersystems in Form der Partikel weiterführend gesteuert werden.

Weiterhin kann das Trägersystem als weiteren Bestandteil, insbesondere als Substanz (viii), mindestens eine grenzflächenaktive amphiphile Substanz, insbesondere grenzflächenaktive und nichtmizellbildende amphiphile Substanz, vorzugsweise mindestens einen Sorbitanfettsäureester, bevorzugt mindestens einen Sorbitanfettsäureester mit bzw. auf Basis von gesättigten und/oder ungesättigten $C_8$-bis $C_{18}$-Fettsäuren, insbesondere $C_{10}$- bis $C_{14}$-Fettsäuren, besonders bevorzugt Sorbitancaprylat, umfassen, insbesondere in einer Menge im Bereich von 0,1 Gew.-% bis 30 Gew.-%, vorzugsweise im Bereich von 1 Gew.-% bis 20 Gew.-%, bevorzugt im Bereich von 5 Gew.-% bis 15 Gew.-%, bezogen auf das Trägersystem und/oder bezogen auf die Gesamtheit der Bestandteile des Trägersystems.

**In** diesem Zusammenhang kann die grenzflächenaktive amphiphile Substanz, vorzugsweise der Sorbitanfettsäureester, einen HLB-Wert nach Griffin im Bereich von 2 bis 5, insbesondere im Bereich von 3 bis 4, aufweisen bzw. kann die grenzflächenaktive amphiphile Substanz, vorzugsweise der Sorbitanfettsäureester, einen Log P-Wert im Bereich von 1,36 bis 3,88, insbesondere im Bereich von 1,76 bis 2,44, aufweisen.

Zudem kann die grenzflächenaktive amphiphile Substanz, vorzugsweise der Sorbitanfettsäureester, einen kritischen Packungsparameter $P_{kr}$ im Bereich von 0,91 bis 1,12, insbesondere im Bereich von 0,98 bis 1,05, aufweisen.

Darüber hinaus kann das Trägersystem als weiteren Bestandteil, insbesondere als Substanz (ix), mindestens einen Ether, insbesondere Ether eines Diols oder Polyols, vorzugsweise Ether eines bicyclischen Diols, bevorzugt Dialkylisosorbit, besonders bevorzugt Dimethylisosorbid (DMI), ganz besonders bevorzugt 2,5-Dimethylisosorbid, umfassen, insbesondere in einer Menge im Bereich von 2,5 Gew.-% bis 30 Gew.-%, vorzugsweise im Bereich von 5 Gew.-% bis 25 Gew.-%, bevorzugt im Bereich von 10 Gew.-% bis 20 Gew.-%, bezogen auf das Trägersystem und/oder bezogen auf die Gesamtheit der Bestandteile des Trägersystems.

Ohne sich auf diese Theorie beschränken oder berufen zu wollen, handelt es sich bei Dimethylisosorbid um einen Penetrationsförderer, was die Aufnahme der Wirksubstanz in eine (Körper-)Zelle erleichtern kann. Zudem werden durch die gezielte Verwendung von Dimethylisosorbid bei der Herstellung des Teilchensystems besonders kleine Partikel, bevorzugt Lipidnanopartikel, in Bezug auf das Trägersystem erhalten, und dies bei gleichzeitig geringer Dispersität bzw. kleiner Varianz.

Erfindungsgemäß kann es weiterhin vorgesehen sein, dass das Trägersystem als weiteren Bestandteil, insbesondere als Substanz (x), mindestens einen Alkohol umfasst, insbesondere wobei der Alkohol ausgewählt wird aus der Gruppe von einwertigen Alkoholen, mehrwertigen Alkoholen, insbesondere Polyolen, und deren Mischungen sowie Kombinationen; und/oder wobei das Trägersystem als weiteren Bestandteil, insbesondere als Substanz (x), mindestens ein Polyol umfasst.

In diesem Zusammenhang kann es vorgesehen sein, dass der Alkohol, insbesondere das Polyol, ausgewählt wird aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen.

Gleichermaßen kann es diesbezüglich vorgesehen sein, das der Alkohol, insbesondere das Polyol, ausgewählt wird aus der Gruppe von Propylenglykol, Polyethylenglykol, Sorbitol, Mannitol, Glycerin, Saccharose, Panthenol und deren Mischungen sowie Kombinationen.

Erfindungsgemäß ist es dabei bevorzugt, dass der Alkohol, insbesondere das Polyol, Panthenol ist. Bei Panthenol handelt es sich gleichermaßen um einen Penetrationsverstärker. Diesbezüglich hat die Anmelderin in völlig überraschender Weise gefunden, dass durch den ziel- und zweckgerichteten Einsatz von Panthenol das Penetrationsverhalten der resultierenden Partikel, insbesondere Lipidnanopartikel des Trägersystems vorgegeben bzw. eingestellt werden kann, wie es auch bereits für das zuvor beschriebene Dimethylisosorbid gemäß Substanz (ix) der Fall ist.

Insgesamt kann Panthenol im Hinblick auf den erfindungsgemäßen Einsatz multifunktionelle Eigenschaften aufweisen. Bei Panthenol handelt es sich um eine kationische Substanz, die zum einen wasserlöslich ist, sich aber um anderen auch in lipidbasierten Strukturen verankert bzw. hiermit interagiert.

Erfindungsgemäß kann der Alkohol, insbesondere das Polyol, zudem Glycerin sein.

Erfindungsgemäß kann die Substanz (x) bzw. der Alkohol, insbesondere das Polyol, in einer Menge im Bereich von 1 Gew.-% bis 90 Gew.-%, insbesondere im Bereich von 2 Gew.-% bis 70 Gew.-%, vorzugsweise im Bereich von 3 Gew.-% bis 60 Gew.-%, bevorzugt im Bereich von 5 Gew.-% bis 55 Gew.-%, bezogen auf das Trägersystem, insbesondere bezogen auf die Gesamtheit der Bestandteile des Trägersystems, vorliegen bzw. eingesetzt werden.

Erfindungsgemäß kann es zudem vorgesehen sein, dass der Alkohol, insbesondere das Polyol, in einer Menge eingesetzt wird derart, dass das gewichtsbezogene Mengenverhältnis von Alkohol, insbesondere das Polyol, zu Trägersystem, insbesondere Gesamtheit der Bestandteile des Trägersystems, [Alkohol, insbesondere Polyol : Trägersystem, insbesondere Gesamtheit der Bestandteile des Trägersystems] im Bereich von 1 : 150 bis 10 : 1, insbesondere im Bereich von 1 : 100 bis 5 : 1, vorzugsweise im Bereich von 1 : 50 bis 1 : 1, bevorzugt im Bereich von 1 : 20 bis 1 : 5, liegt und/oder eingestellt wird.

Gemäß einer erfindungsgemäß bevorzugten Ausführungsform ist es insbesondere vorgesehen, dass der Alkohol kein $C_1$- bis $C_4$-Monoalkohol, vorzugsweise kein Ethanol, ist und/oder insbesondere wobei der Alkohol keinen $C_1$- bis $C_4$-Monoalkohol, insbesondere kein Ethanol, umfasst. Insbesondere ist die Substanz (x) und/oder das Trägermaterial kein $C_1$- bis $C_4$-Monoalkohol, vorzugsweise kein Ethanol. Vorzugsweise verhält es sich derart, dass die Substanz (x) keinen $C_1$- bis $C_4$-Monoalkohol, insbesondere kein Ethanol, umfasst.

Erfindungsgemäß ist es in besonders bevorzugter Weise vorgesehen, dass das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird.

In diesem Zusammenhang ist es insbesondere vorgesehen, dass das flüssige Medium als kontinuierliche Phase ausgebildet wird und/oder vorliegt.

Insbesondere ist es gleichermaßen vorgesehen, dass die Ausbildung des Trägersystems, insbesondere in partikulärer Form, bevorzugt in Form von Lipidnanopartikeln (LNP), in dem flüssigen Medium erfolgt und/oder bewirkt wird.

Erfindungsgemäß verhält es sich dabei insbesondere derart, dass nach Ausbildung des Trägersystems eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert und/oder insbesondere wobei nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert.

Dementsprechend ist es erfindungsgemäß somit insbesondere vorgesehen, dass das Inkontaktbringen bzw. das Inwechselwirkungbringen der Bestandteile zur Ausbildung des Trägersystems in einem flüssigen Medium erfolgt, insbesondere zu Zwecken der Zusammenlagerung bzw. Aggregation der Bestandteile zur Ausbildung der Partikel des Trägersystems, insbesondere Lipidnanopartikel.

Mit anderen Worten verhält es sich erfindungsgemäß insbesondere derart, dass die Bestandteile des Trägersystems in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), (miteinander) in Kontakt gebracht werden und/oder (miteinander) in Wechselwirkung gebracht werden bzw. dass die Zusammenlagerung der Bestandteile zur Ausbildung des Trägersystems, vorzugsweise in partikulärer Form, bevorzugt in Form von Lipidnanopartikeln (LNP), in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), erfolgt und/oder bewirkt wird.

In diesem Zusammenhang kann das flüssige Medium als kontinuierliche Phase ausgebildet werden bzw. vorliegen. Zudem verhält es sich auch diesbezüglich insbesondere derart, dass nach Ausbildung des Trägersystems eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert und/oder insbesondere wobei nach Ausbildung des Trägersystems eine Dispersion des Trägersystems bzw. des mit dem pharmazeutischen Wirkstoff beladenen und/oder ausgerüsteten Trägersystems in dem flüssigen Medium resultiert.

Im Hinblick auf die erfindungsgemäße Verfahrensführung und die damit einhergehende Ausbildung des Trägersystems in Form von Partikeln, insbesondere Lipidnanopartikeln, kommt auch der weiterführenden Ausbildung des flüssigen Mediums sowie der im Rahmen des erfindungsgemäßen Verfahrens eingesetzten Menge, insbesondere auch in Relation zu den Bestandteilen des Trägersystems, eine wichtige Bedeutung zu:

Insbesondere kann das flüssige Medium in einer 0,05fachen bis 10fachen, insbesondere 0,1fachen bis 5fachen, insbesondere 0,15fachen bis 4fachen, bevorzugt 0,2fachen bis 3fachen, Gewichtsmenge zu der Gewichtsmenge des Trägersystems, insbesondere zu der Gewichtsmenge der Gesamtheit der Bestandteile des Trägersystems, vorliegen bzw. eingesetzt werden. Die Gewichtsmenge des flüssigen Mediums bezieht sich dabei insbesondere auf die Gesamtheit des flüssigen Mediums mit den darin gegebenenfalls weiterführend vorgesehenen Komponenten bzw. Bestandteilen des flüssigen Mediums. In entsprechender Weise kann es erfindungsgemäß auch vorgesehen sein, dass das flüssige Medium in einer Menge eingesetzt wird derart, dass das gewichtsbezogene Mengenverhältnis von Trägersystem, insbesondere Gesamtheit der Bestandteile des Trägersystems, zu flüssigem Medium [Trägersystem, insbesondere Gesamtheit der Bestandteile des Trägersystems : flüssiges Medium] im Bereich von 1 : 20 bis 50 : 1, insbesondere im Bereich von 1 : 10 bis 20 : 1, vorzugsweise im Bereich von 1 : 5 bis 10 : 1, bevorzugt im Bereich von 1 : 4 bis 5 : 1, liegt und/oder eingestellt wird.

Erfindungsgemäß kann es sich zudem derart verhalten, dass das flüssige Medium Wasser und/oder mindestens einen Alkohol, vorzugsweise Wasser und mindestens einen Alkohol, umfassen. In diesem Zusammenhang kann der Alkohol ausgewählt werden aus der Gruppe von einwertigen Alkoholen, mehrwertigen Alkoholen, insbesondere Polyolen, und deren Mischungen sowie Kombinationen.

In erfindungsgemäß bevorzugter Weise ist der Alkohol ein mehrwertiger Alkohol, insbesondere Polyol. Durch den Einsatz der diesbezüglich vorgesehenen, insbesondere mehrwertigen Alkoholen, wie Polyolen, kann das Eindringen bzw. Dispergieren der Bestandteile in dem flüssigen Medium gesteuert bzw. verbessert werden, was auch im Hinblick auf die Ausbildung der partikulären Strukturen in dem flüssigen Medium von Vorteil ist. Wie nachfolgend beschrieben, verhält es sich erfindungsgemäß derart, dass der diesbezügliche Alkohol kein Ethanol ist.

Demgegenüber ist es erfindungsgemäß nämlich bevorzugt, dass der Alkohol, insbesondere das Polyol, ausgewählt wird aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen sowie Kombinationen.

Erfindungsgemäß ist es insbesondere vorgesehen, dass der Alkohol, insbesondere das Polyol, ausgewählt wird aus der Gruppe von Propylenglykol, Polyethylenglykol, Sorbitol, Mannitol, Glycerin, Saccharose, Panthenol und deren Mischungen sowie Kombinationen. Gemäß einer bevorzugten Ausführungsform kann der Alkohol, insbesondere das Polyol, Panthenol sein. Somit kann grundsätzlich auch das flüssige Medium Panthenol aufweisen, und zwar insbesondere unabhängig von den Bestandteilen des Trägersystems. Diesbezüglich kann zu den Eigenschaften von Panthenol auch auf die obigen Ausführungen verwiesen werden.

Zudem kann der Alkohol, insbesondere das Polyol, auch Glycerin sein. Glycerin weist im Rahmen der vorliegenden Erfindung dabei in überraschender Weise besonders gute Eigenschaften hinsichtlich der Verwendung für das vorliegend beschriebene flüssige Medium bzw. für das Trägersystem (vgl. zuvor beschriebene Substanz (x)) auf. Insbesondere können unter Verwendung von Glycerin auch höherschmelzende Substanzen, insbesondere höherschmelzende amphiphile Substanzen, wie höherschmelzende Phospholipide (z.B. DSPC), gelöst bzw. optimal in Wechselwirkung zueinander bzw. mit den weiteren Substanzen gebracht werden.

Auch für den Alkohol in dem flüssigen Medium verhält es sich erfindungsgemäß derart, dass der Alkohol kein Ethanol, ist bzw. dass der Alkohol keinen Ethanol umfasst. Mit anderen Worten verhält es sich auch für das flüssige Medium derart, dass dieses keinen Ethanol umfasst bzw. aufweist und somit insbesondere frei von einem $C_1$- bis $C_4$-Monoalkohol ist.

Erfindungsgemäß kann das flüssige Medium das Wasser in einer Menge im Bereich von 10 Gew.-% bis 99,5 Gew.-%, insbesondere im Bereich von 20 Gew.-% bis 99 Gew.-%, vorzugsweise im Bereich von 30 Gew.-% bis 97 Gew.-%, bevorzugt im Bereich von 35 Gew.-% bis 95 Gew.-%, bezogen auf das flüssige Medium, aufweisen. Insbesondere kann das flüssige Medium den Alkohol, insbesondere das Polyol, in einer Menge im Bereich von 0,5 Gew.-% bis 90 Gew.-%, insbesondere im Bereich von 1 Gew.-% bis 80 Gew.-%, vorzugsweise im Bereich von 3 Gew.-% bis 70 Gew.-%, bevorzugt im Bereich von 5 Gew.-% bis 65 Gew.-%, bezogen auf das flüssige Medium, aufweisen.

Zudem kann das flüssige Medium ein Gemisch (Mischung) von Wasser und mindestens einem Alkohol, insbesondere Polyol, insbesondere wie zuvor definiert, umfasst oder hieraus besteht, insbesondere wobei das gewichtsbezogene Mengenverhältnis von Wasser zu Alkohol, insbesondere Polyol, [Wasser : Alkohol, insbesondere Polyol] im Bereich von 200 : 1 bis 1 : 50, insbesondere im Bereich von 100 : 1 bis 1 : 20, vorzugsweise im Bereich von 50 : 1 bis 1 : 5, bevorzugt im Bereich von 20 : 1 bis 1 : 1, insbesondere bezogen auf das flüssige Medium, liegen bzw. eingestellt werden.

Vor diesem Hintergrund ist es erfindungsgemäß insbesondere vorgesehen, dass das Verfahren, insbesondere die Herstellung des Trägersystems bzw. des mit einem pharmazeutischen Wirkstoff beladenen und/oder ausgerüsteten Trägersystems, in Gegenwart mindestens eines Alkohols, insbesondere Polyols, durchgeführt wird, insbesondere als Bestandteil des flüssigen Mediums und/oder als Bestandteil des Trägersystems, bzw. dass die Zusammenlagerung der Bestandteile zur Ausbildung des Trägersystems, vorzugsweise in partikulärer Form, bevorzugt in Form von Lipidnanopartikeln (LNP), in Gegenwart mindestens eines Alkohols, insbesondere Polyols, durchgeführt wird.

Diesbezüglich ist es insbesondere vorgesehen, dass der Alkohol ausgewählt wird aus der Gruppe von einwertigen Alkoholen, mehrwertigen Alkoholen, insbesondere Polyolen, und deren Mischungen sowie Kombinationen, insbesondere wie in zuvor definiert bzw. dass der Alkohol, insbesondere das Polyol, in einer wie zuvor definierten Menge vorliegt bzw. eingesetzt wird.

Erfindungsgemäß kann es weiterhin vorgesehen sein, dass das flüssige Medium mindestens einen Ether, insbesondere Ether eines Diols oder Polyols, vorzugsweise Ether eines bicyclischen Diols, bevorzugt Dialkylisosorbit, besonders bevorzugt Dimethylisosorbid, ganz besonders bevorzugt 2,5-Dimethylisosorbid, umfasst, insbesondere in einer Menge im Bereich von 0,5 Gew.-% bis 25 Gew.-%, vorzugsweise im Bereich von 1 Gew.-% bis 10 Gew.-%, bevorzugt im Bereich von 1 Gew.-% bis 10 Gew.-%, bezogen auf das flüssige Medium.

Insbesondere kann das Verfahren nach der Erfindung, insbesondere die Herstellung des Trägersystems bzw. des mit einem pharmazeutischen Wirkstoff beladenen und/oder ausgerüsteten Trägersystems, in Gegenwart mindestens eines Ethers, insbesondere wie zuvor definiert, durchgeführt werden bzw. kann die Zusammenlagerung der Bestandteile zur Ausbildung des Trägersystems, vorzugsweise in partikulärer Form, bevorzugt in Form von Lipidnanopartikeln (LNP), in Gegenwart mindestens eines Ethers, insbesondere wie zuvor definiert, durchgeführt werden. Diesbezüglich kann der Ether in einer wie zuvor definierten Menge vorliegt bzw. eingesetzt werden.

Gemäß einer weiteren bevorzugten Ausführungsform kann es erfindungsgemäß auch vorgesehen sein, dass das flüssige Medium mindestens ein Antioxidationsmittel, insbesondere Ascorbinsäure (Vitamin C) umfasst, insbesondere in einer Menge im Bereich von 0,1 Gew.-% bis 10 Gew.-%, vorzugsweise im Bereich von 0,2 Gew.-% bis 5 Gew.-%, bevorzugt im Bereich von 0,5 Gew.-% bis 2 Gew.-%, bezogen auf das flüssige Medium.

Erfindungsgemäß kann es insbesondere vorgesehen sein, dass das Verfahren, insbesondere die Herstellung des Trägersystems bzw. des mit einem pharmazeutischen Wirkstoff beladenen und/oder ausgerüsteten Trägersystems, in Gegenwart mindestens eines Antioxidationsmittels, insbesondere Ascorbinsäure (Vitamin C), insbesondere wie zuvor definiert, durchgeführt wird; bzw. die Zusammenlagerung der Bestandteile zur Ausbildung des Trägersystems, vorzugsweise in partikulärer Form, bevorzugt in Form von Lipidnanopartikeln (LNP), in Gegenwart mindestens eines Antioxidationsmittels, insbesondere Ascorbinsäure (Vitamin C), durchgeführt wird. Diesbezüglich kann das Antioxidationsmittel in einer wie zuvor definierten Menge vorliegen bzw. eingesetzt werden.

Weiterhin kann das flüssige Medium mindestens ein Biopolymer, insbesondere ausgewählt aus der Gruppe von Alginaten, Hyaluronsäure und Chitosan (sowie deren jeweiligen Salzen und Derivaten) sowie deren Mischungen und Kombinationen, aufweisen, insbesondere in einer Menge im Bereich von 0,1 Gew.-% bis 50 Gew.-%, insbesondere im Bereich von 0,5 Gew.-% bis 30 Gew.-%, vorzugsweise im Bereich von 1 Gew.-% bis 10 Gew.-%, bezogen auf das flüssige Medium. Der Einsatz der vorgenannten Biopolymere kommt insbesondere für den Fall in Betracht, wonach das flüssige Medium weiterhin zum (nachträglichen) Verdünnen des Trägersystems bzw. der Partikel eingesetzt wird. Hierdurch kann die Zusammensetzung mit weiteren Eigenschaften versehen werden.

Weiterhin kann das flüssige Medium weitere Bestandteile bzw. Inhaltsstoffe aufweisen, wie Additiven oder dergleichen, insbesondere ausgewählt aus der Gruppe von Netzmitteln bzw. oberflächenaktiven Substanzen, Konservierungsmitteln, Stabilisatoren, geschmacks- bzw. geruchsmodifizierenden Substanzen, Rheologiemodifikatoren, Verdickungsmitteln, Farbstoffen, Puffern, pH-Stellmitteln oder deren Mischungen bzw. Kombinationen. Diesbezüglich können auch Geschmacksstoffe, wie (weitere) Süßungsmittel bzw. Süßstoffe eingesetzt werden. Beispielsweise kann Acesulfam als Süßungsmittel zugesetzt werden (beispielsweise um einem bitteren Geschmack bei oraler Applikation der Zusammensetzung entgegenzuwirken).

Wie auch zuvor angeführt, verhält es sich erfindungsgemäß derart, dass das Trägersystem und/oder das flüssige Medium und/oder die Zusammensetzung, insbesondere wie zuvor definiert, zumindest im Wesentlichen frei von Ethanol ist bzw. sind. Diesbezüglich verhält es sich erfindungsgemäß derart, dass das Verfahren in zumindest im Wesentlichen vollständiger Abwesenheit von Ethanol durchgeführt wird. Auf diese Weise wird eine hohe Kompatibilität in Bezug auf die Wirkstoffe sowie eine hohe Verträglichkeit gewährleistet. Insbesondere entfallen aufwendige Verfahrensschritte zur Entfernung des zuvor angeführten Monoalkohols.

Zudem ist es erfindungsgemäß insbesondere vorgesehen, dass das Trägersystem und/oder das flüssige Medium und/oder die Zusammensetzung, insbesondere wie zuvor definiert, zumindest im Wesentlichen frei von halogenierten Kohlenwasserstoffen, insbesondere zumindest im Wesentlichen frei von chlorierten Kohlenwasserstoffen, vorzugsweise zumindest im Wesentlichen frei von Chloroform, ist bzw. sind. Insbesondere kann das Verfahren in zumindest im Wesentlichen vollständiger Abwesenheit von halogenierten Kohlenwasserstoffen, insbesondere in zumindest im Wesentlichen vollständiger Abwesenheit von chlorierten Kohlenwasserstoffen, vorzugsweise in zumindest im Wesentlichen vollständiger Abwesenheit von Chloroform, durchgeführt werden.

Im Hinblick auf die durch die spezielle Verfahrensführung hergestellten bzw. bereitgestellten Partikel des Trägersystems kann insbesondere auch Folgendes angeführt werden.

Insbesondere wird die Zusammenlagerung, insbesondere Aggregation, der Bestandteile bewirkt derart, dass das Trägersystem in partikulärer Form, insbesondere in Form von Lipidnanopartikeln (LNP), bevorzugt Lipidnanopartikeln (LNP) in Form von Mizellen, Vesikeln und/oder diskförmigen (scheibenförmigen) Lipidnanopartikeln (LNP) (Disk-Lipidnanopartikeln bzw. Disk-LNP), bevorzugt diskförmigen (scheibenförmigen) Lipidnanopartikeln (LNP), resultiert. Erfindungsgemäß verhält es sich insbesondere derart, dass das Trägersystem in partikulärer Form, insbesondere in Form von Lipidnanopartikeln (LNP), bevorzugt Lipidnanopartikeln (LNP) in Form von Mizellen, Vesikeln und/oder diskförmigen (scheibenförmigen) Lipidnanopartikeln (LNP) (Disk-Lipidnanopartikeln bzw. Disk-LNP), bevorzugt diskförmigen (scheibenförmigen) Lipidnanopartikeln (LNP), vorliegt. Erfindungsgemäß verhält es sich insbesondere derart, dass aufgrund der speziellen Verfahrensführung insbesondere und in bevorzugter Weise diskförmige (scheibenförmige) Lipidnanopartikel ausgebildet werden. Grundsätzlich können aber auch andere räumliche Strukturen bzw. Partikelformen vorliegen, beispielsweise in Form von Mizellen, insbesondere Kugelmizellen und/oder Wurmmizellen, Vesikeln oder dergleichen.

Die vorgenannten Ausbildungen bzw. Formgebung der Partikel ist sowohl im Hinblick auf die rheologischen Eigenschaften, die (Lager-)Stabilität der Zusammensetzung sowie der Ausrüstung der Partikel mit dem Wirkstoff und dessen Freisetzung am Wirkort von besonderem Vorteil, und zwar insbesondere was die Ausbildung der Partikel in Form von diskförmigen Lipidnanopartikeln (synonym auch als Disk-Lipidnanopartikel bezeichnet) anbelangt. Die Ausbildung der vorgenannten Partikel wird dabei insbesondere auch durch die Auswahl und Abstimmung der für die das Trägersystem eingesetzten Bestandteile bzw. Substanzen und deren verfahrensgemäße Verarbeitung gesteuert. Die in Rede stehenden diskförmigen Lipidnanopartikel weisen aufgrund ihrer speziellen Formgebung besonders gute Eigenschaften hinsichtlich der Aufnahme bzw. Integration von Wirkstoffen und deren zielgerichtete Abgabe auf und führen zudem zu besonders homogenen und stabilen Zusammensetzungen bzw. Dispersionen.

Grundsätzlich können die Partikel insbesondere als vorzugsweise homogene und/oder vorzugsweise zusammenhängende lamellare Struktur oder Form vorliegen oder ausgebildet werden. Demgegenüber kommt auch eine Ausbildung bzw. ein Vorliegen in hexagonaler oder kubischer Form in Betracht.

Insbesondere kann im Rahmen des erfindungsgemäßen Verfahrens in diesem Zusammenhang auch derart vorgegangen werden, dass im Rahmen der Herstellung des Trägersystems bzw. der diesbezüglichen Partikel die Ausbildung von insbesondere großen mehrlagigen Vesikeln (MLVs) zumindest im Wesentlichen vollständig vermieden oder aber zumindest reduziert wird.

Das erfindungsgemäße Verfahren bzw. das auf diese Weise erhältliche Trägersystem zeichnet sich zudem insbesondere dadurch aus, dass die Partikel des Trägersystems, bevorzugt Lipidnanopartikel (LNP), eine zumindest im Wesentlichen homogene Partikelgrößenverteilung, insbesondere mit geringer Dispersität und/oder mit kleiner Varianz, aufweisen. Insbesondere wird im Rahmen des erfindungsgemäßen Verfahrens gewährleistet, dass die Partikel des Trägersystems, bevorzugt Lipidnanopartikel (LNP), zumindest im Wesentlichen eine Normalverteilung und/oder Gaußverteilung aufweisen, insbesondere mit geringer Dispersität und/oder mit kleiner Varianz, aufweisen.

In diesem Zusammenhang können die diesbezüglichen Partikelgrößen, insbesondere die hydrodynamischen Durchmesser, mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09, bestimmt werden.

Insbesondere kann es sich erfindungsgemäß derart verhalten, dass die Partikelgrößen (absolut), insbesondere die hydrodynamischen Durchmesser (absolut) der Partikel des Trägersystems, bevorzugt Lipidnanopartikel, zumindest im Wesentlichen eine Normalverteilung und/oder Standardverteilung aufweisen. Hierdurch werden besonders definierte Eigenschaften der diesbezüglich erhaltenen Zusammensetzung erhalten bzw. gewährleistet.

Im Allgemeinen führt das erfindungsgemäße Verfahren zur Ausbildung von Partikeln mit kleinen Partikelgrößen bzw. Partikeldurchmessern, welche insbesondere im Nanometerbereich liegen.

Insbesondere können die Partikel des Trägersystems, bevorzugt die Lipidnanopartikel (LNP), eine Partikelgröße (absolut), insbesondere einen hydrodynamischen Durchmesser (absolut), im Bereich von 5 nm bis 10.000 nm, insbesondere im Bereich von 10 nm bis 5.000 nm, vorzugsweise im Bereich von 20 nm bis 3.000 nm, bevorzugt im Bereich von 50 nm bis 2.000 nm, besonders bevorzugt im Bereich von 75 nm bis 1.500 nm, ganz besonders bevorzugt im Bereich von 90 nm bis 1.250 nm, weiter bevorzugt im Bereich von 100 nm bis 1.000 nm, nochmals weiter bevorzugt im Bereich von 100 nm bis 900 nm, aufweisen, insbesondere bestimmt mittels Lichtbeugung (Lichdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09.

Beispielsweise können die Partikelgrößen mittels eines Horiba® LA-920 Particle Size Analyzer bestimmt werden.

Insbesondere können die Partikel des Trägersystems, bevorzugt die Lipidnanopartikel (LNP), eine mittlere Partikelgröße, insbesondere einen mittleren hydrodynamischen Durchmesser, im Bereich von 5 nm bis 2.900 nm, insbesondere im Bereich von 10 nm bis 2.500 nm, vorzugsweise im Bereich von 20 nm bis 2.000 nm, bevorzugt im Bereich von 50 nm bis 1.500 nm, besonders bevorzugt im Bereich von 50 nm bis 1.000 nm, aufweisen, insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09.

Wie zuvor angeführt, können die Partikel eine insgesamt geringe Varianz aufweisen: Diesbezüglich kann es sich erfindungsgemäß insbesondere derart verhalten, dass die Varianz der Partikelgröße, insbesondere die Varianz des hydrodynamischen Durchmessers, der Partikel des Trägersystems, bevorzugt der Lipidnanopartikel (LNP), höchstens 45 %, insbesondere höchstens 35 %, vorzugsweise höchstens 25 %, bevorzugt höchstens 20 %, besonders bevorzugt höchstens 15 %, ganz besonders bevorzugt höchstens 10 %, beträgt, insbesondere bezogen auf die mittlere Partikelgöße bzw. den mittleren hydrodynamischen Durchmesser und/oder insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09. Mit anderen Worten können die Partikel des Trägersystems, bevorzugt die Lipidnanopartikel, eine Varianz der Partikelgröße, insbesondere eine Varianz des hydrohynamischen Durchmessers, von höchstens 45 %, insbesondere höchstens 35 %, vorzugsweise höchstens 25 %, bevorzugt höchstens 20 %, besonders bevorzugt höchstens 15 %, ganz besonders bevorzugt höchstens 10 % aufweisen, insbesondere bezogen auf die mittlere Partikelgöße bzw. den mittleren hydrodynamischen Durchmesser und/oder und/oder insbesondere bestimmt mittels Lichtbeugung (Lichtdiffraktometrie), insbesondere wie zuvor angeführt.

Insbesondere können die Partikel des Trägersystems, bevorzugt die Lipidnanopartikel (LNP), eine mittlere Partikelgröße D50, insbesondere einen mittleren hydrodynamischen Durchmesser D50, im Bereich von 2,5 nm bis 950 nm, insbesondere im Bereich von 7,5 nm bis 850 nm, vorzugsweise im Bereich von 10 nm bis 800 nm, bevorzugt im Bereich von 15 nm bis 750 nm, aufweisen, insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09.

Zudem können die Partikel des Trägersystems, bevorzugt die Lipidnanopartikel (LNP), eine mittlere Partikelgröße D90, insbesondere einen mittleren hydrodynamischen Durchmesser D90, im Bereich von 1 nm bis 1.000 nm, insbesondere im Bereich von 5 nm bis 900 nm, vorzugsweise im Bereich von 7,5 nm bis 850 nm, bevorzugt im Bereich von 10 nm bis 800 nm, aufweisen, insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09.

Im Folgenden wird das erfindungsgemäße Verfahren weiterführend anhand von bevorzugten Ausführungsformen beschrieben, auf deren Basis es weiterführend möglich ist, die zuvor definierten Partikelformen und -größen zu erhalten bzw. einzustellen.

Im Rahmen der erfindungsgemäßen Verfahrensführung kann insbesondere die Zusammenlagerung, insbesondere Aggregation, der Bestandteile (insbesondere zur Ausbildung des Trägersystems, vorzugsweise in partikulärer Form) bewirkt werden derart, dass eine insbesondere lyotrope flüssig-kristalline Struktur auf Basis des Trägersystems und des flüssigen Mediums resultiert. Insbesondere kann das Trägersystem zusammen mit dem flüssigen Medium als lyotrope flüssig-kristalline Struktur auf Basis des Trägersystems und des flüssigen Mediums vorliegt und/oder diese Struktur aufweisen. Insbesondere kann im Rahmen der erfindungsgemäßen Verfahrensführung in dem flüssigen Medium unter Einbezug der Bestandteile des Trägersystems eine entsprechende insbesondere lyotrope flüssig-kristalline Struktur bzw. Phase erhalten werden. In diesem Zusammenhang kann im Hinblick auf das flüssige Medium mit den darin vorliegenden Bestandteilen insbesondere eine Gelphase vorliegen bzw. resultieren.

Im Rahmen der erfindungsgemäßen Verfahrensführung kann insbesondere die Zusammenlagerung, insbesondere Aggregation, der Bestandteile zur Ausbildung des Trägersystems unter Eintrag von Energie, bevorzugt Eintrag von Misch- und/oder Rührenergie, insbesondere in das zumindest einen Teil der Bestandteile und/oder Substanzen des Trägersystems aufweisende flüssige Medium und/oder in eine zumindest einen Teil der Bestandteile und/oder Substanzen des Trägersystems aufweisenden Mischung, durchgeführt werden.

Diesbezüglich kann die eingetragene Energie, insbesondere Misch- und/oder Rührenergie, eingestellt werden derart, dass eine zumindest im wesentlichen laminare Strömung, insbesondere in dem zumindest einen Teil der Bestandteile und/oder Substanzen des Trägersystems aufweisenden flüssigen Medium und/oder in der zumindest einen Teil der Bestandteile und/oder Substanzen des Trägersystems aufweisenden Mischung, vorliegt, insbesondere unter zumindest im Wesentlichen vollständiger Vermeidung der Ausbildung einer nichtlaminaren und/oder turbulenten Strömung und/oder insbesondere unter zumindest im Wesentlichen vollständiger Vermeidung der Ausbildung nichtlaminarer und/oder turbulenter Strömungsanteile.

Erfindungsgemäß kann in diesem Zusammenhang beispielsweise derart vorgegangen werden, dass die zur Ausbildung des Trägersystems eingesetzten Bestandteile bzw. Substanzen in ihrer Gesamtheit (beispielsweise gleichzeitig oder aber nacheinander) in das flüssige Medium eingebracht werden und ein diesbezüglicher Eintrag an Energie erfolgt. Wie nachfolgend noch im Detail angeführt, kann erfindungsgemäß aber auch derart vorgegangen werden, dass eine Aufteilung der für die Ausbildung des Trägersystems eingesetzten Bestandteile bzw. Substanzen insbesondere in verschiedenen Ansätzen erfolgt, wobei auch Mischungen von Bestandteilen ohne flüssiges Medium vorliegen können, in welchen der Eintrag an Energie erfolgen kann. Diesbezüglich können auch Ansätze zumindest eines Teils der Bestandteile bzw. Substanzen mit flüssigem Medium hergestellt werden, in welchem gleichermaßen ein Eintrag an Energie erfolgen kann. Die in Rede stehenden Ansätze können dann vereinigt werden, wobei diesbezüglich nochmals einen Eintrag an Energie und gegebenenfalls die Zugabe von weiterem flüssigen Medium zum Erhalt der Zusammensetzung erfolgen kann.

Erfindungsgemäß wird somit vorrangig auf die Einstellung laminarer Strömungsbedingungen abgestellt. In diesem Zusammenhang hat die Anmelderin in völlig überraschender Weise gefunden, dass das Vorliegen laminarer Strömungen in dem fluiden Medium mit den damit eingebrachten Bestandteilen des Trägersystems zur Ausbildung besonders definierter partikulärer Strukturen, insbesondere wie zuvor definiert, führt, beispielsweise was den Erhalt der in Rede stehenden diskförmigen Lipidnanopartikel anbelangt. Infolge des Vorliegens einer laminaren Strömung bzw. durch Mischung der Komponenten wird insbesondere auch der einzubringende Wirkstoff geschont, beispielsweise im Hinblick auf die Verringerung des Einflusses von Scherkräften oder dergleichen, was insbesondere für Wirkstoffe auf Basis von mRNA oder dergleichen von Bedeutung ist.

Im Allgemeinen sollte im Rahmen des erfindungsgemäßen Verfahrens die eingetragene Menge an Energie, insbesondere in den folgenden Bereichen bzw. unter den nachfolgend angeführten Gegebenheiten erfolgen. Einzelfallbedingt kann jedoch auch von den nachfolgend angeführten Werten im Rahmen der vorliegenden Erfindung abgewichen werden, ohne dass die vorliegende Erfindung hierdurch verlassen wird:

- Insbesondere kann die eingetragene Menge an Energie, insbesondere die eingetragene Menge an Misch- und/oder Rührenergie, im Bereich von 0,1 W/kg bis 10 W/kg, insbesondere im Bereich von 0,2 W/kg bis 5 W/kg, vorzugsweise im Bereich von 0,5 W/kg bis 3 W/kg, bevorzugt im Bereich von 1 W/kg bis 2 W/kg, bezogen auf die in Wechselwirkung zu bringenden Bestandteile, ggf. zusammen mit dem flüssigen Medium, liegen.

- Weiterhin kann die eingetragene Menge an Energie, insbesondere die eingetragene Menge an Misch- und/oder Rührenergie, höchstens 2.000 J/kg, insbesondere höchstens 1.000 J/kg, vorzugsweise höchstens 500 J/kg, bevorzugt höchstens 200 J/kg, bezogen auf die in Wechselwirkung zu bringenden Bestandteile, ggf. zusammen mit dem flüssigen Medium, betragen.

- Insbesondere kann die eingetragene Menge an Energie, insbesondere die eingetragene Menge an Misch- und/oder Rührenergie, eingestellt werden derart, dass eine Reynolds-Zahl $R_e$, insbesondere eine auf eine Rührerströmung bezogene Reynolds-Zahl $R_e$, von höchstens 2.000, insbesondere höchstens 1.500, vorzugsweise höchstens 1.000, bevorzugt höchstens 500, vorliegt.

- Insbesondere kann der Eintrag an Energie, insbesondere der Eintrag an Misch- und/oder Rührenergie, für eine Zeitdauer im Bereich von 0,1 s bis 600 s, insbesondere im Bereich von 1 s bis 500 s, vorzugsweise im Bereich von 2 s bis 400 s, bevorzugt im Bereich von 5 s bis 200 s, erfolgen.

- Erfindungsgemäß kann es zudem vorgesehen sein, dass der Eintrag an Energie, insbesondere der Eintrag an Misch- und/oder Rührenergie, mittels mindestens einer Rühreinrichtung (Rührvorrichtung) erfolgt, insbesondere wobei die Rühreinrichtung eine wandgängige Rühreinrichtung ist und/oder insbesondere wobei die Rühreinrichtung ein Wendelrührer oder ein Drahtrührer, vorzugsweise ein Drahtrührer, ist und/oder insbesondere wobei die Rühreinrichtung Bestandteil einer Aufnahmevorrichtung (Verarbeitungsvorrichtung), insbesondere Behälter und/oder Reaktor, vorzugsweise Rührkammer und/oder Rührkessel, ist.

- Insbesondere kann die Partikelgröße, insbesondere die mittlere Partikelgröße, und/oder die Varianz der Partikelgröße der Partikel, insbesondere Lipidnanopartikel (LNP), des Trägersystems über die Zeitdauer des Eintrags an Energie, insbesondere über die Zeitdauer des Eintrags an Misch- und/oder Rührenergie, und/oder über die Menge an eingetragener Energie, insbesondere die Menge an eingetragener Misch- und/oder Rührenergie, gesteuert bzw. eingestellt werden, insbesondere wobei größere Zeitdauern und/oder größere Energiemengen zu kleineren Partikelgrößen und/oder kleineren Varianzen führen.

Erfindungsgemäß ist es von Vorteil, wenn die erfindungsgemäß eingesetzten Behälter bzw. Reaktoren insbesondere rohr- bzw. kesselförmig ausgebildet sind. Hierdurch können insbesondere Toträume vermieden werden. Beispielsweise können sogenannte Ekato-Paravisc®-Rühreinrichtungen eingesetzt werden. Die laminare Strömung kann beispielsweise über die Drehzahl der Rühreinrichtung bzw. der Viskositätswerte des Fluids gesteuert bzw. kontrolliert werden.

Im Hinblick auf das Einbringen von insbesondere Misch- bzw. Rührenergie hat die Anmelderin in völlig überraschender Weise gefunden, dass sowohl der Median der Partikelgröße als auch die Varianz von der Menge an eingetragener Energie und von der Rührdauer im Hinblick auf die gebildeten bzw. zu bildenden zusammenhängenden flüssigkristallinen Strukturen abhängt bzw. hierdurch gesteuert werden kann. Insbesondere verhält es sich derart, dass der Median der Partikelgröße mit zunehmender Rührdauer insbesondere exponentiell abnimmt. Gleichzeitig nimmt die Varianz insbesondere im logarithmischen Maß ebenfalls ab. Insbesondere bestimmt auch die Rührdauer die Partikelgröße bzw. die zugrundeliegende Varianz. Je länger unter Berücksichtigung einer laminaren Strömung gerührt wird, desto kleiner werden die Partikel, insbesondere bei gleichzeitig abnehmender Varianz. Hierdurch können die Teilchengrößen bzw. die diesbezüglichen Zusammensetzungen gezielt eingestellt werden.

Im Hinblick auf die Menge an eingetragener Energie kommt auch, wie zuvor angeführt, der sogenannten Reynolds-Zahl eine große Bedeutung zu. Die Reynolds-Zahl ist eine charakteristische Größe für die Menge an eingetragener Energie in das zugrundeliegende Gemisch bzw. System.

Die Reynolds-Zahl kann insbesondere gemäß der nachfolgenden Formel beschrieben werden:

$$Re = \frac{\rho \cdot N \cdot D^2}{\eta}$$

$\eta$ = dynamische Viskosität [Pa·s]; p = Dichte des Fluids [kg/m³]; N = Drehzahl des Rührers [1/s]; D = Durchmesser des Rührers [m])

Erfindungsgemäß kann es insbesondere vorgesehen sein, dass der Eintrag an Energie nicht über Ultraschall erfolgt. Hierdurch wird ein ungleichmäßiger und schwer kontrollierbarer Energieeintrag vermieden, so dass erfindungsgemäß auch von daher eine besonders schonende Herstellung gewährleistet wird.

Erfindungsgemäß kann das Verfahren, insbesondere die Herstellung des Trägersystems bzw. des mit dem pharmazeutischen Wirkstoff beladenen und/oder ausgerüsteten Trägersystems, und/oder die Zusammenlagerung der Bestandteile zur Ausbildung des Trägersystems bei einer Temperatur von höchstens 100 °C, insbesondere höchstens 95 °C, vorzugsweise höchstens 90 °C, durchgeführt werden bzw. erfolgen.

Insbesondere kann das Verfahren, insbesondere die Herstellung des Trägersystems bzw. des mit dem pharmazeutischen Wirkstoff beladenen und/oder ausgerüsteten Trägersystems, und/oder die Zusammenlagerung der Bestandteile zur Ausbildung des Trägersystems bei einer Temperatur im Bereich von 10 °C bis 100 °C, insbesondere im Bereich von 15 °C bis 95 °C, vorzugsweise im Bereich von 20 °C bis 90 °C, durchgeführt werden bzw erfolgen.

Erfindungsgemäß ist es von Vorteil, wenn das Verfahren, insbesondere die Herstellung des Trägersystems bzw. des mit dem pharmazeutischen Wirkstoff beladenen und/oder ausgerüsteten Trägersystems, und/oder die Zusammenlagerung der Bestandteile zur Ausbildung des Trägersystems bei einer Temperatur unterhalb der Phasenübergangstemperatur des (i) Phospholipids durchgeführt wird und/oder erfolgt. Insbesondere kann die Verfahrenstemperatur, insbesondere die Temperatur zur Herstellung des Trägersystems bzw. des mit dem pharmazeutischen Wirkstoff beladenen und/oder ausgerüsteten Trägersystems, und/oder die Temperatur zur Zusammenlagerung der Bestandteile zur Ausbildung des Trägersystems in Abhängigkeit von dem eingesetzten (i) Phospholipid gewählt und/oder eingestellt werden, vorzugsweise auf einen Temperaturwert unterhalb der Phasenübergangstemperatur des (i) Phospholipids. Hierdurch werden besonders stabile und homogen ausgebildete Trägersysteme erhalten werden.

Erfindungsgemäß kann das Verfahren, insbesondere die Herstellung des Trägersystems bzw. des mit dem pharmazeutischen Wirkstoff beladenen und/oder ausgerüsteten Trägersystems, und/oder die Zusammenlagerung der Bestandteile zur Ausbildung des Trägersystems kontinuierlich, semikontinuierlich oder batchweise, insbesondere kontinuierlich, vorzugsweise kontinuierlich-kaskadiert, durchgeführt werden.

Insbesondere kann das Verfahren, insbesondere die Herstellung des Trägersystems bzw. des mit dem pharmazeutischen Wirkstoff beladenen und/oder ausgerüsteten Trägersystems, und/oder die Zusammenlagerung der Bestandteile zur Ausbildung des Trägersystems in mindestens einer Aufnahmevorrichtung, insbesondere Behälter und/oder Reaktor, vorzugsweise Rührkammer und/oder Rührkessel, durchgeführt werden. In Der Aufnahmevorrichtung keine eine entsprechende Verarbeitung stattfinden.

Diesbezüglich kann, wie vorliegend angeführt, beispielsweise auf Basis eines gemeinsames Ansatzes der Bestandteile bzw. dem flüssigen Medium oder aber ausgehend von verschiedenen Ansätzen, beispielsweise unter Aufteilung der Bestandteile und gegebenenfalls in Kombination mit flüssigem Medium in einem jeweiligen Ansatz, verfahren werden, insbesondere gefolgt von einer Vereinigung bzw. einem Zusammenführen der jeweiligen (Ausgangs-) Ansätze.

Erfindungsgemäß kann insbesondere derart vorgegangen werden, dass die in Kontakt zu bringenden und/oder in Wechselwirkung zu bringenden Bestandteile des Trägersystems zunächst auf verschiedene Ansätze, insbesondere mindestens zwei Ansätze, aufgeteilt werden und/oder in verschiedenen Ansätzen, insbesondere mindestens zwei Ansätzen, vorgelegt werden, gegebenenfalls jeweils in Kombination mit vorzugsweise einen Alkohol, insbesondere Polyol, enthaltendem flüssigem Medium und/oder gegebenenfalls gefolgt von der Zugabe von vorzugsweise einen Alkohol, insbesondere Polyol, enthaltendem flüssigem Medium. Wie vorliegend dargelegt, handelt es sich bei dem Alkohol nicht um einen $C_1$- bis $C_4$-Monoalkohol und nicht um Ethanol.

Die erfindungsgemäße Aufteilung der für die Herstellung des Trägersystems eingesetzten Bestandteile bzw. Substanzen ermöglicht nämlich gerade auch die Vermeidung des Einsatzes der vorgenannten Alkohole, und dies einhergehend mit den vorgenannten Vorteilen.

Für die jeweiligen Ansätze kann, unabhängig voneinander, ein Eintrag an Energie, insbesondere ein Eintrag an Misch- und/oder Rührenergie, insbesondere wie zuvor definiert, vorgenommen werden. Zudem können die jeweiligen Ansätze, unabhängig voneinander, auf eine Temperatur, wie zuvor definiert, gebracht werden. Zudem kann ein jeweiliger Ansatz in einer Aufnahmevorrichtung, insbesondere Behälter und/oder Reaktor, vorzugsweise Rührkammer und/oder Rührkessel, vorliegen bzw. bearbeitet werden.

In diesem Zusammenhang können die verschiedenen Ansätze, insbesondere der erste Ansatz und der zweite Ansatz, anschließend zur Ausbildung des Trägersystems zusammengeführt und/oder vereinigt werden, gegebenenfalls jeweils in Kombination mit vorzugsweise einen Alkohol, insbesondere Polyol, enthaltendem flüssigem Medium und/oder gegebenenfalls gefolgt von der Zugabe von vorzugsweise einen Alkohol, insbesondere Polyol, enthaltendem flüssigem Medium (wobei das flüssige Medium, wie zuvor angeführt, dergestalt ist, dass dieses kein Ethanol aufweist).

In diesem Zusammenhang kann es erfindungsgemäß vorgesehen sein, dass zur Ausbildung des Trägersystems mindestens einer der Ansätze, insbesondere der erste oder der zweite Ansatz, dem mindestens einen anderen Ansatz zugeführt und/oder zugegeben wird und/oder hiermit vereinigt und/oder zusammengeführt werden, insbesondere so dass ein gemeinsamer (zusammengeführter) Ansatz erhalten wird, insbesondere wobei für den gemeinsamen Ansatz ein Eintrag an Energie, insbesondere ein Eintrag an Misch- und/oder Rührenergie, insbesondere wie zuvor definiert, vorgenommen wird; und/oder insbesondere wobei der gemeinsame Ansatz auf eine Temperatur, wie zuvor definiert, gebracht wird; und/oder insbesondere wobei der gemeinsame Ansatz in einer Aufnahmevorrichtung, insbesondere Behälter und/oder Reaktor, vorzugsweise Rührkammer und/oder Rührkessel, vorliegt und/oder bearbeitet wird.

Im Rahmen der vorliegenden Erfindung hat sich auch folgendes Vorgehen als besonders vorteilhaft erwiesen. So kann es erfindungsgemäß vorgesehen sein, dass die hydrophoben Substanzen und gegebenenfalls die amphiphilen Substanzen, gegebenenfalls in Kombination mit vorzugsweise einen Alkohol, insbesondere Polyol, enthaltendem flüssigem Medium, insbesondere wie zuvor definiert, in einem ersten Ansatz vorgelegt werden und/oder dass ein erster Ansatz bereitgestellt wird, welcher die hydrophoben Substanzen und gegebenenfalls die amphiphilen Substanzen, gegebenenfalls in Kombination mit vorzugsweise einen Alkohol, insbesondere Polyol, enthaltendem flüssigem Medium, insbesondere wie zuvor definiert, enthält, und

dass die hydrophilen Substanzen und gegebenenfalls die amphiphilen Substanzen, insbesondere in Kombination mit vorzugsweise einen Alkohol, insbesondere Polyol, enthaltendem flüssigem Medium, insbesondere wie zuvor definiert, in einem zweiten Ansatz vorgelegt werden und/oder wobei ein zweiter Ansatz bereitgestellt wird, welcher die hydrophilen Substanzen und gegebenenfalls die amphiphilen Substanzen, insbesondere in Kombination mit vorzugsweise ein Polyol enthaltendem flüssigem Medium, insbesondere wie zuvor definiert, enthält, und

gegebenenfalls dass der Wirkstoff bzw. die Substanz (vi), insbesondere in Kombination mit vorzugsweise einen Alkohol, insbesondere Polyol, enthaltendem flüssigem Medium, insbesondere wie zuvor definiert, in dem ersten Ansatz und/oder oder in dem zweiten Ansatz und/oder in einem dritten Ansatz, vorzugsweise in einem dritten Ansatz, eingebracht wird und/oder vorgelegt wird und/oder gegebenenfalls dass ein dritter Ansatz bereitgestellt wird, welcher den Wirkstoff bzw. die Substanz (vi), insbesondere in Kombination mit vorzugsweise einen Alkohol, insbesondere Polyol, enthaltendem flüssigem Medium, insbesondere wie zuvor definiert, enthält;

insbesondere wobei zur Ausbildung des Trägersystems der erste Ansatz und der zweite Ansatz sowie gegebenenfalls der dritte Ansatz anschließend zur Ausbildung des Trägersystems zusammengeführt und/oder vereinigt werden.

In diesem Zusammenhang kann insbesondere derart verfahren werden, dass die hydrophilen Substanzen einen HLB-Wert nach Griffin von mindestens 6, vorzugsweise mindestens 7, bevorzugt mindestens 8, aufweisen und/oder dass die hydrophoben Substanzen einen HLB-Wert nach Griffin von weniger als 6, vorzugsweise höchstens 5,5, bevorzugt höchstens 5, aufweisen bzw. dass die hydrophilen Substanzen einen Log P-Wert von höchstens 1,11, vorzugsweise höchstens 0,93, bevorzugt höchstens 0,80, aufweisen und/oder dass die hydrophoben Substanzen einen Log P-Wert von mehr als 1,11, vorzugsweise mindestens 1,22, bevorzugt mindestens 1,36, aufweisen.

Das Einbringen bzw. Vorlegen des Wirkstoffs bzw. der Substanz (vi) in Bezug auf die vorgenannten Ansätze kann in diesem Zusammenhang insbesondere in Abhängigkeit der zugrundeliegenden hydrophoben bzw. hydrophilen Eigenschaften erfolgen.

Gemäß einer erfindungsgemäßen speziellen Ausführungsform kann es insbesondere derart vorgesehen sein, dass die Substanz (i), gegebenenfalls gemeinsam mit mindestens einer von Substanz (ii) verschiedenen Substanz, insbesondere in Kombination mit vorzugsweise einen Alkohol, insbesondere Polyol, enthaltendem flüssigem Medium, insbesondere wie zuvor definiert, in einem ersten Ansatz vorgelegt wird und/oder dass ein erster Ansatz bereitgestellt wird, welcher die Substanz (i), gegebenenfalls gemeinsam mit mindestens einer von Substanz (ii) verschiedenen Substanz, insbesondere in Kombination mit vorzugsweise einen Alkohol, insbesondere Polyol, enthaltendem flüssigem Medium, insbesondere wie zuvor definiert, enthält, und

dass die Substanz (ii), gegebenenfalls gemeinsam mit mindestens einer von Substanz (i) verschiedenen Substanz, insbesondere in Kombination mit vorzugsweise einen Alkohol, insbesondere Polyol, enthaltendem flüssigem Medium, insbesondere wie zuvor definiert, in einem zweiten Ansatz vorgelegt werden dass wobei ein zweiter Ansatz bereitgestellt wird, welcher die Substanz (ii), gegebenenfalls gemeinsam mit mindestens einer von Substanz (i) verschiedenen Substanz, insbesondere in Kombination mit vorzugsweise einen Alkohol, insbesondere Polyol, enthaltendem flüssigem Medium, insbesondere wie zuvor definiert, enthält, und

gegebenenfalls wobei der Wirkstoff bzw. die Substanz (vi), insbesondere in Kombination mit vorzugsweise einen Alkohol, insbesondere Polyol, enthaltendem flüssigem Medium, insbesondere wie zuvor definiert, in dem ersten Ansatz und/oder oder in dem zweiten Ansatz und/oder in einem dritten Ansatz, vorzugsweise in einem dritten Ansatz, eingebracht wird und/oder vorgelegt wird und/oder gegebenenfalls dass ein dritter Ansatz bereitgestellt wird, welcher den Wirkstoff bzw. die Substanz (vi), insbesondere in Kombination mit vorzugsweise einen Alkohol, insbesondere Polyol, enthaltendem flüssigem Medium, insbesondere wie zuvor definiert, enthält,

insbesondere wobei zur Ausbildung des Trägersystems der erste Ansatz und der zweite Ansatz sowie gegebenenfalls der dritte Ansatz anschließend zur Ausbildung des Trägersystems zusammengeführt und/oder vereinigt werden.

Gemäß einer weiteren speziellen Ausführungsform der vorliegenden Erfindung kann es darüber hinaus auch vorgesehen sein, dass die Substanz (i) gemeinsam mit Substanz (ii), insbesondere mit zumindest einem Teil der Substanz (ii), gegebenenfalls gemeinsam mit mindestens einer von Substanz (ii) verschiedenen Substanz, insbesondere in Kombination mit vorzugsweise einen Alkohol, insbesondere Polyol, enthaltendem flüssigem Medium, insbesondere wie zuvor definiert, in einem ersten Ansatz vorgelegt wird und/oder dass ein erster Ansatz bereitgestellt wird, welcher die Substanz (i), gegebenenfalls gemeinsam mit mindestens einer von Substanz (ii) verschiedenen Substanz, insbesondere in Kombination mit vorzugsweise einen Alkohol, insbesondere Polyol, enthaltendem flüssigem Medium, insbesondere wie zuvor definiert, enthält, und

dass die Substanz (ii), gegebenenfalls gemeinsam mit mindestens einer von Substanz (i) verschiedenen Substanz, insbesondere in Kombination mit vorzugsweise einen Alkohol, insbesondere Polyol, enthaltendem flüssigem Medium, insbesondere wie zuvor definiert, in einem zweiten Ansatz vorgelegt wird und/oder wobei ein zweiter Ansatz bereitgestellt wird, welcher die Substanz (ii), gegebenenfalls gemeinsam mit mindestens einer von Substanz (i) verschiedenen Substanz, insbesondere in Kombination mit vorzugsweise einen Alkohol, insbesondere Polyol, enthaltendem flüssigem Medium, insbesondere wie zuvor definiert, enthält, und

gegebenenfalls dass der Wirkstoff bzw. die Substanz (vi), insbesondere in Kombination mit vorzugsweise einen Alkohol, insbesondere Polyol, enthaltendem flüssigem Medium, insbesondere wie zuvor definiert, in dem ersten Ansatz und/oder oder in dem zweiten Ansatz und/oder in einem dritten Ansatz, vorzugsweise in einem dritten Ansatz, eingebracht wird und/oder vorgelegt wird und/oder gegebenenfalls dass ein dritter Ansatz bereitgestellt wird, welcher den Wirkstoff bzw. die Substanz (vi), insbesondere in Kombination mit vorzugsweise einen Alkohol, insbesondere Polyol, enthaltendem flüssigem Medium, insbesondere wie zuvor definiert, enthält,

insbesondere wobei zur Ausbildung des Trägersystems der erste Ansatz und der zweite Ansatz sowie gegebenenfalls der dritte Ansatz anschließend zur Ausbildung des Trägersystems zusammengeführt und/oder vereinigt werden.

Gemäß einer noch weiteren speziellen Ausführungsform der vorliegenden Erfindung kann es darüber hinaus auch vorgesehen sein, dass die Substanz (i), insbesondere in Kombination mit vorzugsweise einen Alkohol, insbesondere Polyol, enthaltendem flüssigem Medium, insbesondere wie zuvor definiert, in einem ersten Ansatz vorgelegt wird und/oder dass ein erster Ansatz bereitgestellt wird, welcher die Substanz (i), insbesondere in Kombination mit vorzugsweise einen Alkohol, insbesondere Polyol, enthaltendem flüssigem Medium, insbesondere wie zuvor definiert, enthält, insbesondere wobei der erste Ansatz auf eine Temperatur im Bereich von 10 °C bis 30 °C, insbesondere im Bereich von 15 °C bis 25 °C gebracht wird, und

dass die Substanzen (ii), (iii) und (vii), gegebenenfalls in Kombination mit vorzugsweise einen Alkohol, insbesondere Polyol, enthaltendem flüssigem Medium, insbesondere wie zuvor definiert, in einem zweiten Ansatz vorgelegt werden und/oder dass ein zweiter Ansatz bereitgestellt wird, welcher die Substanzen (ii), (iii) und (vii), gegebenenfalls in Kombination mit vorzugsweise einen Alkohol, insbesondere Polyol, enthaltendem flüssigem Medium, insbesondere wie zuvor definiert, enthält, insbesondere wobei der zweite Ansatz auf eine Temperatur im Bereich von 60 °C bis 90 °C, insbesondere im Bereich von 70 °C bis 80 °C, gebracht wird, und

gegebenenfalls dass der Wirkstoff bzw. die Substanz (vi), insbesondere in Kombination mit vorzugsweise einen Alkohol, insbesondere Polyol, enthaltendem flüssigem Medium, insbesondere wie zuvor definiert, in dem ersten Ansatz und/oder oder in dem zweiten Ansatz und/oder in einem dritten Ansatz, vorzugsweise in einem dritten Ansatz, eingebracht wird und/oder vorgelegt wird und/oder gegebenenfalls dass ein dritter Ansatz bereitgestellt wird, welcher den Wirkstoff bzw. die Substanz (vi), insbesondere in Kombination mit vorzugsweise einen Alkohol, insbesondere Polyol, enthaltendem flüssigem Medium, insbesondere wie zuvor definiert, enthält;

insbesondere wobei der erste Ansatz und der zweite Ansatz, insbesondere nach Abkühlen des zweiten Ansatzes vorzugsweise auf eine Temperatur unterhalb von 70 °C, insbesondere im Bereich von 40 °C bis 55 °C, sowie gegebenenfalls der dritte Ansatz anschließend zur Ausbildung des Trägersystems zusammengeführt und/oder vereinigt werden, insbesondere wobei der zweite Ansatz und gegebenenfalls der dritte Ansatz dem ersten Ansatz zugegeben werden.

Das Einbringen bzw. Vorlegen des Wirkstoffs bzw. der Substanz (vi) in Bezug auf die vorgenannten Ansätze kann somit unter Berücksichtigung der Thermostabilität des Wirkstoffs erfolgen. Insbesondere kann die Zugabe bzw. das Einbringen in dem ersten Ansatz bzw. einem dritten Ansatz erfolgen, insbesondere sofern es sich um einen Wirkstoff mit geringerer Wärmestabilität handelt. Grundsätzlich kann die Zugabe bzw. das Einbringen auch in dem zweiten Ansatz erfolgen, insbesondere sofern es sich um einen Wirkstoff mit höherer Wärmestabilität handelt.

Gemäß einer nochmals weiteren speziellen Ausführungsform ist es erfindungsgemäß auch möglich, dass die Substanz (ii), insbesondere in Kombination mit vorzugsweise ein Polyol enthaltendem flüssigem Medium, insbesondere wie zuvor definiert, in einem ersten Ansatz vorgelegt wird und/oder dass ein erster Ansatz bereitgestellt wird, welcher die Substanz (ii), insbesondere in Kombination mit vorzugsweise einen Alkohol, insbesondere Polyol, enthaltendem flüssigem Medium, insbesondere wie zuvor definiert, enthält, insbesondere wobei der erste Ansatz auf eine Temperatur im Bereich von 15 °C bis 60 °C, insbesondere im Bereich von 20 °C bis 50 °C gebracht wird, und

dass die Substanzen (i) und gegebenenfalls (iii) und/oder (vii), vorzugsweise die Substanzen (i), (iii) und (vii), gegebenenfalls in Kombination mit vorzugsweise einen Alkohol, insbesondere Polyol, enthaltendem flüssigem Medium, insbesondere wie zuvor definiert, in einem zweiten Ansatz vorgelegt werden und/oder wobei ein zweiter Ansatz bereitgestellt wird, welcher die Substanzen (i) und gegebenenfalls (iii) und/oder (vii), vorzugsweise die Substanzen (i), (iii) und (vii), gegebenenfalls in Kombination mit vorzugsweise einen Alkohol, insbesondere Polyol, enthaltendem flüssigem Medium, insbesondere wie zuvor definiert, enthält, insbesondere wobei der zweite Ansatz auf eine Temperatur im Bereich von 60 °C bis 90 °C, insbesondere im Bereich von 70 °C bis 80 °C, gebracht wird, und

gegebenenfalls dass der Wirkstoff bzw. die Substanz (vi), insbesondere in Kombination mit vorzugsweise einen Alkohol, insbesondere Polyol, enthaltendem flüssigem Medium, insbesondere wie zuvor definiert, in dem ersten Ansatz und/oder oder in dem zweiten Ansatz und/oder in einem dritten Ansatz, vorzugsweise in einem dritten Ansatz, eingebracht wird und/oder vorgelegt wird und/oder gegebenenfalls dass ein dritter Ansatz bereitgestellt wird, welcher den Wirkstoff bzw. die Substanz (vi), insbesondere in Kombination mit vorzugsweise einen Alkohol, insbesondere Polyol, enthaltendem flüssigem Medium, insbesondere wie zuvor definiert, enthält;

insbesondere wobei der erste Ansatz und der zweite Ansatz, insbesondere nach Abkühlen des zweiten Ansatzes vorzugsweise auf eine Temperatur unterhalb von 70 °C, insbesondere im Bereich von 40 °C bis 55 °C, sowie gegebenenfalls der dritte Ansatz anschließend zur Ausbildung des Trägersystems zusammengeführt und/oder vereinigt werden, insbesondere wobei der zweite Ansatz und gegebenenfalls der dritte Ansatz dem ersten Ansatz zugegeben werden.

Im Rahmen der vorliegenden Erfindung ist es völlig überraschend, dass der Einsatz von $C_1$- bis $C_4$-Monoalkoholen bzw. Ethanol sowie Chloroform als Lösemittel für die angeführten Bestandteile vermieden werden kann. Erfindungsgemäß kann zudem im Allgemeinen der abschließend erhaltene gemeinsame Ansatz mit weiterem flüssigen Medium, welches insbesondere einen Alkohol, vorzugsweise einen Polyol, enthält, verdünnt bzw. weiterverarbeitet werden.

Auf dieser Basis kann somit eine endfertige Zusammensetzung erhalten werden, welche das in Partikeln vorliegende Trägersystem enthält. Im Rahmen des erfindungsgemäßen Verfahrens kann auch eine effektive Inkorporation bzw. Anlagerung des Wirkstoffs in bzw. an das Trägersystem erfolgen.

Erfindungsgemäß kann weiterhin eine Aufteilung der eingesetzten Bestandteile auf Basis bzw. in Abhängigkeit ihrer jeweiligen HLB-Werte nach Griffin erfolgen, wobei hierbei in verschiedenen Ansätzen mit einer Zusammenfassung der jeweiligen Bestandteile verfahren werden kann, welche anschließend vereinigt werden, was zur Ausbildung definierter Zusammensetzungen mit definierten Partikeln des Trägersystems führt.

Was die Aufteilung der erfindungsgemäß eingesetzten Bestandteile im Hinblick auf deren HLB-Werten nach Griffin bzw. deren Log P-Wert mit der diesbezüglichen Verteilung auf verschiedene Ansätze anbelangt, so kann gemäß einer weiteren speziellen Ausführungsform der vorliegenden Erfindung insbesondere wie folgt vorgegangen werden: So kann es erfindungsgemäß insbesondere dergestalt sein,

dass die Substanzen mit einem HLB-Wert nach Griffin von mindestens 6, vorzugsweise mindestens 7, bevorzugt mindestens 8, bzw. mit einem Log P-Wert von höchstens 1,11, vorzugsweise höchstens 0,93, bevorzugt höchstens 0,80, insbesondere in Kombination mit vorzugsweise einen Alkohol, insbesondere Polyol, enthaltendem flüssigem Medium, insbesondere wie zuvor definiert, in einem ersten Ansatz vorgelegt werden und/oder dass ein erster Ansatz bereitgestellt wird, welcher die Substanzen mit einem HLB-Wert nach Griffin von mindestens 6, vorzugsweise mindestens 7, bevorzugt mindestens 8, bzw. mit einem Log P-Wert von höchstens 1,11, vorzugsweise höchstens 0,93, bevorzugt höchstens 0,80, insbesondere in Kombination mit vorzugsweise einen Alkohol, insbesondere Polyol, enthaltendem flüssigem Medium, insbesondere wie zuvor definiert, enthält, insbesondere wobei der erste Ansatz auf eine Temperatur im Bereich von 15 °C bis 60 °C, insbesondere im Bereich von 20 °C bis 50 °C gebracht wird, und

dass die Substanzen mit einem HLB-Wert nach Griffin von weniger als 6, vorzugsweise höchstens 5,5, bevorzugt höchstens 5, und/oder mit einem Log P-Wert von mehr als 1,11, vorzugsweise mindestens 1,22, bevorzugt mindestens 1,36, gegebenenfalls in Kombination mit vorzugsweise einen Alkohol, insbesondere Polyol, enthaltendem flüssigem Medium, insbesondere wie zuvor definiert, in einem zweiten Ansatz vorgelegt werden und/oder wobei ein zweiter Ansatz bereitgestellt wird, welcher die Substanzen mit einem HLB-Wert nach Griffin von weniger als 6, vorzugsweise höchstens 5,5, bevorzugt höchstens 5, und/oder mit einem Log P-Wert von mehr als 1,11, vorzugsweise mindestens 1,22, bevorzugt mindestens 1,36, gegebenenfalls in Kombination mit vorzugsweise einen Alkohol, insbesondere Polyol, enthaltendem flüssigem Medium, insbesondere wie zuvor definiert, enthält, insbesondere wobei der zweite Ansatz auf eine Temperatur im Bereich von 60 °C bis 90 °C, insbesondere im Bereich von 70 °C bis 80 °C, gebracht wird, und

gegebenenfalls dass der Wirkstoff bzw. die Substanz (vi), insbesondere in Kombination mit vorzugsweise einen Alkohol, insbesondere Polyol, enthaltendem flüssigem Medium, insbesondere wie zuvor definiert, in dem ersten Ansatz und/oder oder in dem zweiten Ansatz und/oder in einem dritten Ansatz, vorzugsweise in einem dritten Ansatz, eingebracht wird und/oder vorgelegt wird und/oder gegebenenfalls dass ein dritter Ansatz bereitgestellt wird, welcher den Wirkstoff bzw. die Substanz (vi), insbesondere in Kombination mit vorzugsweise einen Alkohol, insbesondere Polyol, enthaltendem flüssigem Medium, insbesondere wie zuvor definiert, enthält;

insbesondere wobei der erste Ansatz und der zweite Ansatz, insbesondere nach Abkühlen des zweiten Ansatzes vorzugsweise auf eine Temperatur unterhalb von 70 °C, insbesondere im Bereich von 40 °C bis 55 °C, sowie gegebenenfalls der dritte Ansatz anschließend zur Ausbildung des Trägersystems zusammengeführt und/oder vereinigt werden, insbesondere wobei der zweite Ansatz und gegebenenfalls der dritte Ansatz dem ersten Ansatz zugegeben werden.

Gemäß einer nochmals weiteren speziellen Ausführungsform kann zudem auch derart vorgegangen werden, dass die Substanzen (i), (ii) und gegebenenfalls (x), vorzugsweise die Substanzen (i), (ii) und (x), insbesondere in Kombination mit vorzugsweise einen Alkohol, insbesondere Polyol, enthaltendem flüssigem Medium, insbesondere wie zuvor definiert, in einem ersten Ansatz vorgelegt werden und/oder dass ein erster Ansatz bereitgestellt wird, welcher die Substanzen (i), (ii) und gegebenenfalls (x), vorzugsweise die Substanzen (i), (ii) und (x), insbesondere in Kombination mit vorzugsweise einen Alkohol, insbesondere Polyol, enthaltendem flüssigem Medium, insbesondere wie zuvor definiert, enthält, insbesondere wobei der erste Ansatz auf eine Temperatur im Bereich von 30 °C bis 90 °C, insbesondere im Bereich von 50 °C bis 80 °C gebracht wird, und

dass die Substanzen (iii) und/oder (v), vorzugsweise die Substanzen (iii) und (v), gegebenenfalls in Kombination mit vorzugsweise einen Alkohol, insbesondere Polyol, enthaltendem flüssigem Medium, insbesondere wie zuvor definiert, in einem zweiten Ansatz vorgelegt werden und/oder wobei ein zweiter Ansatz bereitgestellt wird, welcher die Substanzen (iii) und/oder (v), vorzugsweise die Substanzen (iii) und (v), gegebenenfalls in Kombination mit vorzugsweise einen Alkohol, insbesondere Polyol, enthaltendem flüssigem Medium, insbesondere wie zuvor definiert, enthält, insbesondere wobei der zweite Ansatz auf eine Temperatur im Bereich von 40 °C bis 100 °C, insbesondere im Bereich von 60 °C bis 90 °C, gebracht wird, und

gegebenenfalls dass der Wirkstoff bzw. die Substanz (vi), insbesondere in Kombination mit vorzugsweise einen Alkohol, insbesondere Polyol, enthaltendem flüssigem Medium, insbesondere wie zuvor definiert, in dem ersten Ansatz und/oder in dem zweiten Ansatz und/oder in einem dritten Ansatz, vorzugsweise in einem dritten Ansatz, eingebracht wird und/oder vorgelegt wird und/oder gegebenenfalls dass ein dritter Ansatz bereitgestellt wird, welcher den Wirkstoff bzw. die Substanz (vi), insbesondere in Kombination mit vorzugsweise ein Polyol enthaltendem flüssigem Medium, insbesondere wie zuvor definiert, enthält;

insbesondere wobei der erste Ansatz und der zweite Ansatz anschließend zur Ausbildung des Trägersystems zusammengeführt und/oder vereinigt werden, insbesondere wobei der zweite Ansatz dem ersten Ansatz zugegeben wird, insbesondere gefolgt von der Zugabe von vorzugsweise einen Alkohol, insbesondere Polyol, enthaltendem flüssigem Medium und/oder des dritten Ansatzes.

Gemäß einer weiteren erfindungsgemäß bevorzugten Ausführungsform, insbesondere im Hinblick auf das zuvor angeführte DMI gemäß Substanz (ix), kann erfindungsgemäß auch derart vorgegangen werden, dass die die Substanzen (i), (ii) und (ix) und gegebenenfalls (iii) und gegebenenfalls (v), vorzugsweise die Substanzen (i), (ii), (iii), (v) und (ix), insbesondere in Kombination mit vorzugsweise einen Alkohol, insbesondere Polyol, enthaltendem flüssigem Medium, insbesondere wie zuvor definiert, in einem ersten Ansatz vorgelegt werden und/oder dass ein erster Ansatz bereitgestellt wird, welcher die Substanzen (i), (ii) und (ix) und gegebenenfalls (iii) und gegebenenfalls (v), vorzugsweise die Substanzen (i), (ii), (iii), (v) und (ix), insbesondere in Kombination mit vorzugsweise einen Alkohol, insbesondere Polyol, enthaltendem flüssigem Medium, insbesondere wie zuvor definiert, enthält, insbesondere wobei der erste Ansatz auf eine Temperatur im Bereich von 30 °C bis 80 °C, insbesondere im Bereich von 45 °C bis 65 °C gebracht wird, und

dass ein zweiter Ansatz vorzugsweise einen Alkohol, insbesondere Polyol, enthaltendes flüssiges Medium, insbesondere wie zuvor definiert, enthält oder hieraus gebildet wird, insbesondere wobei der zweite Ansatz auf eine Temperatur im Bereich von 10 °C bis 30 °C, insbesondere im Bereich von 15 °C bis 25 °C, gebracht wird, und gegebenenfalls dass der Wirkstoff bzw. die Substanz (vi), insbesondere in Kombination mit vorzugsweise einen Alkohol, insbesondere Polyol, enthaltendem flüssigem Medium, insbesondere wie zuvor definiert, in dem ersten Ansatz und/oder oder in dem zweiten Ansatz und/oder in einem dritten Ansatz, vorzugsweise in einem dritten Ansatz, eingebracht wird und/oder vorgelegt wird und/oder gegebenenfalls dass ein dritter Ansatz bereitgestellt wird, welcher den Wirkstoff bzw. die Substanz (vi), insbesondere in Kombination mit vorzugsweise ein Polyol enthaltendem flüssigem Medium, insbesondere wie zuvor definiert, enthält;

insbesondere wobei der erste Ansatz und der zweite Ansatz anschließend zur Ausbildung des Trägersystems zusammengeführt und/oder vereinigt werden, insbesondere wobei der zweite Ansatz dem ersten Ansatz zugegeben wird, insbesondere gefolgt von der Zugabe vorzugsweise einen Alkohol, insbesondere Polyol, enthaltendem flüssigem Medium und/oder des dritten Ansatzes.

Gemäß einer weiteren erfindungsgemäßen Ausführungsform kann darüber hinaus auch derart vorgegangen werden, dass die Substanzen (i) und (ii) und gegebenenfalls (iii) und gegebenenfalls (v) und gegebenenfalls (vi) (d.h. der Wirkstoff), vorzugsweise die Substanzen (i), (ii), (iii), (v) und (vi), insbesondere in Kombination mit vorzugsweise einen Alkohol, insbesondere Polyol, enthaltendem flüssigem Medium, insbesondere wie zuvor definiert, in einem ersten Ansatz vorgelegt werden und/oder dass ein erster Ansatz bereitgestellt wird, welcher die Substanzen (i) und (ii) und gegebenenfalls (iii) und gegebenenfalls (v) und gegebenenfalls (vi), vorzugsweise die Substanzen (i), (ii), (iii), (v) und (vi), insbesondere in Kombination mit vorzugsweise einen Alkohol, insbesondere Polyol, enthaltendem flüssigem Medium, insbesondere wie zuvor definiert, enthält, insbesondere wobei der erste Ansatz auf eine Temperatur im Bereich von 30 °C bis 80 °C, insbesondere im Bereich von 45 °C bis 65 °C gebracht wird, und

dass ein zweiter Ansatz vorzugsweise einen Alkohol, insbesondere Polyol, enthaltendes flüssiges Medium, insbesondere wie zuvor definiert, enthält oder hieraus gebildet wird, insbesondere wobei der zweite Ansatz auf eine Temperatur im Bereich von 10 °C bis 30 °C, insbesondere im Bereich von 15 °C bis 25 °C, gebracht wird, und

gegebenenfalls dass der Wirkstoff bzw. die Substanz (vi), insbesondere in Kombination mit vorzugsweise einen Alkohol, insbesondere Polyol, enthaltendem flüssigem Medium, insbesondere wie zuvor definiert, (zudem) in dem ersten Ansatz und/oder oder in dem zweiten Ansatz und/oder in einem dritten Ansatz, vorzugsweise in einem dritten Ansatz, eingebracht wird und/oder vorgelegt wird und/oder gegebenenfalls dass zudem ein dritter Ansatz bereitgestellt wird, welcher den Wirkstoff bzw. die Substanz (vi), insbesondere in Kombination mit vorzugsweise ein Polyol enthaltendem flüssigem Medium, insbesondere wie zuvor definiert, enthält;

insbesondere wobei der erste Ansatz und der zweite Ansatz anschließend zur Ausbildung des Trägersystems zusammengeführt und/oder vereinigt werden, insbesondere wobei der zweite Ansatz dem ersten Ansatz zugegeben wird, insbesondere gefolgt von der Zugabe vorzugsweise einen Alkohol, insbesondere Polyol, enthaltendem flüssigem Medium und/oder des dritten Ansatzes.

Im Hinblick auf die zuvor angeführten Ansätze können weitere Ansätze vorgelegt werden: Insbesondere kann es erfindungsgemäß auch vorgesehen sein, dass der Wirkstoff und/oder die Substanz (iv), gegebenenfalls in Kombination mit Substanz (ii) und/oder (iii) und/oder insbesondere in Kombination mit vorzugsweise einen Alkohol, insbesondere Polyol, enthaltendem flüssigem Medium in einem vierten Ansatz vorgelegt wird und/oder dass zudem ein vierter Ansatz bereitgestellt wird, welcher die Substanz (iv), gegebenenfalls in Kombination mit Substanz (ii) und/oder (iii) und/oder insbesondere in Kombination mit vorzugsweise einen Alkohol, insbesondere Polyol, enthaltendem flüssigem Medium, enthält, insbesondere wobei der vierte Ansatz mindestens einem der weiteren Ansätze und/oder den zuvor vereinigten weiteren Ansätzen und/oder der Zusammensetzung zugegeben wird.

Zudem kann es erfindungsgemäß auch vorgesehen sein, dass die Substanz (iv), gegebenenfalls in Kombination mit Substanz (ii) und/oder (iii) und/oder insbesondere in Kombination mit vorzugsweise einen Alkohol, insbesondere Polyol, enthaltendem flüssigem Medium, insbesondere wie zuvor definiert, in einem fünften Ansatz vorgelegt wird und/oder dass zudem ein fünfter Ansatz bereitgestellt wird, welcher die Substanz (iv), gegebenenfalls in Kombination mit Substanz (ii) und/oder (iii) und/oder insbesondere in Kombination mit vorzugsweise einen Alkohol, insbesondere Polyol, enthaltendem flüssigem Medium, enthält, insbesondere wobei der fünfte Ansatz mindestens einem der weiteren Ansätze und/oder den zuvor vereinigten weiteren Ansätzen und/oder der Zusammensetzung zugegeben wird.

Erfindungsgemäß hat es sich vorteilhaft erwiesen, wenn die Ansätze aufeinander abfolgend und/oder kaskadenförmig, insbesondere in Form einer kontinuierlichkaskadenförmigen Verfahrensführung, vereinigt und/oder zusammengeführt werden, vorzugsweise unter Verwendung jeweiliger Aufnahmevorrichtungen, insbesondere Behälter und/oder Reaktoren, vorzugsweise Rührkammern und/oder Rührkessel, insbesondere wobei die Aufnahmevorrichtung aufeinander abfolgend und/oder kaskadenförmig angeordnet werden.

Erfindungsgemäß kann es aber auch vorgesehen sein, dass sämtliche in Kontakt zu bringende und/oder in Wechselwirkung zu bringende Bestandteile des Trägersystems gemeinsam, insbesondere unmittelbar und/oder direkt (d.h. insbesondere ohne vorangehende Aufteilung in verschiedene Ansätze), insbesondere gemeinsam mit vorzugsweise einen Alkohol, insbesondere Polyol, enthaltendem flüssigem Medium und/oder zur Ausbildung des Trägersystems zusammengeführt und/oder vereinigt werden, insbesondere gefolgt von der (weiteren) Zugabe von vorzugsweise einen Alkohol, insbesondere Polyol, enthaltendem flüssigem Medium, bevorzugt in einem gemeinsamen Ansatz, vorzugsweise in einer gemeinsamen Aufnahmevorrichtung, insbesondere Behälter und/oder Reaktor, vorzugsweise Rührkammer und/oder Rührkessel, oder aber

dass zunächst ein vorzugsweise einen Alkohol, insbesondere Polyol, enthaltendes flüssiges Medium, ggf. zusammen mit wenigstens einem Teil mindestens eines Bestandteils des Trägersystems vorgelegt wird, gefolgt von der Zugabe mindestens eines weiteren Ansatzes, welcher übrige, vorzugsweise sämtliche übrige, in Kontakt zu bringende und/oder in Wechselwirkung zu bringende Bestandteile des Trägersystems gemeinsam, insbesondere gemeinsam mit vorzugsweise einen Alkohol, insbesondere Polyol, enthaltendem flüssigem Medium enthält oder hieraus besteht, insbesondere unter Zusammenführung und/oder Vereinigung aller Ansätze zur Ausbildung des Trägersystems, insbesondere gefolgt von der (weiteren) Zugabe von vorzugsweise einen Alkohol, insbesondere Polyol, enthaltendem flüssigem Medium, bevorzugt in einem gemeinsamen Ansatz, vorzugsweise in einer gemeinsamen Aufnahmevorrichtung, insbesondere Behälter und/oder Reaktor, vorzugsweise Rührkammer und/oder Rührkessel.

Hierzu kann es vorgesehen sein, dass für den gemeinsamen Ansatz, ein Eintrag an Energie, insbesondere ein Eintrag an Misch- und/oder Rührenergie, insbesondere wie in zuvor definiert, vorgenommen wird; und/oder dass, vorzugsweise für den gemeinsamen Ansatz, eine Temperatur, wie zuvor definiert, eingestellt wird.

Ebenfalls kann es insbesondere vorgesehen sein, dass die Bestandteile des Trägersystems vor und/oder bei Zusammenführen und/oder Vereinigung aufgeschmolzen und/oder in einen flüssigen Zustand überführt werden und/oder dass das flüssige Medium und/oder der das flüssige Medium enthaltende Ansatz auf eine Temperatur von höchstens 40 °C eingestellt und/oder gebracht wird.

Im Hinblick auf die vorgenannten Verfahrensführungen verhält es sich insbesondere derart, dass das vorzugsweise einen Alkohol, insbesondere Polyol, enthaltende flüssige Medium, insbesondere unabhängig für die jeweiligen Ansätze, ein wie zuvor definiertes Medium ist; bzw. dass der Alkohol, insbesondere Polyol, insbesondere unabhängig für die jeweiligen Ansätze, ein wie zuvor definierter Alkohol, insbesondere Polyol, ist.

Diesbezüglich verhält es sich erfindungsgemäß derart, dass der Alkohol kein Ethanol ist bzw. wobei der Alkohol kein Ethanol umfasst.

Insbesondere können für die jeweiligen Ansätze insbesondere in Abstimmung mit den diesbezüglich vorliegenden Bestandteilen jeweils voneinander verschiedene flüssige Medien bzw. Alkohole, insbesondere Polyole, insbesondere gemäß den zuvor genannten Ausführungen eingesetzt werden.

Erfindungsgemäß kann zudem derart vorgegangen werden, dass das verfahrensgemäß erhaltene Trägersystem bzw. das verfahrensgemäß erhaltene und mit dem pharmazeutischen Wirkstoff beladene und/oder ausgerüstete Trägersystem bzw. die verfahrensgemäß erhaltene Zusammensetzung, welche das Trägersystem bzw. das mit dem pharmazeutischen Wirkstoff beladene und/oder ausgerüstete Trägersystem und das flüssige Medium umfasst, abgekühlt wird, insbesondere durch Zugabe von insbesondere auf eine Temperatur unterhalb der Temperatur des erhaltenen Trägersystems und/oder der erhaltenen Zusammensetzung eingestelltem und/oder gekühltem flüssigem (weiterem) Medium, insbesondere wie zuvor definiert.

Diesbezüglich kann das zuzugebende flüssige Medium eine Temperatur aufweisen, welche mindestens 5 °C, insbesondere mindestens 10 °C, vorzugsweise mindestens 15 °C, unterhalb der Temperatur des erhaltenen Trägersystems bzw. des erhaltenen und mit dem pharmazeutischen Wirkstoff beladene und/oder ausgerüstete Trägersystems bzw. der erhaltenen Zusammensetzung liegt.

Insbesondere kann das zuzugebende flüssige Medium eine Temperatur im Bereich von -20 °C bis 40 °C, insbesondere im Bereich von -10 °C bis 30 °C, vorzugsweise im Bereich von 0 °C bis 25 °C, bevorzugt im Bereich von -5 °C bis 20 °C, aufweisen.

Erfindungsgemäß kann es in diesem Zusammenhang insbesondere vorgesehen sein, dass das (weitere) flüssige Medium, welches auf eine Temperatur unterhalb der Temperatur des erhaltenen Trägersystems und/oder der erhaltenen Zusammensetzung eingestellt ist und/oder gekühlt ist, zumindest im Wesentlichen unmittelbar und/oder zeitnah (z.B. höchstens 60 s, insbesondere höchstens 30 s, vorzugsweise höchstens 10 s) nach Erhalt des Trägersystems und/oder der Zusammensetzung zugegeben wird.

Das insbesondere auf eine definierte Temperatur eingestellte bzw. gekühlte flüssige Mediums kann somit dem Trägersystem bzw. der Zusammensetzung zugegeben werden. Durch die damit einhergehende Abkühlung wird das Trägersystem bzw. der diesbezügliche Wirkstoff bzw. die Zusammensetzung weiterführend stabilisiert. Durch den Kühleffekt wird eine weiterführende Thermostabilisierung des Trägersystems bzw. des Wirkstoffs bzw. der Zusammensetzung gewährleistet.

Erfindungsgemäß wird insbesondere derart vorgegangen, dass die kontinuierliche Verfahrens- und/oder Prozesskontrolle (Produktionskontrolle), insbesondere *online*- und/oder *inline*-Prozesskontrolle, wie zuvor beschrieben, zur Überwachung des Verfahrensstatus und/oder des Verfahrensfortschritts, vorzugsweise zur Überwachung und/oder Einstellung der Ausbildung der Partikel des Trägersystems, insbesondere Lipidnanopartikel (LNP), und/oder insbesondere zur Überwachung und/oder Einstellung von Verfahrensparametern, ausgewählt aus Eintrag von Energie, bevorzugt Eintrag von Misch- und/oder Rührenergie, Eintrag und/oder Abfuhr von Wärmenergie, Strömungsverhalten, Massendurchsatz, Volumenströmen oder dergleichen, durchgeführt wird, e wobei die Verfahrens- und/oder Prozesskontrolle über die Erfassung von mindestens einem Prozessparameter, insbesondere mindestens zwei Prozessparametern erfolgt, insbesondere wobei der Prozessparameter ausgewählt wird aus der Gruppe von Temperatur, Leitfähigkeit, Viskosität und pH-Wert, bevorzugt Leitfähigkeit jeweils des flüssigen Mediums mitsamt den darin eingebrachten Bestandteilen und/oder Substanzen, bzw. der Zusammensetzung und/oder der jeweiligen Ansätze, insbesondere wie zuvor definiert, und deren Kombinationen.

Erfindungsgemäß kann die kontinuierliche Verfahrens- und/oder Prozesskontrolle (Produktionskontrolle) über die Erfassung der Leitfähigkeit des flüssigen Mediums mitsamt den darin eingebrachten Bestandteilen und/oder Substanzen, bzw. der Zusammensetzung und/oder der jeweiligen Ansätze, insbesondere wie vorliegend definiert, erfolgen, gegebenenfalls in Kombination mit mindestens einem weiteren Prozessparameter, insbesondere wie vorliegend definiert. Insbesondere kann als der mindestens eine Prozessparameter die Leitfähigkeit, insbesondere die Leitfähigkeit des flüssigen Mediums, vorzugsweise mitsamt den darin eingebrachten Bestandteilen und/oder Substanzen, bzw. die Leitfähigkeit der Zusammensetzung und/oder die Leitfähigkeit der jeweiligen Ansätze, insbesondere wie zuvor definiert, ausgewählt werden, gegebenenfalls wobei die Verfahrens- und/oder Prozesskontrolle über die zusätzliche Erfassung mindestens eines weiteren Prozessparameters, insbesondere wie zuvor definiert, erfolgt. Erfindungsgemäß stellt somit die zuvor angeführte Leitfähigkeit den bevorzugten Prozessparameter dar.

Im Hinblick auf die zu erfassenden Prozessparameter können beispielsweise diesbezügliche (relative) Änderungen, beispielsweise (relative) Leitfähigkeitsänderungen zur Regelung bzw. Adjustierung von Verfahrensparametern, wie Energieeintrag oder dergleichen, herangezogen werden. Auf dieser Basis wird eine effektive Prozesssteuerung bzw. Verfahrensführung ermöglicht, welche auch automatisiert ablaufen kann

Im Rahmen der Steuerung bzw. Kontrolle der erfindungsgemäßen Verfahrensführung ist es insbesondere möglich, die Produktion der zugrundeliegenden Partikel computergesteuert durchzuführen. Was die Leitfähigkeit anbelangt, so kann diese insbesondere als Maß im Hinblick auf eine Korrelation zur Partikelkonzentration bzw. -bildung herangezogen werden. Gleichermaßen liegt eine Korrelation beispielsweise zur Elektrolytkonzentration vor. Auf diese Weise kann auch beispielsweise eine Verdünnung eingestellt werden. Die Viskosität als Charakteristikum insbesondere von Gelphasen kann gleichermaßen im Hinblick auf die Ausbildung der Partikel herangezogen werden. Die Viskosität kann beispielsweise direkt über das Drehmoment des Rührers bestimmt werden, was der Funktionsweise eines Brookfield-Viskosimeters ähnlich ist. Insbesondere kann das zur Aufrechterhaltung der Bewegung erforderliche Drehmoment gemessen werden, woraus sich dann die Spannung und damit die Viskosität ableiten lässt. Insbesondere kann die Viskosität mittels Brookfield bzw. als Brookfield-Viskosität bestimmt werden. Im Übrigen kann im Falle von viskoelastischem Vehalten dieses auch durch den Verlustfaktor tan $\delta$ charakterisiert werden. In diesem Zusammenhang ist ein viskoelastisches Verhalten insbesondere durch tan $\delta$ < 1 charakterisiert. Im Allgemeinen nimmt das viskoelastische Verhalten mit steigender Temperatur ab. Bei Phasenübergangstemperatur wird ein Wert tan $\delta$ > 1 eingenommen.

Die Viskosität kann insbesondere beispielsweise gemäß der EN ISO 3219, insbesondere ISO 3219-1:2021 bzw. EN ISO 3219-1:2021, gemessen werden. Diesbezüglich kann auch ein Viskosimeter herangezogen werden. Zudem kann die Partikelgrößenverteilung ermittelt werden. Beispielsweise können Methoden auf Basis von Lichtstreuung bzw. Laserdiffraktometrie eingesetzt werden.

Das erfindungsgemäße Verfahren kann insbesondere automatisiert, vorzugsweise zumindest im Wesentlichen vollautomatisiert, zumindest aber teilautomatisiert, bzw. computergesteuert durchgeführt werden. Im Rahmen des erfindungsgemäßen Verfahrens kann insgesamt eine Zusammensetzung auf Basis von in einem flüssigen Medium vorliegenden Partikeln des Trägersystems mit diesbezüglicher Ausrüstung bzw. Beladung auf Basis eines Wirkstoffs bereitgestellt werden, welche insgesamt definierte Eigenschaften aufweist.

Insbesondere kann nach Ausbildung des Trägersystems eine vorzugweise disperse Zusammensetzung, insbesondere eine Dispersion, vorzugsweise wie zuvor definiert, welche das Trägersystem bzw. das mit dem pharmazeutischen Wirkstoff beladene und/oder ausgerüstete Trägersystem und das flüssige Medium umfassen, resultieren und/oder insbesondere wobei nach Ausbildung des Trägersystems eine Zusammensetzung, insbesondere wie zuvor definiert, in Form einer Dispersion des Trägersystems bzw. des mit einem pharmazeutischen Wirkstoff beladenen und/oder ausgerüsteten Trägersystems in dem flüssigen Medium resultiert.

Die verfahrensgemäß erhältliche Zusammensetzung kann das Trägersystem, vorzugsweise die Partikel, bevorzugt die Lipidnanopartikel, in einer Menge im Bereich von 5 Gew.-% bis 95 Gew.-%, insbesondere im Bereich von 10 Gew.-% bis 90 Gew.-%, vorzugsweise im Bereich von 15 Gew.-% bis 85 Gew.-%, bevorzugt im Bereich von 20 Gew.-% bis 80 Gew.-%, besonders bevorzugt im Bereich von 25 Gew.-% bis 75 Gew.-%, bezogen auf die Zusammensetzung, aufweisen.

Insbesondere kann das Trägersystem in der Zusammensetzung in partikulärer Form, insbesondere in Form von Lipidnanopartikeln (LNP), bevorzugt Lipidnanopartikeln (LNP) in Form von Mizellen, Vesikeln und/oder diskförmigen (scheibenförmigen) Lipidnanopartikeln (LNP) (Disk-Lipidnanopartikeln bzw. Disk-LNP), bevorzugt diskförmigen (scheibenförmigen) Lipidnanopartikeln (LNP), insbesondere wie zuvor definiert, vorliegen.

Die auf Basis des erfindungsgemäßen Verfahrens hergestellten Zusammensetzung zeichnet sich insbesondere durch eine geringe Wasseraktivität auf. Insbesondere kann die Zusammensetzung eine Wasseraktivität von weniger als 1, insbesondere höchstens 0,9, vorzugsweise höchstens 0,7, bevorzugt höchstens 0,5, besonders bevorzugt höchstens 0,3, aufweisen.

Im Allgemeinen kann die Zusammensetzung eine Wasseraktivität im Bereich von 0 bis 1, insbesondere im Bereich von 0 bis 0,9, vorzugsweise im Bereich von 0 bis 0,7, bevorzugt im Bereich von 0 bis 0,5, besonders bevorzugt im Bereich von 0 bis 0,3, aufweisen.

Die Wasseraktivität kann insbesondere gemäß ISO 21807:2004 und/oder gemäß ISO 18787:2017 und/oder 21 CFR Part 11, insbesondere wie in der ISO 29621:2017 bestimmt werden.

Die Wasseraktivität (auch $a_w$-Wert oder Activity of Water bezeichnet) einer diesbezüglichen Zusammensetzung ist ein Maß für das sozusagen verfügbare bzw. aktive Wasser, welches sich zur bloßen Angabe des Wassergehalts unterscheiden kann. Die Wasseraktivität beeinflusst u.a. das Wachstum von Mikroorganismen, den Ablauf chemischer Prozesse, wie Fettoxidation bzw. Oxidationsprozesse im Allgemeinen und auch die physikochemischen Eigenschaften der Zusammensetzungen. Die Wasseraktivität ist insbesondere definiert als Verhältnis des Wasserdampfpartialdrucks (p) einer Zusammensetzung zum Sättigungsdampfdruck von reinem Wasser ($p_0$) und kann insbesondere anhand der Formel $a_w = p / p_0$ ermittelt werden (beispielsweise bei Raumtemperatur bzw. 20 °C). Bei den erfindungsgemäßen Zusammensetzungen kann es sich insbesondere derart verhalten - ohne sich auf diese Theorie beschränken oder berufen zu wollen - dass Wasser teilweise in gebundener Form vorliegt, was zu einer verringerten Wasseraktivität führt. In Ergänzung zu den obigen Ausführungen kann die Wasseraktivität auch gemäß der Norm ISO 29621 bestimmt werden, insbesondere im Hinblick auf einen sogenannten Konservierungsbelastungstest.

Die erfindungsgemäßen Zusammensetzungen weisen somit insgesamt relativ niedrige Wasseraktivitäten auf. Auch hieraus resultiert eine hohe Lagerstabilität bzw. ein hoher Oxidationsschutz in Bezug auf die zugrundeliegenden Bestandteile bzw. Inhaltsstoffe.

Mit der erfindungsgemäßen Vorgehensweise ist es insbesondere auch möglich, in gezielter Weise Trägersysteme bzw. Zusammensetzungen mit definierten bzw. maßgeschneiderten Log P-Werten bereitzustellen. Hierdurch kann z. B. die Stabilität entsprechender Zusammensetzung bzw. die Bioverfügbarkeit des Trägersystems bzw. des Wirkstoffs erhöht werden.

So kann das Trägersystem und/oder die Zusammensetzung, insbesondere das Trägersystem, einen Log P-Wert, insbesondere Gesamt-Log P-Wert, von höchstens 5, insbesondere höchstens 4, bevorzugt höchstens 3, besonders bevorzugt höchstens 2, weiter bevorzugt höchstens 1, aufweisen. In diesem Zusammenhang kann das Trägersystem und/oder die Zusammensetzung, insbesondere das Trägersystem, einen Log P-Wert, insbesondere Gesamt-Log P-Wert, im Bereich von 0,1 bis 5, insbesondere im Bereich von 0,2 bis 4, bevorzugt im Bereich von 0,3 bis 3, besonders bevorzugt im Bereich von 0,4 bis 2, aufweisen.

Insbesondere können die Bestandteile und/oder Substanzen des Trägersystems, insbesondere die Substanzen (i), (ii), (iii), (iv) und/oder (v), mit der Maßgabe ausgewählt werden, dass das resultierende Trägersystem einen Log P-Wert, insbesondere Gesamt-Log P-Wert, von höchstens 5, insbesondere höchstens 4, bevorzugt höchstens 3, besonders bevorzugt höchstens 2, weiter bevorzugt höchstens 1, aufweist. Zudem können die Bestandteile und/oder Substanzen des Trägersystems, insbesondere die Substanzen (i), (ii), (iii), (iv) und/oder (v), mit der Maßgabe ausgewählt werden, dass das resultierende Trägersystem einen Log P-Wert, insbesondere Gesamt-Log P-Wert, im Bereich von 0,1 bis 5, insbesondere im Bereich von 0,2 bis 4, bevorzugt im Bereich von 0,3 bis 3, besonders bevorzugt im Bereich von 0,4 bis 2, aufweist.

Insgesamt führen die vorgenannten Gesamt-Log P-Werte für das Trägersystem auch zu einer optimierten Ein- bzw. Anlagerung von Wirkstoffen, wie beispielsweise von mRNA. Die erfindungsgemäß bereitgestellten Trägersysteme, insbesondere in Form von Lipidnanopartikeln und insbesondere diskförmigen Lipidnanopartikeln, zeichnen sich dabei insbesondere auch dadurch aus, dass eine hohe Bioverfügbarkeit des Wirkstoffs bzw. der Partikel als solche auch von daher vorliegt, dass in großen (Oberflächen-)Bereichen des Trägersystems bzw. der Partikel relativ niedrige Log P-Werte vorliegen und in diesem Zusammenhang auch die sogenannte Fünferregel nach Lipinski erfüllt ist. Dabei kann gewährleistet werden, dass zudem auch für die gesamte (Ober-)Fläche der erfindungsgemäß bereitgestellten Trägersysteme bzw. diskförmigen Lipidnanopartikel ein Gesamt-Log P-Wert vorliegt, welcher die Fünferregel gemäß Lipinski erfüllt.

Mit abnehmender Partikelgröße der Lipidnanopartikel kann es sich zudem insbesondere derart verhalten, dass ein größerer Kontakt zwischen Zelle und Lipidnanopartikel ausgebildet wird, wobei die durchschnittliche Kontaktfläche pro Zelle zunimmt. Auch dies führt zu einer hohen Wirkstoffabgabe bzw. -freisetzung. Hierdurch wird insgesamt auch eine hohe Bioverfügbarkeit ermöglicht. In diesem Zusammenhang liegt auch eine optimale Hydrophilie bzw. Lipophilie in Bezug auf das Trägersystem nach der Erfindung vor. Hierzu ist auch beachtlich, dass Wirkstoffe mit einer ingesamt zu hohen Hydrophilie bzw. mit einer zu hohen Lipophilie eine im Allgemeinen unzureichende bzw. nicht optimale Bioverfügbarkeit aufweisen, beispielsweise da keine optimalen Permeabilitätseigenschaften oder dergleichen vorliegen. In diesem Zusammenhang kann auf die sogenannte Fünferregel gemäß Lipinski verwiesen werden, welche anführt, dass der Log P-Wert für eine Drogenähnlichkeit bzw. Wirkstoffe im Allgemeinen den Wert von fünf nicht überschreiten sollte. Zudem liegt auf Basis der definierten Gesamt-Log P-Werte eine stabile Zusammensetzung mit den darin befindlichen erfindungsgemäßen Trägersystemen vor. Insbesondere liegt auch eine hohe Kompatibilität zu dem zugrundeliegenden Medium vor, und zwar auch, was die zusätzliche Verwendung von Polyol oder dergleichen in dem Medium anbelangt, wie zuvor angeführt. Auf Basis der speziellen HLD- bzw. (Gesamt-)Log P-Werte liegt auch eine optimale Kompatibilität der eingesetzten Komponenten vor, so dass auch von daher optimierte Trägersysteme resultieren. Somit können insgesamt auch auf dieser Basis insgesamt maßgeschneiderte Trägersysteme mit hoher Kompatibilität zu dem zugrundeliegenden Medium bereitgestellt werden.

Erfindungsgemäß kann es sich derart verhalten, dass sich die HLB-Werte und/oder die Log P-Werte auf eine Temperatur, insbesondere Verfahrenstemperatur, vorzugsweise Mischtemperatur, unterhalb der Phaseninversionstemperatur der eingesetzten Substanz (ii) und/oder unterhalb der Phaseninversionstemperatur der auf der eingesetzten Substanz (ii) basierenden Zusammensetzung beziehen bzw. dass das Verfahren unterhalb der Phaseninversionstemperatur der eingesetzten Substanz (ii) und/oder unterhalb der Phaseninversionstemperatur der auf der eingesetzten Substanz (ii) basierenden Zusammensetzung durchgeführt wird. Insbesondere kann dabei die Verfahrens- und/oder Prozesskontrolle, insbesondere Überwachung, über die Erfassung der Leitfähigkeit und/der Oberflächenspannung, insbesondere dynamischen Oberflächenspannung, insbesondere jeweils des flüssigen Mediums, vorzugsweise mitsamt den darin eingebrachten Bestandteilen und/oder Substanzen, und/oder der Zusammensetzung, erfolgen. Auch hierdurch können definierte Trägersysteme bzw. Zusammensetzungen bereitgestellt werden.

Die Kontrolle bzw. Verhinderung einer Phaseninversion kann beispielsweise über die Erfassung von Prozessparametern erfolgen, beispielsweise durch die Erfassung bzw. Messung der Leitfähigkeit des zugrundeliegenden Mediums, welches insbesondere die Bestandteile bzw. Substanzen zur Ausbildung des Trägersystems bzw. das Trägersystem als solches umfasst. Diesbezüglich kann auch die Oberflächenspannung, insbesondere dynamische Oberflächenspannung, des zugrundeliegenden Mediums bzw. Systems als Prozessparameter herangezogen werden.

Erfindungsgemäß wird das Verfahren nach der Erfindung im Allgemeinen unterhalb des Trübungspunkts und/oder unterhalb des Entmischungspunkts des zugrundeliegenden Systems und/oder des flüssigen Mediums, vorzugsweise mitsamt den darin eingebrachten Bestandteilen und/oder Substanzen, und/oder der Zusammensetzung durchgeführt. Auf dieser Basis können insgesamt optimierte und einheitliche Trägersysteme erhalten werden.

Gemäß dem vorliegenden Aspekt betrifft die vorliegende Erfindung auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten, lipidbasierten Trägersystems in partikulärer Form, bevorzugt in Form von Lipidnanopartikeln (LNP), insbesondere Verfahren wie vorstehend definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin;
wobei das Phosholipid einen HLB-Wert nach Griffin von höchstens 11, insbesondere höchstens 8, aufweist und/oder wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern;
wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D);
wobei das Steroid und/oder Secosteroid, insbesondere das Sterol, einen HLB-Wert nach Griffin von höchstens 1,5 aufweist und/oder wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins;
wobei das kationische Lipid einen HLB-Wert nach Griffin von höchstens 4 aufweist und/oder insbesondere wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;
wobei der Mono-, Di- oder Tricarbonsäureester von Glycerin einen HLB-Wert nach Griffin von höchstens 8 aufweist und/oder insbesondere wobei der Mono-, Di- oder Tricarbonsäureester von Glycerin einen Log P-Wert von mindestens 0,80 aufweist;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

insbesondere wobei die Substanzen zunächst zumindest teilweise auf unterschiedliche Ansätze (Chargen) aufgeteilt werden bzw. in unterschiedlichen Ansätzen (Chargen) vorgelegt werden und anschließend zur Ausbildung des Trägersystems zusammengeführt und/oder vereinigt werden, vorzugsweise wobei die Aufteilung der Substanzen auf die unterschiedlichen Ansätze und/oder das Vorlegen der Substanzen in unterschiedlichen Ansätzen in Abhängigkeit von ihrer jeweiligen Hydrophilie und/oder Lipophilie und/oder in Abhängigkeit von ihren HLB-Werten nach Griffin erfolgt und/oder in Abhängigkeit von ihren LogP -Werten erfolgt, insbesondere wobei die Aufteilung derart erfolgt, dass einander entsprechende Substanzen, insbesondere Substanzen mit gleicher oder gleichartiger Hydrophilie und/oder Lipophilie bzw. Substanzen mit gleichen oder gleichartigen HLB-Werten nach Griffin und/oder mit gleichen oder gleichartigen LogP -Werten, jeweils in demselben Ansatz vorgelegt werden und/oder insbesondere wobei gleichartige Substanzen, insbesondere Substanzen mit gleicher oder gleichartiger Hydrophilie und/oder Lipophilie bzw. Substanzen mit gleichen oder gleichartigen HLB-Werten nach Griffin und/oder mit gleichen oder gleichartigen LogP -Werten jeweils in demselben Ansatz vorgelegt werden.

Gemäß dem vorliegenden Aspekt betrifft die vorliegende Erfindung auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten, lipidbasierten Trägersystems in partikulärer Form, bevorzugt in Form von Lipidnanopartikeln (LNP), insbesondere Verfahren wie vorstehend definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten (polyoxyethylierten) Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern, insbesondere Polyolestern von PEGylierten Fettsäuren;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D);

(iv) ggf. mindestens ein kationisches Lipid, insbesondere aminbasiertes kationisches Lipid, vorzugsweise ein kationisches Lipid auf Basis eines tertiären Amins;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;

wobei das Verfahren durch mindestens eine(s) der nachfolgenden Merkmale und/oder Maßnahmen (1a) bis (5a), insbesondere durch eine Kombination von mindestens zwei, vorzugsweise mindestens drei, bevorzugt mindestens vier, besonders bevorzugt sämtlichen fünf, der nachfolgenden Merkmale und/oder Maßnahmen (1a) bis (5a), gekennzeichnet ist:

(1a) die Substanz (i) weist einen HLB-Wert nach Griffin von höchstens 11, insbesondere höchstens 8, insbesondere im Bereich von 0,5 bis 8, vorzugsweise im Bereich von 2 bis 7, bevorzugt im Bereich von 3 bis 6, auf und/oder die Substanz (i) weist eine einen Log P-Wert von mindestens 0,55, insbesondere mindestens 0,80, insbesondere im Bereich von 0,80 bis 18,97, vorzugsweise im Bereich von 0,93 bis 3,88, bevorzugt im Bereich von 1,11 bis 2,44, auf;

(2a) die Substanz (ii) weist einen HLB-Wert nach Griffin von mindestens 13, insbesondere im Bereich von 13 bis 19, vorzugsweise im Bereich von 13 bis 17,9, bevorzugt im Bereich von 13,5 bis 17,5, besonders bevorzugt im Bereich von 14 bis 17, auf und/oder die Substanz (ii) weist einen Log P-Wert von höchstens 0,46, insbesondere im Bereich von 0,32 bis 0,46, vorzugsweise im Bereich von 0,33 bis 0,44, bevorzugt im Bereich von 0,34 bis 0,42, auf;

(3a) die Substanz (iii) weist einen HLB-Wert nach Griffin von höchstens 1,5, insbesondere im Bereich von 0,5 bis 1,5, vorzugsweise im Bereich von 0,6 bis 1,4, bevorzugt im Bereich von 0,7 bis 1,3, auf und/oder die Substanz (iii) weist einen Log P-Wert von mindestens 5,40, insbesondere im Bereich von 5,40 bis 18,97, vorzugsweise im Bereich von 5,84 bis 15,40, bevorzugt im Bereich von 6,36 bis 12,91, auf;

(4a) die Substanz (iv) weist einen HLB-Wert nach Griffin von höchstens 4, insbesondere im Bereich von 1 bis 4, vorzugsweise im Bereich von 1,5 bis 3,5, bevorzugt im Bereich von 2 bis 3, auf und/oder die Substanz (iv) weist einen Log P-Wert von mindestens 1,76, insbesondere im Bereich von 1,76 bis 8,58, vorzugsweise im Bereich von 2,05 bis 5,40, bevorzugt im Bereich von 2,44 bis 3,88, auf;

(5a) die Substanz (v) weist einen HLB-Wert nach Griffin von höchstens 8, insbesondere im Bereich von 1 bis 8, vorzugsweise im Bereich von 1,25 bis 7, bevorzugt im Bereich von 1,5 bis 6,5, auf und/oder die Substanz (v) weist einen Log P-Wert von mindestens 0,80, insbesondere im Bereich von 0,80 bis 8,58, vorzugsweise im Bereich von 0,93 bis 6,65, bevorzugt im Bereich von 1,01 bis 5,40, auf;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

insbesondere wobei die Substanzen zunächst zumindest teilweise auf unterschiedliche Ansätze (Chargen) aufgeteilt werden bzw. in unterschiedlichen Ansätzen (Chargen) vorgelegt werden und anschließend zur Ausbildung des Trägersystems zusammengeführt und/oder vereinigt werden, vorzugsweise wobei die Aufteilung der Substanzen auf die unterschiedlichen Ansätze und/oder das Vorlegen der Substanzen in unterschiedlichen Ansätzen in Abhängigkeit von ihrer jeweiligen Hydrophilie und/oder Lipophilie und/oder in Abhängigkeit von ihren HLB-Werten nach Griffin erfolgt und/oder in Abhängigkeit von ihren LogP -Werten erfolgt, insbesondere wobei die Aufteilung derart erfolgt, dass einander entsprechende Substanzen, insbesondere Substanzen mit gleicher oder gleichartiger Hydrophilie und/oder Lipophilie bzw. Substanzen mit gleichen oder gleichartigen HLB-Werten nach Griffin und/oder mit gleichen oder gleichartigen LogP -Werten, jeweils in demselben Ansatz vorgelegt werden und/oder insbesondere wobei gleichartige Substanzen, insbesondere Substanzen mit gleicher oder gleichartiger Hydrophilie und/oder Lipophilie bzw. Substanzen mit gleichen oder gleichartigen HLB-Werten nach Griffin und/oder mit gleichen oder gleichartigen LogP -Werten jeweils in demselben Ansatz vorgelegt werden.

Gemäß den vorgenannten Verfahren verhält es sich insbesondere gleichermaßen derart, dass die Substanzen mit einem HLB-Wert nach Griffin von mindestens 6, vorzugsweise mindestens 7, bevorzugt mindestens 8, bzw. mit einem Log P-Wert von höchstens 1,11, vorzugsweise höchstens 0,93, bevorzugt höchstens 0,80, insbesondere in Kombination mit vorzugsweise ein Polyol enthaltendem flüssigem Medium, insbesondere wie zuvor definiert, in einem ersten Ansatz vorgelegt werden und/oder insbesondere wobei ein erster Ansatz bereitgestellt wird, welcher die Substanzen mit einem HLB-Wert nach Griffin von mindestens 6, vorzugsweise mindestens 7, bevorzugt mindestens 8, bzw. mit einem Log P-Wert von höchstens 1,11, vorzugsweise höchstens 0,93, bevorzugt höchstens 0,80, insbesondere in Kombination mit vorzugsweise ein Polyol enthaltendem flüssigem Medium, insbesondere wie zuvor definiert, enthält und/oder insbesondere wobei die Substanzen mit einem HLB-Wert nach Griffin von weniger als 6, vorzugsweise höchstens 5,5, bevorzugt höchstens 5, bzw. mit einem Log P-Wert von mehr als 1,11, vorzugsweise mindestens 1,22, bevorzugt mindestens 1,36, gegebenenfalls in Kombination mit vorzugsweise ein Polyol enthaltendem flüssigem Medium, insbesondere wie zuvor definiert, in einem zweiten Ansatz vorgelegt werden und/oder insbesondere wobei ein zweiter Ansatz bereitgestellt wird, welcher die Substanzen mit einem HLB-Wert nach Griffin von weniger als 6, vorzugsweise höchstens 5,5, bevorzugt höchstens 5, bzw. mit einem Log P-Wert von mehr als 1,11, vorzugsweise mindestens 1,22, bevorzugt mindestens 1,36, gegebenenfalls in Kombination mit vorzugsweise ein Polyol enthaltendem flüssigem Medium, insbesondere wie zuvor definiert, enthält.

Diesbezüglich können auch die vorgenannten Verfahren gemäß dem vorliegenden Aspekt in zumindest im Wesentlichen vollständiger Abwesenheit von aliphatischen Monoalkoholen, insbesondere in zumindest im Wesentlichen vollständiger Abwesenheit von aliphatischen $C_1$- bis $C_4$-Monoalkoholen, vorzugsweise in zumindest im Wesentlichen vollständiger Abwesenheit von Ethanol, durchgeführt werden.

Durch die gezielte Verwendung und Kombination der Substanzen bzw. Bestandteile des Trägersystems bzw. des Mediums kann die Ausbildung des Trägersystems weiterführend gesteuert bzw. maßgeschneidert werden. Aufgrund der speziellen HLB-Werte bzw. Log P-Werte der eingesetzten Bestandteile bzw. Substanzen für das Trägersystem liegt dabei auch eine hohe Kompatibilität und Abstimmung der Komponenten vor, so dass auch auf dieser Basis ziel- und zweckgerichtet definierte Trägersysteme bereitgestellt werden können. Beispielsweise kann durch die Verwendung eines Polyols die Aggregatbildung weiterführend beeinflusst bzw. gesteuert werden.

Gemäß dem vorliegenden Aspekt betrifft die vorliegende Erfindung darüber hinaus auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten lipidbasierten Trägersystems in partikulärer Form, bevorzugt in Form von Lipidnanopartikeln (LNP), insbesondere Verfahren wie zuvor definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin;
wobei das Phosholipid einen HLB-Wert nach Griffin von höchstens 11, insbesondere höchstens 8, aufweist und/oder wobei die nichtionogene amphiphile und/oder tensidische Substanz einen Log P-Wert von höchstens 0,46 aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern;
wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D);
wobei das Steroid und/oder Secosteroid, insbesondere das Sterol, einen HLB-Wert nach Griffin von höchstens 1,5 aufweist und/oder wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins;
wobei das kationische Lipid einen HLB-Wert nach Griffin von höchstens 4 aufweist und/oder wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;

wobei der Mono-, Di- oder Tricarbonsäureester von Glycerin einen HLB-Wert nach Griffin von höchstens 8 aufweist und/oder insbesondere wobei der Mono-, Di- oder Tricarbonsäureester von Glycerin einen Log P-Wert von mindestens 0,80 aufweist;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

wobei das flüssige Medium Wasser und mindestens einen Alkohol umfasst;

insbesondere wobei der Alkohol ausgewählt wird aus der Gruppe von einwertigen Alkoholen, mehrwertigen Alkoholen, insbesondere Polyolen, und deren Mischungen sowie Kombinationen; und/oder

insbesondere wobei der Alkohol ein mehrwertiger Alkohol, insbesondere Polyol, ist; und/oder

insbesondere wobei der Alkohol, insbesondere das Polyol, ausgewählt wird aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen sowie Kombinationen; und/oder

insbesondere wobei der Alkohol, insbesondere das Polyol, ausgewählt wird aus der Gruppe von Propylenglykol, Polyethylenglykol, Sorbitol, Mannitol, Glycerin, Saccharose, Panthenol und deren Mischungen sowie Kombinationen; und/oder

insbesondere wobei der Alkohol, insbesondere das Polyol, Glycerin ist; und/oder

insbesondere wobei der Alkohol, insbesondere das Polyol, Panthenol ist; und/oder

wobei das flüssige Medium keinen Ethanol umfasst.

Gemäß dem vorliegenden Aspekt betrifft die vorliegende Erfindung zudem auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten lipidbasierten Trägersystems in partikulärer Form, bevorzugt in Form von Lipidnanopartikeln (LNP), insbesondere Verfahren wie zuvor definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin;
wobei das Phosholipid einen HLB-Wert nach Griffin von höchstens 11, insbesondere höchstens 8, aufweist und/oder wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern; wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D); wobei das Steroid und/oder Secosteroid, insbesondere das Sterol, einen HLB-Wert nach Griffin von höchstens 1,5 aufweist und/oder wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins; wobei das kationische Lipid einen HLB-Wert nach Griffin von höchstens 4 aufweist und/oder wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin; wobei der Mono-, Di- oder Tricarbonsäureester von Glycerin einen HLB-Wert nach Griffin von höchstens 8 aufweist und/oder wobei der Mono-, Di- oder Tricarbonsäureester von Glycerin einen Log P-Wert von mindestens 0,80 aufweist;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

wobei das flüssige Medium Wasser und mindestens einen Alkohol umfasst und

wobei das flüssige Medium ein Gemisch (Mischung) von Wasser und mindestens einem Alkohol, insbesondere Polyol, umfasst oder hieraus besteht, wobei das gewichtsbezogene Mengenverhältnis von Wasser zu Alkohol, insbesondere Polyol, [Wasser : Alkohol, insbesondere Polyol] im Bereich von 200 : 1 bis 1 : 50, insbesondere im Bereich von 100 : 1 bis 1 : 20, vorzugsweise im Bereich von 50 : 1 bis 1 : 5, bevorzugt im Bereich von 20 : 1 bis 1 : 1, insbesondere bezogen auf das flüssige Medium, liegt und/oder eingestellt wird;

insbesondere wobei der Alkohol ausgewählt wird aus der Gruppe von einwertigen Alkoholen, mehrwertigen Alkoholen, insbesondere Polyolen, und deren Mischungen sowie Kombinationen; und/oder

insbesondere wobei der Alkohol ein mehrwertiger Alkohol, insbesondere Polyol, ist; und/oder

insbesondere wobei der Alkohol, insbesondere das Polyol, ausgewählt wird aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen sowie Kombinationen; und/oder

insbesondere wobei der Alkohol, insbesondere das Polyol, ausgewählt wird aus der Gruppe von Propylenglykol, Polyethylenglykol, Sorbitol, Mannitol, Glycerin, Saccharose, Panthenol und deren Mischungen sowie Kombinationen; und/oder

insbesondere wobei der Alkohol, insbesondere das Polyol, Glycerin ist; und/oder

insbesondere wobei der Alkohol, insbesondere das Polyol, Panthenol ist; und/oder

wobei das flüssige Medium keinen Ethanol umfasst.

Gemäß dem vorliegenden Aspekt betrifft die vorliegende Erfindung zudem auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten lipidbasierten Trägersystems in partikulärer Form, bevorzugt in Form von Lipidnanopartikeln (LNP), insbesondere Verfahren wie zuvor definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin;
wobei das Phosholipid einen HLB-Wert nach Griffin von höchstens 11, insbesondere höchstens 8, aufweist und/oder wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern;
wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D);
wobei das Steroid und/oder Secosteroid, insbesondere das Sterol, einen HLB-Wert nach Griffin von höchstens 1,5 aufweist und/oder wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins;
wobei das kationische Lipid einen HLB-Wert nach Griffin von höchstens 4 aufweist und/oder wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;

wobei der Mono-, Di- oder Tricarbonsäureester von Glycerin einen HLB-Wert nach Griffin von höchstens 8 aufweist und/oder insbesondere wobei der Mono-, Di- oder Tricarbonsäureester von Glycerin einen Log P-Wert von mindestens 0,80 aufweist;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

wobei das flüssige Medium Wasser und mindestens einen Alkohol umfasst und

wobei das flüssige Medium in einer Menge eingesetzt wird derart, dass das gewichtsbezogene Mengenverhältnis von Trägersystem, insbesondere Gesamtheit der Bestandteile des Trägersystems, zu flüssigem Medium [Trägersystem, insbesondere Gesamtheit der Bestandteile des Trägersystems : flüssiges Medium] im Bereich von 1 : 20 bis 50 : 1, insbesondere im Bereich von 1 : 10 bis 20 : 1, vorzugsweise im Bereich von 1 : 5 bis 10 : 1, bevorzugt im Bereich von 1 : 4 bis 5 : 1, liegt und/oder eingestellt wird, und/oder wobei das flüssige Medium ein Gemisch (Mischung) von Wasser und mindestens einem Alkohol, insbesondere Polyol, umfasst oder hieraus besteht, wobei das gewichtsbezogene Mengenverhältnis von Wasser zu Alkohol, insbesondere Polyol, [Wasser : Alkohol, insbesondere Polyol] im Bereich von 200 : 1 bis 1 : 50, insbesondere im Bereich von 100 : 1 bis 1 : 20, vorzugsweise im Bereich von 50 : 1 bis 1 : 5, bevorzugt im Bereich von 20 : 1 bis 1 : 1, insbesondere bezogen auf das flüssige Medium, liegt und/oder eingestellt wird;

insbesondere wobei der Alkohol ausgewählt wird aus der Gruppe von einwertigen Alkoholen, mehrwertigen Alkoholen, insbesondere Polyolen, und deren Mischungen sowie Kombinationen; und/oder

insbesondere wobei der Alkohol ein mehrwertiger Alkohol, insbesondere Polyol, ist; und/oder

insbesondere wobei der Alkohol, insbesondere das Polyol, ausgewählt wird aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen sowie Kombinationen; und/oder

insbesondere wobei der Alkohol, insbesondere das Polyol, ausgewählt wird aus der Gruppe von Propylenglykol, Polyethylenglykol, Sorbitol, Mannitol, Glycerin, Saccharose, Panthenol und deren Mischungen sowie Kombinationen; und/oder insbesondere wobei der Alkohol, insbesondere das Polyol, Glycerin ist; und/oder

insbesondere wobei der Alkohol, insbesondere das Polyol, Panthenol ist; und/oder

wobei das flüssige Medium keinen Ethanol umfasst.

Gemäß dem vorliegenden Aspekt betrifft die vorliegende Erfindung weiterführend auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten, vorzugsweise lipidbasierten Trägersystems in partikulärer Form, bevorzugt in Form von Lipidnanopartikeln (LNP), insbesondere Verfahren wie zuvor definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin;
wobei das Phosholipid einen HLB-Wert nach Griffin von höchstens 11, insbesondere höchstens 8, aufweist und/oder wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern;
wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D);
wobei das Steroid und/oder Secosteroid, insbesondere das Sterol, einen HLB-Wert nach Griffin von höchstens 1,5 aufweist und/oder wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins;
wobei das kationische Lipid einen HLB-Wert nach Griffin von höchstens 4 aufweist und/oder wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;
wobei der Mono-, Di- oder Tricarbonsäureester von Glycerin einen HLB-Wert nach Griffin von höchstens 8 aufweist und/oder wobei der Mono-, Di- oder Tricarbonsäureester von Glycerin einen Log P-Wert von mindestens 0,80 aufweist;

(vi) mindestens einen Wirkstoff, insbesondere wie in einem der Ansprüche 23 bis 25 definiert, insbesondere wobei der Wirkstoff ein therapeutischer und/oder prophylaktischer Wirkstoff, insbesondere therapeutischer und/oder prophylaktischer pharmazeutischer Wirkstoff, ist und/oder insbesondere wobei der Wirkstoff ausgewählt wird aus der Gruppe von Vakzinwirkstoffen (Impfwirkstoffen), antibiotischen Wirkstoffen, antimykotischen Wirkstoffen, antiviralen Wirkstoffen, antiphlogistischen Wirkstoffen, antipyretischen Wirkstoffen, antiallergischen Wirkstoffen, Antikrebswirkstoffen, Hormonwirkstoffen, antikoagulativen Wirkstoffen, Antitumorwirkstoffen (wie Zytostatika), Nahrungsergänzungsstoffen, Vitaminen, Mineralstoffen, UVabsorbierenden Stoffen und deren Mischungen sowie Kombinationen, vorzugsweise Vakzinwirkstoffen (Impfwirkstoffen);

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

wobei das flüssige Medium Wasser und mindestens einen Alkohol umfasst;

insbesondere wobei der Alkohol ausgewählt wird aus der Gruppe von einwertigen Alkoholen, mehrwertigen Alkoholen, insbesondere Polyolen, und deren Mischungen sowie Kombinationen; und/oder

insbesondere wobei der Alkohol ein mehrwertiger Alkohol, insbesondere Polyol, ist; und/oder

insbesondere wobei der Alkohol, insbesondere das Polyol, ausgewählt wird aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen sowie Kombinationen; und/oder

insbesondere wobei der Alkohol, insbesondere das Polyol, ausgewählt wird aus der Gruppe von Propylenglykol, Polyethylenglykol, Sorbitol, Mannitol, Glycerin, Saccharose, Panthenol und deren Mischungen sowie Kombinationen; und/oder

insbesondere wobei der Alkohol, insbesondere das Polyol, Glycerin ist; und/oder

insbesondere wobei der Alkohol, insbesondere das Polyol, Panthenol ist; und/oder

wobei das flüssige Medium keinen Ethanol umfasst.

Gemäß dem vorliegenden Aspekt betrifft die vorliegende Erfindung nochmals weiterführend auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten, vorzugsweise lipidbasierten Trägersystems in partikulärer Form, bevorzugt in Form von Lipidnanopartikeln (LNP), insbesondere Verfahren wie zuvor definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin;
insbesondere wobei das Phosholipid einen HLB-Wert nach Griffin von höchstens 11, insbesondere höchstens 8, aufweist und/oder insbesondere wobei das Phosholipid einen Log P-Wert von mindestens 0,55, insbesondere mindestens 0,80, aufweist;
(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern;
insbesondere wobei die nichtionogene amphiphile und/oder tensidische Substanz einen HLB-Wert nach Griffin von mindestens 13 aufweist und/oder insbesondere wobei die nichtionogene amphiphile und/oder tensidische Substanz einen Log P-Wert von höchstens 0,46 aufweist;

(iii) ggf. mindestens ein Sterol (Sterin), insbesondere ausgewählt aus der Gruppe von Zoosterolen, Phytosterolen und Mycosterolen sowie deren Kombinationen und Mischungen, vorzugsweise Cholesterol und/oder Phytosterol; insbesondere wobei das Sterol einen HLB-Wert nach Griffin von höchstens 1,5 aufweist und/oder insbesondere wobei das Sterol einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins; insbesondere wobei das kationische Lipid einen HLB-Wert nach Griffin von höchstens 4 aufweist und/oder insbesondere wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;

insbesondere wobei der Mono-, Di- oder Tricarbonsäureester von Glycerin einen HLB-Wert nach Griffin von höchstens 8 aufweist und/oder insbesondere wobei der Mono-, Di- oder Tricarbonsäureester von Glycerin einen Log P-Wert von mindestens 0,80 aufweist;

wobei das gewichtsbezogene Mengenverhältnis von Substanz (i) zu Substanz (ii) [Substanz (i) : Substanz (ii)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, liegt und/oder auf die vorgenannten Werte eingestellt wird; und/oder

wobei das gewichtsbezogene Mengenverhältnis von Substanz (i) zu Substanz (ii) zu Substanz (iii) zu Substanz (iv) zu Substanz (v) [Substanz (i) : Substanz (ii) : Substanz (iii) : Substanz (iv) : Substanz (v)] im Bereich von (0,5 bis 50) : (1 bis 80) : (0,2 bis 40) : (1 bis 80) : (2 bis 85), insbesondere (1 bis 40) : (2 bis 75) : (0,5 bis 25) : (2 bis 75) : (4 bis 80), vorzugsweise (1,25 bis 30) : (3 bis 70) :(1 bis 20) : (3 bis 70) : (6 bis 75), bevorzugt (1,5 bis 25) : (5 bis 65) : (3 bis 15) : (5 bis 65) : (8 bis 70), liegt und/oder auf die vorgenannten Werte eingestellt wird;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert.

Gemäß dem vorliegenden Aspekt betrifft die vorliegende Erfindung auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten lipidbasierten Trägersystems in partikulärer Form, bevorzugt in Form von Lipidnanopartikeln (LNP), insbesondere Verfahren wie zuvor definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin; insbesondere wobei das Phosholipid einen HLB-Wert nach Griffin von höchstens 11, insbesondere höchstens 8, aufweist und/oder insbesondere wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern; insbesondere wobei die nichtionogene amphiphile und/oder tensidische Substanz einen HLB-Wert nach Griffin von mindestens 13 aufweist und/oder insbesondere wobei die nichtionogene amphiphile und/oder tensidische Substanz einen Log P-Wert von höchstens 0,46 aufweist;

(iii) ggf. mindestens ein Sterol (Sterin), insbesondere ausgewählt aus der Gruppe von Zoosterolen, Phytosterolen und Mycosterolen sowie deren Kombinationen und Mischungen, vorzugsweise Cholesterol und/oder Phytosterol; insbesondere wobei das Sterol einen HLB-Wert nach Griffin von höchstens 1,5 aufweist und/oder insbesondere wobei das Sterol einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins;

insbesondere wobei das kationische Lipid einen HLB-Wert nach Griffin von höchstens 4 aufweist und/oder insbesondere wobei das kationische Lipid einen Log P-Wert von mindestens 1,76 aufweist;;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;

insbesondere wobei der Mono-, Di- oder Tricarbonsäureester von Glycerin einen HLB-Wert nach Griffin von höchstens 8 aufweist und/oder insbesondere wobei der Mono-, Di- oder Tricarbonsäureester von Glycerin einen Log P-Wert von mindestens 0,80 aufweist;

wobei der Wirkstoff, insbesondere als Substanz (vi), Bestandteil des Trägersystems ist und/oder einen Bestandteil des Trägersystems ausbildet und/oder wobei das Trägersystem den Wirkstoff, insbesondere als Substanz (vi), aufweist;

insbesondere wobei das gewichtsbezogene Mengenverhältnis von Substanz (i) zu Substanz (ii) zu Wirkstoff bzw. Substanz (vi) [Substanz (i) : Substanz (ii) : Substanz (vi)] im Bereich von (0,5 bis 50) : (1 bis 80) : (0,01 bis 80), insbesondere im Bereich von (1 bis 40) : (2 bis 75) : (0,1 bis 70), vorzugsweise im Bereich von (1,25 bis 30) : (3 bis 70) : (0,5 bis 60), bevorzugt im Bereich von (1,5 bis 25) : (5 bis 65) : (1 bis 50) liegt und/oder auf die vorgenannten Werte eingestellt wird; und/oder

insbesondere wobei das gewichtsbezogene Mengenverhältnis von Substanz (i) zu Substanz (ii) zu Substanz (iii) zu Substanz (iv) zu Substanz (v) zu Wirkstoff bzw. Substanz (vi) [Substanz (i) : Substanz (ii) : Substanz (iii) : Substanz (iv) : Substanz (v) : Substanz (vi)] im Bereich von (0,5 bis 50) : (1 bis 80) : (0,2 bis 40) : (1 bis 80) : (2 bis 85): (0,01 bis 80), insbesondere im Bereich von (1 bis 40) : (2 bis 75) : (0,5 bis 25) : (2 bis 75) : (4 bis 80) : (0,1 bis 70), vorzugsweise im Bereich von (1,25 bis 30) : (3 bis 70) : (1 bis 20) : (3 bis 70) : (6 bis 75) : (0,5 bis 60), bevorzugt im Bereich von (1,5 bis 25) : (5 bis 65) : (3 bis 15) : (5 bis 65) :(8 bis 70) : (1 : 50), liegt und/oder auf die vorgenannten Werte eingestellt wird; und/oder

insbesondere wobei das Trägersystem den Wirkstoff und/oder die Substanz (vi) in einer Menge im Bereich von 0,01 Gew.-% bis 80 Gew.-%, insbesondere im Bereich von 0,1 Gew.-% bis 70 Gew.-%, vorzugsweise im Bereich von 0,5 Gew.-% bis 60 Gew.-%, bevorzugt im Bereich von 1 Gew.-% bis 50 Gew.-%, bezogen auf das Trägersystem und/oder bezogen auf die Gesamtheit der Bestandteile des Trägersystems, aufweist;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert.

Gemäß dem vorliegenden Aspekt betrifft die vorliegende Erfindung auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten lipidbasierten Trägersystems in partikulärer Form, bevorzugt in Form von Lipidnanopartikeln (LNP), insbesondere Verfahren wie zuvor definiert,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin; wobei das Phosholipid einen HLB-Wert nach Griffin von höchstens 11, insbesondere höchstens 8, aufweist und/oder wobei das Phosholipid einen Log P-Wert von mindestens 0,55, insbesondere mindestens 0,80, aufweist;
(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern; wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;
(iii) ggf. mindestens ein Sterol (Sterin), insbesondere ausgewählt aus der Gruppe von Zoosterolen, Phytosterolen und Mycosterolen sowie deren Kombinationen und Mischungen, vorzugsweise Cholesterol und/oder Phytosterol; wobei das Sterol einen HLB-Wert nach Griffin von höchstens 1,5 aufweist und/oder wobei das Sterol einen Log P-Wert von mindestens 5,40 aufweist;
(iv) ggf. mindestens ein kationisches Lipid, insbesondere aminbasiertes kationisches Lipid, vorzugsweise ein kationisches Lipid auf Basis eines tertiären Amins; wobei das kationische Lipid einen HLB-Wert nach Griffin von höchstens 4 aufweist und/oder insbesondere wobei das kationische Lipid einen Log P-Wert von mindestens 1,76 aufweist;
(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin; wobei der Mono-, Di- oder Tricarbonsäureester von Glycerin einen HLB-Wert nach Griffin von höchstens 8 aufweist und/oder wobei der Mono-, Di- oder Tricarbonsäureester von Glycerin einen Log P-Wert von mindestens 0,80 aufweist;

wobei das gewichtsbezogene Mengenverhältnis von Substanz (i) zu Substanz (ii) [Substanz (i) : Substanz (ii)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, liegt und/oder auf die vorgenannten Werte eingestellt wird; und/oder

wobei das gewichtsbezogene Mengenverhältnis von Substanz (i) zu Substanz (ii) zu Substanz (iii) zu Substanz (iv) zu Substanz (v) [Substanz (i) : Substanz (ii) : Substanz (iii) : Substanz (iv) : Substanz (v)] im Bereich von (0,5 bis 50) : (1 bis 80) : (0,2 bis 40) : (1 bis 80) : (2 bis 85), insbesondere (1 bis 40) : (2 bis 75) : (0,5 bis 25) : (2 bis 75) : (4 bis 80), vorzugsweise (1,25 bis 30) : (3 bis 70) : (1 bis 20) : (3 bis 70) : (6 bis 75), bevorzugt (1,5 bis 25) : (5 bis 65) : (3 bis 15) : (5 bis 65) : (8 bis 70), liegt und/oder auf die vorgenannten Werte eingestellt wird;

wobei das Verfahren, insbesondere die Herstellung des Trägersystems bzw. des mit dem pharmazeutischen

EP 4 436 558 B1

Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

wobei das flüssige Medium Wasser und mindestens einen Alkohol umfasst und

wobei das flüssige Medium in einer Menge eingesetzt wird derart, dass das gewichtsbezogene Mengenverhältnis von Trägersystem, insbesondere Gesamtheit der Bestandteile des Trägersystems, zu flüssigem Medium [Trägersystem, insbesondere Gesamtheit der Bestandteile des Trägersystems : flüssiges Medium] im Bereich von 1 : 20 bis 50 : 1, insbesondere im Bereich von 1 : 10 bis 20 : 1, vorzugsweise im Bereich von 1 : 5 bis 10 : 1, bevorzugt im Bereich von 1 : 4 bis 5 : 1, liegt und/oder eingestellt wird, und/oder wobei das flüssige Medium ein Gemisch (Mischung) von Wasser und mindestens einem Alkohol, insbesondere Polyol, umfasst oder hieraus besteht, wobei das gewichtsbezogene Mengenverhältnis von Wasser zu Alkohol, insbesondere Polyol, [Wasser : Alkohol, insbesondere Polyol] im Bereich von 200 : 1 bis 1 : 50, insbesondere im Bereich von 100 : 1 bis 1 : 20, vorzugsweise im Bereich von 50 : 1 bis 1 : 5, bevorzugt im Bereich von 20 : 1 bis 1 : 1, insbesondere bezogen auf das flüssige Medium, liegt und/oder eingestellt wird.

Gemäß dem vorliegenden Aspekt betrifft die vorliegende Erfindung weiterhin auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten lipidbasierten Trägersystems in partikulärer Form, bevorzugt in Form von Lipidnanopartikeln (LNP), wie zuvor definiert,

wobei die Bestandteile des Trägersystems (miteinander) in Kontakt gebracht werden und/oder (miteinander) in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin;
insbesondere wobei das Phosholipid einen HLB-Wert nach Griffin von höchstens 11, insbesondere höchstens 8, aufweist und/oder insbesondere wobei das Phosholipid einen Log P-Wert von mindestens 0,55, insbesondere mindestens 0,80, aufweist;
(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern;
insbesondere wobei die nichtionogene amphiphile und/oder tensidische Substanz einen HLB-Wert nach Griffin von mindestens 13 aufweist und/oder insbesondere wobei die nichtionogene amphiphile und/oder tensidische Substanz einen Log P-Wert von höchstens 0,46 aufweist;
(iii) ggf. mindestens ein Sterol (Sterin), insbesondere ausgewählt aus der Gruppe von Zoosterolen, Phytosterolen und Mycosterolen sowie deren Kombinationen und Mischungen, vorzugsweise Cholesterol und/oder Phytosterol;
insbesondere wobei das Sterol einen HLB-Wert nach Griffin von höchstens 1,5 aufweist und/oder insbesondere wobei das Sterol einen Log P-Wert von mindestens 5,40 aufweist;
(iv) ggf. mindestens ein kationisches Lipid, insbesondere aminbasiertes kationisches Lipid, vorzugsweise ein kationisches Lipid auf Basis eines tertiären Amins;
insbesondere wobei das kationische Lipid einen HLB-Wert nach Griffin von höchstens 4 aufweist und/oder insbesondere wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;

insbesondere wobei der Mono-, Di- oder Tricarbonsäureester von Glycerin einen HLB-Wert nach Griffin von höchstens 8 aufweist und/oder insbesondere wobei der Mono-, Di- oder Tricarbonsäureester von Glycerin einen Log P-Wert von mindestens 0,80 aufweist;

wobei das Verfahren, insbesondere die Herstellung des Trägersystems bzw. des mit dem pharmazeutischen Wirkstoff beladene und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine vorzugsweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladene und/oder ausgerüsteten Trägersystems in dem flüssigen Medium resultiert,

wobei das erhaltene Trägersystem bzw. das erhaltene und mit dem pharmazeutischen Wirkstoff beladene und/oder ausgerüstete Trägersystem bzw. die erhaltene Zusammensetzung, welche das Trägersystem bzw. das mit dem pharmazeutischen Wirkstoff beladene und/oder ausgerüstete Trägersystem und das flüssige Medium umfasst, abgekühlt wird, insbesondere durch (weitere) Zugabe von insbesondere gekühltem flüssigem Medium, insbesondere wie zuvor definiert,

insbesondere wobei das zuzugebende flüssige Medium eine Temperatur aufweist, welche mindestens 5 °C, insbesondere mindestens 10 °C, vorzugsweise mindestens 15 °C, unterhalb der Temperatur des erhaltenen Trägersystems bzw. des erhaltenen und mit dem pharmazeutischen Wirkstoff beladene und/oder ausgerüstete Trägersystems bzw. der erhaltenen Zusammensetzung liegt und/oder insbesondere wobei das zuzugebende flüssige Medium eine Temperatur im Bereich von -20 °C bis 40 °C, insbesondere im Bereich von -10 °C bis 30 °C, vorzugsweise im Bereich von 0 °C bis 25 °C, bevorzugt im Bereich von -5 °C bis 20 °C, aufweist.

Auch bei den vorgenannten Verfahren gemäß dem vorliegenden erfindungsgemäßen Aspekt kann es sich insbesondere derart verhalten,

dass das flüssige Medium Wasser und mindestens einen Alkohol umfasst;

dass bzw. insbesondere wobei der Alkohol ausgewählt wird aus der Gruppe von einwertigen Alkoholen, mehrwertigen Alkoholen, insbesondere Polyolen, und deren Mischungen sowie Kombinationen; und/oder

dass bzw. insbesondere wobei der Alkohol ein mehrwertiger Alkohol, insbesondere Polyol, ist; und/oder

dass bzw. insbesondere wobei der Alkohol, insbesondere das Polyol, ausgewählt wird aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen sowie Kombinationen; und/oder

dass bzw. insbesondere wobei der Alkohol, insbesondere das Polyol, ausgewählt wird aus der Gruppe von Propylenglykol, Polyethylenglykol, Sorbitol, Mannitol, Glycerin, Saccharose, Panthenol und deren Mischungen sowie Kombinationen; und/oder

dass bzw. insbesondere wobei der Alkohol, insbesondere das Polyol, Glycerin ist; und/oder

dass bzw. insbesondere wobei der Alkohol, insbesondere das Polyol, Panthenol ist; und/oder

dass bzw. insbesondere wobei der Alkohol kein $C_1$- bis $C_4$-Monoalkohol, vorzugsweise kein Ethanol, ist und/oder insbesondere wobei der Alkohol keinen $C_1$-bis $C_4$-Monoalkohol, insbesondere kein Ethanol, umfasst; und/oder

dass bzw. insbesondere wobei das flüssige Medium keinen $C_1$- bis $C_4$-Monoalkohol, insbesondere kein Ethanol, umfasst.

Gemäß dem vorliegenden Aspekt betrifft die vorliegende Erfindung darüber hinaus auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten, lipidbasierten Trägersystems in partikulärer Form, bevorzugt in Form von Lipidnanopartikeln (LNP), wie zuvor definiert,

wobei die Bestandteile des Trägersystems (miteinander) in Kontakt gebracht werden und/oder (miteinander) in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems, vorzugsweise in partikulärer Form, bevorzugt in Form von Lipidnanopartikeln (LNP), bewirkt wird, wobei die Bestandteile des Trägersystems die folgenden Substanzen umfassen:

(i) mindestens ein Phospholipid, vorzugsweise Phosphatidylcholin (Lecithin); insbesondere wobei das Phosholipid einen HLB-Wert nach Griffin von höchstens 11, insbesondere höchstens 8, aufweist und/oder insbesondere wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern; insbesondere wobei die nichtionogene amphiphile und/oder tensidische Substanz einen HLB-Wert nach Griffin von mindestens 13 aufweist und/oder insbesondere wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D); insbesondere wobei das Steroid und/oder Secosteroid, insbesondere das Sterol, einen HLB-Wert nach Griffin von höchstens 1,5 aufweist und/oder insbesondere wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins; insbesondere wobei das kationische Lipid einen HLB-Wert nach Griffin von höchstens 4 aufweist und/oder insbesondere wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin; insbesondere wobei der Mono-, Di- oder Tricarbonsäureester von Glycerin einen HLB-Wert nach Griffin von höchstens 8 aufweist und/oder insbesondere wobei der Mono-, Di- oder Tricarbonsäureester von Glycerin einen Log P-Wert von mindestens 0,80 aufweist;

insbesondere wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert.

Gemäß dem vorliegenden Aspekt betrifft die vorliegende Erfindung zudem auch das Verfahren zur Herstellung eines lipidbasierten Trägersystems in partikulärer Form, bevorzugt in Form von Lipidnanopartikeln (LNP), insbesondere für einen pharmazeutischen Wirkstoff; und/oder zur Herstellung eines mit einem pharmazeutischen Wirkstoff beladenen und/oder ausgerüsteten lipidbasierten Trägersystems, vorzugsweise in partikulärer Form, bevorzugt in Form von Lipidnanopartikeln (LNP), wie zuvor definiert,

wobei die Bestandteile des Trägersystems (miteinander) in Kontakt gebracht werden und/oder (miteinander) in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems, vorzugsweise in partikulärer Form, bevorzugt in Form von Lipidnanopartikeln (LNP), bewirkt wird,

wobei die Bestandteile des Trägersystems die folgenden Substanzen umfassen:

(i) mindestens ein Phospholipid, vorzugsweise Phosphatidylcholin (Lecithin);

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D);

(iv) ggf. mindestens ein kationisches Lipid, insbesondere aminbasiertes kationisches Lipid, vorzugsweise ein kationisches Lipid auf Basis eines tertiären Amins;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin.

In diesem Zusammenhang kann es vorgesehen sein, dass das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), durchgeführt wird und nach Ausbildung des Trägersystems eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert.

Für die vorgenannten weiteren Gegenstände des vorliegenden Aspekts kann es vorgesehen sein,

dass die Zusammenlagerung, insbesondere Aggregation, der Bestandteile zur Ausbildung des Trägersystems unter Eintrag von Energie, bevorzugt Eintrag von Misch- und/oder Rührenergie, insbesondere in das zumindest einen Teil der Bestandteile und/oder Substanzen des Trägersystems aufweisende flüssige Medium und/oder in eine zumindest einen Teil der Bestandteile und/oder Substanzen des Trägersystems aufweisenden Mischung, durchgeführt wird, wobei die eingetragene Energie, insbesondere Misch- und/oder Rührenergie, eingestellt wird derart, dass eine zumindest im Wesentlichen laminare Strömung, insbesondere in dem zumindest einen Teil der Bestandteile und/oder Substanzen des Trägersystems aufweisenden flüssigen Medium und/oder in der zumindest einen Teil der Bestandteile und/oder Substanzen des Trägersystems aufweisenden Mischung, vorliegt, insbesondere unter zumindest im Wesentlichen vollständiger Vermeidung der Ausbildung einer nichtlaminaren und/oder turbulenten Strömung und/oder insbesondere unter zumindest im Wesentlichen vollständiger Vermeidung der Ausbildung nichtlaminarer und/oder turbulenter Strömungsanteile; und/oder

dass eine insbesondere kontinuierliche Verfahrens- und/oder Prozesskontrolle (Produktionskontrolle), insbesondere *online*- und/oder *inline*-Prozesskontrolle, durchgeführt wird, insbesondere zur Überwachung des Verfahrensstatus und/oder des Verfahrensfortschritts, vorzugsweise zur Überwachung und/oder Einstellung der Ausbildung der Partikel des Trägersystems, insbesondere Lipidnanopartikel (LNP), und/oder insbesondere zur Überwachung und/oder Einstellung von Verfahrensparametern, wie Eintrag von Energie, bevorzugt Eintrag von Misch- und/oder Rührenergie, Eintrag und/oder Abfuhr von Wärmenergie, Strömungsverhalten, Massendurchsatz, Volumenströmen oder dergleichen; wobei die Verfahrens- und/oder Prozesskontrolle über die Erfassung von mindestens einem Prozessparameter, insbesondere mindestens zwei Prozessparametern, erfolgt, wobei der Prozessparameter ausgewählt wird aus der Gruppe von Temperatur, Leitfähigkeit, Viskosität, pH-Wert, elektrischem Widerstand und Oberflächenspannung, insbesondere dynamischer Oberflächenspannung, vorzugsweise aus der Gruppe von Temperatur, Leitfähigkeit, Viskosität und pH-Wert, insbesondere jeweils des flüssigen Mediums, vorzugsweise mitsamt den darin eingebrachten Bestandteilen und/oder Substanzen, und/oder der Zusammensetzung und/oder der jeweiligen Ansätze und deren Kombinationen.

Für weitere Ausgestaltungen des erfindungsgemäßen Verfahrens gemäß dem vorliegenden Aspekt kann auch auf die Ausführungen zu den weiteren erfindungsgemäßen Aspekten verwiesen werden, welche vorliegend entsprechend gelten.

Darüber hinaus ist Gegenstand der vorliegenden Erfindung - gemäß einem **weiteren** Aspekt der vorliegenden Erfindung - zudem das lipidbasierte Trägersystem in partikulärer Form, bevorzugt in Form von Lipidnanopartikeln (LNP), insbesondere für einen pharmazeutischen Wirkstoff, und/oder mit einem pharmazeutischen Wirkstoff beladenes und/oder ausgerüstetes, vorzugsweise lipidbasiertes Trägersystem, vorzugsweise in partikulärer Form, bevorzugt in Form von Lipidnanopartikeln (LNP), erhältlich durch ein wie zuvor definiertes Verfahren.

Gemäß dem vorliegenden Aspekt betrifft die vorliegende Erfindung gleichermaßen auch das lipidbasierte Trägersystem nach der Erfindung in partikulärer Form, bevorzugt in Form von Lipidnanopartikeln (LNP), insbesondere für einen pharmazeutischen Wirkstoff, und/oder mit einem mit einem pharmazeutischen Wirkstoff beladenes und/oder ausgerüstetes, lipidbasiertes Trägersystem in partikulärer Form, bevorzugt in Form von Lipidnanopartikeln (LNP), wie zuvor definiert,

wobei die Bestandteile des Trägersystems zur Ausbildung des Trägersystems in partikulärer Form, bevorzugt in Form von Lipidnanopartikeln (LNP), zusammengelagert, insbesondere aggregiert sind und/oder wobei das Trägersystem vorzugsweise in partikulärer Form, bevorzugt in Form von Lipidnanopartikeln (LNP), vorliegt,

wobei die Bestandteile des Trägersystems die folgenden Substanzen umfassen:

(i) mindestens ein Phospholipid, vorzugsweise Phosphatidylcholin; wobei das Phosholipid einen HLB-Wert nach Griffin von höchstens 11, insbesondere höchstens 8, aufweist und/oder wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern; wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D); wobei das Steroid und/oder Secosteroid, insbesondere das Sterol, einen HLB-Wert nach Griffin von höchstens 1,5 aufweist und/oder wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins; wobei das kationische Lipid einen HLB-Wert nach Griffin von höchstens 4 aufweist und/oder wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin; wobei der Mono-, Di- oder Tricarbonsäureester von Glycerin einen HLB-Wert nach Griffin von höchstens 8 aufweist und/oder wobei der Mono-, Di- oder Tricarbonsäureester von Glycerin einen Log P-Wert von mindestens 0,80 aufweist;

insbesondere wobei der Mono-, Di- oder Tricarbonsäureester von Glycerin einen HLB-Wert nach Griffin von höchstens 8 aufweist;

wobei das Trägersystem bzw. das mit dem pharmazeutischen Wirkstoff beladene und/oder ausgerüstete Trägersystem in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), enthalten ist und/oder vorliegt;

wobei das Trägersystem mit dem flüssigen Medium eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, bildet und/oder insbesondere wobei das Trägersystem mit dem flüssigen Medium eine Dispersion des Trägersystems bzw. des mit einem pharmazeutischen Wirkstoff beladenen und/oder ausgerüsteten Trägersystems in dem flüssigen Medium bildet;

wobei die Zusammenlagerung, insbesondere Aggregation, der Bestandteile zur Ausbildung des Trägersystems unter Eintrag von Energie, bevorzugt Eintrag von Misch- und/oder Rührenergie, insbesondere in das zumindest einen Teil der Bestandteile und/oder Substanzen des Trägersystems aufweisende flüssige Medium und/oder in eine zumindest einen Teil der Bestandteile und/oder Substanzen des Trägersystems aufweisenden Mischung, erfolgt ist, wobei die eingetragene Energie, insbesondere Misch- und/oder Rührenergie, eingestellt worden ist derart, dass eine zumindest im Wesentlichen laminare Strömung, insbesondere in dem zumindest einen Teil der Bestandteile und/oder Substanzen des Trägersystems aufweisenden flüssigen Medium und/oder in der zumindest einen Teil der Bestandteile und/oder Substanzen des Trägersystems aufweisenden Mischung, vorgelegen hat, insbesondere unter zumindest im Wesentlichen vollständiger Vermeidung der Ausbildung einer nichtlaminaren und/oder turbulenten Strömung und/oder insbesondere unter zumindest im Wesentlichen vollständiger Vermeidung der Ausbildung nichtlaminarer und/oder turbulenter Strömungsanteile, und

wobei eine insbesondere kontinuierliche Verfahrens- und/oder Prozesskontrolle (Produktionskontrolle), insbesondere *online*- und/oder *inline*-Prozesskontrolle, erfolgt ist, insbesondere zur Überwachung des Verfahrensstatus und/oder des Verfahrensfortschritts, vorzugsweise zur Überwachung und/oder Einstellung der Ausbildung der Partikel des Trägersystems, insbesondere Lipidnanopartikel (LNP), und/oder insbesondere zur Überwachung und/oder Einstellung von Verfahrensparametern, wie Eintrag von Energie, bevorzugt Eintrag von Misch- und/oder Rührenergie, Eintrag und/oder Abfuhr von Wärmenergie, Strömungsverhalten, Massendurchsatz, Volumenströmen oder dergleichen; wobei die Verfahrens- und/oder Prozesskontrolle über die Erfassung von mindestens einem Prozessparameter, insbesondere mindestens zwei Prozessparametern, erfolgt ist, wobei der Prozessparameter ausgewählt ist aus der Gruppe von Temperatur, Leitfähigkeit, Viskosität, pH-Wert, insbesondere jeweils des flüssigen Mediums, vorzugsweise mitsamt den darin eingebrachten Bestandteilen und/oder Substanzen, und/oder der Zusammensetzung;

wobei das Trägersystem und/oder das flüssige Medium und/oder die Zusammensetzung zumindest im Wesentlichen frei von Ethanol ist bzw. sind.

Erfindungsgemäß kann es im Hinblick auf das Trägersystem insbesondere vorgesehen sein,

dass die Substanz (i) einen HLB-Wert nach Griffin im Bereich von 0,5 bis 8, vorzugsweise im Bereich von 2 bis 7, bevorzugt im Bereich von 3 bis 6, aufweist und/oder dass die Substanz (i) einen Log P-Wert im Bereich von 0,80 bis 18,97, vorzugsweise im Bereich von 0,93 bis 3,88, bevorzugt im Bereich von 1,11 bis 2,44, aufweist; und/oder

dass die Substanz (ii) einen HLB-Wert nach Griffin im Bereich von 13 bis 19, insbesondere im Bereich von 13 bis 17,9, vorzugsweise im Bereich von 13,5 bis 17,5, bevorzugt im Bereich von 14 bis 17, aufweist und/oder dass die Substanz (ii) einen Log P-Wert im Bereich von 0,30 bis 0,46, insbesondere im Bereich von 0,32 bis 0,46, vorzugsweise im Bereich von 0,33 bis 0,44, bevorzugt im Bereich von 0,34 bis 0,42, aufweist; und/oder

dass die Substanz (iii) einen HLB-Wert nach Griffin im Bereich von 0,5 bis 1,5, vorzugsweise im Bereich von 0,6 bis 1,4, bevorzugt im Bereich von 0,7 bis 1,3, aufweist und/oder dass die Substanz (iii) einen Log P-Wert im Bereich von 5,40 bis 18,97, vorzugsweise im Bereich von 5,84 bis 15,40, bevorzugt im Bereich von 6,36 bis 12,91, aufweist; und/oder

dass die Substanz (iv) einen HLB-Wert nach Griffin im Bereich von 1,5 bis 3,5, bevorzugt im Bereich von 2 bis 3, aufweist und/oder dass die Substanz (iv) einen Log P-Wert im Bereich von 2,05 bis 5,40, bevorzugt im Bereich von 2,44 bis 3,88, aufweist; und/oder

dass die Substanz (v) einen HLB-Wert nach Griffin im Bereich von 1 bis 8, vorzugsweise im Bereich von 1,25 bis 7, bevorzugt im Bereich von 1,5 bis 6,5, aufweist und/oder dass die Substanz (v) einen Log P-Wert im Bereich von 0,80 bis 8,58, vorzugsweise im Bereich von 0,93 bis 6,65, bevorzugt im Bereich von 1,01 bis 5,40, aufweist.

Insbesondere kann das Trägersystem durch mindestens eine(s) der nachfolgenden Merkmale und/oder Maßnahmen (1a) bis (5a), insbesondere durch eine Kombination von mindestens zwei, vorzugsweise mindestens drei, bevorzugt mindestens vier, besonders bevorzugt sämtlichen fünf, der nachfolgenden Merkmale und/oder Maßnahmen (1a) bis (5a), gekennzeichnet sein:

(1a) die Substanz (i) weist einen HLB-Wert nach Griffin von höchstens 11, insbesondere höchstens 8, insbesondere im Bereich von 0,5 bis 8, vorzugsweise im Bereich von 2 bis 7, bevorzugt im Bereich von 3 bis 6, auf und/oder die Substanz (i) weist eine einen Log P-Wert von mindestens 0,55, insbesondere mindestens 0,80, insbesondere im Bereich von 0,80 bis 18,97, vorzugsweise im Bereich von 0,93 bis 3,88, bevorzugt im Bereich von 1,11 bis 2,44, auf;

(2a) die Substanz (ii) weist einen HLB-Wert nach Griffin von mindestens 13, insbesondere im Bereich von 13 bis 19, vorzugsweise im Bereich von 13 bis 17,9, bevorzugt im Bereich von 13,5 bis 17,5, besonders bevorzugt im Bereich von 14 bis 17, auf und/oder die Substanz (ii) weist einen Log P-Wert von höchstens 0,46, insbesondere im Bereich von 0,32 bis 0,46, vorzugsweise im Bereich von 0,33 bis 0,44, bevorzugt im Bereich von 0,34 bis 0,42, auf;

(3a) die Substanz (iii) weist einen HLB-Wert nach Griffin von höchstens 1,5, insbesondere im Bereich von 0,5 bis 1,5, vorzugsweise im Bereich von 0,6 bis 1,4, bevorzugt im Bereich von 0,7 bis 1,3, auf und/oder die Substanz (iii) weist einen Log P-Wert von mindestens 5,40, insbesondere im Bereich von 5,40 bis 18,97, vorzugsweise im Bereich von 5,84 bis 15,40, bevorzugt im Bereich von 6,36 bis 12,91, auf;

(4a) die Substanz (iv) weist einen HLB-Wert nach Griffin von höchstens 4, insbesondere im Bereich von 1 bis 4, vorzugsweise im Bereich von 1,5 bis 3,5, bevorzugt im Bereich von 2 bis 3, auf und/oder die Substanz (iv) weist einen Log P-Wert von mindestens 1,76, insbesondere im Bereich von 1,76 bis 8,58, vorzugsweise im Bereich von 2,05 bis 5,40, bevorzugt im Bereich von 2,44 bis 3,88, auf;

(5a) die Substanz (v) weist einen HLB-Wert nach Griffin von höchstens 8, insbesondere im Bereich von 1 bis 8, vorzugsweise im Bereich von 1,25 bis 7, bevorzugt im Bereich von 1,5 bis 6,5, auf und/oder die Substanz (v) weist einen Log P-Wert von mindestens 0,80, insbesondere im Bereich von 0,80 bis 8,58, vorzugsweise im Bereich von 0,93 bis 6,65, bevorzugt im Bereich von 1,01 bis 5,40, auf.

Gemäß dem vorliegenden Aspekt betrifft die vorliegende Erfindung auch das vorzugsweise lipidbasierte Trägersystem, vorzugsweise in partikulärer Form, bevorzugt in Form von Lipidnanopartikeln (LNP), insbesondere für einen pharmazeutischen Wirkstoff, und/oder mit einem mit einem pharmazeutischen Wirkstoff beladenes bzw. ausgerüstetes, vorzugsweise lipidbasiertes Trägersystem, vorzugsweise in partikulärer Form, bevorzugt in Form von Lipidnanopartikeln (LNP), insbesondere wie zuvor definiert,

wobei die Bestandteile des Trägersystems zur Ausbildung des Trägersystems, vorzugsweise in partikulärer Form, bevorzugt in Form von Lipidnanopartikeln (LNP), zusammengelagert, insbesondere aggregiert sind und/oder wobei das Trägersystem vorzugsweise in partikulärer Form, bevorzugt in Form von Lipidnanopartikeln (LNP), vorliegt,

wobei die Bestandteile des Trägersystems die folgenden Substanzen umfassen:

(i) mindestens ein Phospholipid, vorzugsweise Phosphatidylcholin;
(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten (polyoxyethylierten) Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden (PEGylierten Fetten und PEGylierten Ölen), PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern, insbesondere Polyolestern von PEGylierten Fettsäuren;
(iii) ggf. mindestens ein Steroid und/oder Secosteroid, insbesondere mindestens ein Sterol (Sterin), vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol (Vitamin D);
(iv) ggf. mindestens ein kationisches Lipid, insbesondere aminbasiertes kationisches Lipid, vorzugsweise ein kationisches Lipid auf Basis eines tertiären Amins;
(v) ggf. mindestens einen Mono-, Di-, Tri- oder Polycarbonsäureester eines Polyols, insbesondere mindestens einen Mono-, Di- oder Tricarbonsäureester von Glycerin (Mono-, Di- oder Tricarbonsäureglycerid), vorzugsweise mindestens einen Mono-, Di- oder Trifettsäureester von Glycerin (Mono-, Di- oder Trifettsäureglycerid), bevorzugt mindestens einen C8- bis C16-Mono-, C8- bis C16-Di- oder C8- bis C16-Trifettsäureester von Glycerin, besonders bevorzugt mindestens einen C8- bis C14-Mono-, C8- bis C14-Di- oder C8- bis C14-Trifettsäureester von Glycerin;

wobei das Trägersystem durch mindestens eine(s) der nachfolgenden Merkmale und/oder Maßnahmen (1a) bis (5a), insbesondere durch eine Kombination von mindestens zwei, vorzugsweise mindestens drei, bevorzugt mindestens vier, besonders bevorzugt sämtlichen fünf, der nachfolgenden Merkmale und/oder Maßnahmen (1a) bis (5a), gekennzeichnet ist:

(1a) die Substanz (i) weist einen HLB-Wert nach Griffin von höchstens 11, insbesondere höchstens 8, insbesondere im Bereich von 0,5 bis 8, vorzugsweise im Bereich von 2 bis 7, bevorzugt im Bereich von 3 bis 6, auf und/oder die Substanz (i) weist eine einen Log P-Wert von mindestens 0,55, insbesondere mindestens 0,80, insbesondere im Bereich von 0,80 bis 18,97, vorzugsweise im Bereich von 0,93 bis 3,88, bevorzugt im Bereich von 1,11 bis 2,44, auf;

(2a) die Substanz (ii) weist einen HLB-Wert nach Griffin von mindestens 13, insbesondere im Bereich von 13 bis 19, vorzugsweise im Bereich von 13 bis 17,9, bevorzugt im Bereich von 13,5 bis 17,5, besonders bevorzugt im Bereich von 14 bis 17, auf und/oder die Substanz (ii) weist einen Log P-Wert von höchstens 0,46, insbesondere im Bereich von 0,32 bis 0,46, vorzugsweise im Bereich von 0,33 bis 0,44, bevorzugt im Bereich von 0,34 bis 0,42, auf;

(3a) die Substanz (iii) weist einen HLB-Wert nach Griffin von höchstens 1,5, insbesondere im Bereich von 0,5 bis 1,5, vorzugsweise im Bereich von 0,6 bis 1,4, bevorzugt im Bereich von 0,7 bis 1,3, auf und/oder die Substanz (iii) weist einen Log P-Wert von mindestens 5,40, insbesondere im Bereich von 5,40 bis 18,97, vorzugsweise im Bereich von 5,84 bis 15,40, bevorzugt im Bereich von 6,36 bis 12,91, auf;

(4a) die Substanz (iv) weist einen HLB-Wert nach Griffin von höchstens 4, insbesondere im Bereich von 1 bis 4, vorzugsweise im Bereich von 1,5 bis 3,5, bevorzugt im Bereich von 2 bis 3, auf und/oder die Substanz (iv) weist einen Log P-Wert von mindestens 1,76, insbesondere im Bereich von 1,76 bis 8,58, vorzugsweise im Bereich von 2,05 bis 5,40, bevorzugt im Bereich von 2,44 bis 3,88, auf;

(5a) die Substanz (v) weist einen HLB-Wert nach Griffin von höchstens 8, insbesondere im Bereich von 1 bis 8, vorzugsweise im Bereich von 1,25 bis 7, bevorzugt im Bereich von 1,5 bis 6,5, auf und/oder die Substanz (v) weist einen Log P-Wert von mindestens 0,80, insbesondere im Bereich von 0,80 bis 8,58, vorzugsweise im Bereich von 0,93 bis 6,65, bevorzugt im Bereich von 1,01 bis 5,40, auf;

insbesondere wobei das Trägersystem bzw. das mit dem pharmazeutischen Wirkstoff beladene und/oder ausgerüstete Trägersystem in einem flüssigen Medium, insbesondere in einem bei einer Temperatur im Bereich von 20 °C bis 40 °C, vorzugsweise Raumtemperatur (20 °C), und Umgebungsdruck (1.013,25 hPa) flüssigen Medium, vorzugsweise in Form eines Dispersionsmediums (Dispersionsmittels), enthalten ist und/oder vorliegt;

insbesondere wobei das Trägersystem mit dem flüssigen Medium eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, bildet und/oder insbesondere wobei das Trägersystem mit dem flüssigen Medium eine Dispersion des Trägersystems bzw. des mit einem pharmazeutischen Wirkstoff beladenen und/oder ausgerüsteten Trägersystems in dem flüssigen Medium bildet.

Erfindungsgemäß kann es sich insbesondere derart verhalten, dass das Trägersystem durch eine Kombination der Merkmale und/oder Maßnahmen (1a) und (2a), insbesondere durch eine Kombination der Merkmale und/oder Maßnahmen (1a), (2a) und (3a), vorzugsweise durch eine Kombination der Merkmale und/oder Maßnahmen (1a), (2a), (3a) und (4a) oder vorzugsweise durch eine Kombination der Merkmale und/oder Maßnahmen (1a), (2a), (3a) und (5a), bevorzugt durch eine Kombination der Merkmale und/oder Maßnahmen (1a), (2a), (3a), (4a) und (5a), gekennzeichnet ist.

Im Hinblick auf das Trägersystem nach der Erfindung kann es insbesondere vorgesehen sein, dass die Substanzen (i) und (iii) oder die Substanzen (i) und (iv) oder die Substanzen (i) und (v) einen mittleren HLB-Wert nach Griffin (jeweiliger arithmetischer Mittelwert der HLB-Werte nach Griffin der vorgenannten Substanzenkombinationen) im Bereich von 1 bis 10, insbesondere im Bereich von 2 bis 9, vorzugsweise im Bereich von 3 bis 8, aufweisen und/oder dass die Substanzen (i) und (iii) oder die Substanzen (i) und (iv) oder die Substanzen (i) und (v) einen mittleren Log P-Wert (jeweiliger arithmetischer Mittelwert der Log P-Werte der vorgenannten Substanzenkombinationen) im Bereich von 0,62 bis 8,58, insbesondere im Bereich von 0,70 bis 3,88, vorzugsweise im Bereich von 0,80 bis 2,44, aufweisen; und/oder dass die Substanzen (i), (iii) und (iv) oder die Substanzen (i), (iii) und (v) oder die Substanzen (i), (iv) und (v) einen mittleren HLB-Wert nach Griffin (jeweiliger arithmetischer Mittelwert der HLB-Werte nach Griffin der vorgenannten Substanzenkombinationen) im Bereich von 1 bis 10, insbesondere im Bereich von 2 bis 9, vorzugsweise im Bereich von 3 bis 8, aufweisen und/oder dass die Substanzen (i), (iii) und (iv) oder die Substanzen (i), (iii) und (v) oder die Substanzen (i), (iv) und (v) einen mittleren Log P-Wert (jeweiliger arithmetischer Mittelwert der Log P-Werte der vorgenannten Substanzenkombinationen) der vorgenannten Substanzenkombinationen im Bereich von 0,62 bis 8,58, insbesondere im Bereich von 0,70 bis 3,88, vorzugsweise im Bereich von 0,80 bis 2,44, aufweisen; und/oder dass die Substanzen (i), (iii), (iv) und (v) einen mittleren HLB-Wert nach Griffin (jeweiliger arithmetischer Mittelwert der HLB-Werte nach Griffin der vorgenannten Substanzenkombinationen) im Bereich von 1 bis 10, insbesondere im Bereich von 2 bis 9, vorzugsweise im Bereich von 3 bis 8, aufweisen und/oder dass die Substanzen (i), (iii), (iv) und (v) einen mittleren Log P-Wert (jeweiliger arithmetischer Mittelwert der Log P-Werte der vorgenannten Substanzenkombinationen) der vorgenannten Substanzenkombinationen) im Bereich von 0,62 bis 8,58, insbesondere im Bereich von 0,70 bis 3,88, vorzugsweise im Bereich von 0,80 bis 2,44, aufweisen.

Gleichermaßen kann es vorgesehen sein, dass die Substanz (i) einen kritischen Packungsparameter $P_{kr}$ von mindestens 0,5, insbesondere mindestens 0,7, insbesondere im Bereich von 0,7 bis 1,225, vorzugsweise im Bereich von 0,77 bis 1,12, bevorzugt im Bereich von 0,84 bis 1,05, aufweist; und/oder

dass die Substanz (ii) einen kritischen Packungsparameter $P_{kr}$ von höchstens 0,35, insbesondere im Bereich von 0,007 bis 0,35, vorzugsweise im Bereich von 0,035 bis 0,315, bevorzugt im Bereich von 0,07 bis 0,28, aufweist; und/oder

dass die Substanz (iii) einen kritischen Packungsparameter $P_{kr}$ von mindestens 1,155, insbesondere im Bereich von 1,155 bis 1,225, vorzugsweise im Bereich von 1,162 bis 1,218, bevorzugt im Bereich von 1,169 bis 1,211, aufweist;

und/oder

dass die Substanz (iv) einen kritischen Packungsparameter $P_{kr}$ von mindestens 0,98, insbesondere im Bereich von 0,98 bis 1,19, vorzugsweise im Bereich von 1,015 bis 1,155, bevorzugt im Bereich von 1,05 bis 1,12, aufweist; und/oder

dass die Substanz (v) einen kritischen Packungsparameter $P_{kr}$ von mindestens 0,7, insbesondere im Bereich von 0,7 bis 1,19, vorzugsweise im Bereich von 0,77 bis 1,1725, bevorzugt im Bereich von 0,805 bis 1,155, aufweist.

Insbesondere kann das Trägersystem durch mindestens eine(s) der nachfolgenden Merkmale und/oder Maßnahmen (1b) bis (5b), insbesondere durch eine Kombination von mindestens zwei, vorzugsweise mindestens drei, bevorzugt mindestens vier, besonders bevorzugt sämtlichen fünf, der nachfolgenden Merkmale und/oder Maßnahmen (1b) bis (5b), gekennzeichnet sein:

(1b) die Substanz (i) weist einen kritischen Packungsparameter $P_{kr}$ von mindestens 0,5, insbesondere mindestens 0,7, insbesondere im Bereich von 0,7 bis 1,225, vorzugsweise im Bereich von 0,77 bis 1,12, bevorzugt im Bereich von 0,84 bis 1,05, auf;

(2b) die Substanz (ii) weist einen kritischen Packungsparameter Pkr von höchstens 0,35, insbesondere im Bereich von 0,007 bis 0,35, vorzugsweise im Bereich von 0,035 bis 0,315, bevorzugt im Bereich von 0,07 bis 0,28, auf;

(3b) wobei die Substanz (iii) weist einen kritischen Packungsparameter Pkr von mindestens 1,155, insbesondere im Bereich von 1,155 bis 1,225, vorzugsweise im Bereich von 1,162 bis 1,218, bevorzugt im Bereich von 1,169 bis 1,211, auf;

(4b) die Substanz (iv) weist einen kritischen Packungsparameter Pkr von mindestens 0,98, insbesondere im Bereich von 0,98 bis 1,19, vorzugsweise im Bereich von 1,015 bis 1,155, bevorzugt im Bereich von 1,05 bis 1,12, auf;

(5b) die Substanz (v) weist einen kritischen Packungsparameter Pkr von mindestens 0,7, insbesondere im Bereich von 0,7 bis 1,19, vorzugsweise im Bereich von 0,77 bis 1,1725, bevorzugt im Bereich von 0,805 bis 1,155, auf.

Insbesondere kann das Trägersystem durch eine Kombination der Merkmale und/oder Maßnahmen (1b) und (2b), insbesondere durch eine Kombination der Merkmale und/oder Maßnahmen (1b), (2b) und (3b), vorzugsweise durch eine Kombination der Merkmale und/oder Maßnahmen (1b), (2b), (3b) und (4b) oder vorzugsweise durch eine Kombination der Merkmale und/oder Maßnahmen (1b), (2b), (3b) und (5b), bevorzugt durch eine Kombination der Merkmale und/oder Maßnahmen (1b), (2b), (3b), (4b) und (5b), gekennzeichnet sein.

Insbesondere kann es vorgesehen sein, dass das Trägersystem die Substanz (i) in einer Menge im Bereich von 0,5 Gew.-% bis 50 Gew.-%, insbesondere im Bereich von 1 Gew.-% bis 40 Gew.-%, vorzugsweise im Bereich von 1,25 Gew.-% bis 30 Gew.-%, bevorzugt im Bereich von 1,5 Gew.-% bis 25 Gew.-%, bezogen auf das Trägersystem und/oder bezogen auf die Gesamtheit der Bestandteile des Trägersystems, aufweist; und/oder

dass die Substanz (i) in einer Menge eingesetzt ist derart, dass das Trägersystem die Substanz (i) in einer Menge im Bereich von 0,1 Gew.-% bis 50 Gew.-%, insbesondere im Bereich von 0,25 Gew.-% bis 40 Gew.-%, vorzugsweise im Bereich von 0,5 Gew.-% bis 30 Gew.-%, bevorzugt im Bereich von 1 Gew.-% bis 25 Gew.-%, bezogen auf das Trägersystem und/oder bezogen auf die Gesamtheit der Bestandteile des Trägersystems, aufweist.

Für das Trägersystem kann es sich gleichermaßen insbesondere derart verhalten, dass das Trägersystem die Substanz (ii) in einer Menge im Bereich von 1 Gew.-% bis 80 Gew.-%, insbesondere im Bereich von 2 Gew.-% bis 75 Gew.-%, vorzugsweise im Bereich von 3 Gew.-% bis 70 Gew.-%, bevorzugt im Bereich von 5 Gew.-% bis 65 Gew.-%, bezogen auf das Trägersystem und/oder bezogen auf die Gesamtheit der Bestandteile des Trägersystems, aufweist; und/oder

dass die Substanz (ii) in einer Menge eingesetzt ist derart, dass das Trägersystem die Substanz (ii) in einer Menge im Bereich von 1 Gew.-% bis 80 Gew.-%, insbesondere im Bereich von 2 Gew.-% bis 75 Gew.-%, vorzugsweise im Bereich von 3 Gew.-% bis 70 Gew.-%, bevorzugt im Bereich von 5 Gew.-% bis 65 Gew.-%, bezogen auf das Trägersystem und/oder bezogen auf die Gesamtheit der Bestandteile des Trägersystems, aufweist.

Zudem kann es vorgesehen sein, dass das Trägersystem die Substanz (iii) in einer Menge im Bereich von 0,2 Gew.-% bis 40 Gew.-%, insbesondere im Bereich von 0,5 Gew.-% bis 25 Gew.-%, vorzugsweise im Bereich von 1 Gew.-% bis 20 Gew.-%, bevorzugt im Bereich von 3 Gew.-% bis 15 Gew.-%, bezogen auf das Trägersystem und/oder bezogen auf die Gesamtheit der Bestandteile des Trägersystems, aufweist; und/oder

dass die Substanz (iii) in einer Menge eingesetzt ist derart, dass das Trägersystem die Substanz (iii) in einer Menge im Bereich von 0,2 Gew.-% bis 40 Gew.-%, insbesondere im Bereich von 0,5 Gew.-% bis 25 Gew.-%, vorzugsweise im Bereich von 1 Gew.-% bis 20 Gew.-%, bevorzugt im Bereich von 3 Gew.-% bis 15 Gew.-%, bezogen auf das Trägersystem und/oder bezogen auf die Gesamtheit der Bestandteile des Trägersystems, aufweist.

Weiterhin kann es vorgesehen sein, dass das Trägersystem die Substanz (iv) in einer Menge im Bereich von 1 Gew.-% bis 80 Gew.-%, insbesondere im Bereich von 2 Gew.-% bis 75 Gew.-%, vorzugsweise im Bereich von 3 Gew.-% bis 70 Gew.-%, bevorzugt im Bereich von 5 Gew.-% bis 65 Gew.-%, bezogen auf das Trägersystem und/oder bezogen auf die Gesamtheit der Bestandteile des Trägersystems, aufweist; und/oder

dass die Substanz (iv) in einer Menge eingesetzt ist derart, dass das Trägersystem die Substanz (iv) in einer Menge im Bereich von 1 Gew.-% bis 80 Gew.-%, insbesondere im Bereich von 2 Gew.-% bis 75 Gew.-%, vorzugsweise im Bereich von 3 Gew.-% bis 70 Gew.-%, bevorzugt im Bereich von 5 Gew.-% bis 65 Gew.-%, bezogen auf das Trägersystem und/oder bezogen auf die Gesamtheit der Bestandteile des Trägersystems, aufweist.

Zudem kann es vorgesehen sein, dass das Trägersystem die Substanz (v) in einer Menge im Bereich von 2 Gew.-% bis 85 Gew.-%, insbesondere im Bereich von 4 Gew.-% bis 80 Gew.-%, vorzugsweise im Bereich von 6 Gew.-% bis 75 Gew.-%, bevorzugt im Bereich von 8 Gew.-% bis 70 Gew.-%, bezogen auf das Trägersystem und/oder bezogen auf die Gesamtheit der Bestandteile des Trägersystems, aufweist; und/oder

dass die Substanz (v) in einer Menge eingesetzt ist derart, dass das Trägersystem die Substanz (v) in einer Menge im Bereich von 2 Gew.-% bis 85 Gew.-%, insbesondere im Bereich von 4 Gew.-% bis 80 Gew.-%, vorzugsweise im Bereich von 6 Gew.-% bis 75 Gew.-%, bevorzugt im Bereich von 8 Gew.-% bis 70 Gew.-%, bezogen auf das Trägersystem und/oder bezogen auf die Gesamtheit der Bestandteile des Trägersystems, aufweist.

Weiterführend kann es auch vorgesehen sein, dass das gewichtsbezogene Mengenverhältnis von Substanz (i) zu Substanz (ii) [Substanz (i) : Substanz (ii)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, liegt und/oder auf die vorgenannten Werte eingestellt ist; und/oder

dass die Substanzen (i) und (ii) in einer Menge eingesetzt sind derart, dass das Trägersystem die Substanzen (i) und (ii) in einem gewichtsbezogenen Mengenverhältnis von Substanz (i) zu Substanz (ii) [Substanz (i) : Substanz (ii)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, aufweist.

Zudem verhält es sich insbesondere derart, dass das gewichtsbezogene Mengenverhältnis von Substanz (i) zu Substanz (iii) [Substanz (i) : Substanz (iii)] im Bereich von 250 : 1 bis 1 : 80, insbesondere im Bereich von 80 : 1 bis 1 : 25, vorzugsweise im Bereich von 30 : 1 bis 1 : 16, bevorzugt im Bereich von 8 : 1 bis 1 : 10, liegt und/oder auf die vorgenannten Werte eingestellt ist; und/oder

dass die Substanzen (i) und (iii) in einer Menge eingesetzt sind derart, dass das Trägersystem die Substanzen (i) und (iii) in einem gewichtsbezogenen Mengenverhältnis von Substanz (i) zu Substanz (iii) [Substanz (i) : Substanz (iii)] im Bereich von 250 : 1 bis 1 : 80, insbesondere im Bereich von 80 : 1 bis 1 : 25, vorzugsweise im Bereich von 30 : 1 bis 1 : 16, bevorzugt im Bereich von 8 : 1 bis 1 : 10, aufweist.

Insbesondere kann es vorgesehen sein, dass das gewichtsbezogene Mengenverhältnis von Substanz (i) zu Substanz (iv) [Substanz (i) : Substanz (iv)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, liegt und/oder auf die vorgenannten Werte eingestellt ist; und/oder

dass die Substanzen (i) und (iv) in einer Menge eingesetzt sind derart, dass das Trägersystem die Substanzen (i) und (iv) in einem gewichtsbezogenen Mengenverhältnis von Substanz (i) zu Substanz (iv) [Substanz (i) : Substanz (iv)] im Bereich von 50 : 1 bis 1 : 160, insbesondere im Bereich von 20 : 1 bis 1 : 75, vorzugsweise im Bereich von 10 : 1 bis 1 : 56, bevorzugt im Bereich von 5 : 1 bis 1 : 43, aufweist.

Insbesondere kann es vorgesehen sein, dass das gewichtsbezogene Mengenverhältnis von Substanz (i) zu Substanz (v) [Substanz (i) : Substanz (v)] im Bereich von 25 : 1 bis 1 : 170, insbesondere im Bereich von 10 : 1 bis 1 : 80, vorzugsweise im Bereich von 5 : 1 bis 1 : 60, bevorzugt im Bereich von 4 : 1 bis 1 : 46, liegt und/oder auf die vorgenannten Werte eingestellt ist; und/oder

dass die Substanzen (i) und (v) in einer Menge eingesetzt sind derart, dass das Trägersystem die Substanzen (i) und (v) in einem gewichtsbezogenen Mengenverhältnis von Substanz (i) zu Substanz (ii) [Substanz (i) : Substanz (v)] im Bereich von 25 : 1 bis 1 : 170, insbesondere im Bereich von 10 : 1 bis 1 : 80, vorzugsweise im Bereich von 5 : 1 bis 1 : 60, bevorzugt im Bereich von 4 : 1 bis 1 : 46, aufweist.

Erfindungsgemäß kann es auch vorgesehen sein, dass das gewichtsbezogene Mengenverhältnis von Substanz (i) zu Substanz (ii) zu Substanz (iii) zu Substanz (iv) [Substanz (i) : Substanz (ii) : Substanz (iii) : Substanz (iv)] im Bereich von (0,5 bis 50) : (1 bis 80) : (0,2 bis 40) : (1 bis 80), insbesondere im Bereich von (1 bis 40) : (2 bis 75) : (0,5 bis 25) : (2 bis 75), vorzugsweise im Bereich von (1,25 bis 30) : (3 bis 70) :(1 bis 20) : (3 bis 70), bevorzugt im Bereich von (1,5 bis 25) : (5 bis 65) : (3 bis 15) : (5 bis 65), liegt und/oder auf die vorgenannten Werte eingestellt ist; und/oder

dass die Substanzen (i), (ii), (iii) und (iv) in einer Menge eingesetzt sind derart, dass das Trägersystem die Substanzen (i), (ii), (iii) und (iv) in einem gewichtsbezogenen Mengenverhältnis von Substanz (i) zu Substanz (ii) zu Substanz (iii) zu Substanz (iv) [Substanz (i) : Substanz (ii) : Substanz (iii) : Substanz (iv)] im Bereich von (0,5 bis 50) : (1 bis 80) : (0,2 bis 40) : (1 bis 80), insbesondere im Bereich von (1 bis 40) : (2 bis 75) : (0,5 bis 25) : (2 bis 75), vorzugsweise im Bereich von (1,25 bis 30) : (3 bis 70) :(1 bis 20) : (3 bis 70), bevorzugt im Bereich von (1,5 bis 25) : (5 bis 65) : (3 bis 15) : (5 bis 65), aufweist.

Zudem kann es im Rahmen der vorliegenden Erfindung auch vorgesehen sein, dass das gewichtsbezogene Mengenverhältnis von Substanz (i) zu Substanz (ii) zu Substanz (iii) zu Substanz (v) [Substanz (i) : Substanz (ii) : Substanz (iii) : Substanz (v)] im Bereich von (0,5 bis 50) : (1 bis 80) : (0,2 bis 40) : (2 bis 85), insbesondere im Bereich von (1 bis 40) : (2 bis 75) : (0,5 bis 25) : (4 bis 80), vorzugsweise im Bereich von (1,25 bis 30) : (3 bis 70) :(1 bis 20) : (6 bis 75), bevorzugt im Bereich von (1,5 bis 25) : (5 bis 65) : (3 bis 15) : (8 bis 70), liegt und/oder auf die vorgenannten Werte eingestellt ist; und/oder dass die Substanzen (i), (ii), (iii) und (v) in einer Menge eingesetzt sind derart, dass das Trägersystem die Substanzen (i), (ii), (iii) und (v) in einem gewichtsbezogenen Mengenverhältnis von Substanz (i) zu Substanz (ii) zu Substanz (iii) zu Substanz (v) [Substanz (i) : Substanz (ii) : Substanz (iii) : Substanz (v)] im Bereich von (0,5 bis 50) : (1 bis 80) : (0,2 bis 40) : (2 bis 85), insbesondere im Bereich von (1 bis 40) : (2 bis 75) : (0,5 bis 25) : (4 bis 80), vorzugsweise im Bereich von (1,25 bis 30) : (3 bis 70) : (1 bis 20) : (6 bis 75), bevorzugt im Bereich von (1,5 bis 25) : (5 bis 65) : (3 bis 15) : (8 bis 70), aufweist.

Zudem kann es aber auch vorgesehen sein, dass das gewichtsbezogene Mengenverhältnis von Substanz (i) zu Substanz (ii) zu Substanz (iii) zu Substanz (iv) zu Substanz (v) [Substanz (i) : Substanz (ii) : Substanz (iii) : Substanz (iv) : Substanz (v)] im Bereich von (0,5 bis 50) : (1 bis 80) : (0,2 bis 40) : (1 bis 80) : (2 bis 85), insbesondere (1 bis 40) : (2 bis 75) : (0,5 bis 25) : (2 bis 75) : (4 bis 80), vorzugsweise (1,25 bis 30) : (3 bis 70) :(1 bis 20) : (3 bis 70) : (6 bis 75), bevorzugt (1,5 bis 25) : (5 bis 65) : (3 bis 15) : (5 bis 65) : (8 bis 70), liegt und/oder auf die vorgenannten Werte eingestellt ist; und/oder dass die Substanzen (i), (ii), (iii) und (iv) in einer Menge eingesetzt sind derart, dass das Trägersystem die Substanzen (i), (ii), (iii) und (iv) in einem gewichtsbezogenen Mengenverhältnis von Substanz (i) zu Substanz (ii) zu Substanz (iii) zu Substanz (iv) zu Substanz (v) [Substanz (i) : Substanz (ii) : Substanz (iii) : Substanz (iv)] im Bereich von (0,5 bis 50) : (1 bis 80) : (0,2 bis 40) : (1 bis 80), insbesondere (1 bis 40) : (2 bis 75) : (0,5 bis 25) : (2 bis 75), vorzugsweise (1,25 bis 30) : (3 bis 70) :(1 bis 20) : (3 bis 70), bevorzugt (1,5 bis 25) : (5 bis 65) : (3 bis 15) : (5 bis 65), aufweist.

Insbesondere können die Substanz (ii) und die hydrophoben Substanzen, insbesondere die hydrophoben amphiphilen Substanzen, vorzugsweise ausgewählt aus der Gruppe von Substanz (i), Substanz (iii), Substanz (iv), Substanz (v) und deren Kombinationen, in einer Menge eingesetzt sein derart, dass das Trägersystem die Substanzen in einem gewichtsbezogenen Mengenverhältnis von Substanz (ii) zu den hydrophoben Substanzen, insbesondere hydrophoben amphiphilen Substanzen, vorzugsweise ausgewählt aus der Gruppe von Substanz (i), Substanz (iii), Substanz (iv), Substanz (v) und deren Kombinationen, vorzugsweise zu Substanz (i) und/oder Substanz (iii) und ggf. Substanz (iv) und ggf. Substanz (v) [Substanz (ii) : hydrophobe Substanzen] im Bereich von 10 : 1 bis 1 : 20, vorzugsweise im Bereich von 5 : 1 bis 1 : 15, bevorzugt im Bereich von 2 : 1 bis 1 : 10, aufweist.

Insbesondere kann die Substanz (i) 1,2-Distearoyl-sn-glycero-3-phosphocholin sein oder umfassen.

Zudem kann es erfindungsgemäß vorgesehen sein, dass die Substanz (ii) ein PEG-Stearat, insbesondere PEG 40-Stearat, und/oder ein Macrogol-Stearat, insbesondere Macrogol 40-Stearat, ist und/oder umfasst; und/oder wobei die Substanz (ii) PEG 2000-DMG (1,2-Dimyristoyl-rac-glycero-3-methoxypolyethyleneglykol-2000) ist und/oder umfasst; und/oder wobei die Substanz (ii) ein Cetromacrogol (Polyethylenglycolhexadecylether) ist und/oder umfasst; und/oder wobei die Substanz (ii) 2-[(Polyethylenglycol)-2000]-N,N-ditetradecylacetamid (ALC-0159) ist und/oder umfasst; und/oder wobei die Substanz (ii) ein Polaxamer ist und/oder umfasst; und/oder wobei die Substanz (ii) ein Alkylpolyglykosid, insbesondere $C_8$- bis $C_{14}$-Alkylpolyglykosid, vorzugsweise $C_{10}$- bis $C_{12}$-Alkylpolyglykosid, ist oder umfasst; und/oder wobei die Substanz (ii) ein ethoxylierter Sorbitanfettsäureester, insbesondere Polysorbat, vorzugsweise Polysorbat 80 und/oder Polysorbat 20, ist oder umfasst; und/oder

dass die Substanz (iii) Cholesterol oder Phytosterol oder deren Mischungen, insbesondere Phytosterol, ist oder umfasst; und/oder

dass die Substanz (iv) [(4-Hydroxybutyl)azandiyl]-bis(hexan-6,1-diyl)-bis(2-hexyldecanoat) (ALC-0315) ist oder umfasst; und/oder wobei die Substanz (iv) Heptadecan-9-yl-8-((2-hydroxyethyl)(6-oxo-6-(undecyloxy)-hexyl)amino)octanoat (SM-102) ist oder umfasst; und/oder

dass die Substanz (v) Glycerinmonolaurat ist oder umfasst; und/oder wobei die Substanz (v) Caprinsäure/Caprylsäure-Triglycerid ist oder umfasst.

Insbesondere kann die Substanz (iv) bzw. das kationische Lipid mindestens eine insbesondere quaternierbare tertiäre aminofunktionelle Gruppe aufweisen.

Erfindungsgemäß kann der Wirkstoff, insbesondere als Substanz (vi), Bestandteil des Trägersystems sein und/oder einen Bestandteil des Trägersystems ausbilden und/oder wobei das Trägersystem den Wirkstoff, insbesondere als Substanz (vi), aufweist.

Insbesondere kann es erfindungsgemäß vorgesehen sein, dass der Wirkstoff ein therapeutischer und/oder prophylaktischer Wirkstoff, insbesondere therapeutischer und/oder prophylaktischer pharmazeutischer Wirkstoff, ist;

insbesondere wobei der Wirkstoff ein zumindest im Wesentlichen wasserunlöslicher, vorzugsweise ein zumindest im Wesentlichen wasserunlöslicher amphiphiler Wirkstoff, ist; und/oder insbesondere wobei der Wirkstoff ein wasserlöslicher Wirkstoff ist; undoder

insbesondere wobei der Wirkstoff ausgewählt ist aus der Gruppe von Vakzinwirkstoffen, antibiotischen Wirkstoffen, antimykotischen Wirkstoffen, antiviralen Wirkstoffen, antiphlogistischen Wirkstoffen, antipyretischen Wirkstoffen, antiallergischen Wirkstoffen, Antikrebswirkstoffen, Hormonwirkstoffen, antikoagulativen Wirkstoffen, Vitaminen, Mineralstoffen, UV-absorbierenden Stoffen und deren Mischungen sowie Kombinationen, vorzugsweise Vakzinwirkstoffen; und/oder

insbesondere wobei der Wirkstoff ausgewählt ist aus der Gruppe von RNA, insbesondere messenger-RNA (mRNA); Retinol; Tocopherol; Kortikosteroiden, insbesondere Kortison; Antibiotika, insbesondere Tetracyklinen und Benzoylpenicillinen; Diclofenac; Estradiol, Estradiol-Hemihydrat, Salicylsäure, Acetylsalicylsäure; Indometacin; ätherischen Ölen; insbesondere amphiphilen UVA-Filtern und UVB-Filter; Nicotinamid; und deren Mischungen sowie Kombinationen; und/oder

insbesondere wobei der Wirkstoff ein Vakzinwirkstoff, insbesondere ein Nukleinsäure-basierter Vakzinwirkstoff und/oder genbasierter Vakzinwirkstoff, vorzugsweise ein Antigen eines Erregers und/oder eines Pathogens, insbesondere ein Antigen eines Virus, codierender Vakzinwirkstoff ist; und/oder

insbesondere wobei der Vakzinwirkstoff ausgewählt ist aus der Gruppe von DNA; RNA, insbesondere messenger-RNA (mRNA); viralen Vektoren; Plasmiden, insbesondere Plasmid-DNA (pDNA); vorzugsweise RNA; bevorzugt mRNA; und deren Mischungen sowie Kombinationen; und/oder

insbesondere wobei der Wirkstoff ein mRNA-basierter Vakzinwirkstoff und/oder mRNA ist, insbesondere wobei die mRNA einzelsträngig ausgebildet ist und/oder insbesondere wobei die mRNA eine 5'-Cap-Struktur aufweist; und/oder

insbesondere wobei der Wirkstoff ein mRNA-basierter Vakzinwirkstoff und/oder mRNA ist, insbesondere wobei die mRNA ein Protein, insbesondere Antigen, eines Coronavirus, vorzugsweise SARS-CoV-2, codiert, insbesondere wobei das Protein, insbesondere Antigen, das insbesondere membranverankerte Spike-Protein (Spike-Glykoprotein bzw. S-Protein bzw. S-Glykoprotein) des Coronavirus, vorzugsweise SARS-CoV-2, zumindest teilweise, vorzugsweise zumindest im Wesentlichen vollständig, codiert; und/oder

insbesondere wobei der Wirkstoff ein Protein und/oder Antigen, insbesondere eines pathogenen Erregers, wie eines Bakteriums, Virus oder dergleichen, ist.

Erfindungsgemäß kann das Trägersystem den Wirkstoff, insbesondere als Substanz (vi), und/oder die Substanz (vi) in einer Menge im Bereich von 0,01 Gew.-% bis 80 Gew.-%, insbesondere im Bereich von 0,1 Gew.-% bis 70 Gew.-%, vorzugsweise im Bereich von 0,5 Gew.-% bis 60 Gew.-%, bevorzugt im Bereich von 1 Gew.-% bis 50 Gew.-%, bezogen auf das Trägersystem und/oder bezogen auf die Gesamtheit der Bestandteile des Trägersystems, aufweisen.

Erfindungsgemäß insbesondere mRNA als Wirkstoff eingesetzt werden. Dabei wird aufgrund der hervorragenden Eigenschaften des Trägersystems auch eine hohe Zellaufnahme bzw. Bioverfügbarkeit ermöglicht.

Insbesondere kann der Wirkstoff ein Vitamin-A bzw. Vitamin-A-Derivat, insbesondere Retinol bzw. Vitamin A$_1$, sein. Auch diesbezüglich wird eine hohe Wirkstoffaufnahme ermöglicht.

Zudem kann es erfindungsgemäß vorgesehen sein, dass das Trägersystem als weiteren Bestandteil, insbesondere als Substanz (vii), mindestens einen Fettalkohol, insbesondere C$_8$- bis C$_{25}$-Fettalkohol, vorzugsweise mindestens einen verzweigten Fettalkohol, bevorzugt Octyldodecanol, besonders bevorzugt 2-Octyldocen-1-ol, umfasst, insbesondere in einer Menge im Bereich von 1 Gew.-% bis 80 Gew.-%, vorzugsweise im Bereich von 5 Gew.-% bis 70 Gew.-%, bevorzugt im Bereich von 10 Gew.-% bis 60 Gew.-%, bezogen auf das Trägersystem und/oder bezogen auf die Gesamtheit der Bestandteile des Trägersystems;

insbesondere wobei der Fettalkohol, bevorzugt Octyldodecanol, einen HLB-Wert nach Griffin im Bereich von 1 bis 5, insbesondere im Bereich von 2 bis 4, aufweist und/oder insbesondere wobei der Fettalkohol, bevorzugt Octyldodecanol, einen Log P-Wert im Bereich von 1,36 bis 8,58, insbesondere im Bereich von 1,76 bis 3,88, aufweist; und/oder

insbesondere wobei der Fettalkohol, bevorzugt Octyldodecanol, einen kritischen Packungsparameter P$_{kr}$ im Bereich von 0,91 bis 1,19, insbesondere im Bereich von 0,98 bis 1,12, aufweist.

Im Allgemeinen kann das Trägersystem als weiteren Bestandteil, insbesondere als Substanz (viii), mindestens eine grenzflächenaktive amphiphile Substanz, insbesondere grenzflächenaktive und nichtmizellbildende amphiphile Substanz, vorzugsweise mindestens einen Sorbitanfettsäureester, bevorzugt mindestens einen Sorbitanfettsäureester mit bzw. auf Basis von gesättigten und/oder ungesättigten $C_8$-bis $C_{18}$-Fettsäuren, insbesondere $C_{10}$- bis $C_{14}$-Fettsäuren, besonders bevorzugt Sorbitancaprylat, umfassen, insbesondere in einer Menge im Bereich von 0,1 Gew.-% bis 30 Gew.-%, vorzugsweise im Bereich von 1 Gew.-% bis 20 Gew.-%, bevorzugt im Bereich von 5 Gew.-% bis 15 Gew.-%, bezogen auf das Trägersystem und/oder bezogen auf die Gesamtheit der Bestandteile des Trägersystems;

insbesondere wobei die grenzflächenaktive amphiphile Substanz, vorzugsweise der Sorbitanfettsäureester, einen HLB-Wert nach Griffin im Bereich von 2 bis 5, insbesondere im Bereich von 3 bis 4, aufweist und/oder insbesondere wobei die grenzflächenaktive amphiphile Substanz, vorzugsweise der Sorbitanfettsäureester, einen Log P-Wert im Bereich von 1,36 bis 3,88, insbesondere im Bereich von 1,76 bis 2,44, aufweist; und/oder

insbesondere wobei die grenzflächenaktive amphiphile Substanz, vorzugsweise der Sorbitanfettsäureester, einen kritischen Packungsparameter $P_{kr}$ im Bereich von 0,91 bis 1,12, insbesondere im Bereich von 0,98 bis 1,05, aufweist.

Zudem kann es sich derart verhalten, dass das Trägersystem als weiteren Bestandteil, insbesondere als Substanz (ix), mindestens einen Ether, insbesondere Ether eines Diols oder Polyols, vorzugsweise Ether eines bicyclischen Diols, bevorzugt Dialkylisosorbit, besonders bevorzugt Dimethylisosorbid (DMI), ganz besonders bevorzugt 2,5-Dimethylisosorbid, umfasst, insbesondere in einer Menge im Bereich von 2,5 Gew.-% bis 30 Gew.-%, vorzugsweise im Bereich von 5 Gew.-% bis 25 Gew.-%, bevorzugt im Bereich von 10 Gew.-% bis 20 Gew.-%, bezogen auf das Trägersystem und/oder bezogen auf die Gesamtheit der Bestandteile des Trägersystems.

Erfindungsgemäß kann es vorgesehen sein, dass das Trägersystem als weiteren Bestandteil, insbesondere als Substanz (x), mindestens einen Alkohol umfasst, insbesondere wobei der Alkohol ausgewählt ist aus der Gruppe von einwertigen Alkoholen, mehrwertigen Alkoholen, insbesondere Polyolen, und deren Mischungen sowie Kombinationen; und/oder wobei das Trägersystem als weiteren Bestandteil, insbesondere als Substanz (x), mindestens ein Polyol umfasst;

insbesondere wobei der Alkohol, insbesondere das Polyol, ausgewählt ist aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen sowie Kombinationen; und/oder

insbesondere wobei der Alkohol, insbesondere das Polyol, ausgewählt ist aus der Gruppe von Propylenglykol, Polyethylenglykol, Sorbitol, Mannitol, Glycerin, Saccharose, Panthenol und deren Mischungen sowie Kombinationen; und/oder

insbesondere wobei der Alkohol, insbesondere das Polyol, Glycerin ist; und/oder

insbesondere wobei der Alkohol, insbesondere das Polyol, Panthenol ist; und/oder

insbesondere wobei der Alkohol, insbesondere das Polyol, in einer Menge im Bereich von 1 Gew.-% bis 90 Gew.-%, insbesondere im Bereich von 2 Gew.-% bis 70 Gew.-%, vorzugsweise im Bereich von 3 Gew.-% bis 60 Gew.-%, bevorzugt im Bereich von 5 Gew.-% bis 55 Gew.-%, bezogen auf das Trägersystem, insbesondere bezogen auf die Gesamtheit der Bestandteile des Trägersystems, vorliegt und/oder eingesetzt ist; und/oder

insbesondere wobei der Alkohol, insbesondere das Polyol, in einer Menge eingesetzt ist derart, dass das gewichtsbezogene Mengenverhältnis von Alkohol, insbesondere das Polyol, zu Trägersystem, insbesondere Gesamtheit der Bestandteile des Trägersystems, [Alkohol, insbesondere Polyol : Trägersystem, insbesondere Gesamtheit der Bestandteile des Trägersystems] im Bereich von 1 : 150 bis 10 : 1, insbesondere im Bereich von 1 : 100 bis 5 : 1, vorzugsweise im Bereich von 1 : 50 bis 1 : 1, bevorzugt im Bereich von 1 : 20 bis 1 : 5, liegt und/oder eingestellt ist.

Im Hinblick auf das Trägersystem kann das flüssige Medium als kontinuierliche Phase ausgebildet sein und/oder vorliegen.

In diesem Zusammenhang kann das flüssige Medium in einer 0,05fachen bis 10fachen, insbesondere 0,1fachen bis 5fachen, insbesondere 0,15fachen bis 4fachen, bevorzugt 0,2fachen bis 3fachen, Gewichtsmenge zu der Gewichtsmenge des Trägersystems, insbesondere zu der Gewichtsmenge der Gesamtheit der Bestandteile des Trägersystems, vorliegen bzw. eingesetzt sein.

Zudem kann das flüssige Medium in einer Menge eingesetzt sein derart, dass das gewichtsbezogene Mengenverhältnis von Trägersystem, insbesondere Gesamtheit der Bestandteile des Trägersystems, zu flüssigem Medium [Trägersystem, insbesondere Gesamtheit der Bestandteile des Trägersystems : flüssiges Medium] im Bereich von 1 : 20 bis 50 : 1, insbesondere im Bereich von 1 : 10 bis 20 : 1, vorzugsweise im Bereich von 1 : 5 bis 10 : 1, bevorzugt im Bereich von 1 : 4 bis 5 : 1, liegt und/oder eingestellt ist.

Erfindungsgemäß kann es insbesondere vorgesehen sein, dass das flüssige Medium Wasser und/oder mindestens einen Alkohol, vorzugsweise Wasser und mindestens einen Alkohol, umfasst;

insbesondere wobei der Alkohol ausgewählt ist aus der Gruppe von einwertigen Alkoholen, mehrwertigen Alkoholen, insbesondere Polyolen, und deren Mischungen sowie Kombinationen; und/oder

insbesondere wobei der Alkohol ein mehrwertiger Alkohol, insbesondere Polyol, ist.

Erfindungsgemäß kann es insbesondere vorgesehen sein, dass der Alkohol, insbesondere das Polyol, ausgewählt ist aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen sowie Kombinationen; und/oder

dass der Alkohol, insbesondere das Polyol, ausgewählt ist aus der Gruppe von Propylenglykol, Polyethylenglykol, Sorbitol, Mannitol, Glycerin, Saccharose, Panthenol und deren Mischungen sowie Kombinationen; und/oder

dass der Alkohol, insbesondere das Polyol, Glycerin ist; und/oder

dass der Alkohol, insbesondere das Polyol, Panthenol ist und/oder

dass der Alkohol kein $C_1$- bis $C_4$-Monoalkohol, vorzugsweise kein Ethanol, ist und/oder insbesondere wobei der Alkohol keinen $C_1$- bis $C_4$-Monoalkohol, insbesondere kein Ethanol, umfasst; und/oder

dass das flüssige Medium keinen $C_1$- bis $C_4$-Monoalkohol, insbesondere kein Ethanol, umfasst.

Zudem kann es erfindungsgemäß vorgesehen sein, dass das flüssige Medium das Wasser in einer Menge im Bereich von 10 Gew.-% bis 99,5 Gew.-%, insbesondere im Bereich von 20 Gew.-% bis 99 Gew.-%, vorzugsweise im Bereich von 30 Gew.-% bis 97 Gew.-%, bevorzugt im Bereich von 35 Gew.-% bis 95 Gew.-%, bezogen auf das flüssige Medium, enthält; und/oder

dass das flüssige Medium den Alkohol, insbesondere das Polyol, in einer Menge im Bereich von 0,5 Gew.-% bis 90 Gew.-%, insbesondere im Bereich von 1 Gew.-% bis 80 Gew.-%, vorzugsweise im Bereich von 3 Gew.-% bis 70 Gew.-%, bevorzugt im Bereich von 5 Gew.-% bis 65 Gew.-%, bezogen auf das flüssige Medium, enthält; und/oder

dass das flüssige Medium ein Gemisch (Mischung) von Wasser und mindestens einem Alkohol, insbesondere Polyol, umfasst oder hieraus besteht, insbesondere wobei das gewichtsbezogene Mengenverhältnis von Wasser zu Alkohol, insbesondere Polyol, [Wasser : Alkohol, insbesondere Polyol] im Bereich von 200 : 1 bis 1 : 50, insbesondere im Bereich von 100 : 1 bis 1 : 20, vorzugsweise im Bereich von 50 : 1 bis 1 : 5, bevorzugt im Bereich von 20 : 1 bis 1 : 1, insbesondere bezogen auf das flüssige Medium, liegt und/oder eingestellt ist.

Darüber hinaus kann es sich in Bezug auf das Trägersystem nach der Erfindung auch derart verhalten, dass das flüssige Medium mindestens einen Ether, insbesondere Ether eines Diols oder Polyols, vorzugsweise Ether eines bicyclischen Diols, bevorzugt Dialkylisosorbit, besonders bevorzugt Dimethylisosorbid, ganz besonders bevorzugt 2,5-Dimethylisosorbid, umfasst, insbesondere in einer Menge im Bereich von 0,5 Gew.-% bis 25 Gew.-%, vorzugsweise im Bereich von 1 Gew.-% bis 10 Gew.-%, bevorzugt im Bereich von 1 Gew.-% bis 10 Gew.-%, bezogen auf das flüssige Medium.

Insbesondere kann das flüssige Medium mindestens ein Antioxidationsmittel, insbesondere Ascorbinsäure (Vitamin C) umfassen, insbesondere in einer Menge im Bereich von 0,1 Gew.-% bis 10 Gew.-%, vorzugsweise im Bereich von 0,2 Gew.-% bis 5 Gew.-%, bevorzugt im Bereich von 0,5 Gew.-% bis 2 Gew.-%, bezogen auf das flüssige Medium.

Weiterhin kann das flüssige Medium mindestens ein Biopolymer, insbesondere ausgewählt aus der Gruppe von Alginaten, Hyaluronsäure und Chitosan (sowie deren jeweiligen Salzen und Derivaten) sowie deren Mischungen und Kombinationen, aufweisen, insbesondere in einer Menge im Bereich von 0,1 Gew.-% bis 50 Gew.-%, insbesondere im Bereich von 0,5 Gew.-% bis 30 Gew.-%, vorzugsweise im Bereich von 1 Gew.-% bis 10 Gew.-%, bezogen auf das flüssige Medium.

Erfindungsgemäß ist es vorgesehen, dass das Trägersystem und/oder das flüssige Medium und/oder die Zusammensetzung, wie zuvor definiert, zumindest im Wesentlichen frei von Ethanol ist bzw. sind.

Zudem kann es vorgesehen sein, dass das Trägersystem bzw. das flüssige Medium bzw. die Zusammensetzung, insbesondere wie zuvor definiert, zumindest im Wesentlichen frei von halogenierten Kohlenwasserstoffen, insbesondere zumindest im Wesentlichen frei von chlorierten Kohlenwasserstoffen, vorzugsweise zumindest im Wesentlichen frei von Chloroform, ist bzw. sind.

Im Allgemeinen verhält es sich auch in Bezug auf das Trägersystem derart, dass die Zusammenlagerung, insbesondere Aggregation, der Bestandteile derart ist, dass das Trägersystem in partikulärer Form, insbesondere in Form von Lipidnanopartikeln (LNP), bevorzugt Lipidnanopartikeln (LNP) in Form von Mizellen, Vesikeln und/oder diskförmigen (scheibenförmigen) Lipidnanopartikeln (LNP) (Disk-Lipidnanopartikeln bzw. Disk-LNP), bevorzugt diskförmigen (scheibenförmigen) Lipidnanopartikeln (LNP), resultiert; und/oder

dass das Trägersystem in partikulärer Form, insbesondere in Form von Lipidnanopartikeln (LNP), bevorzugt Lipidnanopartikeln (LNP) in Form von Mizellen, Vesikeln und/oder diskförmigen (scheibenförmigen) Lipidnanopartikeln (LNP) (Disk-Lipidnanopartikeln bzw. Disk-LNP), bevorzugt diskförmigen (scheibenförmigen) Lipidnanopartikeln (LNP), vorliegt.

Im Allgemeinen können die Partikelgrößen (absolut), insbesondere die hydrodynamischen Durchmesser (absolut), der Partikel des Trägersystems, bevorzugt Lipidnanopartikel (LNP), zumindest im Wesentlichen eine Normalverteilung und/oder Gaußverteilung aufweisen, insbesondere wobei die Partikelgrößen, insbesondere die hydrodynamischen Durchmesser, mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09, bestimmt werden.

Erfindungsgemäß verhält es sich zudem insbesondere derart, dass die Partikel des Trägersystems, bevorzugt die Lipidnanopartikel (LNP), eine Partikelgröße (absolut), insbesondere einen hydrodynamischen Durchmesser (absolut), im Bereich von 5 nm bis 10.000 nm, insbesondere im Bereich von 10 nm bis 5.000 nm, vorzugsweise im Bereich von 20 nm bis 3.000 nm, bevorzugt im Bereich von 50 nm bis 2.000 nm, besonders bevorzugt im Bereich von 75 nm bis 1.500 nm, ganz besonders bevorzugt im Bereich von 90 nm bis 1.250 nm, weiter bevorzugt im Bereich von 100 nm bis 1.000 nm, nochmals weiter bevorzugt im Bereich von 100 nm bis 900 nm, aufweisen, insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09.

Zudem kann es sich derart verhalten, dass die Partikel des Trägersystems, bevorzugt die Lipidnanopartikel (LNP), eine mittlere Partikelgröße, insbesondere einen mittleren hydrodynamischen Durchmesser, im Bereich von 5 nm bis 2.900 nm, insbesondere im Bereich von 10 nm bis 2.500 nm, vorzugsweise im Bereich von 20 nm bis 2.000 nm, bevorzugt im Bereich von 50 nm bis 1.500 nm, besonders bevorzugt im Bereich von 50 nm bis 1.000 nm, aufweisen, insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09.

Zudem kann es vorgesehen sein, dass die Varianz der Partikelgröße, insbesondere die Varianz des hydrodynamischen Durchmessers, der Partikel des Trägersystems, bevorzugt der Lipidnanopartikel (LNP), höchstens 45 %, insbesondere höchstens 35 %, vorzugsweise höchstens 25 %, bevorzugt höchstens 20 %, besonders bevorzugt höchstens 15 %, ganz besonders bevorzugt höchstens 10 %, beträgt, insbesondere bezogen auf die mittlere Partikelgöße bzw. den mittleren hydrodynamischen Durchmesser und/oder insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09; und/oder

dass die Varianz der Partikelgröße, insbesondere die Varianz des hydrodynamischen Durchmessers, der Partikel des Trägersystems, bevorzugt der Lipidnanopartikel (LNP), im Bereich von 5 % bis 45 %, insbesondere im Bereich von 10 % bis 40 %, vorzugsweise im Bereich von 15 % bis 35 %, bevorzugt im Bereich von 20 % bis 35 %, liegt, insbesondere bezogen auf die mittlere Partikelgöße bzw. den mittleren hydrodynamischen Durchmesser und/oder insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09.

Weiterhin kann es auch vorgesehen sein, dass die Partikel des Trägersystems, bevorzugt die Lipidnanopartikel (LNP), eine mittlere Partikelgröße D50, insbesondere einen mittleren hydrodynamischen Durchmesser D50, im Bereich von 2,5 nm bis 950 nm, insbesondere im Bereich von 7,5 nm bis 850 nm, vorzugsweise im Bereich von 10 nm bis 800 nm, bevorzugt im Bereich von 15 nm bis 750 nm, aufweisen, insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09; und/oder dass die Partikel des Trägersystems, bevorzugt die Lipidnanopartikel (LNP), eine mittlere Partikelgröße D90, insbesondere einen mittleren hydrodynamischen Durchmesser D90, im Bereich von 1 nm bis 1.000 nm, insbesondere im Bereich von 5 nm bis 900 nm, vorzugsweise im Bereich von 7,5 nm bis 850 nm, bevorzugt im Bereich von 10 nm bis 800 nm, aufweisen, insbesondere bestimmt mittels Lichtbeugung (Lichtdiffraktometrie), insbesondere Laserdiffraktometrie, vorzugsweise Dynamischer Lichtstreuung (DLS), bevorzugt gemäß der ISO 22412:2017 und/oder DIN ISO 22412:2018-09.

Erfindungsgemäß kann es sich zudem derart verhalten, dass die Zusammenlagerung, insbesondere Aggregation, der Bestandteile derart ist, dass eine insbesondere lyotrope flüssig-kristalline Struktur auf Basis des Trägersystems und des flüssigen Mediums resultiert; und/oder

dass das Trägersystem zusammen mit dem flüssigen Medium als lyotrope flüssig-kristalline Struktur auf Basis des Trägersystems und des flüssigen Mediums vorliegt und/oder diese Struktur aufweist.

Insbesondere kann es erfindungsgemäß vorgesehen sein, dass das Trägersystem in einem flüssigen Medium, insbesondere wie zuvor definiert, enthalten und/oder eingebracht ist, insbesondere dass das Trägersystem mit dem flüssigen Medium eine Zusammensetzung, insbesondere pharmazeutische Zusammensetzung, bildet; und/oder insbesondere dass die Zusammensetzung eine vorzugweise disperse Zusammensetzung, insbesondere eine Dispersion, vorzugsweise wie zuvor definiert, ist, welche das Trägersystem bzw. das mit dem pharmazeutischen Wirkstoff beladene und/oder ausgerüstete Trägersystem und das flüssige Medium umfasst, und/oder wobei die Zusammensetzung, insbesondere wie zuvor definiert, in Form einer Dispersion des Trägersystems bzw. des mit einem pharmazeutischen Wirkstoff beladenen und/oder ausgerüsteten Trägersystems in dem flüssigen Medium vorliegt.

Insbesondere kann es erfindungsgemäß vorgesehen sein, dass die Zusammensetzung das Trägersystem, vorzugsweise die Partikel, bevorzugt die Lipidnanopartikel, in einer Menge im Bereich von 5 Gew.-% bis 95 Gew.-%, insbesondere im Bereich von 10 Gew.-% bis 90 Gew.-%, vorzugsweise im Bereich von 15 Gew.-% bis 85 Gew.-%, bevorzugt im Bereich von 20 Gew.-% bis 80 Gew.-%, besonders bevorzugt im Bereich von 25 Gew.-% bis 75 Gew.-%, bezogen auf die Zusammensetzung, aufweist; und/oder

dass das Trägersystem in der Zusammensetzung in partikulärer Form, insbesondere in Form von Lipidnanopartikeln (LNP), bevorzugt Lipidnanopartikeln (LNP) in Form von Mizellen, Vesikeln und/oder diskförmigen (scheibenförmigen) Lipidnanopartikeln (LNP) (Disk-Lipidnanopartikeln bzw. Disk-LNP), bevorzugt diskförmigen (scheibenförmigen) Lipidnanopartikeln (LNP), insbesondere wie zuvor definiert, vorliegt.

In Bezug auf die Zusammensetzung kann es sich zudem derart verhalten, dass die Zusammensetzung eine Wasseraktivität von weniger als 1, insbesondere höchstens 0,9, vorzugsweise höchstens 0,7, bevorzugt höchstens 0,5, besonders bevorzugt höchstens 0,3, aufweist; und/oder

dass die Zusammensetzung eine Wasseraktivität im Bereich von 0 bis 1, insbesondere im Bereich von 0 bis 0,9, vorzugsweise im Bereich von 0 bis 0,7, bevorzugt im Bereich von 0 bis 0,5, besonders bevorzugt im Bereich von 0 bis 0,3, aufweist;

insbesondere bestimmt gemäß ISO 21807:2004 und/oder gemäß ISO 18787:2017 und/oder 21 CFR Part 11, insbesondere wie in der ISO 29621:2017 hinterlegt.

Insbesondere kann das Trägersystem und/oder die Zusammensetzung, insbesondere das Trägersystem, einen Log P-Wert, insbesondere Gesamt-Log P-Wert, im Bereich von 0,1 bis 5, insbesondere im Bereich von 0,1 bis 5, insbesondere im Bereich von 0,2 bis 4, bevorzugt im Bereich von 0,3 bis 3, besonders bevorzugt im Bereich von 0,4 bis 2, aufweisen. Insbesondere kann es zudem vorgesehen sein, dass die Bestandteile und/oder Substanzen des Trägersystems, insbesondere die Substanzen (i), (ii), (iii), (iv) und/oder (v), mit der Maßgabe ausgewählt sind, dass das resultierende Trägersystem einen Log P-Wert, insbesondere Gesamt-Log P-Wert, von höchstens 5, insbesondere höchstens 4, bevorzugt höchstens 3, besonders bevorzugt höchstens 2, weiter bevorzugt höchstens 1, aufweist. Weiterhin kann es vorgesehen sein, dass die Bestandteile und/oder Substanzen des Trägersystems, insbesondere die Substanzen (i), (ii), (iii), (iv) und/oder (v), mit der Maßgabe ausgewählt sind, dass das resultierende Trägersystem einen Log P-Wert, insbesondere Gesamt-Log P-Wert, im Bereich von 0,1 bis 5, insbesondere im Bereich von 0,2 bis 4, bevorzugt im Bereich von 0,3 bis 3, besonders bevorzugt im Bereich von 0,4 bis 2, aufweist.

Darüber hinaus ist Gegenstand der vorliegenden Erfindung - gemäß einem **weiteren** Aspekt der vorliegenden Erfindung - auch die erfindungsgemäße Zusammensetzung, insbesondere pharmazeutische Zusammensetzung, vorzugsweise Arzneimittel oder Medikament, wobei die Zusammensetzung mindestens ein vorzugsweise lipidbasiertes Trägersystem, vorzugsweise in partikulärer Form, bevorzugt in Form von Lipidnanopartikeln (LNP), insbesondere für einen pharmazeutischen Wirkstoff; und/oder mindesten ein mit einem pharmazeutischen Wirkstoff beladenes und/oder ausgerüstetes, vorzugsweise lipidbasiertes Trägersystem, vorzugsweise in partikulärer Form, bevorzugt in Form von Lipidnanopartikeln (LNP), nach einem der Ansprüche 82 bis 129 in einem flüssigen Medium, insbesondere in einem bei einer Temperatur im Bereich von 20 °C bis 40 °C, vorzugsweise Raumtemperatur (20°C), und Umgebungsdruck (1.013,25 hPa) flüssigen Medium, vorzugsweise in Form eines Dispersionsmediums (Dispersionsmittels), enthält.

Insbesondere ist die Zusammensetzung eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst. Insbesondere liegt die Zusammensetzung nach der Erfindung in Form einer Dispersion des Trägersystems bzw. des mit einem pharmazeutischen Wirkstoff beladenen und/oder ausgerüsteten Trägersystems in dem flüssigen Medium vor.

Die Zusammensetzung kann das Trägersystem, vorzugsweise die Partikel, bevorzugt die Lipidnanopartikel, in einer Menge im Bereich von 5 Gew.-% bis 95 Gew.-%, insbesondere im Bereich von 10 Gew.-% bis 90 Gew.-%,

vorzugsweise im Bereich von 15 Gew.-% bis 85 Gew.-%, bevorzugt im Bereich von 20 Gew.-% bis 80 Gew.-%, besonders bevorzugt im Bereich von 25 Gew.-% bis 75 Gew.-%, bezogen auf die Zusammensetzung, aufweisen.

Zudem kann das Trägersystem in der Zusammensetzung in partikulärer Form, insbesondere in Form von Lipidnanopartikeln (LNP), bevorzugt Lipidnanopartikeln (LNP) in Form von Mizellen, Vesikeln und/oder diskförmigen (scheibenförmigen) Lipidnanopartikeln (LNP) (Disk-Lipidnanopartikeln bzw. Disk-LNP), bevorzugt diskförmigen (scheibenförmigen) Lipidnanopartikeln (LNP), insbesondere wie zuvor definiert, vorliegen.

Insbesondere kann die Zusammensetzung eine Wasseraktivität von weniger als 1, insbesondere höchstens 0,9, vorzugsweise höchstens 0,7, bevorzugt höchstens 0,5, besonders bevorzugt höchstens 0,3, aufweisen. Insbesondere kann die Zusammensetzung eine Wasseraktivität im Bereich von 0 bis 1, insbesondere im Bereich von 0 bis 0,9, vorzugsweise im Bereich von 0 bis 0,7, bevorzugt im Bereich von 0 bis 0,5, besonders bevorzugt im Bereich von 0 bis 0,3, aufweisen. Die Wasseraktivität kann insbesondere bestimmt werden gemäß ISO 21807:2004 und/oder gemäß ISO 18787:2017 und/oder 21 CFR Part 11, insbesondere wie in der ISO 29621:2017 hinterlegt.

Im Allgemeinen kann der Wirkstoff Bestandteil des Trägersystems sein bzw. einen Bestandteil des Trägersystems ausbilden. Insbesondere kann das Trägersystem den Wirkstoff aufweisen bzw. enthalten.

Erfindungsgemäß kann im Hinblick auf die Zusammensetzung nach der Erfindung der Wirkstoff ein therapeutischer und/oder prophylaktischer Wirkstoff, insbesondere therapeutischer und/oder prophylaktischer pharmazeutischer Wirkstoff, sein,

insbesondere wobei der Wirkstoff ein zumindest im Wesentlichen wasserunlöslicher, vorzugsweise ein zumindest im Wesentlichen wasserunlöslicher amphiphiler Wirkstoff, ist; und/oder insbesondere wobei der Wirkstoff ein wasserlöslicher Wirkstoff ist; und/oder

insbesondere wobei der Wirkstoff ausgewählt wird aus der Gruppe von Vakzinwirkstoffen (Impfwirkstoffen), antibiotischen Wirkstoffen, antimykotischen Wirkstoffen, antiviralen Wirkstoffen, antiphlogistischen Wirkstoffen, antipyretischen Wirkstoffen, antiallergischen Wirkstoffen, Antikrebswirkstoffen, Hormonwirkstoffen, antikoagulativen Wirkstoffen, Antitumorwirkstoffen (wie Zytostatika), Nahrungsergänzungsstoffen, Vitaminen, Mineralstoffen, UV-absorbierenden Stoffen und deren Mischungen sowie Kombinationen, vorzugsweise Vakzinwirkstoffen (Impfwirkstoffen); und/oder

insbesondere wobei der Wirkstoff ausgewählt wird aus der Gruppe von RNA, insbesondere messenger-RNA (mRNA); Retinol; Tocopherol; Kortikosteroiden, insbesondere Kortison; Antibiotika, insbesondere Tetracyklinen und Benzoylpenicillinen; Diclofenac; Estradiol, Estradiol-Hemihydrat, Salicylsäure, Acetylsalicylsäure; Indometacin; ätherischen Ölen; insbesondere amphiphilen UVA-Filtern und UVB-Filter; Nicotinamid; und deren Mischungen sowie Kombinationen; und/oder

insbesondere wobei der Wirkstoff ein Vakzinwirkstoff (Impfwirkstoff), insbesondere ein Nukleinsäure-basierter Vakzinwirkstoff und/oder genbasierter Vakzinwirkstoff, vorzugsweise ein Antigen eines Erregers und/oder eines Pathogens, insbesondere ein Antigen eines Virus, codierender Vakzinwirkstoff ist; und/oder

insbesondere wobei der Vakzinwirkstoff ausgewählt wird aus der Gruppe von DNA; RNA, insbesondere messenger-RNA (mRNA); viralen Vektoren; Plasmiden, insbesondere Plasmid-DNA (pDNA); vorzugsweise RNA; bevorzugt mRNA; und deren Mischungen sowie Kombinationen; und/oder

insbesondere wobei der Wirkstoff ein mRNA-basierter Vakzinwirkstoff und/oder mRNA ist, insbesondere wobei die mRNA einzelsträngig ausgebildet ist und/oder insbesondere wobei die mRNA eine 5'-Cap-Struktur aufweist; und/oder

insbesondere wobei der Wirkstoff ein mRNA-basierter Vakzinwirkstoff und/oder mRNA ist, insbesondere wobei die mRNA ein Protein, insbesondere Antigen, eines Coronavirus, vorzugsweise SARS-CoV-2, codiert, insbesondere wobei das Protein, insbesondere Antigen, das insbesondere membranverankerte Spike-Protein (*Spike*-Glykoprotein bzw. S-Protein bzw. S-Glykoprotein) des Coronavirus, vorzugsweise SARS-CoV-2, zumindest teilweise, vorzugsweise zumindest im Wesentlichen vollständig, codiert; und/oder

insbesondere wobei der Wirkstoff ein Protein und/oder Antigen, insbesondere eines pathogenen Erregers, wie eines Bakteriums, Virus oder dergleichen, ist.

In diesem Zusammenhang kann das Trägersystem den Wirkstoff in einer Menge im Bereich von 0,01 Gew.-% bis 80 Gew.-%, insbesondere im Bereich von 0,1 Gew.-% bis 70 Gew.-%, vorzugsweise im Bereich von 0,5 Gew.-% bis 60 Gew.-%, bevorzugt im Bereich von 1 Gew.-% bis 50 Gew.-%, bezogen auf das Trägersystem und/oder bezogen auf die Gesamtheit der Bestandteile des Trägersystems, aufweisen.

Zudem kann die Zusammensetzung den Wirkstoff in einer Menge im Bereich von 0,001 Gew.-% bis 75 Gew.-%, insbesondere im Bereich von 0,01 Gew.-% bis 65 Gew.-%, vorzugsweise im Bereich von 0,1 Gew.-% bis 55 Gew.-%, bevorzugt im Bereich von 0,5 Gew.-% bis 50 Gew.-%, bezogen auf die Zusammensetzung, aufweisen.

Weiterhin kann das flüssige Medium als kontinuierliche Phase ausgebildet sein bzw. vorliegen.

Erfindungsgemäß kann es insbesondere vorgesehen sein, dass das flüssige Medium in einer 0,05fachen bis 10fachen, insbesondere 0,1fachen bis 5fachen, insbesondere 0,15fachen bis 4fachen, bevorzugt 0,2fachen bis 3fachen, Gewichtsmenge zu der Gewichtsmenge des Trägersystems, insbesondere zu der Gewichtsmenge der Gesamtheit der Bestandteile des Trägersystems, vorliegt bzw. eingesetzt ist.

Diesbezüglich kann das flüssige Medium in einer Menge eingesetzt werden derart, dass das gewichtsbezogene Mengenverhältnis von Trägersystem, insbesondere Gesamtheit der Bestandteile des Trägersystems, zu flüssigem Medium [Trägersystem, insbesondere Gesamtheit der Bestandteile des Trägersystems : flüssiges Medium] im Bereich von 1 : 20 bis 50 : 1, insbesondere im Bereich von 1 : 10 bis 20 : 1, vorzugsweise im Bereich von 1 : 5 bis 10 : 1, bevorzugt im Bereich von 1 : 4 bis 5 : 1, liegt und/oder eingestellt ist.

Im Hinblick auf die erfindungsgemäße Zusammensetzung kann das flüssige Medium Wasser bzw. mindestens einen Alkohol, vorzugsweise Wasser und mindestens einen Alkohol, umfassen.

In diesem Zusammenhang kann der Alkohol ausgewählt sein aus der Gruppe von einwertigen Alkoholen, mehrwertigen Alkoholen, insbesondere Polyolen, und deren Mischungen sowie Kombinationen.

In erfindungsgemäß bevorzugter Weise ist der Alkohol ein mehrwertiger Alkohol, insbesondere Polyol.

Diesbezüglich kann es sich erfindungsgemäß insbesondere derart verhalten, dass der Alkohol, insbesondere das Polyol, ausgewählt ist aus der Gruppe von Alkylenglykolen, insbesondere Propylenglykol; Polyalkylenglykolen, insbesondere Polyethylenglykol; Zuckeralkoholen, insbesondere Sorbitol, Mannitol und Glycerin; Sacchariden, insbesondere Disacchariden, vorzugsweise Saccharose; Panthenol; und deren Mischungen sowie Kombinationen.

In erfindungsgemäß bevorzugter Weise ist der Alkohol, insbesondere das Polyol, ausgewählt aus der Gruppe von Propylenglykol, Polyethylenglykol, Sorbitol, Mannitol, Glycerin, Saccharose, Panthenol und deren Mischungen sowie Kombinationen.

Erfindungsgemäß kann es vorgesehen sein, dass Alkohol, insbesondere das Polyol, Glycerin ist.

Erfindungsgemäß kann es insbesondere vorgesehen sein, dass der Alkohol, insbesondere das Polyol, Panthenol ist. Hierzu kann auch auch die zuvor beschriebenen Vorteile und Eigenschaften von Panthenol verwiesen werden.

Auch im Hinblick auf das flüssige Medium der Zusammensetzung gilt insbesondere, dass der Alkohol kein $C_1$- bis $C_4$-Monoalkohol, vorzugsweise kein Ethanol, ist bzw. dass der Alkohol keinen $C_1$- bis $C_4$-Monoalkohol, insbesondere kein Ethanol, umfasst.

Insbesondere ist es erfindungsgemäß vorgesehen, dass das flüssige Medium keinen $C_1$- bis $C_4$-Monoalkohol, insbesondere kein Ethanol, umfasst.

Im Allgemeinen kann das flüssige Medium das Wasser in einer Menge im Bereich von 10 Gew.-% bis 99,5 Gew.-%, insbesondere im Bereich von 20 Gew.-% bis 99 Gew.-%, vorzugsweise im Bereich von 30 Gew.-% bis 97 Gew.-%, bevorzugt im Bereich von 35 Gew.-% bis 95 Gew.-%, bezogen auf das flüssige Medium, enthalten.

Zudem kann das flüssige Medium den Alkohol, insbesondere das Polyol, in einer Menge im Bereich von 0,5 Gew.-% bis 90 Gew.-%, insbesondere im Bereich von 1 Gew.-% bis 80 Gew.-%, vorzugsweise im Bereich von 3 Gew.-% bis 70 Gew.-%, bevorzugt im Bereich von 5 Gew.-% bis 65 Gew.-%, bezogen auf das flüssige Medium, enthalten.

Weiterhin kann das flüssige Medium ein Gemisch (Mischung) von Wasser und mindestens einem Alkohol, insbesondere Polyol, umfassen oder hieraus bestehen, insbesondere wobei das gewichtsbezogene Mengenverhältnis von Wasser zu Alkohol, insbesondere Polyol, [Wasser : Alkohol, insbesondere Polyol] im Bereich von 200 : 1 bis 1 : 50, insbesondere im Bereich von 100 : 1 bis 1 : 20, vorzugsweise im Bereich von 50 : 1 bis 1 : 5, bevorzugt im Bereich von 20 : 1 bis 1 : 1, insbesondere bezogen auf das flüssige Medium, liegt und/oder eingestellt ist.

Zudem kann das flüssige Medium einen Ether, insbesondere Ether eines Diols oder Polyols, vorzugsweise Ether eines bicyclischen Diols, bevorzugt Dialkylisosorbit, besonders bevorzugt Dimethylisosorbid, ganz besonders bevorzugt 2,5-Dimethylisosorbid, umfassen, insbesondere in einer Menge im Bereich von 0,5 Gew.-% bis 25 Gew.-%, vorzugsweise im Bereich von 1 Gew.-% bis 10 Gew.-%, bezogen auf das flüssige Medium.

Zudem kann das flüssige Medium mindestens ein Antioxidationsmittel, insbesondere Ascorbinsäure (Vitamin C) umfassen, insbesondere in einer Menge im Bereich von 0,1 Gew.-% bis 10 Gew.-%, vorzugsweise im Bereich von 0,2 Gew.-% bis 5 Gew.-%, bevorzugt im Bereich von 0,5 Gew.-% bis 2 Gew.-%, bezogen auf das flüssige Medium.

Weiterhin kann das flüssige Medium mindestens ein Biopolymer, insbesondere ausgewählt aus der Gruppe von Alginaten, Hyaluronsäure und Chitosan (sowie deren jeweiligen Salzen und Derivaten) sowie deren Mischungen und Kombinationen, aufweisen, insbesondere in einer Menge im Bereich von 0,1 Gew.-% bis 50 Gew.-%, insbesondere im Bereich von 0,5 Gew.-% bis 30 Gew.-%, vorzugsweise im Bereich von 1 Gew.-% bis 10 Gew.-%, bezogen auf das flüssige Medium.

Erfindungsgemäß verhält es sich insbesondere derart, dass das Trägersystem bzw. das flüssige Medium bzw. die Zusammensetzung, insbesondere wie zuvor definiert, zumindest im Wesentlichen frei von aliphatischen Monoalkoholen, insbesondere zumindest im Wesentlichen frei von aliphatischen $C_1$- bis $C_4$-Monoalkoholen, vorzugsweise zumindest im Wesentlichen frei von Ethanol, ist bzw. sind.

Zudem verhält es sich vorliegend insbesondere derart, dass das Trägersystem bzw. das flüssige Medium bzw. die Zusammensetzung, insbesondere wie zuvor definiert, zumindest im Wesentlichen frei von halogenierten Kohlenwasserstoffen, insbesondere zumindest im Wesentlichen frei von chlorierten Kohlenwasserstoffen. vorzugsweise zumindest im Wesentlichen frei von Chloroform, ist bzw. sind.

Die erfindungsgemäße Zusammensetzung zeichnet sich durch eine hohe (Lager-) Stabilität aus: So kann die Zusammensetzung in einem Temperaturbereich von im Allgemienen - 20 °C bis 45 °C, insbesondere in einem Temperaturbereich von - 15 °C bis 40 °C, bevorzugt in einem Temperaturbereich von - 15 °C bis 20 °C, lagerstabil sein, vorzugsweise über einen Lagerzeitraum von mindestens 4 Wochen, insbesondere mindestens 6 Wochen, vorzugsweise einhergehend mit sich über den Lagerzeitraum zumindest im Wesentlichen nicht verändernden Partikelgrößen. Die diesbezüfliche Lagerung kann insbesondere unter Umgebungsdruck (1.013,25 hPa) und/oder bei einer relativen Luftfeuchtigkeit der Umgebungsatmosphäre von etwa 60 % bestimmt werden. Die Zusammensetzung kann dabei in üblichen Bevoratungsvorrichtungen, wie Glasgefäßen bzw -ampullen, vorliegen.

Insbesondere ist es im Hinblick auf die erfindungsgemäße Zusammensetzung vorgesehen, dass das Trägersystem in partikulärer Form, insbesondere in Form von Lipidnanopartikeln (LNP), bevorzugt Lipidnanopartikeln (LNP) in Form von Mizellen, Vesikeln und/oder diskförmigen (scheibenförmigen) Lipidnanopartikeln (LNP) (Disk-Lipidnanopartikeln bzw. Disk-LNP), bevorzugt diskförmigen (scheibenförmigen) Lipidnanopartikeln (LNP), vorliegt.

Insbesondere kann das Trägersystem und/oder die Zusammensetzung, insbesondere das Trägersystem, einen Log P-Wert, insbesondere Gesamt-Log P-Wert, im Bereich von 0,1 bis 5, insbesondere im Bereich von 0,2 bis 4, bevorzugt im Bereich von 0,3 bis 3, besonders bevorzugt im Bereich von 0,4 bis 2, aufweisen. Insbesondere kann es zudem vorgesehen sein, dass die Bestandteile und/oder Substanzen des Trägersystems, insbesondere die Substanzen (i), (ii), (iii), (iv) und/oder (v), mit der Maßgabe ausgewählt sind, dass das resultierende Trägersystem einen Log P-Wert, insbesondere Gesamt-Log P-Wert, von höchstens 5, insbesondere höchstens 4, bevorzugt höchstens 3, besonders bevorzugt höchstens 2, weiter bevorzugt höchstens 1, aufweist. Weiterhin kann es vorgesehen sein, dass die Bestandteile und/oder Substanzen des Trägersystems, insbesondere die Substanzen (i), (ii), (iii), (iv) und/oder (v), mit der Maßgabe ausgewählt sind, dass das resultierende Trägersystem einen Log P-Wert, insbesondere Gesamt-Log P-Wert, im Bereich von 0,1 bis 5, insbesondere im Bereich von 0,2 bis 4, bevorzugt im Bereich von 0,3 bis 3, besonders bevorzugt im Bereich von 0,4 bis 2, aufweist.

Im Hinblick auf weitere Ausgestaltungen der erfindungsgemäßen Zusammensetzung gemäß dem vorliegenden Aspekt kann auf die Ausführungen zu den weiteren erfindungsgemäßen Aspekten verwiesen werden, welche vorliegend entsprechend gelten.

Weiterer Gegenstand der vorliegenden Erfindung - gemäß einem **weiteren** Aspekt der vorliegenden Erfindung - ist zudem das erfindungsgemäße Arzneimittel oder Medikament, insbesondere zur Verwendung bei der prophylaktischen und/oder therapeutischen Behandlung von Erkrankungen des menschlichen oder tierischen Körper,
wobei das Arzneimittel oder Medikament mindestens ein lipidbasiertes Trägersystem, wie zuvor definiert, oder eine Zusammensetzung, wie zuvor definiert, insbesondere zusammen mit mindestens einem pharmazeutisch akzeptablen und/oder pharmazeutisch verträglichen Exzipienten, aufweist oder hieraus besteht.

Gemäß dem vorliegenden Aspekt betrifft die vorliegende Erfindung auch ein lipidbasiertes Trägersystem, wie zuvor definiert, oder Zusammensetzung, wie zuvor definiert, zur Verwendung bei der prophylaktischen und/oder therapeutischen Behandlung von Erkrankungen des menschlichen oder tierischen Körpers.

Diesbezüglich können die Erkrankungen ausgewählt sein aus der Gruppe von bakteriellen, mykotischen oder viralen, vorzugsweise viralen, Erkrankungen, entzündlichen Erkrankungen, mit Fieber einhergehenden Erkrankungen bzw. Fieber, allergischen Erkrankungen, Krebserkrankungen, hormonell bedingten Erkrankungen, Blutgerinnungsstörungen, Krebserkrankungen, Mangelzuständen bzw. Stoffwechselerkrankungen, Hauterkrankungen und deren Kombinationen.

Insbesondere kann die Erkrankung eine virale Infektionserkrankung, insbesondere eine durch Coronaviren hervorgerufene und/oder hiermit in Zusammenhang stehende Erkrankung, insbesondere Infektionskrankheit, vorzugsweise eine durch SARS-CoV-2 hervorgerufene und/oder hiermit in Zusammenhang stehende Erkrankung, insbesondere Infektionskrankheit, bevorzugt COVID-19 *(coronavirus disease 2019),* sein.

Die vorliegende Erfindung betrifft gemäß dem vorliegenden Aspekt auch das zuvor definierte Arzneimittel bzw. Medikament bzw. das Trägersystem oder die Zusammensetzung zur Verwendung bei der prophylaktischen Behandlung von insbesondere viralen Erkrankung bzw. Infektionskrankheit, insbesondere einer durch Coronaviren hervorgerufenen und/oder hiermit in Zusammenhang stehenden Erkrankung bzw. Infektionskrankheit, vorzugsweise einer durch SARS-CoV-2 hervorgerufenen und/oder hiermit in Zusammenhang stehende Erkrankung und/oder Infektionskrankheit, bevorzugt COVID-19 *(coronavirus disease 2019),* insbesondere wobei das Arzneimittel bzw. Medikament oder das Trägersystem oder die Zusammensetzung als Impfstoff, vorzugsweise mRNA-Impfstoff, ausgebildet ist.

Das Arzneimittel bzw. Medikament oder das Trägersystem oder die Zusammensetzung nach der Erfindung kann weiterhin als Antikrebsmittel, insbesondere gegen Hautkrebs, vorzugsweise gegen weißen Hautkrebs, ausgebildet sein. Im Hinblick auf Hautkrebs kann insbesondere eine topische Anwendung bzw. Verabreichung in Betracht kommen bzw. vorliegen.

Gemäß dem vorliegenden Aspekt betrifft die vorliegende Erfindung darüber hinaus auch die Verwendung des zuvor definierten lipidbasierten Trägersystems nach der Erfindung oder die Verwendung der zuvor definierten Zusammensetzung nach der Erfindung (zur Herstellung eines Arzneimittels) oder die Verwendung des zuvor definierten Arzneimittels bzw. Medikaments

zur prophylaktischen und/oder therapeutischen Behandlung von Erkrankungen des menschlichen oder tierischen Körpers, wobei die Erkrankungen ausgewählt sind aus der Gruppe von bakteriellen, mykotischen oder viralen, vorzugsweise viralen, Erkrankungen, entzündlichen Erkrankungen, mit Fieber einhergehenden Erkrankungen bzw. Fieber, allergischen Erkrankungen, Krebserkrankungen, hormonell bedingten Erkrankungen, Blutgerinnungsstörungen, Krebserkrankungen, Mangelzuständen bzw. Stoffwechselerkrankungen, Hauterkrankungen und deren Kombinationen; und/oder wobei die Erkrankung eine virale Infektionserkrankung, insbesondere eine durch Coronaviren hervorgerufene und/oder hiermit in Zusammenhang stehende Erkrankung bzw. Infektionskrankheit, vorzugsweise eine durch SARS-CoV-2 hervorgerufene und/oder hiermit in Zusammenhang stehende Erkrankung bzw. Infektionskrankheit, bevorzugt COVID-19 (*coronavirus disease 2019*), ist, oder

zur prophylaktischen Behandlung von insbesondere viralen Erkrankungen bzw. Infektionskrankheiten, insbesondere einer durch Coronaviren hervorgerufenen und/oder hiermit in Zusammenhang stehenden Erkrankungen und/oder Infektionskrankheiten, vorzugsweise einer durch SARS-CoV-2 hervorgerufenen und/oder hiermit in Zusammenhang stehenden Erkrankung und/oder Infektionskrankheit, bevorzugt COVID-19 *(coronavirus disease 2019),* insbesondere wobei das Arzneimittel bzw. Medikament oder das Trägersystem oder die Zusammensetzung als Impfstoff, vorzugsweise mRNA-Impfstoff, ausgebildet ist.

Gemäß dem vorliegenden Aspekt betrifft die vorliegende Erfindung auch ein Verfahren zur Behandlung einer Erkrankung des menschlichen oder tierischen Körpers, umfassend den Schritt der Verabreichung einer wirksamen, insbesondere prophylaktisch und/oder therapeutisch wirksamen, Menge des zuvor definierten Trägersystems nach der Erfindung oder der zuvor definierten Zusammensetzung nach der Erfindung oder des zuvor definierten Arzneimittels bzw. Medikaments nach der Erfindung,
insbesondere wobei die Erkrankung ausgewählt ist aus der Gruppe von bakteriellen, mykotischen oder viralen, vorzugsweise viralen, Erkrankungen, entzündlichen Erkrankungen, mit Fieber einhergehenden Erkrankungen bzw. Fieber, allergischen Erkrankungen, Krebserkrankungen, hormonell bedingten Erkrankungen, Blutgerinnungsstörungen, Krebserkrankungen, Mangelzuständen bzw. Stoffwechselerkrankungen, Hauterkrankungen und deren Kombinationen; und/oder insbesondere zur Behandlung einer viralen Infektionserkrankung, insbesondere einer durch Coronaviren hervorgerufenen und/oder hiermit in Zusammenhang stehenden Erkrankung bzw. Infektionskrankheit, vorzugsweise einer durch SARS-CoV-2 hervorgerufenen und/oder hiermit in Zusammenhang stehenden Erkrankung bzw. Infektionskrankheit, bevorzugt COVID-19 *(coronavirus disease 2019).*

Im Rahmen der vorliegenden Erfindung kann das Arzneimittel bzw. Medikament oder Trägersystem oder die Zusammensetzung topisch oder systemisch, insbesondere systemisch, appliziert werden. Dementsprechend kann das Arzneimittel bzw. Medikament oder das Trägersystem oder die Zusammensetzung zur topischen oder systemischen, insbesondere systemischen, Applikation hergerichtet sein.

Insbesondere kann das Arzneimittel bzw. Medikament oder das Trägersystem oder die Zusammensetzung systemisch, insbesondere parenteral oder peroral, vorzugweise parenteral, wie intravenös, intraarteriell, intramuskulär, subkutan oder dergleichen, bevorzugt intramuskulär, appliziert werden. Diesbezüglich kann das Arzneimittel bzw. Medikament oder das Trägersystem oder die Zusammensetzung zur systemischen, insbesondere parenteralen oder peroralen, Applikation, vorzugweise parenteralen Applikation, wie intravenösen, intraarteriellen, intramuskulären, subkutanen Applikation oder dergleichen, bevorzugt intramuskulären Applikation, hergerichtet sein.

Insbesondere kann das Arzneimittel bzw. Medikament oder das Trägersystem oder die Zusammensetzung topisch, insbesondere kutan, appliziert werden. Diesbezüglich kann das Arzneimittel bzw. Medikament oder das Trägersystem oder die Zusammensetzung zur topischen, insbesondere kutanen, Applikation hergerichtet sein.

Darüber hinaus kann das Trägersystem oder die Zusammensetzung als Desinfektionsmittel, insbesondere Flächendesinfektionsmittel, eingesetzt werden, insbesondere unter Verwendung desinfizierend wirkender Inhalts- bzw. Wirkstoffen. Hierzu kann insbesondere auch Glycerinmonolaurat eingesetzt werden.

Zu weiteren Ausgestaltungen des erfindungsgemäßen Gegenstands gemäß diesem Aspekt kann auch auf die Ausführungen zu den weiteren erfindungsgemäßen Aspekten verwiesen werden, welche entsprechend gelten.

Weiterer Gegenstand der vorliegenden Erfindung - gemäß einem **weiteren** Aspekt der vorliegenden Erfindung - ist die Verwendung mindestens eines Prozessparameters zur Steuerung, insbesondere zur Verfahrens- und/oder Prozesskontrolle, des vorliegend definierten Verfahrens, wobei der Prozessparameter ausgewählt wird aus der Gruppe von Temperatur, Leitfähigkeit, Viskosität, pH-Wert, elektrischem Widerstand und Oberflächenspannung, insbesondere dynamischer Oberflächenspannung, vorzugsweise aus der Gruppe von Temperatur, Leitfähigkeit, Viskosität und pH-Wert, insbesondere jeweils des flüssigen Mediums, vorzugsweise mitsamt den darin eingebrachten Bestandteilen und/oder Substanzen, und/oder der Zusammensetzung, wie zuvor definiert.

Zu weiteren Ausgestaltungen des erfindungsgemäßen Gegenstands gemäß diesem Aspekt kann auch auf die Ausführungen zu den weiteren erfindungsgemäßen Aspekten verwiesen werden, welche entsprechend gelten

Weitere vorteilhafte Eigenschaften, Aspekte und Merkmale der vorliegenden Erfindung ergeben sich auch aus den angeführten Figuren und deren Beschreibung, wie sie auch nachfolgend noch im Detail angeführt wird.

In den Figurendarstellungen zeigt:

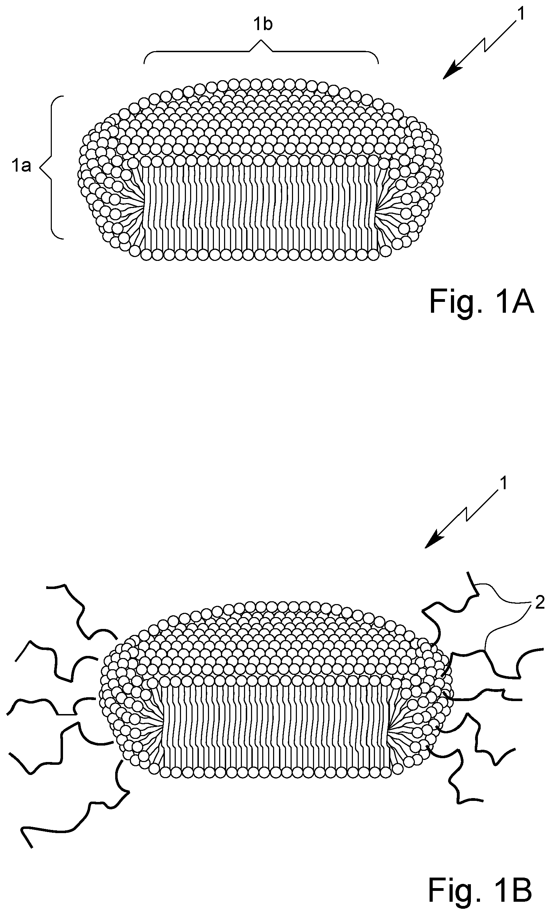

Fig. 1A      eine schematische und veranschaulichende Darstellung eines Aufbaus bzw. der Formgebung eines Partikels des erfindungsgemäßen Trägersystems in Form eines diskförmigen (scheibenförmigen) Lipidnanopartikels 1 (synonym auch als Disk-Lipidnanopartikel (Disk-LNP), Disk-Vesikel oder Disk-Lamelle bezeichnet); das diskförmige Lipidnanopartikel weist insbesondere eine zylinderartige bzw. -förmige bzw. scheibenförmige Geometrie mit einem Randbereich bzw. einer Mantelfläche 1a sowie insbesondere kreisartig bzw. insbesondere kreisförmig ausgebildeten Grundflächen 1b auf;

Fig. 1B      eine schematische und veranschaulichende Darstellung eines diskförmigen Lipidnanopartikels 1, wonach die Mantelfläche bzw. der Randbereich nichtionogene amphiphile bzw. tensidische Substanzen, insbesondere PEGylierte Substanzen bzw. PEGylierte Tenside 2 (vgl. Substanz (ii)), aufweist, während die Mantelflächen maßgeblich Phospholipide (vgl. Substanz (i)) aufweisen; hierbei ist auch der jeweils zugrundeliegende HLB-Wert nach Griffin bzw. der jeweilige kritische Packungsparameter für die Anordnung der Substanzen in dem diskförmigen Lipidnanopartikel von Relevanz, wonach nämlich Substanzen mit relativ großem HLB-Wert nach Griffin bzw. relativ kleinem kritischem Packungsparameter vorrangig im Randbereich bzw. im Bereich der Mantelfläche angeordnet werden, während Substanzen mit relativ kleinem HLB-Wert nach Griffin bzw. relativ großem kritischen Packungsparameter vorrangig im Bereich der Grundflächen angeordnet werden;

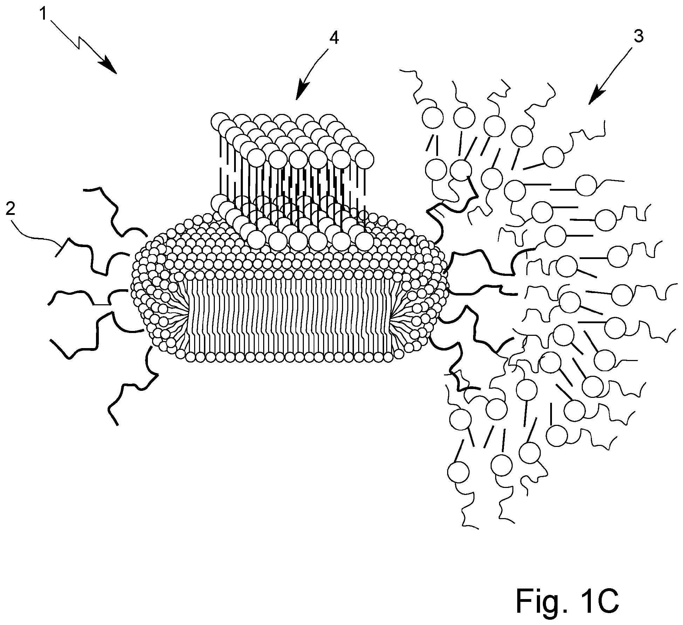

Fig. 1C      eine schematische und veranschaulichende Darstellung eines diskförmigen Lipidnanopartikels 1 des erfindungsgemäßen Trägersystems, welcher im Randbereich gleichermaßen die zuvor in Fig. 1B angeführten Substanzen 2 aufweist und wonach das Lipidnanopartikel mehrlagig bzw. multilamellar ausgebildet ist, und zwar insbesondere auf Basis weiterer Phospholipide 4, welche insbesondere parallel zu den Grundflächen der zugrundeliegenden diskförmigen Struktur angeordnet werden und weiteren Substanzen 3, welche im Mantelbereich angeordnet werden und insbesondere mit der Substanz 2 interagieren können; die Partikel gemäß Fig. 1A bis Fig. 1C sind insbesondere zu Zwecken der Veranschaulichung als perspektivische Schnittdarstellung aufgezeigt, wobei die jeweiligen Kreisdarstellungen die hydrophilen Bereiche und die jeweiligen Strich- bzw. Liniendarstellungen die lipophilen Bereiche der zugrundeliegenden Moleküle veranschaulichen;

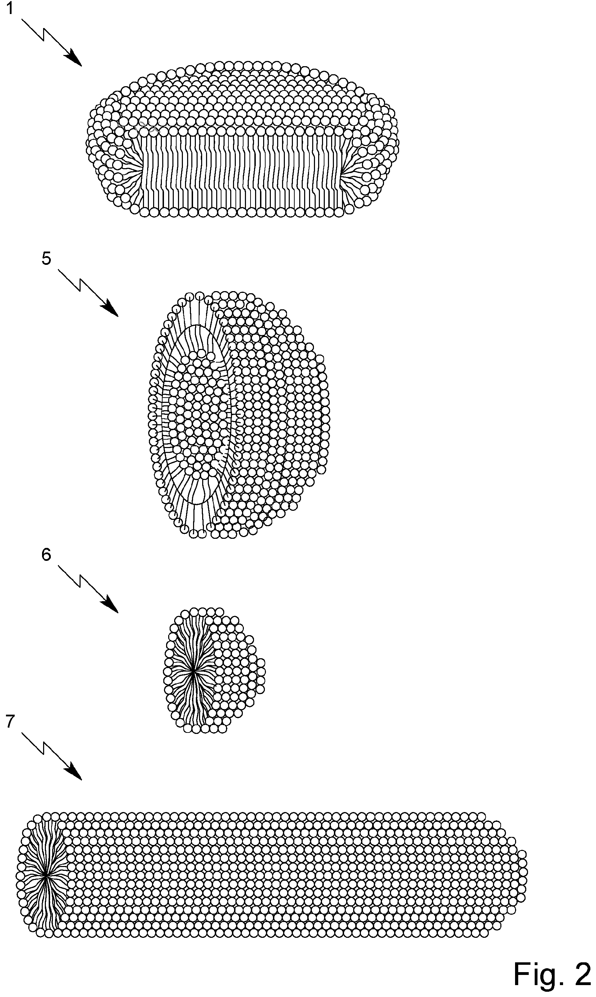

Fig. 2      eine schematische und veranschaulichende Darstellung der Ausbildung der Partikel des Trägersystems nach der Erfindung auf Basis jeweiliger Grundstrukturen in Form eines diskförmigen Lipidnanopartikels 1; Fig. 2 zeigt zudem weitere, wenn auch weniger bevorzugte Ausgestaltungen der Partikel, und zwar in Form eines Vesikels 5, einer Kugelmizelle 6 sowie einer Wurmmizelle 7; die Partikel 1, 5, 6 und 7 sind insbesondere in Form einer Schnittdarstellung veranschaulicht, wobei die jeweiligen Kreisdarstellungen und die jeweiligen Strich- bzw. Liniendarstellungen insbesondere den lipophilen Bereich der zugrundeliegenden Moleküle darstellen;

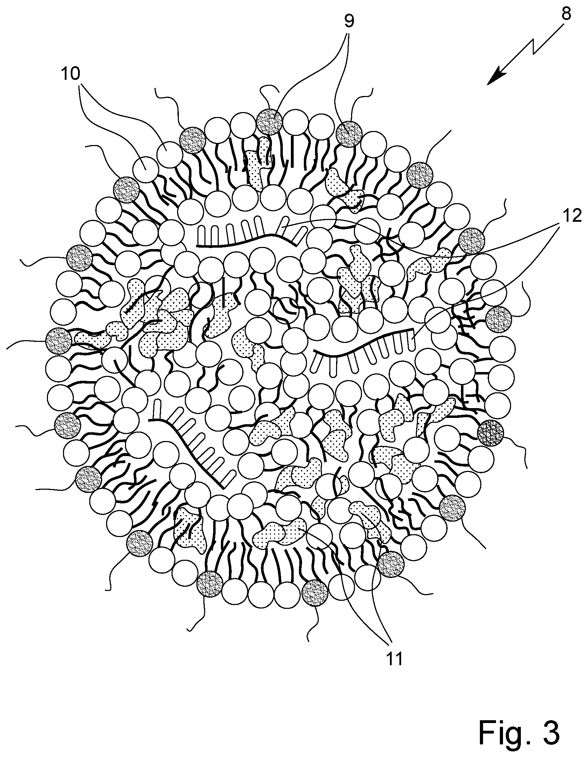

Fig. 3      eine schematische und veranschaulichende Darstellung einer weiteren partikulären Form bzw. eines Partikels 8 mit Bestandteilen auf Basis von PEGylierten Substanzen 9, Phospholipiden 10, Cholesterol 11 und Wirkstoff 12;

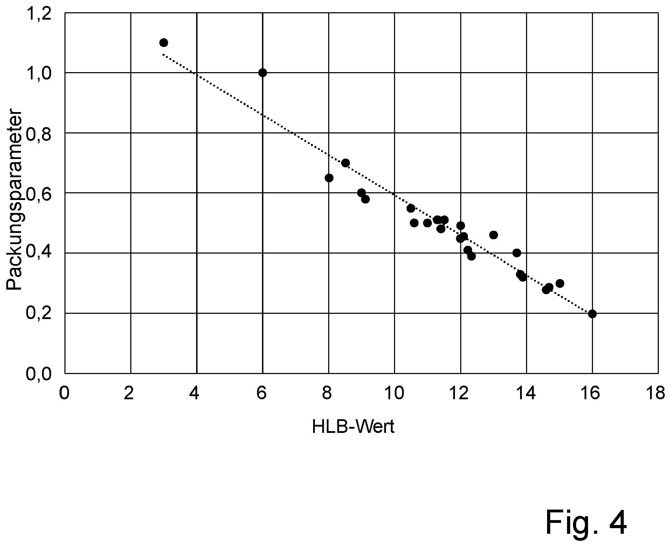

Fig. 4      eine grafische Darstellung in Form eines Diagramms zur Veranschaulichung der Beziehung zwischen Packungsparameter $P_{kr}$ einerseits und HLB-Wert nach Griffin andererseits;

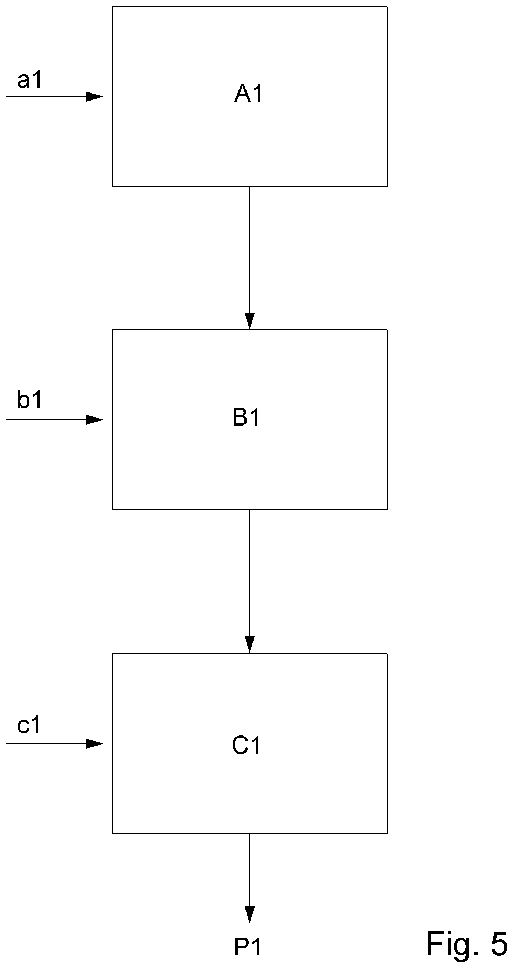

Fig. 5      eine schematische Darstellung eines erfindungsgemäßen Verfahrensablaufs auf Basis jeweiliger Ansätze bzw. einer aufgeteilten Zufuhr der Bestandteile bzw. Komponenten a1 bis c1 in jeweils zugeordnete Aufnahme- bzw. Verarbeitungsvorrischtungen A1 bis C1 im Rahmen einer kontinuierlichen bzw. kaskadierten und/oder konsekutiven Verfahrensführung zum Erhalt der Zusammensetzung ;

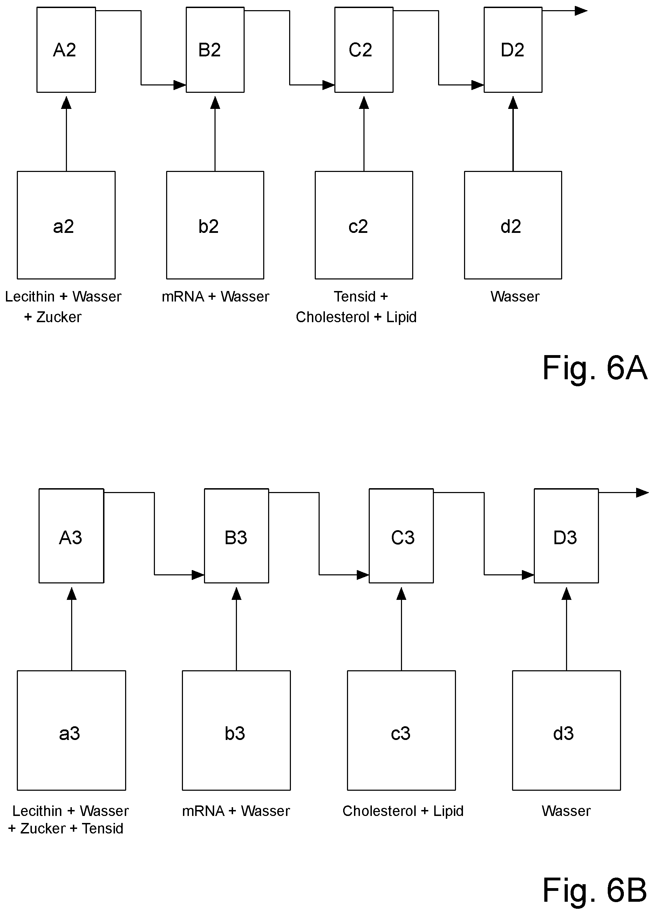

Fig. 6A eine schematische Darstellung eines erfindungsgemäßen Verfahrensablaufs mit einer beispielhaften Aufteilung der Substanzen des Trägersystems bzw. der Komponenten für die herzustellende Zusammensetzung zu Zwecken der Ausbildung entsprechender Ansätze bzw. ausgehend von jeweiligen Bevoratungsvorrichtungen a2 bis d2 sowie mit der Verarbeitung in entsprechenden Aufnahmevorrichtungen (A2 bis D2) im Rahmen einer kontinuierlichen und insbesondere kaskadierten bzw. konsekutiven Verfahrensführung mit aufeinander abfolgender Zusammenführung der Ansätze gemäß einer erfindungsgemäßen Ausführungsform;

Fig. 6B eine schematische Darstellung in Anlehnung an Fig. 6A gemäß einer weiteren erfindungsgemäßen Ausführungsform mit einer alternativen Aufteilung der Substanzen bzw. Komponenten; die Bezugszeichen a3 bis d3 korrespondieren zu a2 bis d2 gemäß Fig. 6A, wobei Entsprechendes auch für A3 bis D3 und A2 bis D3 gilt;

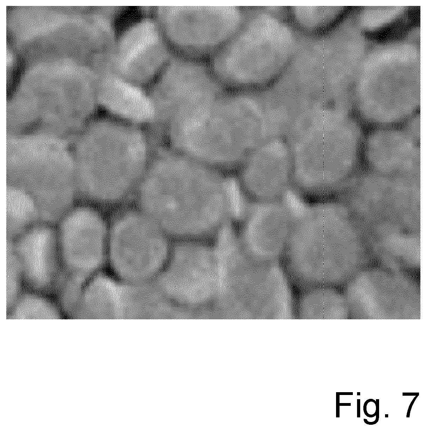

Fig. 7 eine kryorasterelektronenmikroskopische Aufnahme einer erfindungsgemäßen Zusammensetzung mit dem erfindungsgemäßen Trägersystem auf Basis von Partikeln in Form von disk-förmigen Lipidnanopartikeln; der mittlere Partikeldurchmesser beträgt 150 nm $\pm$ 70 nm; als amphiphile Hauptkomponenten werden Polysorbat 20 und Retinol eingesetzt;

Fig. 8 eine grafische Darstellung in Form eines Diagramms zur Veranschaulichung der im Rahmen der Erfindung aufgezeigten Beziehung zwischen HLB-Wert nach Griffin einerseits und Log P-Wert andererseits (in halblogarithmischer Auftragung).

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Das erfindungsgemäße Verfahren ermöglicht weiterführend auch eine standardisierte bzw. normierte industrielle Produktion, und zwar auch im Hinblick auf (Prozess-)Automatisierung und (Prozess-)Digitalisierung. Insbesondere erfüllt die vorliegende Erfindung auch wesentliche Kriterien bzw. Anforderungen, wie sie unter dem Begriff "Industrie 4.0" zusammengefasst werden. Der Begriff "Industrie 4.0" geht auf die Forschungsunion der Deutschen Bundesregierung und ein gleichnamiges Projekt in der Hightech-Strategie der Bundesregierung zurück. Die vorliegende Erfindung kann in diesem Zusammenhang beispielsweise unter Heranziehung moderner Informations- und Kommunikationstechnik auf Basis intelligenter und digital vernetzter Systeme durchgeführt werden, wobei auch eine selbstorganisierte Produktion möglich ist, wobei auch die gesamte Wertschöpfungskette optimiert werden kann.

Insbesondere ist im Rahmen der vorliegenden Erfindung eine flexible Produktion möglich: Für die Herstellung und Entwicklung zugrundeliegender Produkte können durch digitale Vernetzung die jeweiligen Prozessschritte gut aufeinander abgestimmt und insgesamt die Auslastungen der verfahrensgemäß eingesetzten Produktionsvorrichtungen besser geplant und optimiert werden. Zudem ist auch das Kriterium der sogenannten wandelbaren Fabrik erfüllt. So können Produktionsstraßen in Modulen und unabhängig von deren Größe bzw. Kapazität aufgebaut werden, wobei ein schneller und unkomplizierter Aufbau bzw. Bereitstellung im Hinblick auf heranzuziehende Aufgaben ermöglicht wird. Insbesondere kann auch eine verbesserte Produktivität bzw. Wirtschaftlichkeit bereitgestellt werden, wobei individualisierte Produkte auch in kleiner Stückzahl mit reduziertem Kostenaufwand hergestellt werden können. Zudem können auch kundenorientierte bzw. -zentrierte Lösungen bereitgestellt werden, wobei Produzent einerseits und Kunden bzw. Abnehmer andererseits näher zusammenrücken, zumal Kunden bzw. Abnehmer Produkte nach ihren Anforderungen mitgestalten können bzw. eine Anpassung entsprechender Produkte an das individuelle Anforderungsprofil jeweiliger Kunden bzw. Abnehmer möglich ist. Zudem liegt auch eine optimierte Logistik vor, zumal die erfindungsgemäßen Zusammensetzungen bzw. LNP-Produkte global bzw. sozusagen ortsunabhängig produziert werden können. Im Hinblick auf die vorliegende Erfindung ist auch ein fokussierter Einsatz von Daten möglich. So können Daten zum Ablauf der Produktion und zum Zustand entsprechender Produkte erfasst, zusammengeführt und ausgewertet werden. In diesem Zusammenhang kann die Datenanalyse auch dazu herangezogen werden, wie ein Produkt mit nochmals verbesserter Effizienz bzw. Eigenschaften hergestellt werden kann. Entsprechende Daten können auch als Grundlage für weiterführende Geschäftsmodelle und Serviceleistungen dienen.

Zu den vorgenannten Kriterien ist im Hinblick auf die vorliegende Erfindung zusammenfassend und ergänzend Folgendes von Relevanz:

- Flexible Produktion: Die erfindungsgemäß bereitgestellten Trägersysteme, bevorzugt in Form von Lipidnanopartikeln (LNP), bzw. die entsprechenden Zusammensetzungen können unter Heranziehung der entsprechenden HLB-Werte bzw. Log P-Werte der hierzu eingesetzten Bestandteile bzw. Substanzen individuell ausgestaltet bzw. maßgeschneidert werden, wobei ein insgesamt berechenbares bzw. individuell anpassbares System bereitgestellt wird, und zwar auch was die individuelle Ausgestaltung und Formgebung der Lipidnanopartikel anbelangt. Insbesondere können auf dieser Basis schnell und sicher beispielsweise sowohl Liposom-LNPs als auch Disk-LNPs bereitgestellt bzw. entwickelt werden, und zwar auch unter Berücksichtigung jeweiliger Anforderungs- und Anwendungsbereiche. Zudem können auch kritische Verfahrensparameter bereits während der Entwicklung bestimmt bzw. optimiert werden. Zudem ist im gesamten Herstellungsprozess eine kontinuierliche Kontrolle zugrundeliegender Prozessparameter möglich, wobei eine insbesondere kontinuierliche Verfahrens- bzw. Prozess- und Produktionskontrolle, insbesondere *online- bzw. inline*-Prozesskontrolle, durchgeführt werden kann.

- Wandelbare Fabrik: Die spezielle erfindungsgemäße Verfahrensführung ermöglicht eine verbesserte Produktivität und Wirtschaftlichkeit, wobei individualisierte bzw. maßgeschneiderte Produkte auch in kleiner Menge bzw. Stückzahl bei optimierter Kosteneffizienz bereitgestellt werden können.

- Optimierte Logistik: Auf Basis des erfindungsgemäßen Verfahrens können die im Rahmen der vorliegenden Erfindung bereitgestellten Trägersysteme bzw. Zusammensetzungen global bzw. sozusagen ortsunabhängig produziert werden. Insbesondere liegt auch eine optimierte Energieeffizienz vor. Die energiearme Produktion ermöglicht die Verwendung beispielsweise von Solarenergie. Dies ermöglicht beispielsweise eine effiziente Produktion von sogenannten Coating-Applikationen auch vor Ort.

Die obigen Ausführungen zeigen insgesamt die weiterführenden Vorteile und Eigenschaften, welche mit der vorliegenden Erfindung einhergehen.

Die folgenden Ausführungsbeispiele dienen lediglich der Veranschaulichung der vorliegenden Erfindung, ohne die vorliegende Erfindung jedoch hierauf zu beschränken. In diesem Zusammenhang werden nachfolgend auch weitere allgemeine Aspekte zu der vorliegenden Erfindung angeführt, welche die vorliegende Erfindung weiterführend charakterisieren, und zwar auch unabhängig von den angeführten Ausführungsbeispielen.

## AUSFÜHRUNGSBEISPIELE UND WEITERE ALLGEMEINE AUSFÜHRUNGEN ZUR VORLIEGENDEN ERFINDUNG:

Die nachfolgenden Ausführungsbeispiele verdeutlichen die Vorteile und speziellen Eigenschaften und Besonderheiten, wie sie mit der erfindungsgemäßen Konzeption einhergehen. Nachfolgend werden weiterführend auch ergänzende bzw. weitere Merkmale, Eigenschaften und Vorteile der vorliegenden Erfindung aufgezeigt, welche die folgende Erfindung weiter charakterisieren, und zwar auch unabhängig von den angeführten Ausführungsbeispielen.

Im Allgemeinen werden die nachfolgend beschriebenen Ausführungsbeispiele bzw. Untersuchungen im Technikumsmaßstab bzw. im Labormaßstab durchgeführt. Dabei wird im Hinblick auf die Ausbildung entsprechender Trägersysteme im Allgemeinen unter Eintrag von Energie bzw. Misch- bzw. Rührenergie vorgegangen, und zwar dahingehend, dass die eingetragene Energie derart eingestellt wird, dass eine zumindest im Wesentlichen laminare Strömung in dem zugrundeliegenden Medium eingestellt wird. Zudem wird im Allgemeinen insbesondere eine kontinuierliche Verfahrens- bzw. Prozesskontrolle durchgeführt, und zwar unter Erfassung entsprechender relevanter Prozessparameter, wie Temperatur und Leitfähigkeit. Auf Basis der Erfassung der Prozessparameter können die Verfahrensparameter zur Ausbildung optimaler Zusammensetzungen bzw. Trägersysteme gegebenenfalls entsprechend angepasst bzw. adjustiert werden.

## TEIL A:

2,5-Dimethylisosorbid (DMI) ist eine klare, farblose Flüssigkeit mit schwachem Geruch, welche sich mit Wasser und vielen organischen Lösungsmitteln mischt. DMI weist als biobasierter Ether insgesamt günstige Eigenschaften auf (z.B. geringe Augen- und Hautreizung und sehr niedrige Toxizität). Dimethylisosorbid eignet sich als wenig toxisches Lösungsmittel auch für pharmakologisch aktive Wirkstoffe, deren Penetrationsvermögen zum Beispiel in die Haut gefördert werden soll.

Überraschend ist insbesondere auch die Lösungseigenschaft gegenüber relativ hochschmelzenden Substanzen, wie zum Beispiel Cholesterol, Phytosterol oder Distearoylphosphatidylcholin, bei relativ niedrigen Temperaturen und hoher Konzentration der zu lösenden Substanzen. Dieses Verhalten wird eingehend am Beispiel Phytosterol getestet.

Das hydrophobe Phytosterol Stigmasterin, dessen Schmelzpunkt bei etwa 170 °C liegt, kann in dem hydrophilen 2,5-Dimethylisosorbid insbesondere im Temperaturbereich von 55 °C bis 70 °C auch in Konzentrationen von über 50 Prozent vollkommen klar bzw. vollständig gelöst werden. In dem vorgenannten Temperaturbereich können auch Cholesterol und bei hohen Temperaturen schmelzende Phosphatidylcholine in hoher Konzentration bzw. Menge gelöst werden.

2,5-Dimethylisosorbid fördert die Bildung von LNP und führt zu einer sehr kleinen Partikelgröße der resultierenden Lipidnanopartikel, und zwar auch wenn es in relativ hoher Konzentration zu- bzw. eingesetzt wird, wie das folgende Beispiel gemäß Tabelle 1 zeigt.

**Tabelle 1:**

| 1. Ansatz (Phase A) | |
|---|---|
| 2,5-Dmethyl-Isosorbid (DMI) [Gew.-%] | 20,0 |
| Phytosterol [Gew.-%] | 3,7 |
| Phosphatidylcholin [Gew.-%] | 6,5 |
| PEG 40 Stearate [Gew.-%] | 14,1 |
| Caprinsäure/Caprylsäure-Triglycerid (CCT) [Gew.-%] | 22,2 |
| 2. Ansatz (Phase B) | |
| Wasser [Gew.-%] | 33,4 |

Bei der Herstellung dieser LNP Emulsion werden zuerst sämtliche Komponenten der Phase A in ein Rührgefäß gegeben und anschließend auf 55°C erwärmt. Dabei einsteht eine homogene klare Lösung. Zu Dieser Lösung wird das Wasser gemäß Phase B, welches eine Temperatur von ca. 20 °C aufweist, unter Rühren zugegeben, insbesondere wobei sich ein liquid-kristallines Gel bzw. ein in dem flüssigen Medium vorliegendes Trägersystem auf Basis von definierten Lipidnanopartikeln bildet. Die erhaltene Zusammensetzung kann zum Erhalt jeweiliger Konzentrationen beliebig mit Wasser verdünnt werden. Die Partikelgröße des Trägersystems auf Basis von Lipidnanopartikeln LNP wird mittels Laserdiffraktometrie bestimmt. Es wird ein Wert von 150 nm $\pm$ 40 nm ermittelt. Bedingt durch den niedrigen Schmelzpunkt von -70 °C können die entsprechenden partikelhaltigen Zusammensetzungen, die 2,5-Dimethylisosorbid enthalten, auch ohne negative Beeinflussung der Partikelstruktur bzw. der zugrundeliegenden Dispersion bei tiefen Temperaturen gelagert werden. Der mittlere HLB-Wert nach Griffin der in dem ersten Ansatz bzw. in Phase A eingesetzten amphiphilen grenzflächenaktiven Komponenten und dem PEGyliertem Tensid beträgt HLB = 6,4. Zudem liegt ein Log P-Wert von etwa 1,03 vor.

## TEIL B:

### Zusammensetzungen auf Basis von Phosphatidylcholin, pegyliertem Tensid und Glycerinmonolaurat

Glycerinmonolaurat weist weiterführend auch keimreduzierende bzw. antimikrobielle bzw. antbakterielle Eigenschaften auf, wobei insbesondere gegenüber Helicobacter pylori eine sehr hohe Wirksamkeit vorliegt. Insbesondere können Lipidnanopartikel, die mit Glycerinmonolaurat als insbesondere verkapsulierten Bestandteil ausgerüstet oder beladen sind, ein antimikrobielles Spektrum aufweisen.

Infektionen mit Helicobacter pylori werden für eine Reihe von Magenerkrankungen verantwortlich gemacht, weolche mit einer verstärkten Sekretion von Magensäure einhergehen. Die erfindungsgemäßen Lipidnanopartikel, die auf Phosphatidylcholin und Glycerinmonolaurat basieren, weisen zudem den Vorteil auf, dass sie durch eine Protonierung von Phosphatidylcholin zudem die Konzentration der Magensäure reduziert werden kann.

Für eine zugrundeliegende orale Anwendung können die Lipidnanopartikel bzw. das Teilchensystem bzw. die die Lipidnanopartikel enthaltende Zusammensetzung nach der Erfindung mit Sorbitol und/oder einem Süßstoff, wie z.B. Acesulfam, versehen werden, und zwar insbesondere um bei der oralen Applikation den Geschmack zu verbessern, insbesondere kann ein etwaiger bitterer Geschmack durch Süße (d.h. Zugabe insbesondere von Süßstoffen) kompensiert werden.

In diesem Zusammenhang werden die nachfolgend in den Tabellen 2 und 3 angeführten Zusammensetzungen mit den angegebenen Eigenschaften hergestellt. Der in den Tabellen angeführte mittlere HLB-Wert nach Griffin bezieht sich auf den Mittelwert der für das Trägersystem eingesetzten Bestandteile bzw. Substanzen. Entsprechendes gilt für den angegeben mittleren Log P-Wert und somit den Gesamt-Log P-Wert.

Legt man die Zusammensetzungen auf Basis von Phosphatidylcholin, pegyliertem Tensid und Glycerinmonolaurat zugrunde und fügt diesen noch ein kurzkettiges Glycerincaprylat oder Sorbitancaprylat hinzu, erhält man durch das breite antimikrobielle und antibakterielle Spektrum auch ein hervorragend wirksames Hautdesinfektionsmittel bzw.

Flächendesinfektionsmittel. Derartige Zusammensetzungen zeigen nach Applikation bzw. Auftragen eine hervorragende Filmbildung und können somit die zu behandelnden Flächen zumindest im Wesentlichen vollständig abdecken. In alternativen Zusammensetzungen bzw. Ansätzen mit einem (weiteren) Wirkstoff (antimikrobieller Wirkstoff oder Impfwirkstoff (mRNA)) werden vergleichbare Eigenschaften erhalten.

**Tabelle 2:**

| | | |
|---|---|---|
| Phosphatidylcholin [Gew.-%] | 3,5 | 3,1 |
| PEG 40-Stearat [Gew.-%] | 7,0 | 11,0 |
| Glycerinmonolaurate [Gew.-%] | 5,2 | 5,5 |
| Phytosterol [Gew.-%] | 2,2 | 2,3 |
| Caprinsäure/Caprylsäure-Triglycerid [Gew.-%] | 9,2 | 9,1 |
| Glycerin [Gew.-%] | 16,0 | 16,8 |
| Wasser [Gew.-%] | 57,0 | 52,3 |
| | | |
| | 100,00 | 100,00 |
| | | |
| Partikelgröße [nm] | 1.200 ± 300 | 620 ± 160 |
| | | |
| mittlerer HLB nach Griffin | 6,4 | 7,8 |
| mittlerer Log P-Wert | 1,03 | 0,82 |

**Tabelle 3:**

| | |
|---|---|
| Phosphatidylcholin [Gew.-%] | 1,9 |
| PEG 40-Stearat [Gew.-%] | 7,8 |
| Glycerinmonolaurat [Gew.-%] | 8,4 |
| Sorbitancaprylat | 3,6 |
| Caprinsäure/Caprylsäure-Triglycerid [Gew.-%] | 10,7 |
| Glycerin [Gew.-%] | 3,8 |
| Wasser [Gew.-%] | 63,8 |
| | |
| | 100,00 |
| | |
| Partikelgröße [nm] | 750 ± 200 |
| | |
| mittlerer HLB nach Griffin | 7 |
| mittlerer Log P-Wert | 0,93 |

**Partikelgröße als Charakteristikum für die erfindungsgemäßen Trägersysteme insbesondere in Form von diskförmigen Lipidnanopartikeln (Disk-LNP)**

Den folgenden Ausführungen liegt die Herstellung, Anwendung und Formulierung von erfindungsgemäßen Trägersystemen in Form von diskförmigen Lipidnanopartikeln zugrunde, wobei das Trägersystem auf Phosphatidylcholin und einem pegyliertem Tensid sowie amphiphilen Substanzen, deren gemeinsamer mittlerer HLB nach Griffin kleiner als 8 ist, sowie ggf. einen Wirkstoffs basiert. Die diskförmigen Lipidnanopartikeln sind in ihrer Größe insbesondere dadurch definiert, dass eine Gaußverteilung mit einer Varianz im Bereich von ± 20-35 % um den Mittewert liegt (Tabelle 4).

**Tabelle 4:**

| Partikelgröße | Rührzeit | Varianz | Varianz |
|---|---|---|---|
| [nm] | [s] | [$\pm$] | [%] |
| 1.900 | 30 | 0,6 | 31,6 |
| 1.400 | 60 | 0,38 | 27,1 |
| 1.200 | 90 | 0,33 | 27,5 |
| 1.000 | 120 | 0,25 | 25,0 |
| 900 | 150 | 0,2 | 22,2 |
| 750 | 180 | 0,16 | 21,3 |

Insbesondere kann sowohl die Partikelgröße, insbesondere die mittlere Partikelgröße, der Partikel des Trägersystem als auch die Varianz über die Rührdauer bzw. -zeit der zu bildenden zusammenhängenden flüssigkristallienen Struktur (Zeitdauer des Eintrags von Misch- bzw. Rührenergie) gesteuert werden. Der Median der Partikelgröße nimmt mit zunehmender Rührdauer insbesondere exponentiell ab. Zudem nimmt auch die prozentuale Varianz insbesondere im logarithmischen Maß ab. Dieses Verhalten kann man als ein Charakteristikum bzw. spezielle Eigenschaft des erfindungsgemäßen Teilchensystems, insbesondere in Form von Disk-LNPs, gesehen werden. Das Spektrum bzw. die Werte des mittleren HLB-Wertes nach Griffin bei Kombinationen auf Basis von pegylierten Tensiden und Phosphatidylcholin verschiebt sich durch den Zusatz von nicht-ionogenen Substanzen, die einen höheren HLB-Wert als das eingesetzte Phosphatidylcholin aufweisen, im Allgemeinen in Richtung höherer Werte. Der Log P-Wert kann sich sich dementsprechend in Richtung kleinerer Werte verschieben.

**Ausbildung von Partikeln in Form von diskförmigen Lipidnanopartikeln in Beziehung zum HLB-Wert nach Griffin**

Wie bereits beschrieben und wie seitens der Anmelderin in überraschender Weise gefunden, ist es erfindungsgemäß im Hinblick auf die Ausbildung speziell von Disk-LNP (vgl. auch Fig. 1A, 1B und 1C sowie Fig. 2 (Bezugzeichen 1)) von Vorteil, wenn für die Gesamtheit an pegylierten Tensiden und amphiphilen Substanzen des Trägersystems ein mittlerer HLB-Wert nach Griffin im Bereich von 2 bis 8 gewählt wird. Überraschend ist hier sicherlich, dass im Stand der Technik der HLB-Bereich von 3 bis 8 im Allgemeinen W/O-Emulgatoren zugeordnet ist.

**TEIL C:**

**Aufgabenstellung**

Strukturierte Arzneiformen, wie Liposome und lipidbasierte Partikel, bieten neue Therapieoptionen, wie sie auch bei der schnellen Entwicklung von Covid-19-Impfstoffen sichtbar geworden sind. Erste liposomale Fertigarzneimittel wurden bereits vor mehr als 20 Jahren in die Therapie eingeführt, aber in den Folgejahren haben nur wenige weitere Produkte die Marktreife erlangt.

Die Struktur der Partikel kann - wie in biologischen Membranen auch - im Allgemeinen von Phospholipiden mit darin eingelagertem Cholesterin gebildet werden. Die Lipidpartikel können darüber hinaus weitere Lipidbestandteile, die spezielle Eigenschaften vermitteln, enthalten.

Liposomen werden als Träger für Arzneistoffe in der Medizin schon seit über 20 Jahren eingesetzt werden. In einem weiteren zugelassenen Arzneimittel sind therapeutische RNA-Moleküle in Lipidpartikel verpackt (vgl. Onpattro®). Bei diesen Arzneimitteln werden im Vergleich zur Impfung deutlich höhere Lipidmengen intravenös verabreicht. Lipidpartikel werden auch als Träger für mRNA eingesetzt und sorgen dafür, dass die eingesetzte mRNA leichter in die Körperzellen transportiert werden kann. Dort setzt sie verschiedene Reaktionen in Gang, an deren Ende zum Beispiel eine Immunisierung gegen das SARS-CoV-2-Virus steht.

Lipidpartikel, und zwar insbesondere im Hinblick auf Lipid(nano)partikel, insbesondere wenn sie als Träger für mRNA dienen, sind allenfalls sehr aufwendig herzustellen. Insbesondere resultieren im Stand der Technik oftmals relativ große und insbesondere in Bezug auf ihre Partikelgrößenverteilung nicht homogen ausgebildete Partikel, und zwar oftmals mit übermäßig großer Dispersität der zugrundeliegenden Partikel. Hieraus resultierende Dispersionen sind häufig nicht ausreichend stabil und nur unter großem Aufwand zu lagern, insbesondere bei sehr niedrigen Temperaturen.

Für die medizinische Anwendung stehen Vesikelstrukturen bzw. -partikel im Vordergrund. Diese Strukturen bestehen zumeist vorwiegend aus Phosphatidylcholinen und Cholesterol. Dabei wird zu deren Herstellung häufig derart vorgegangen, dass ein Trägeröl, Phospholipid und etwaige weitere hydrophobe Bestandteile in einem Lösemittel gelöst werden, nämlich oftmals Ethanol, welches im Anschluss wieder entfernt werden muss. Zudem wird erfolgt die Zugabe von Pufferlösung und eines Wirkstoffs (beispielsweise eines Antigens oder einer für ein Antigen codierender mRNA im Fall von Impfwirkstoffen), wobei insbesondere derart verfahren wird, dass große mehrlagige Vesikel (*multilayer* bzw. *multilamellar vesicles;* MLVs), welche anschließend in einem Microfluidizer®-Hochdruckhomogenisator zu kleineren Vesikeln verarbeitet werden. Sofern das Trägermaterial flüssig ist oder in Wasser geschmolzen werden kann, kann man die hydrophoben Bestandteile auch einfach vermischen und der wässrigen Phase zufügen, bevor das Material im Homogenisator verarbeitet wird.

Die Herstellung ist also sehr aufwendig und führt nicht immer zu den gewünschten Produktqualitäten. Ein solcher Prozess ist vor allem in der für eine gleichbleibende Batch/Batch-Qualität nicht sonderlich geeignet. Im Allgemeinen sollen die LNP-Strukturen als Träger der mRNA auf Vesikel aufgebaut sein. Aufgrund der im Träger des Impfstoffs vorliegenden Verhältnisse der pegylierten Substanzen zu den wasserunlösliche amphiphilen Substanzen ist eine homogene Vesikelstruktur im Allgemeinen nicht gegeben, zumal die vorliegenden Packungsparameter der beteiligten Substanzen einer solche Struktur entgegenstehen bzw eine solche Struktur nicht zulassen. Dies führt auch zu kurzen Haltbarkeiten bzw. Lagerzeiträumen. So sind beispielsweise mRNA-Impfstoffe innerhalb von kurzen Zeiträumen zu verwenden sind. Wird dieser Zeitraum überschritten, ist die physikalisch-chemische Stabilität des Produkts nicht mehr ausreichend sichergestellt. demgegenüber kann ein anzustrebendes Vorliegen der Lipidpartikel in homogener Form zu einer höheren Stabilität und einer Beständigkeit über einen viel größeren Zeitraum führen.

Auch die notwendige starke Kühlung insbesondere von mRNA-Impfstoffen, beispielsweise auf eine Temperatur von -20 °C bis -70 °C stellt insbesondere bei einer Pandemie eine fast nicht zu bewältigende Schwierigkeit dar, zumal weltweit nicht alle Regionen die dazu notwendigen Kühlketten aufbauen können.

Auch die äußerst komplexe Produktion kann ein Hindernis für die Verbreitung des Impfstoffes in bestimmte Regionen darstellen. Die Ausrüstung beispielsweise mit hydrophilen Antigenen, welche insbesondere nach der Herstellung der Lipidpartikel an deren Oberfläche erfolgt, ist gleichermaßen nicht immer optimal, insbesondere da die zugrundeliegenden Partikel mitunter keine auch im Hinblick auf deren Volumen optimierte Oberflächengröße aufweisen.

Die Herstellmethoden des Standes der Technik sind darüber hinaus zudem oftmals nur im kleinen Maßstab realisierbar.

## Problemlösung und weitere Ausführungen

Die Lösung des Problems soll eine einfache Herstellung ermöglichen, die zum einen eine hohe Batchqualität garantiert und zum anderen die Möglichkeit eröffnet, Lipidnanopartikel (LNP) im großen Maßstab und in gleichbleibender Qualität mit definierten Größeneigenschaften herzustellen.

Lipid-Nano-Partikel (LNP) sind Partikel, deren Struktur durch lyotrope flüssig-kristalline (liquid-kristalline) Bereiche aufgebaut ist und deren Größe im Allgemeinen im skalierten Nanobereich angesiedelt ist, wie auch zuvor angeführt. Zusammenhängende flüssig-kristalline Strukturen zählen, ohne sich auf dies Theorie berufen oder beschränken zu wollen, zu den selbstorganisierten Systemen und sind thermodynamisch stabil. Sie formen also unter gegebenen äußeren Bedingungen definierte Strukturen und führen auch von daher zu einer gleichbleibenden Qualität. Bekannt sind sowohl wasserlösliche als auch inverse, also öllösliche Strukturen. Für die Formulierung von pharmazeutischen Trägersystemen sind insbesondere die wasserlöslichen Strukturen von Bedeutung.

Lyotrope flüssig-kristalline wasserlösliche Strukturen können in drei Haupttypen vorliegen, nämlich in einer insbesondere homogenen und/oder insbesondere zusammenhängenden lamellaren Form, in einer hexagonalen oder einer kubischen Form. Diese lyotropen flüssig-kristallinen Formen können grundsätzlich noch weitere Unterstrukturen aufweisen. Die drei Strukturen sind insbesondere auch dadurch charakterisiert, dass ihre Kompartimente eine gegebenenfalls zusammenhängende Struktur bilden. Die Strukturen der laminaren und hexagonalen Phase erscheinen milchig und repräsentieren Gele mit viskoelastischem Verhalten.

Die kubische Phase ist im Allgemeinen transparent ausgebildet. Eine Voraussetzung für die Bildung einer kubischen Struktur ist, dass die Partikel sphärisch oder ellipsoid sind. Eine Sonderform der kubischen Struktur ist die Formierung aus Vesikeln, deren Aufbau aus Lamellen besteht. Flüssig-kristalline Lamellarstrukturen zeigen eine Reihe von Unterstrukturen. Von diesen Unterstrukturen ist die aus einzelnen Disk-Kompartimenten bestehende Struktur für die Formierung von LNPs, die in pharmazeutischen Anwendungen als Träger für Aktivstoffe dienen, von Bedeutung und welche erfindungsgemäß ziel- und zweckgerichtet bereitgestellt werden kann. Lyotrope flüssig-kristalline Phasen können beim Verdünnen mit wässrigen Phasen eine Dispersion auf Basis der Kompartimente, aus denen sie aufgebaut sind, bilden. Dieser Vorgang ist reversibel. Verdunstet man Wasser aus über lyotrope flüssig-kristalline Systeme gebildeten LNP-Dispersionen, so kann sich beim Erreichen der kritischen Wasserkonzentration die ursprünglich zusammenhängende Phase zurückbilden.

Die lyotropen flüssig-kristallinen Strukturen können in hohem Maße mit Wasser verdünnt werden. Das zum Verdünnen verwendete Wasser kann auch Elektrolyte oder andere nicht-tensidische wasserlösliche Substanzen enthalten. Beim Verdünnen der lyotropen flüssig-kristalline Phase nimmt die Viskosität nach der kritische Verdünnungskonzentration exponentiell ab und die Wasseraktivität exponentiell zu. Die lyotropen flüssig-kristalline Strukturen sind nicht durch eine bestimmte Wasserkonzentration bestimmt, sondern je nach Struktur über einen Konzentrationsbereich des gebundenen Wassers stabil. Der Konzentrationsbereich des notwendigen Wassers in einer bereits gebildeten flüssig-kristallinen Struktur kann mehrere Gewichtsprozent betragen.

Bei der Bildung flüssig-kristalliner Strukturen liegt das vorhandene Wasser im hohen Maß in gebundener Form vor. Der Bindungsgrad des Wassers kann über die Wasseraktivität berechnet werden. Die Bestimmung der Wasseraktivität ist bereits ein etabliertes Verfahren, das oftmals als Ersatz für einen Konservierungsbelastungstests (KBT) verwendet wird. Bei entsprechend niedriger Wasseraktivität unterhalb des Wertes 0,5 vermehren sich Mikroorganismen nicht mehr. Die Bestimmung der Wasseraktivität als KBT ist in der Norm ISO 29621 hinterlegt. Die Bestimmung der Wasseraktivität ist heute ein wichtiges Instrument für die Qualitätskontrolle von Produkten und Inhaltsstoffen in der Lebensmittel-, Pharma- und Kosmetikindustrie. Die Messung der Wasseraktivität erfolgt nach ISO 21807 und ISO 18787 + 21 CFR Part 11. Dies ist auch in der Norm ISO 29621 hinterlegt. Die Wasseraktivität beeinflusst auch den Ablauf chemischer Prozesse, wie Fettoxidation.

Insbesondere liegt eine Wasserkonzentration vor, bei welcher die Struktur insbesondere irreversibel in ihre einzelnen Kompartimente zerfällt und eine wässrige Dispersion bildet.

Lytrope flüssig-kristalline Strukturen sind innerhalb eines Temperaturfensters stabil und können demgegenüber und mitunter irreversibel zumeist bei Lagerung unterhalb oder oberhalb der kritischen Temperatur zerfallen.

Eine konkret auszubildende flüssig-kristalline Struktur hängt, wie überraschend gefunden, insbesondere auch vom Packungsparameter der beteiligten Substanzen ab. Dem kritischen Packungsparameter $P_{kr}$ liegt gemäß dem Modell nach Israelachvili die Formel gemäß $P_{kr} = V / a_0 \cdot l_C$ zugrunde, insbesondere wie zuvor ausgeführt. $P_{kr}$ beschreibt, ähnlich wie der HLB Wert, ein Verhältnis hydrophiler Kopfgruppen zu den lipophilen Gruppen einer insbesondere amphiphilen Substanz bzw. Verbindung, wobei der Schwerpunkt auf geometrische und raumfüllende Komponenten liegt. Der $P_{kr}$ ist dimensionslos und charakteristisch für jedes die herangezogene Verbindung. Über den insbesondere mittleren $P_{kr}$ können geometrische Formen lyotroper flüssig-kristalliner Strukturen gesteuert bzw. vorhergesagt werden.

Der Packungsparameter einer insbesondere amphiphilen Substanz bzw. Verbindung kann auch über deren HLB-Wert nach Griffin abgeschätzt werden. Berechnet man z.B. den $P_{kr}$-Wert von Fettalkoholen mit unterschiedlicher C-Kettenlänge und unterschiedlichem Ethoxylierungsgrad, so korrelieren diese Werte mit $R^2 = 0,94$ sehr gut mit den HLB-Werten (vgl. auch Fig. 4). Diese Werte können entsprechend auf andere Substanzen übertragen werden. Zudem kann der Packungsparameter auch auf Basis diesbezüglicher Log P-Werte ermittelt werden.

Da man, wenn bei einer Stoffmischung insbesondere von tensidischen bzw. amphiphilen Substanzen der HLB-Wert jeder einzelnen Substanz bekannt ist, den mittleren HLB-Wert berechnen kann, ist es aufgrund der Beziehung zwischen HLB-Wert und dem Packungsparameter $P_{kr}$ möglich, auch der Mischung als solchen einen mittleren Packungsparameter zuzuordnen. Hierdurch kann in überraschender Weise eine Steuerung oder Abschätzung der sich aus der vorgegebenen Mischung bildenden flüssig-kristallinen Kristallstruktur vorgenommen werden.

Grundsätzlich kann auch der Log P-Wert diesbezüglich herangezogen werden, was gleichermaßen überraschend ist.

Die Beziehung zwischen dem Packungsparameter einer insbesondere nicht ionogenen Substanz und deren HLB-Wert kann wie folgt näherungsweise mathematisch formuliert werden: $P_{kr} = 0,07 \cdot HLB + 1,26$ (mit $P_{kr}$ = kritischer Packungsparameter; HLB = HLB-Wert eines einer insbesondere amphiphilen bzw. tensidischen Substanz bzw. einer diesbezüglichen Mischung; vgl. Fig. 4). Der HLB-Wert einer Substanz ist also direkt proportional zum kritischen Packungsparameter und auch auf dieser Basis zu ermitteln.

Griffin stellte für insbesondere nichtionische bzw. insbesondere grenzflächenaktive Verbindungen ein HLB-System auf, welches den Substanzen bzw. Verbindungen numerische, dimensionslose Zahlenwerte zwischen 0-20 entsprechend des Gewichtsverhältnisses des hydrophilen Molekularanteiles zum Gesamtmolekül zuordnet und somit Aussagen über die Wasserlöslichkeit des Stoffes zulässt.

Als Berechnnungsgrundlage gilt, wie auch zuvor angeführt, die Formel $HLB = 20 \cdot M_{hydrophil} / M_{gesamt}$. Aus Kombinationen mehrerer insbesondere amphiphiler Substanzen kann ein mittlerer HLB-Wert nach Griffin berechnet werden.

Das Verhalten einer amphiphilen bzw. Substanz kann von Parametern, wie dem pH-Wert, beeinflusst werden. Für Nichtionogene Tenside können ihre grenzflächenaktiven Eigenschaften mitunter im Hinblick auf die vorliegende Temperatur ändern. Bei nichtionogenen Tensiden ist der Einfluss des Elektrolyten moderat.

Der Trübungspunkt ist die Temperatur, bei der eine nichtionischen Tensidlösung trüb wird. Etwa 0,5 °C oberhalb des Trübungspunktes ist das Tensid vollständig wasserunlöslich. Je höher der HLB-Wert ist (bzw. je kleiner der Log P-Wert ist), desto höher liegt auch der Trübungspunkt. Der Trübungspunkt ist damit ein charakteristisches Merkmal für das jeweilige Tensid und seinen Einsatzbereich. Der Trübungspunkt hängt von der Konzentration der Elektrolyte ab.

Bei ionischen oder amphoteren Tensiden bzw. Lipiden kann der HLB-Wert vom pH-Wert beeinflusst werden. In diesem Fall können Ähnlichkeiten zu nichtionischen Substanzen im Verhalten in wässrigen Medien herangezogen werden.

Für ionische, insbesondere kationische, und/oder amphotere Substanzen kann der HLB-Wert im Rahmen der vorliegenden Erfindung insbesondere für den ungeladenen Zustand bzw. am isoelektrischen Punkt bestimmt werden. Zudem kann der HLB-Wert erfindungsgemäß insbesondere bei Raumtemperatur (20 °C) ermittelt werden.

Für das Phosphatidylcholin Distearoylphophatidylcholin (DSPC) ist in der Literatur ein Wert von HLB-Wert von 4,5 angegeben (was einem Log P-Wert von 1,54 entspricht). Dieser Wert ist auch konsistent mit dem daraus abgeleiteten Packungsparameter.

Amphotere Tenside bzw. Lipide, wie Phospholipide, verlieren in der Nähe des isoelektrischen Punkt ihre Wasserlöslichkeit und wandeln sich in Co-Tenside um. In diesem Bereich verhalten sich Phospholipide ähnlich wie nichtionogene Tenside. Kalkuliert man für Phospholipide nach Griffin einen HLB Wert, dann entspricht dieser dem am isoelektrischen Punkt. Unterhalb und oberhalb des isoelektrischen Punktes steigt der kalkulierte HLB-Wert an.

## Einfluss der Bildung von lamellaren lyotropen flüssig-kristallinen Strukturen auf die LNP-Bildung

Ein möglicher Packungsparameter für die Bildung lamellarer flüssig-kristalliner (liquid kristalliner) Strukturen liegt insbesondere im Bereich $P_{kr} = 0,5$, was einem HLB-Wertebereich nach Griffin von HLB = 10 bis 12 bzw. einem Log P-Wertebereich von Log P = 0,50 bis 0,62 entspricht. In diesem HLB-Bereich können zunächst insbesondere vesikelartige Strukturen gebildet, die insbesondere eine kubische Form aufweisen können. Die kubische Vesikelstruktur kann insbesondere transparent sein. Wird diese Form mit Wasser verdünnt, bilden sich insbesondere sphärische LNPs. Grundsätzlich sind auch hexagonale Kugelpackungen von Vesikeln möglich.

Wird ein PEGyliertes Tensid zur Bildung dieser Phasen eingesetzt, ist dieses über die gesamte Lamelle des Vesikels gleichmäßig verteilt. Als Träger für Aktiv- bzw Wirkstoffe, die in eine menschliche Zelle eingeschleust werden sollen, sind Vesikel, die mit PEGylierten Substanzen gebildet werden, welche sich über die gesamte Oberfläche des Partikels verteilen, nur bedingt geeignet, da die PEG-Reste der pegylierten Substanz eine sterische Barriere auch mit bzw. gegenüber der Zelle bilden.

Die flüssig-kristallinen Gelstrukturen mit einem mittleren HLB Wert von 11,7 und 10,1 (insbesondere Log P-Wert von 0,52 und 0,61) repräsentieren insbesondere klare Gele, welche man der kubischen Struktur zuordnen kann. Es handelt sich hierbei um sphärische oder ellipsoide Vesikelstrukturen.

Insbesondere verhält es sich derart, dass eine Unterstruktur der vesikelartigen flüssig-kristallinen Lamellarstruktur durch die Disk-Struktur bzw. Disk-LNP gebildet wird, welche insbesondere durch ein Tensid bzw. (Phospho-)Lipid mit einem nach Griffin kalkulierten HLB-Wert von mindestens 13 (insbesondere Log P-Wert von höchstens 0,46) und insbesondere mit vorzugsweise nicht wasserlöslichen amphiphilen Substanzen mit einem HLB von höchstens 6 (insbesondere Log P-Wert von mindestens 1,11) gebildet wird. Der aus den HLB-Werten der Tenside bzw. Lipide mit einem HLB von mindestens 13 und den nicht wasserlöslichen amphiphilen Substanzen mit einem HLB von höchstens 6 gemittelte HLB-Wert liegt diesbezüglich vorzugsweise bei einem HLB von höchstens 5 (insbesondere Log P-Wert von mindestens 1,36).

Liquid kristalline bzw. flüsssig-kristalline Disk-Strukturen zeigen eine zylinderartige Geometrie. In dieser Geometrie ist die Mantelfläche dem Gesetz der Packungsparameter vorrangig bzw. überwiegend mit beispielsweise mit PEGylierten Tensiden belegt, während die kreisförmigen Grundflächen mit den nicht wasserlöslichen amphiphilen Komponenten belegt sind. Ein mittlerer HLB-Wert nach Griffin, welcher die Bedingungen für die Zusammensetzung der amphiphilen Komponenten für die Bildung von Disk-Strukturen beschreibt, ist bislang nicht bekannt, und es ist völlig überraschend, dass dieser im Hinblick auf die Ausbildung von diskförmigen LNPs herangezogen werden kann. Entsprechendes gilt für den Log P-Wert.

## Herstellung von LNP-Strukturen

### Eintrag von Misch- und/oder Rührenergie

Die (Selbst-)Organisation lyotroper flüssig-kristalliner Strukturen aus einem amphiphilen Gemisch erfolgt erfindungsgemäß über den Eintrag von Misch- bzw. Rührenergie. Auf dieser Basis wird die Strukturgebung bzw. Organisation der partikulären Strukturen induziert bzw. hervorgerufen bzw. sozusagen katalysiert "Katalyse über Misch-bzw. Rührenergie"). Um eine Bildung der gewünschten Struktur zu erreichen werden also die Teilchen in eine für die Bildung flüssig-kristalliner Strukturen günstige Position geschoben werden. Erfindungsgemäß hat e sich dabei in überraschender Weise als vorteilhaft erwiesen, wenn die durch das Rühren erzeugte bzw. eingetragene Energie im laminaren Strömungsbereich liegt. Turbulente Strömungen stören die gewünschte Selbstorganisation. Die dabei hohe eingetragene Energie $\Delta W$ kann deutlich zu hoch werden ($\Delta U = \Delta Q + \Delta W$; $\Delta U$ kann dadurch deutlich zu hohe Werte annehmen).

Die für die Bildung von flüssig-kristallinen Strukturen eingetragene Rührenergie liegt erfindungsgemäß insbesondere nur im laminaren Fließbereich. Das laminare Fließverhalten wird über die Reynolds-Zahl Re beschrieben, wie zuvor angeführt.

Insbesondere kann ein Übergang von der laminaren Strömung in die turbulente bei $R_{e,krit}$ oberhalb von 2.000, insbesondere bei $R_{e,krit}$ = 2.200 bis 2.300, vorliegen, was erfindungsgemäß vermieden werden sollte ($R_e > R_{e,krit}$ => Strömung ist turbulent $R_e < R_{e,krit}$ => Strömung ist laminar). Für die Einhaltung der laminaren Strömung kommen übliche Rührwerkzeuge bzw. -vorrichtungenin Betracht, die insbesondere zumindest im Wesentlichen keine oder allenfalls nur eine geringe örtliche Wärmeentwicklung bei der Anwendung erzeugen.

Einsatz von Polyolen

Überraschenderweise hat ein Zusatz von Polyolen zu der für die Bildung der flüssig-kristallinen Phasen eingesetzten Wasserphase bzw. dem flüssigen Medium einen deutlichen Einfluss auf die Partikelgröße. Innerhalb eines kritischen Konzentrationsbereiches des Polyols in der Wasserphase werden deutlich kleinere Partikel gebildet als in reinen Wasserphasen. Eine bevorzugte Polyolkonzentration in der Wasserphase liegt zwischen 10 und 60 Prozent, bezogen auf die Wasserphase. Der besonders bevorzugte Bereich richtet sich insbesondere nach der Struktur des verwendeten Polyols. Besonders geeignet sind Polyole, wie Propylenglycol, Glycerin, Polethylenglycol, Sorbitol, Mannitol, Saccharose oder Panthenol.

Insbesondere Panthenol kann in den LNP-Formulierungen über seine Struktur multifunktionelle Eigenschaften zeigen. Bei Panthenol handelt es sich aufgrund der vorhandenen Aminopropanol-Gruppe, die ähnlich der in mRNA Impfstoffen eingesetzten amphiphilen Substanzen ist, um eine bei physiologischem pH-Wert wasserlösliche Substanz ist, welche sich aber nachweislich in bzw. auf lamellare Strukturen verankern kann. Panthenol fördert auch das Pentrationsverhalten, was in der Literatur beschrieben an mehreren Beispielen unter anderem an Progesterone nachgewiesen wurde.

Der Grund für die verbesserte Formulierung der flüssig-kristallinen Disk-Struktur scheint auf die verminderten Wasseraktivität von Polyollösungen zurückzuführen zu sein. Optimal bei der Bildung von LNP erscheint für die dazu eingesetzten Polyollösungen eine Wasseraktivität von 0,9 bis 4,5.

Da jedes Polyol, das mit Wasser gemischt wird, zu einer spezifischen Reduktion der Wasseraktivität führt, kann der der optimale Konzentrationsbereich für die Bildung von optimalen Bedingungen, insbesondere im Hinblick auf eine spezifische Reduktion der Wasseraktivität, dabei insbesondere auch in Abhängigkeit von dem konkret eingesetzten Polyol eingestellt werden. Bezogen auf die Wasseraktivität liegt ein möglicher Bereich für wässrige Saccharose in dem vorgenannten Bereich.

In diesem Zusammenhang werden im Rahmen der vorliegenden Erfindungen entsprechende Zusammensetzungen auf Basis des Trägersystems und des flüssigen Mediums hergestellt, und zwar in Bezug auf das flüssige (wässrige) Medium (1. Ansatz bzw. Phase B) zum einen unter Verwendung von Saccharose als Polyol (12 Gew.-%, bezogen auf die Zusammensetzung) und zum anderen ohne Saccharose (Ersatz durch Wasser). Die Bestandteile des Trägersystems werde als erster Ansatz bzw. Phase A vorgegeben. Die Ansätze werden zum Erhalt der Zusammensetzung zusammengeführt. Für die Saccharose-haltige Zusammensetzung kann eine mittlere Partikelgröße von 590 nm $\pm$ 170 nm im Vergleich zu 1.500 nm $\pm$ 350 nm für die Zusammensetzung ohne Saccharose ermittelt werden.

Einfluss des mittleren HLB-Werts bzw. mittleren Loq P-Wertes auf die Partikelgröße von Disk-LNP-Strukturen

Auch der mittlere HLB-Wert nach Griffin, der sich aus den HLB-Werten der hydrophoben amphiphilen Komponenten und den pegylierten Tensiden errechnet, spielt für die Bildung stabiler Disk-LNP eine wichtige Rolle. Entsprechendes gilt für den diesbezüglichen Log P-Wert. Um submicron- bzw. nanoskalige Partikel, wie LNPs, zu erzeugen, sollte der mittlere HLB-Wert vorzugsweise im Bereich von 3,5 bis 5,5 liegen (Log P-Wertebereich von 1,22 bis 2,04). Der optimale mittlere HLB-Bereich bzw. mittlere Log P-Bereich aller amphiphilen Strukturen für die Bildung von Dik-LNP richtet sich nach der Struktur der eingesetzten amphiphilen Substanzen. Auch das Gewichtsverhältnis der PEGylierten Komponenten bzw. der Substanz (ii) zu den hydrophoben amphiphilen Substanzen spielt eine wichtige Rolle. Ein bevorzugter Bereich liegt hier beispielsweise bei etwa 2 : 1 bis 1 : 10.

Neben den PEGylierten Substanzen, wie ALC-0159, PEG2000-DMG, Polysorbate, Cetromacrogole, PEG-Stearate, können für die Formulierung von Disk-LNP, die in der Pharmazie zum Einsatz kommen, auch Poloxamere eingesetzt werden.

Als hydrophobe amphiphile Substanzen, die als Trägersubstanz eingesetzt werden, kommen Phosphatidylcholine, ALC-0315, SM-102, flüssige Triglyceride, Octyldodecanol, Phytosterol, Cholesterol, Monoglyceride, Diglyceride, Sorbitanfettsäureester in Betracht.

Auffällig und überraschend ist, dass die Disk-Strukturen auch oberhalb des submicron-Bereiches, also im μ-Bereich erhalten bleiben können, was an der relativ engen Gaußschen Partikelgrößenverteilung zu beobachten ist.

Derartige Disk-Strukturen können sicherlich auch als pharmazeutische Träger in Betracht gezogen werden, weil hier die Beladungsmenge an amphiphilen Wirkstoffen deutlich größer ist, als im submicron-Bereich.

Die nachfolgende Tabelle 5 zeigt entsprechende Zusammensetzungen. In alternativen Zusammensetzungen bzw. Ansätzen mit Phosphatidylcholin bzw. einem (weiteren) Wirkstoff können vergleichbare Eigenschaften erhalten werden.

**Tabelle 5:**

| 1. Ansatz (Phase A) | | | | |
|---|---|---|---|---|
| Glycerinmonolaurat [Gew.-%] | 6,4 | 4,5 | 5,2 | 4,1 |
| PEG 40-Stearat [Gew.-%] | 8,1 | 5,7 | 6,5 | 5,2 |
| Caprinsäure/Caprylsäure-Triglycerid [Gew.-%] | 43,1 | 33,1 | 38,2 | 37,6 |
| Phytosterol [Gew.-%] | 2,6 | 2,8 | 3,3 | 5,8 |
| 2. Ansatz (Phase B) | | | | |
| Saccharose [Gew.-%] | 11,4 | 10,7 | 13,4 | 13,5 |
| Wasser [Gew.-%] | 28,4 | 43,1 | 33,4 | 33,7 |
| Summe | 100,00 | 100,00 | 100,00 | 100,00 |
| | | | | |
| Partikelgröße [nm] | 460 ± 100 | 360 ± 90 | 530 ± 150 | 180 ± 80 |
| | | | | |
| Mittelwert HLB | 4,17 | 3,93 | 3,96 | 3,47 |
| Mittelwert Log P (ca.) | 1,66 | 1,81 | 1,76 | 2,05 |

Um LNPs mit einer Disk-Struktur herzustellen, sollten die PEGylierten Tenside mit einem nach Griffin berechneten HLB-Wert von mindestens 13 (Log P-Wert höchstens 0,46) und amphiphile nicht wasserlösliche Substanzen mit einem HLB von höchstens 7 (Log P-Wert mindestens 0,93) bzw. von höchstens 11 (Log P-Wert mindestens 0,55) für das Phospholipid verwendet werden. Der mittlere HLB Wert aus den amphiphilen und pegylierten Substanzen kann vorzugsweise im Bereich von 3,5 bis 5,5 (Log P im Bereich von1,22 bis 2,05) liegen.

Variation der wasserunlöslichen amphiphilen Substanzen

In den hier beschriebenen submicron Disk-LNP können die Konzentrationen innerhalb der wasserunlöslichen amphiphilen Substanzen variiert werden, wenn der mittlere HLB-Wert bzw. Log P-Wert im beschriebenen günstigen Bereich bleibt. In dem hier beschriebenen Beispiel (Tabelle 6) wird die Konzentration von Phytosterol gezielt erhöht, ohne dass der gewünschte Partikelgrößenbereich verlassen wird. Zudem ist anzuführen, dass Phytosterole einen antioxidativen Effekt zeigen. In alternativen Zusammensetzungen bzw. Ansätzen mit Phosphatidylcholin werden vergleichbare Eigenschaften erhalten.

**Tabelle 6:**

| 1. Ansatz (Phase A) | |
|---|---|
| Glycerinmonolaurat [Gew.-%] | 5,5 |
| PEG 40-Stearat [Gew.-%] | 8,7 |
| Caprinsäure/Caprylsäure-Triglycerid [Gew.-%] | 25,1 |
| Phytosterol [Gew.-%] | 8,2 |
| Glycerin [Gew.-%] | 3,8 |
| 2. Ansatz (Phase B) | |
| Saccharose [Gew.-%] | 9,6 |
| Wasser [Gew.-%] | 39,1 |
| Summe | 100,00 |
| | |

(fortgesetzt)

| 2. Ansatz (Phase B) | |
|---|---|
| Partikelgröße [nm] | 480 ± 130 |
| | |
| Mittelwert HLB | 4,8 |
| Mittelwer Log P | 1,43 |

Formulierungen mit wasserunlöslichen amphiphilen Wirkstoffen

Wie die oben ausgeführte Rezeptur am Beispiel des wasserunlöslichen amphiphilen Wirkstoffs zeigt, ist es also auch möglich, eine solche in eine Disk-LNP Formulierung einzubauen.

Andere mögliche, insbesondere nicht wasserlösliche amphiphilen Wirkstoffe sind in nicht beschränkender Weise z.B. mRNA, Retinol, Cortison, Antibiotika wie z.B. Tetracykline oder Benzoylpenicilline, Diclofenac, Estradiol, Estradiol-Hemihydrat, Salicylsäure, Aspirin, Indometacin, ätherische Öle, amphiphile UVA und UVB Filter.

Als wasserlösliche pharmazeutische Aktivstoffe kommen sämtliche wasserlösliche Verbindungen in Betracht, die mit den Trägersubstanzen der Disk-LNP eine Wechselwirkung, wie eine elektrostatische Bindung, eingehen können, wie z.B. mRNA. Disk-LNP, die pharmazeutische Wirkstoffe enthalten, können sowohl intramuskulär, intravenös, oral als auch topisch angewandt werden. In der topischen Anwendung zeichnen sich Disk-LNP durch gutes und kontrolliertes Penetrationsverhalten aus. Über den Einsatz von Panthenol kann das Penetrationsverhalten der hier beschriebenen LNP weiter gesteuert werden.

Tabelle 7 zeigt eine weitere Zusammensetzung bzw. diesbezügliche Ansätze. Eine Ausrüstung mit Wirkstoffen führt zu vergleichbaren Eigenschaften.

**Tabelle 7:**

| 1. Ansatz (Phase A) | |
|---|---|
| Polysorbat 80 [Gew.-%] | 14 |
| Tocopherol [Gew.-%] | 12 |
| Phospatidylcholin [Gew.-%] | 1,6 |
| Phytosterol [Gew.-%] | 2,5 |
| Caprinsäure/Caprylsäure-Triglycerid [Gew.-%] | 41,9 |
| **2. Ansatz (Phase B)** | |
| Saccherose [Gew.-%] | 4,5 |
| Wasser [Gew.-%] | 23,5 |
| | |
| | 100 |
| | |
| Partikelgröße [nm] | 380 ± 80 |
| | |
| mittlerer HLB bzw. Log P | 4,2 bzw. 1,66 |

Herstellungsverfahren für Disk-LNP

Bezüglich der Herstellung von Disk-LNP werden nachfolgen entsprechende Verfahren beschrieben. Insbesondere kann auf Basis eines direkten Herstellungsverfahren oder eines inversen Verfahrens vorgegangen werden.

Bei dem inversen Verfahren wird die gesamte Trägerphase auf Basis des PEGylierten Tensids und den nicht wasserlöslichen Substanzen in ihrer durch Aufschmelzen gebildeten flüssigen Phase vorgelegt und die Wasserphase portionsweise zugegeben.

Überraschenderweise kann man bei der Herstellung von Disk-LNP nach dem inversen Verfahren auf ein heiß-kalt Verfahren zurückgreifen. Obwohl die in den vorherigen aufgeführten Beispielen beschriebene Phase A auf 80 °C bis 90 °C erwärmt wird, kann in diese Phase die Wasserphase (Phase B) mit einer Temperatur von 25 °C bis 30 °C entweder langsam zugegeben oder portionsweise eingerührt werden. Eine Temperatur der Wasser-Phase B von größer 40 °C beim Einrühren führt demgegenüber nicht zu optimalen Ergebnissen.

Für die Anwendung des inversen Verfahrens ist es vorteilhaft, der aufgeschmolzenen Phase A, die eine Temperatur von 50 °C bis 65 °C aufweist, eine wässrige Phase B, die einen Polyolgehalt von vorzugsweise 20 Gew.-% bis 50 Gew.-% enthält, insbesondere mit einer Temperatur von 25°C bis 30°C unter Rühren zuzuführen und anschließend die Phase C unter Rühren einzuarbeiten.

Hierzu kann auch auf Tabelle 8 mit den diesbezüglichen Ansätzen verwiesen werden:

**Tabelle 8:**

| 1. Ansatz (Phase A) | |
|---|---|
| Glycerinmonolaurat [Gew.-%] | 5,5 |
| PEG 40-Stearat [Gew.-%] | 8,7 |
| Caprinsäure/Caprylsäure-Triglycerid [Gew.-%] | 25,1 |
| Phytosterol [Gew.-%] | 8,2 |
| Glycerin [Gew.-%] | 3,8 |
| **2. Ansatz (Phase B)** | |
| Saccherose [Gew.-%] | 9,6 |
| Wasser [Gew.-%] | 28,8 |
| **weiterer Ansatz (Phase C)** | |
| Saccherose [Gew.-%] | 10,3 |
| | |
| | 100 |
| | |
| Partikelgröße [nm] | 480 ± 130 |
| | |
| mittlerer HLB bzw. mittlerer Log P | 4,8 bzw. 1,43 |

Bei der direkten Einarbeitung (Tabelle 9) der Phase A, die eine Temperatur von 80 °C bis 90 °C aufweist und eine homogene flüssige Phase darstellt, in die wässrige Phase B, die vorzugsweise 15 Gew.-% bis 25 Gew.-% der PEGylierten Substanz enthält, ist folgende Vorgehensweise für den Erhalt einer flüssig-kristallinen Disk-Phase zielführend.

Die Phase A wird langsam oder portionsweise unter Rühren in die Phase B gegeben, die eine Temperatur von vorzugsweise 25 °C bis 40 °C hat. Während der Zugabe der Phase steigt die Viskosität der erhaltenen Mischung mit zunehmender Konzentration und erreicht nach Ende der Zugabe einen stark viskoelastischen Gel-Bereich. Die erhaltene Gelphase wird auf 20 °C abgekühlt.

**Tabelle 9:**

| 1. Ansatz (Phase A) | |
|---|---|
| Glycerinmonolaurat [Gew.-%] | 5,7 |
| PEG 40-Stearat [Gew.-%] | 6,8 |
| Caprinsäure/Caprylsäure-Triglycerid [Gew.-%] | 26 |
| Phytosterol [Gew.-%] | 8,5 |
| Glycerin [Gew.-%] | 3,9 |
| **2. Ansatz (Phase B)** | |
| Saccherose [Gew.-%] | 13,3 |

(fortgesetzt)

| 2. Ansatz (Phase B) | |
|---|---|
| Wasser [Gew.-%] | 33,5 |
| Saccherose [Gew.-%] | 2,2 |
| | |
| | 100 |
| | |
| Partikelgröße [nm] | 640 ± 170 |

Die Verfahren, insbesondere das direkte Verfahren, können auch mit einem kaskadierten kontinuierlichen Verfahren eingesetzt werden. Beide Verfahren eignen sich sowohl für die Herstellung von lamellaren flüssig kristallinen als auch kubischen flüssig-kristallinen Strukturen, die bei ihrer Verdünnung stabile LNP-Strukturen zeigen. In alternativen Zusammensetzungen bzw. Ansätzen mit Phosphatidylcholin bzw. einem (weiteren) Wirkstoff können vergleichbare Eigenschaften erhalten werden.

Beim Rühren der geligen Phase kann es der Fall sein, dass die Rührenergien in Wärme dissipiert. Dem kann durch eine Kühlung entgegengewirkt werden.

Elektrolytverträglichkeit

Die Wasserphase kann auch neben den Polyolen auch Elektrolyte enthalten, die beispielsweise zur pH-Einstellung des Systems herangezogen werden können. Die hierfür einsetzbaren Elektrolytkonzentrationen haben keinen nennenswerten negativen Einfluss auf die gebildete Struktur der lyotropen flüssig-kristallinen Phase. Zur Einstellung des pH-Wertes über eine wässrige Elektrolytlösung sollte die Wasserkonzentration so gewählt werden, dass der liquid-kristalline Bereich nicht verlassen wird, damit alle Bereiche der einzelnen Kompartimente erreicht werden können.

Herstellung von LNP mit PEGylierten Tensiden und Phosphatidylcholin

Ein günstigster Packungsparameter für die Bildung lamellarer flüssig-kristalliner Strukturen mit sphärischem Aufbau liegt insbesondere im Bereich $P_{kr}$ = 0,5, was einem HLB-Wertbereich von 10 bis 12 (insbesondere Log P im Bereich von 0,50 bis 0,62) entspricht. Insbesondere in diesem HLB-Bereich können insbesondere vesikelartige Strukturen gebildet werden, die eine kubische Form zeigen. Wird die kubische Form mit Wasser verdünnt, bilden sich sphärische LNPs.

Wird ein PEGyliertes Tensid zur Bildung dieser Phasen eingesetzt, ist dieses über die gesamte Lamelle des Vesikels gleichmäßig verteilt. Als Träger für Aktivstoffe, die in eine menschliche Zelle eingeschleust werden sollen, sind Vesikel, die mit PEGylierten Substanzen gebildet werden, jedoch weniger gut geeignet, da die PEG-Reste der pegylierten Substanz eine sterische Barriere mit der Zelle bilden können.

Auch die Beladung mit wasserunlöslichen amphiphilen Substanzen, wie zum Beispiel Phosphatidylcholin, Cholesterol oder Phytosterol ist mitunter stark begrenzt. Phytosterole zeigen gegenüber Cholesterol den Vorteil, dass diese nachweislich auch antioxidative Eigenschaften haben, was einen Schutz gegen die Oxidation empfindliche Aktivstoffe darstellt.

Nachfolgend werden weitere Formulierungen gemäß der Tabelle 10 angeführt, auch zur Demonstration des der obigen Ausführungen. Dabei zeigt die Tabelle auch die Ergebnisse der Formulierungen von flüssig-kristallinen Gelstrukturen.

**Tabelle 10:**

| 1. Ansatz (Phase A) | | | | |
|---|---|---|---|---|
| Phospatidylcholin [Gew.-%] | 4,5 | 4,4 | 4,4 | 4,5 |
| Caprinsäure/Caprylsäure-Triglycerid [Gew.-%] | 5,4 | 9,1 | 16,8 | 16,6 |
| PEG 40-Stearat [Gew.-%] | 19,1 | 16,8 | 9,1 | 10,1 |
| Phytosterol [Gew.-%] | 0,9 | 3,5 | 3,5 | 4,1 |
| Glycerin [Gew.-%] | 26,6 | 25,6 | 25,7 | 23,8 |

(fortgesetzt)

| 2. Ansatz (Phase B) | | | | |
|---|---|---|---|---|
| Wasser [Gew.-%] | 43,5 | 40,6 | 40,6 | 41,0 |
| | | | | |
| | 100,00 | 100,00 | 100,00 | 100,00 |
| | | | | |
| Partikelgröße [nm] | 400 ± 100 | 320 ± 70 | 6.000 | 8.000 |
| | | | | |
| Mittelwert HLB | 11,7 | 10,1 | 9,5 | 9,1 |
| Mittelwert Log P | 0,52 | 0,61 | 0,65 | 0,69 |

Die liquid kristallinen Gelstrukturen mit einem mittleren HLB Wert von 11,7 (Log P = 0,52) und 10,1 (Log P = 0,61) repräsentieren klare Gele, die man der kubischen Struktur zuordnen kann. Es handelt sich hierbei um sphärische oder ellipsoide Vesikelstrukturen.

Die Gelstrukturen der Formulierungen mit einem mittleren HLB Wert von 9,5 und 9,1 (Log P = 0,65 und 0,69) ) zeigen eine milchige Struktur und können somit nicht der kubischen Form zugeordnet werden. Man kann davon ausgehen, dass sich in der Gelphase zumindest zwei Strukturen überlagern. Die breite Streuung der Partikelgröße, die sich im skalierten $\mu$m-Bereich befindet, zeigt, dass das Gel eine uneinheitliche liquid kristalline Struktur aufweist und demnach kein homogenes System bildet. Ein solcher unerwünschter Effekt zeigt sich mitunter auch bei den derzeit verwendeten mRNA-Impfstoffen.

Für die Messung der Partikelgröße wurde ein Horiba la-920 Particle Size Analyzer benutzt.

Die Herstellung der flüssig-kristallinen Gele erfolgt nach der folgenden Prozedur. Die in der Phase A enthaltenen Komponenten Phospatidylcholin, das dem Sojalecithin entstammt, PEG 40 Stearate und Glycerin werden auf 70°C erwärmt und solange gerührt, bis eine homogene klare Lösung entsteht. Parallel wird Phytosterol in Caprinsäure/Caprylsäure-Triglycerid bei 80°C unter Rühren gelöst und anschließend in die aus Phospatidylcholin, PEG 40 Stearate und Glycerin bestehende Lösung homogen eingerührt. Zu dieser Phase A wird Wasser der Phase B, das eine Temperatur von 20 °C bis 30 °C aufweist, portionsweise unter Rühren mit laminarem Fluss eingearbeitet.

Eine Unterstruktur der vesikelartigen liquid kristallinen Lamellarstruktur ist die Disk-Struktur, die durch ein Tensid mit einem nach Griffin kalkulierten HLB-Wert von mindestens 13 (insbesondere Log P von höchstens 0,46) und nicht wasserlöslichen amphiphilen Substanzen mit einem HLB von insbesondere höchstens 6 (insbesondere Log P von mindestens 1,11) gebildet wird. Der aus den HLB Werten der Tenside mit HLB von mindestens 13 und den nicht wasserlöslichen amphiphilen Substanzen mit HLB von höchstens 6 gemittelte HLB Wert liegt hier vorzugsweise bei einem HLB von höchstens 5 (insbesondere Log P von mindestens 1,36).

Flüssig-kristalline Disk-Strukturen zeigen eine zylinderartige Geometrie (vgl. z.B. Fig. 1A). Dabei kann es sich insbesondere derart verhalten, dass in dieser Geometrie die Mantelfläche dem Gesetz der Packungsparameter vorrangig, insbesondere fast ausschließlich, mit pegylierten Tensiden belegt ist, während die kreisförmigen Grundflächen maßgeblich mit den nicht wasserlöslichen amphiphilen Komponenten belegt sind (vgl. z.B. Fig. 1B).

**Tabelle 11:**

| 1. Ansatz (Phase A) | | | |
|---|---|---|---|
| Phospatidylcholin [Gew.-%] | 5,9 | 6,8 | 7,7 |
| Caprinsäure/Caprylsäure-Triglycerid [Gew.-%] | 14,0 | 16,4 | 18,1 |
| PEG 40-Stearat [Gew.-%] | 5,0 | 6,8 | 8,6 |
| Phytosterol [Gew.-%] | 3,1 | 3,7 | 4,1 |
| Glycerin [Gew.-%] | 27,1 | 29,6 | 30,1 |
| 2. Ansatz (Phase B) | | | |
| Wasser [Gew.-%] | 44,9 | 36,6 | 31,4 |
| | | | |
| | 100,00 | 100,00 | 100,00 |

(fortgesetzt)

| 2. Ansatz (Phase B) | | | |
|---|---|---|---|
| | | | |
| mittlerer HLB bzw. Log P | 4,8 bzw. 1,43 | 5,1 bzw. 1,33 | 5,4 bzw. 1,25 |
| | | | |
| Partikelgröße [nm] | 1.200 ± 300 | 540 ± 150 | 400 ± 90 |

Überraschenderweise kann man mit derselben Komponenten-Matrix, die zur Herstellung liquid kristalliner sphärischer Vesikel-Strukturen eingesetzt wurde, auch liquid kristalline Disk-LNP Strukturen herstellen (Tabelle 11).

Die Herstellung der hier gezeigten Herstellung von Disk-Strukturen erfolgt nach derselben Prozedur wie die oben gezeigte Herstellung der Vesikel-Strukturen. Die Herstellung der Disk-Strukturen kann sowohl nach der direkten und inversen Phase erfolgen. Um eine Disk-Struktur herzustellen, sollten PEGylierte Tenside mit einem nach Griffin berechneten HLB-Wert von mindestens 13 (insbesondere Log P von höchstens 0,46) und amphiphile nicht wasserlösliche Substanzen mit einem HLB von insbesondere höchstens 5 (insbesondere Log P von mindestens 1,36) verwendet werden. Der mittlere HLB Wert aus den amphiphilen und pegylierten Substanzen liegt bei der Kombination von PEGylierten Substanzen und Phosphatidylcholin vorzugsweise im Bereich von 4,5 bis 6 (Log P im Bereich von 1,11 bis 1,54).

In den vorliegenden Rezepturen wird als PEGyliertes Tensid PEG 40 Stearat eigesetzt. Gegebenenfalls vorliegendes freies PEG eingesetzt, das bekanntlich mindestens 20 % bis 30 % freies PEG enthält. Das bedeutet, dass die effektive Einsatzkonzentration von PEG 40 Stearate in den vorliegenden Rezepturen um etwa 30 Prozent niedriger angesetzt werden muss. Dieses kann gegebenenfalls freies PEG enthalten (insbesondere etwa 20 % bis 30 %), was dem Fachmann bekannt ist. Für diesen Fall gilt, dass die effektive Einsatzkonzentration bzw. -menge von PEG 40 Stearate bzw. des entsprechenden PEGylierten Tensids in den vorliegenden Rezepturen um den entsprechenden Prozentwert niedriger anzusetzen ist.

Die vorliegend beschriebenen Formulierungen sind über einen weiten Temperaturbereich von etwa -15°C bis 40°C lagerstabil. Die Partikelgröße ändert sich über einen Lagerzeitraum von 6 Wochen allenfalls nur unwesentlich.

In alternativen Zusammensetzungen mit einem (weiteren) Wirkstoff können vergleichbare Eigenschaften erhalten werden.

Herstellung von sphärischen lamellaren Vesikelstrukturen als Träger für mRNA Systeme

In den bereits zugelassenen mRNA-Impfstoffen gegen SARS-CoV-2 sind die im folgenden beschriebenen PEGylierten Tenside und wasserunlöslichen amphiphilen Substanzen enthalten, denen auch ein HLB-Wert zugeordnet werden kann. Die Trägersubstanzen ALC-0159, DMG-PEG 2000, ALC-0315 und SM-102 werden zusammen mit DSPC und Cholesterol kombiniert. Soll aus diesen Substanzen eine homogene und zusammenhängende flüssig-kristalline Vesikel-Struktur gebildet werden, sollte der mittlere HLB-Wert, der aus allen beteiligten Substanzen gebildet wird, vorzugsweise im Bereich HLB von 9,8 bis 12 liegen. Als Beispiel für eine geeignete Zusammensetzung amphiphiler Komponenten soll insbesondere unter Einbezug der obigen Ausführungen die folgende prozentuale Zusammensetzung der Komponenten dienen.

**Tabelle 12:**

| | |
|---|---|
| ALC-0159 [Gew.-%] | 67,7 |
| ALC-0315 [Gew.-%] | 20,8 |
| DSPC [Gew.-%] | 7,8 |
| Cholesterol [Gew.-%] | 3,6 |
| | |
| Summe | 100,0 |

Eine derartige Zusammensetzung wird in einer daraus erzeugten flüssig-kristallinen Struktur lamellare sphärische Vesikel-Kompartimente erzeugen, die beim Verdünnen mit einer wässrigen Lösung in eine nano-skalierte stabile Struktur übergehen. Wässrige mRNA-Lösungen können einfach und homogen verteilt in die flüssig-kristalline Struktur eingerührt werden.

Herstellung von Disk-Lamellen als Träger für mRNA Systeme

Für die Formierung von Disk-Lamellen bzw. Disk-LNPs ist ein bevorzugter mittlerer HLB-Wert, der aus den pegylierten und wasserunlöslichen amphiphilen Substanzen gebildet wird, von HLB = 4,5 bis 6 bzw. Log P = 1,11 bis 1,54 von Vorteil.

Beispielsweise kann die folgende Zusammensetzung herangezogen werden:

**Tabelle 13:**

| ALC-0159 [Gew.-%] | 17,1 |
|---|---|
| ALC-0315 [Gew.-%] | 57,1 |
| DSPC [Gew.-%] | 11,4 |
| Cholesterol [Gew.-%] | 14,3 |
| | |
| Summe | 100,0 |

Eine derartige Zusammensetzung wird in einer daraus erzeugten flüssig-kristallinen Struktur lamellare Disk-Kompartimente bzw. Disk-LNPs erzeugen, die beim Verdünnen mit einer wässrigen Lösung in eine nano-skalierte stabile Struktur übergehen. Wässrige mRNA-Lösungen können einfach und homogen verteilt in die liquid kristalline Struktur eingerührt werden.

Vorteile von submicron bzw. nanoskaligen Disk-LNP bei der Einarbeitung von mRNA

Die kleinen Disk-LNP weisen eine sehr große spezifisch Oberfläche auf, welche es erlaubt, mRNA insbesondere bei niedriger Temperatur so einzuarbeiten, dass sie mit insbesondere kationischen Trägerkomponenten in elektrostatische Wechselwirkung treten kann und so sehr gleichmäßig und gezielt in den Disk-Kompartimenten unter laminarem Rühreinfluss verteilt werden können.

Da die lytrope flssig-kristalline Phase eine sehr geringe Wasseraktivität zeigt, ist damit auch das Risiko von Oxidationserscheinungen oder anderen chemischen Reaktionen stark unterdrückt und mRNA sehr gut schon bei Temperaturen oberhalb des Gefrierpunktes von Wasser geschützt.

Die niedrige Wasseraktivität der flüssig-kristallinen Phase beruht zum einen auf das Bindungsvermögen des Wassers an den hydrophilen Kopfgruppen der PEGylierten Tenside und der wasserunlöslichen amphiphilen Komponenten und zum anderen auf das Wasserbindungsvermögen der einsetzten Polyole.

Die Partikelgröße wird unterhalb der Phasenübergangstemperatur durch intensives Rühren im laminaren Strömungsbereich gebildet. Der Temperaturbereich des Phasenübergangs befindet sich im Bereich der Temperatur, bei der die Kristallisation von Substanzen aus der flüssigen homogenen geschmolzenen Phase der PEGylierten und amphiphilen Substanzen einsetzt. Die Rührdauer in diesem Bereich bestimmt die Partikelgröße. Je länger intensiv unter der Berücksichtigung der laminaren Strömung gerührt wird, desto kleiner werden die Partikel.

Die kritische Phasenübergangstemperatur kann über DSC-Messungen bestimmt werden. Die DSC-Messungen zeigen auch den Übergangsbereich der bei der Phasenübergangstemperatur vorliegenden flüssigen Form der lamellaren Disk-Struktur in eine feste Gelstruktur.

Herstellung mit Inhaltsstoffen Phosphatidylcholin

Für eine Herstellung flüssig-kristalliner LNP-Disk-Strukturen sollte im ersten Schritt die gesamte Phase aller PEGylierten und amphiphilen Substanzen soweit erwärmt wird, bis ein homogenes klares bis ein leicht transparentes Gemisch erscheint.

Sind in der Formulierung Phosphatidylcholine und Cholesterol oder Phytosterol enthalten, ist folgende Vorgehensweise vorteilhaft. Zuerst wird das PEGylierte Tensid zusammen mit Glycerin oder einem anderen vorzugsweisen flüssigem Polyol und einem Phosphatidylcholine bei 70 °C bis 80 °C aufgeschmolzen. Das Polyol wird in diese Phase eingebracht, um schwer lösliche Phosphatidylcholine, wie zum Beispiel Distearoylphosphatidylcholin, in Lösung zu bringen. Phosphatidylcholine sind sehr gut bezüglich der Phasen-Übergangstemperatur beschrieben. Um ein Phosphatidylcholin in einem flüssigen Polyol zu lösen, sollte die Temperatur oberhalb Phasen-Übergangstemperatur liegen.

Der Polyolgehalt, bezogen auf die Lipidphase, die insbesondere aus amphiphilen wasserunlöslichen Substanzen zusammengesetzt ist, sollte vorzugsweise 5 Gew.-% bis 50 Gew.-% betragen.

Anschließend werden die restlichen amphiphilen Substanzen zugeführt und homogen gerührt. Zuletzt wir das Cholesterol oder Phytosterol zu gegeben und solange gerührt, bis eine homogene Flüssigkeit entstanden ist. Die Temperatur sollte jetzt vorzugsweise 80 °C bis 90°C betragen.

Überraschenderweise kann jetzt die Wasserphase, die vorzugsweise aus einer Poyollösung besteht, mit einer Temperatur von vorzugsweise 20 °C bis 50 °C langsam oder Schrittweise unter Rühren zugegeben werden, und zwar in einer Konzentration bis sich eine wasserlösliche Gelphase eingestellt hat.

### Übertragen der Ergebnisse auf mRNA-Impfstoffe

Glycerinmonolaurat wird gewählt, weil es mit einem von HLB von 5,2 (Log P von 1,30) in etwa dem des Distearoylphosphatidylcholin HLB = 4,5 (Log P = 1,54) entspricht. Es konnte gezeigt werden, dass etwa gleiche HLB Werte bzw. Log P-Werte in der Formulierung von Disk-LNP zu gleichen strukturellen Ergebnissen führen.Um mit den in mRNA Impfstoffen verwendeten Rohstoffe in einen für Disk-LNP Bereich zu gelangen, ist die Formulierung insbesondere so zu gestalten, dass die Konzentration der pegylerten Substanz zusammen mit den wasserunlöslichen Komponenten einen mittleren HLB Wert von HLB = 4,5 - 6,0 (Log P = 1,11 - 1,54) ergibt.

Bei der Produktion von mRNA Disk-LNP wird nach der abgeschlossenen liquid kristallinen Gelbildung eine wässrige mRNA Lösung zugegeben. Anschließend wird mit einer weiteren wässrigen Phase der endgültige pH-Wert eingestellt.

### Herstellung von Disk LNP für topische Anwendungen

Disk-LNP eigen sich, insbesondere wenn sie in ihrer Matrix Phosphatidylcholine, die für eine gute Bioverfügbarkeit sorgen, enthalten, sehr gut für topische Anwendungen, da sie zum einen nach der Applikation durch die gute Filmbildung auf der Haut eine gute Penetration des Wirkstoffes garantieren und zum anderen für den Anwender ein angenehmes Hautgefühl während und nach der Applikation zeigen. Enthalten Disk-LNP auch als amphiphile Substanz beispielsweise Phytosterole, die einen antioxidativen Effekt zeigen, eignen sich diese insbesondere auch für Aktivstoffe, die an der Luft zur Oxidation neigen, wie zum Beispiel Tocopherole oder Retinol. In diesem Zuammenhang kann auch unter Verwendung von Vitamin C, insbesondere zusammen mit Panthenol, ein Oxidationsschutz bereitgestellt werden. Auch führt die Verwendung von Vitamin D insbesondere als Lamellen- bzw. Membran-Antioxidans zu einem Oxidationsschutz.

Beispielsweise können in diesem Zusammenhang Konzentrate oder dergleichen in Disk-Lamellen eingearbeitet werden.

### Vorteile der erfindungsgemäß vorliegenden Disk-LNP

Ein überraschendes Ergebnis der vorliegend beschriebenen Disk-LNP ist die erfindungsgemäß hierfür bereitgestellte Produktion bzw. das diesbezügliche Verfahren, welches auch mit einer Vereinfachung der Herstellung der Partikel einhergeht. Insbesondere können auch in der Pharmazie vorhandene bzw. herkömmliche Produktionsanlagen verwendet werden. Mit herkömmlichen Mischanlagen können auch größere Batches problemlos produziert werden. Ein weiteres Merkmal ist die konstante Qualität der erhaltenen Produkte. Formulierungen können auch schnell auf andere Wirkstoffe ohne großen Forschungsaufwand adaptiert werden.

### Online Produktionskontrolle

Für die Produktionsüberwachung können zudem beispielsweise folgende für jedes System charakteristische Parameter online überprüft werden. Charakteristische Größen sind z.B. Temperatur, Viskosität und Leitfähigkeit. Bei einer online-Messung dieser charakteristischen Parameter ist es möglich, die Produktion von Disk-LNP und anderen liquidkristallinen LNP beispielsweise Computer-gesteuert durchzuführen.

Eine über Computer gesteuerte und überwachte Herstellung ist auch für eine Produktion in den meisten Regionen weltweit von Vorteil, um Pandemien schneller in den Griff zu bekommen.

### Zusammenfassung

Herstellung und Formulierung von lytropen flüssig-kristallinen Vesikel- und Disk-LNPs bzw. diesbezügliche Zusammensetzungen, welche

- Phosphatidylcholin insbesondere in Kombination mit Phytosterol oder Cholesterol, aufweisen,
- deren mittlerer HLB-Wert der eingesetzten pegylierten Substanzen HLB ≥ 13 und der wasserunlöslichen amphiphilen Substanzen insbesondere HLB ≤ 6 beträgt (entsprechendes gilt für die korrelierenden Log P-Werte),

- im heiß-kalt Verfahren hergestellt werden können,
- eine gute Lagerstabilität über einen weiten Temperaturbereich aufweisen,
- insbesondere eine Wasseraktivität unter 1 aufweisen,
- deren Herstellung in wenigen Schritten erfolgen kann,
- insbesondere unter laminaren Strömungsbedingungen insbesondere unter Rühren hergestellt werden,
- es erlauben, bei der Herstellung die wichtigsten Prozessparameter zu überwachen und über eine IT-Technologie das Verfahren zu steuern,
- in einem direkten oder inversen Herstellungsverfahren hergestellt werden können,
- derart hergestellt werden können, dass zusammenhängende liquid kristalline Phasen durch Zusätze von Polyolen ermöglicht bzw. verbessert werden,
- es ermöglichen, wasserlösliche Aktivstoffe wie zum Beispiel mRNA, bei niedrigen Temperaturen durch Rühren homogen zu verteilen,
- den stabilen Einbau amphiphiler Wirkstoffe gezielt in die LNP-Matrix ermöglichen,
- beim Verdünnen mit Wasser eine enge Gaußsche Partikelgröße zeigen,
- im pharmazeutischen Bereich sowohl intramuskulär, intravenös, oral als auch topisch angewandt werden können.

## TEIL D:

### Weitere Ausführungen zur Aufgabenstellung

Die Herstellung LNPs, insbesondere mRNA-LNPs, ist schwierig und führt im Stand der Technik mitunter zu sehr inhomogenen Systemen, was auch eine sehr komplexe Anwendung bei der Applikation erfordert. Bekannte Systeme müssen zur Lagerung auf tiefe Temperaturen gekühlt werden. Zudem ist eine aufwendige Handhabung vor Verabreichung erforderlich (unbedingtes Vermeiden von Scherkräften, die beim Schütteln entstehen würden, aufwendiges Verdünnen, vertikales Kippen vor Verdünnung, kurze Lagerzeit nach Auftauen, Verwendung spezieller Kanülen, kein längerer Transport nach Verdünnung etc.). Zudem liegen auch oftmals Partikel im hohen $\mu$m-Bereich vorm

Die Gründe für die Inhomogenität des Impfstoffes kann auf mehrere Gründe zurückgeführt werden. Insbesondere ist bislang eine ausreichende Löslichkeit der Hauptkomponenten Phosphatidylcholin und Cholesterol problematisch. Phosphatidylcholine werden in den bisherigen Verfahren zunächst in Ethanol gelöst, das dann in einem nächsten Schritt über Vakuum aufwendig abgedampft werden muss. Die Löslichkeit von Cholesterol wird meist über Chloroform erreicht, das dem System ebenfalls über Vakuum entzogen werden muss. Bei den bisherigen Herstellungsverfahren kann es zudem zu mitunter erheblichen Batch-zu-Batch-Schwankungen bezüglich der Qualität kommen.

Oftmals werden die Partikel, insbesondere Vesikel, die Phosphatidylcholine zusammen mit Cholesterol enthalten, im Ultraschall-Verfahren hergestellt, der Energieeintrag solcher Verfahren ist dabei nicht unerheblich. So können im Beschallungsfeld implodierende Kavitationsblasen intensive Schockwellen in der Flüssigkeit und Flüssigkeitsstrahlen von sehr hoher Geschwindigkeit verursachen. Diese Flüssigkeitsstrahlen verringern die Größe der einzelnen Tropfen in der beschallten Flüssigkeit und "durchmischen" diese. Ein solches Verfahren führt also oftmals zu einem sehr hohen lokalen Energieeintrag und stellt somit insgesamt ein nicht gut zu kontrollierendes Verfahren dar. Der Energieeintrag von Ultraschallprozessoren beträgt für das Labor 50 W bis 400 W, für die industrielle Produktion werden 500 Watt- bis 16 kW-Prozessoren eingesetzt. Insgesamt sind übliche Verfahren zur Herstellung von LNPs, die als Träger für mRNA dienen, in der Fachliteratur als sehr kompliziert und aufwendig beschrieben. Der Grund für das komplexe Herstellungsverfahren ist die sehr schlechte Wasserlöslichkeit von DSCP und Cholesterol.

### Weitere Ausführungen zur Problemlösung

Dieses Problem kann insbesonder dadurch gelöst werden, dass DSCP in einer wässrigen Polyollösung gelöst wird. Als Polyole eignen sich zum Beispiel Propylenglycol, Glycerin oder Saccharose. Überraschenderweise lassen sich die meisten Phosphatidylcholine schon bei Raumtemperatur in einem Saccharose-Wassergemisch oder in anderen wässrigen Polyolmischungen vollständig

Sojalecithin enthält auch Glykolipide. Glykolipide sind phosphorfreie Strukturlipide oder Membranlipide (Bestandteile von Zellmembranen), bei denen ein oder mehrere Mono- oder Oligosaccharide glycosidisch an ein Lipid-Molekül gebunden sind. Glykolipide kommen in allen Geweben vor, allerdings ausschließlich auf der Außenseite der Lipiddoppelschicht. Das nachfolgend verwendete Sojalecithin ist ein auf 45 Prozent Phosphatidylcholin angereichertes Produkt (Lipoid® S45). Der Anteil an Glykolipiden kann mit etwa 5 Prozent angenommen werden. In Lecithin-Gelen, die auf wässrige Polyollösungen basieren, können erfindungsgemäß auch wasserlösliche Tenside mit einem HLB Wert von mindesten 13 insbesondere kalt eingearbeitet werden. Da die in den mRNA-Impfstoffen verwendeten Tenside ALC-0159 und DMG-PEG 2000 einen ausreichen hohen HLB Wert haben, können auch diese in Lecithingele kalt eingearbeitet werden. In derartige Lecithin-Tensid-Gele können auch unter lamellarem Rührfluss relativ hohe verzweigtkettige Alkohole, die einen HLB von 2 bis 4 aufweisen, wie Octyldodecanol, ALC-0315 oder SM-102, bei Raumtemperatur oder niedrigeren Temperaturen eingearbeitet werden. Dabei entstehen erfindungsgemäß stabile gelartige semitransparente Strukturen, die mit Wasser verdünnbar sind und bei langsamer Zuführung von Wasser eine submicron bzw. nanoskalige Partikelgröße aufweisen. In derartigen Gelen kann mRNA auch bei niedrigen Temperaturen, z.B. unterhalb von Raumtemperatur, eingearbeitet werden.

Die nachfolgende Tabelle zeigt eine entsprechende Rezeptur:

**Tabelle 14:**

| Sojalecithin [Gew.-%] | 13,0 |
|---|---|
| Zucker [Gew.-%] | 27,9 |
| Wasser [Gew.-%] | 35,7 |
| Octyldodecanol [Gew.-%] | 20,5 |
| Polysorbat 80 [Gew.-%] | 2,9 |
| | |
| Summe | 100,0 |

Die Struktur, die aus dieser Rezeptur entsteht, ist ein semitransparentes Gel, das beim Verdünnen mit Wasser submicron bzw. nanoskalige Partikel bildet. Im verwendeten Tensid können auch Phytosterol oder Cholesterol gelöst werden. In dieses System lassen sich auch andere lipophile Wirkstoffe insbesondere mit einem HLB-Wert im Bereich von 1 bis 5, wie Retinol, Tocopherol, Tetracycline etc. einarbeiten. Hydrocortison mit einem HLB = 4,5 ist zwar schlecht wasserlöslich aber sehr gut löslich in Polyolen. Man kann davon ausgehen, dass Substanzen mit einem HLB Wert HLB = 4-5 das gleich Löslichkeitsspektrum wie Kortison hat. Entsprechendes gilt unter Heranziehung der korrelierenden Log P-Werte.

**Herstellung von LNPs**

Zur der Herstellung eines weiteren stabilen LNP-Systems ist es bevorzugt, dass die Komponenten in zwei Phasen aufgeteilt werden. Die bevorzugte Aufteilung der Komponenten hängt vorliegend von der Löslichkeit des ausgewählten Phosphatidylcholin in einem wässrigen Polyolgemisch ab. Vorliegend beträgt die zur Zerkleinerung der Partikel eingetragene Energie insbesondere 1 bis 2 Watt/kg Emulsion. Bei ausreichend schneller Löslichkeit des ausgewählten Phosphatidylcholins in einem wässrigen Polyolgemisches wie zum Beispiel in einer wässrigen Zuckerlösung bildet diese Lösung eine Phase. Die zweite Phase besteht aus einem PEGylierten Tensid, einem verzweigtkettigen Alkohol und ein Cholesterol bzw. Phytostearin. Vorzugsweise sollte das PEGyliertes Tensid einen HLB-Wert mehr als 13, der verzweigtkettige Alkohol einen HLB-Wert insbesondere im Bereich von 2,5 bis 3,5 und das Cholesterol bzw. Phytostearin einen HLB = 0,9 bis 1,1 aufweisen. Entsprechendes gilt unter Heranziehung der korrelierenden Log P-Werte. In dem vorliegenden Beispiel bleibt die Phosphatidylcholin-Phase bei etwa 20°C. Die zweite Phase wird auf 70 °C bis 80 °C aufgewärmt und gerührt, bis das Cholesterol vollständig aufgelöst ist. Anschließend wird die Phase auf 45 °V bis 50 °C abgekühlt und unter Rühren, das ein lamellares Fließverhalten garantiert, langsam der Phosphatidylcholin-Phase zugegeben. Hierbei wird ein nicht fließfähiges flüssig-kristallines Gel aufgebaut. Das so entstandene Gel kann unter Rühren unter laminarem Fluss mit Wasser verdünnt werden. Die dabei erzeugten Disk-Partikel haben in Abhängigkeit von der Rührdauer und Rührgeschwindigkeit eine Partikelgröße von 170 nm bis 250 nm.

Eine andere Aufteilung der Phasen ergibt sich aus der HLB-Wert bzw. Log P-Wert bedingten natürlichen Löslichkeit der PEGylierten Komponente in Wasser und wässrigen Polyol-Systemen. Vor der Zugabe der zweiten Phase sollte diese hier beschriebene Phase eine Temperatur von 25 °C bis 45 °C aufweisen. Die zweite Phase besteht dann aus dem Phosphatidylcholin und Cholesterol, die bei ca. 70 °C bis 80°C in dem verzweigtkettigen Alkohol gelöst werden. Vor der Zugabe in die erste Phase wird die Lösung vorzugsweise auf eine Temperatur von < 70°C abgekühlt.

Bei der Phasenaufteilung ist es auch möglich, dass ein Teil des Phosphatidylcholins und/oder Cholesterols sowohl in der ersten wie auch zweiten Phase vorhanden sind.

Alle in diesem Versuch verwendeten Stoffe sind in der Europäischen Pharmacopoeia gelistet.

**Tabelle 15:**

| Sojalecithin [Gew.-%] | 3,5 |
|---|---|
| Zucker [Gew.-%] | 16,3 |

(fortgesetzt)

| | |
|---|---|
| Wasser [Gew.-%] | 51,2 |
| Macrogol-40-Stearat [Gew.-%] | 2,9 |
| Octyldodecanol [Gew.-%] | 17,8 |
| Phytosterol [Gew.-%] | 8,3 |
| | |
| Summe | 100,0 |

Eine Substitution des Phytosterols mit Cholesterol führt zu gleichen Ergebnissen. Das PEGylierte Tensid Macrogol-40-Stearat ist ein PEG-40 Stearat mit einem HLB Wert = 16,9 und dem ALC-0159 sehr ähnlich. Der verzweigtkettige Fettalkohol Octyldodecanol hat einen HLB = 3 und ist somit dem ALC-0315 in seinen physikalischen Eigenschaften sehr ähnlich. Bei ALC-0315 handelt es sich um ein verzweigtkettiges amphiphiles kationisches synthetisches Lipid mit einer endständigen OH-Gruppe.

Den Disk-Strukturen bzw. Disk-LNPs liegt geometrisch eine Zylinder-Struktur zugrunde. Der Mantel eines Disk-Zylinders ist maßgeblich mit hydrophilen Tensiden, die insbesondere einen Packungsparameter von 0,3 bis 0,5 aufweisen, belegt, was einem HLB-Wert der Substanzen von 10 bis 17 (insbesondere Log P von etwa 0,34 bis 0,61) entspricht. Die Grundfläche wird durch amphiphile Substanzen mit einem Packungsparameter von etwa 1 belegt. Aus dem Verhältnis der Hydrophilen und lipophilen amphiphilen Komponenten kann man die lamellare Belegung und die dazugehörigen Durchmesser der LNPs ermittelt bzw. gesteuert werden.

**Kontinuierliche Herstellung**

Hierzu kann auch auf Fig. 6A und 6B verwiesen werden. Die Verweilzeit richtet sich nach Durchfluss und Gefäßgröße. Werden bei der Rührkaskade Gefäße mit einem Volumen von 300 ml eingesetzt, kann die mittlere Verweilzeit etwa 10 bis 15 Sekunden je Rührstufe betragen. Hierbei werden die Rührkammern mit aliqouten Teilen aus jeweiligen Vorratvorrichtungen bzw. Vorratskammern gemäß a2 bis d2 befüllt und von Rührkammer A2 (Fig. 6A) bis zur Endstufe D4 weitergeleitet (Fig. 6A). Das Endprodukt kann der Rührstufe D2 entnommen werden. Entspricht die nachfolgende Qualitätskontrolle den Spezifikationen müssen die einzelnen Rührstufen für die nachfolgende Batch-Produktion nicht mehr gereinigt werden. Das Verfahren kann auch semi-kontinuierlich durchgeführt werden, insbesondere um eine Batch-Qualitätskontrolle zu ermöglichen. Die vorliegende Herstellung ist mit sehr geringer Verweilzeit in der jeweiligen Rührkammer möglich. Durch das Verfahren ist eine äußerst geringe Temperaturbelastung des zu verkapselnden Wirkstoffs, wie mRNA, gewährleistet. In entsprechender Weise kann auch auf Fig. 6B verwiesen werden.

**TEIL E: Rührer zur Ausbildung einer laminaren Strömung**

Wendelrührer

Zum Homogenisieren hochviskoser Medien eignet sich der Wendelrührer. Diese Rühreinrichtung weist eine bandförmige Wendel auf, die mit Stegen an der Welle gehalten wird. Im Gegensatz zum Propeller wird in diesem Fall die axiale Strömung nicht durch Druckdifferenzen, sondern durch Verdrängungswirkung im laminaren Strömungsbereich hervorgerufen. Mit einem Durchmesserverhältnis von 0,9 bis 0,99 zählt der Wendelrührer zu den wandgängigen Rührorganen.

Drahtrührer

Auch Drahtrührer eignen sich im besonderen Maße zum Homogenisieren hochviskoser Medien, Durch Abstimmung der Drahtanordnung und Drahtdicke kann die Ausbildung einer turbulenten Strömung entgegengewirkt werden.

Ekato-Paravisc® -Rühreinrichtungen

Diese Rühreinrichtung gehört zur Gruppe der wandgängigen Rührorgane. Diese Rührorgane arbeiten insbesondere im laminaren Bereich, eine Umwälzung des Behälterinhalts wird durch Zwangsförderung erreicht. Die axiale Durchmischung wird durch die spezielle Form und Anordnung der beiden Hauptblätter erreicht. Diese Rühreinrichtung kann je nach Anwendungsfall sowohl nach unten als auch nach oben fördernd betrieben werden.

TEIL F: **Vitamin C als Wirkstoff-Modellsubstanz**

Auf Basis der in Tabelle 16 angeführten Rezeptur wird ein weitere Zusammensetzung bzw. ein diesbezügliches Trägersystem hergestellt, wobei Vitamin C vorliegend als Modellsubstanz für mRNA-Wirkstoffe eingesetzt wird.

**Tabelle 16:**

| 1. Ansatz (Phase A) | |
| --- | --- |
| Panthenol [Gew.-%] | 20,3 |
| Wasser [Gew.-%] | 5,9 |
| Vitamin C [Gew.-%] | 16,9 |
| **2. Ansatz (Phase B)** | |
| Phosphatidylcholin [Gew.-%] | 6,0 |
| PEG 40-Stearat [Gew.-%] | 11,3 |
| Glycerinmonolaurat [Gew.-%] | 2,2 |
| Caprinsäure/Caprylsäure-Triglycerid [Gew.-%] | 23,0 |
| **3. Ansatz (Phase C)** | |
| Wasser [Gew.-%] | 14,4 |
| | |
| Summe | 100 |
| | |
| Partikelgröße [nm] | $140 \pm 0,03$ |
| mittlerer HLB bzw. Log P | 6,1 bzw. 1,08 |

Festes Vitamin C ist relativ stabil, zersetzt sich aber normalerweise in wässriger Lösung insbesondere aufgrund von Oxidationsvorgängen relativ schnell. Demgegenüber weist das Vitamin C in der vorliegenden Zusammensetzung bzw. dem Trägersystem eine hohe Stabilität auf, und dies sogar im Hinblick auf die eingesetzte relativ hohe Vitamin C-Menge von mehr als 15 Gew.-%. Überraschend ist auch die hohe Löslichkeit in Panthenol, welche sogar die Löslichkeit in Wasser übertrifft. In anderen Polyolen, wie Glycerin und Propylenglykol ist die Löslichkeit geringer. Ohne sich auf diese Theorie beschränken oder berufen zu wollen, kann die hohe Löslichkeit von Vitamin C in Panthenol mit dessen kationischem Verhalten erklärt werden. Wenn sogar für hohe Vitamin C-Mengen von mehr als 15 Gew.-% insbesondere in Kombination mit Panthenol oder anderen Polyolen eine hohe Stabilität erreicht wird, dann ist dies ein deutlicher Hinweis darauf, dass auch mRNA-Wirkstoffe auf dieser Basis stabilisiert werden können. Insgesamt zeigt die exzellente Wirkung anhand von Vitamin C als Modellsubstanz für mRNA-Wirkstoffe, dass mRNA-Wirkstoffe entsprechend stabilisiert werden können.

Insgesamt zeigen die Ausführungsbeispiele die mit der vorliegenden Erfindung einhergehenden Vorteile und Besonderheiten.

**Patentansprüche**

**1.** Verfahren zur Herstellung eines lipidbasierten Trägersystems in partikulärer Form, bevorzugt in Form von Lipidnanopartikeln, insbesondere für einen pharmazeutischen Wirkstoff; und/oder zur Herstellung eines mit einem pharmazeutischen Wirkstoff beladenen und/oder ausgerüsteten lipidbasierten Trägersystems in partikulärer Form, bevorzugt in Form von Lipidnanopartikeln,

wobei die Bestandteile des Trägersystems miteinander in Kontakt gebracht werden und/oder miteinander in Wechselwirkung gebracht werden, insbesondere physikalisch und/oder chemisch in Wechselwirkung gebracht werden, derart, dass eine Zusammenlagerung, insbesondere Aggregation, der Bestandteile zur Ausbildung des Trägersystems in partikulärer Form, bevorzugt in Form von Lipidnanopartikeln, bewirkt wird,
wobei die Bestandteile des Trägersystems die folgenden Substanzen umfassen:

(i) mindestens ein Phospholipid, vorzugsweise Phosphatidylcholin;

wobei das Phosholipid einen HLB-Wert nach Griffin von höchstens 11, insbesondere höchstens 8, aufweist und/oder wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden, PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern;

wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol, vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol;

wobei das Steroid und/oder Secosteroid, insbesondere das Sterol, einen HLB-Wert nach Griffin von höchstens 1,5 aufweist und/oder wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins;

wobei das kationische Lipid einen HLB-Wert nach Griffin von höchstens 4 aufweist und/oder wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin, vorzugsweise mindestens einen Mono-, Di- oder Trifettsäureester von Glycerin, bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;

wobei der Mono-, Di- oder Tricarbonsäureester von Glycerin einen HLB-Wert nach Griffin von höchstens 8 aufweist und/oder wobei der Mono-, Di- oder Tricarbonsäureester von Glycerin einen Log P-Wert von mindestens 0,80 aufweist;

wobei das Verfahren, insbesondere die Herstellung des Trägersystems bzw. des mit dem pharmazeutischen Wirkstoff beladenen und/oder ausgerüsteten Trägersystems, in einem flüssigen Medium, insbesondere in einem bei einer Temperatur im Bereich von 20 °C bis 40 °C und Umgebungsdruck flüssigen Medium, vorzugsweise in Form eines Dispersionsmediums, durchgeführt wird und nach Ausbildung des Trägersystems eine disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, resultiert bzw. nach Ausbildung des Trägersystems eine Dispersion des Trägersystems bzw. des mit einem pharmazeutischen Wirkstoff beladenen und/oder ausgerüsteten Trägersystems in dem flüssigen Medium resultiert;

wobei die Zusammenlagerung, insbesondere Aggregation, der Bestandteile zur Ausbildung des Trägersystems unter Eintrag von Energie, bevorzugt Eintrag von Misch- und/oder Rührenergie, in das zumindest einen Teil der Bestandteile und/oder Substanzen des Trägersystems aufweisende flüssige Medium und/oder in eine zumindest einen Teil der Bestandteile und/oder Substanzen des Trägersystems aufweisenden Mischung durchgeführt wird, wobei die eingetragene Energie eingestellt wird derart, dass eine zumindest im Wesentlichen laminare Strömung in dem zumindest einen Teil der Bestandteile und/oder Substanzen des Trägersystems aufweisenden flüssigen Medium und/oder in der zumindest einen Teil der Bestandteile und/oder Substanzen des Trägersystems aufweisenden Mischung vorliegt, insbesondere unter zumindest im Wesentlichen vollständiger Vermeidung der Ausbildung einer nichtlaminaren und/oder turbulenten Strömung und/oder insbesondere unter zumindest im Wesentlichen vollständiger Vermeidung der Ausbildung nichtlaminarer und/oder turbulenter Strömungsanteile; und

wobei eine kontinuierliche Verfahrens- und/oder Prozesskontrolle, insbesondere *online*- und/oder inline-Prozesskontrolle, zur Überwachung des Verfahrensstatus und/oder des Verfahrensfortschritts, vorzugsweise zur Überwachung und/oder Einstellung der Ausbildung der Partikel des Trägersystems, insbesondere Lipidnanopartikel, und zur Überwachung und/oder Einstellung von Verfahrensparametern, ausgewählt aus Eintrag von Energie, bevorzugt Eintrag von Misch- und/oder Rührenergie, Eintrag und/oder Abfuhr von Wärmenergie, Strömungsverhalten, Massendurchsatz, Volumenströmen oder dergleichen, durchgeführt wird; wobei die Verfahrens- und/oder Prozesskontrolle über die Erfassung von mindestens einem Prozessparameter, insbesondere mindestens zwei Prozessparametern, erfolgt, wobei der Prozessparameter ausgewählt wird aus der Gruppe von Temperatur, Leitfähigkeit, Viskosität und pH-Wert jeweils des flüssigen Mediums mitsamt den darin eingebrachten Bestandteilen und/oder Substanzen und/oder der Zusammensetzung;

wobei das Verfahren in zumindest im Wesentlichen vollständiger Abwesenheit von Ethanol durchgeführt wird.

2. Verfahren nach Anspruch 1,

wobei die eingetragene Menge an Energie, insbesondere die eingetragene Menge an Misch- und/oder Rührenergie, eingestellt wird derart, dass eine Reynolds-Zahl $R_e$, insbesondere eine auf eine Rührerströmung bezogene Reynolds-Zahl $R_e$, von höchstens 2.000, insbesondere höchstens 1.500, vorzugsweise höchstens 1.000, bevorzugt höchstens 500, vorliegt; und/oder

wobei der Wirkstoff, insbesondere als Substanz (vi), Bestandteil des Trägersystems ist und/oder einen Bestandteil des Trägersystems ausbildet und/oder wobei das Trägersystem den Wirkstoff, insbesondere als Substanz (vi), aufweist.

3. Lipidbasiertes Trägersystem in partikulärer Form, bevorzugt in Form von Lipidnanopartikeln, insbesondere für einen pharmazeutischen Wirkstoff, und/oder mit einem pharmazeutischen Wirkstoff beladenes und/oder ausgerüstetes lipidbasiertes Trägersystem in partikulärer Form, bevorzugt in Form von Lipidnanopartikeln, erhältlich durch ein Verfahren nach einem der vorangehenden Ansprüche.

4. Lipidbasiertes Trägersystem in partikulärer Form, bevorzugt in Form von Lipidnanopartikeln, insbesondere für einen pharmazeutischen Wirkstoff, und/oder mit einem mit einem pharmazeutischen Wirkstoff beladenes und/oder ausgerüstetes lipidbasiertes Trägersystem, vorzugsweise in partikulärer Form, bevorzugt in Form von Lipidnanopartikeln, wie in Anspruch 3 definiert,

wobei die Bestandteile des Trägersystems zur Ausbildung des Trägersystems zusammengelagert, insbesondere aggregiert sind und/oder wobei das Trägersystem vorzugsweise in partikulärer Form, bevorzugt in Form von Lipidnanopartikeln, vorliegt,

wobei die Bestandteile des Trägersystems die folgenden Substanzen umfassen:

(i) mindestens ein Phospholipid, vorzugsweise Phosphatidylcholin;
wobei das Phosholipid einen HLB-Wert nach Griffin von höchstens 11, insbesondere höchstens 8, aufweist und/oder wobei das Phosholipid einen Log P-Wert von mindestens 0,55, insbesondere mindestens 0,80, aufweist;

(ii) mindestens eine nichtionogene amphiphile und/oder tensidische Substanz, insbesondere ausgewählt aus der Gruppe von Polyglycerinestern, Alkylpolyglykosiden, amphiphilen und/oder tensidischen PEGylierten Substanzen und deren Mischungen oder Kombinationen; vorzugsweise ausgewählt aus tensidischen PEGylierten Substanzen, bevorzugt ausgewählt aus der Gruppe von PEGylierten Lipiden, besonders bevorzugt ausgewählt aus der Gruppe von PEGylierten Fettsäuren und deren Salzen und Estern, PEGylierten Triglyceriden, PEGylierten Wachsen, PEGylierten Phosholipiden, PEGylierten Sphingolipiden, PEGylierten Lipopolysacchariden und PEGylierten Isoprenoiden sowie deren Kombinationen und Mischungen, ganz besonders bevorzugt ausgewählt aus PEGylierten Fettsäuren und deren Salzen und Estern;
wobei die nichtionogene amphiphile und/oder tensidische Substanz einen HLB-Wert nach Griffin von mindestens 13 aufweist und/oder wobei die nichtionogene amphiphile und/oder tensidische Substanz einen Log P-Wert von höchstens 0,46 aufweist;

(iii) ggf. mindestens ein Steroid und/oder Secosteroid, insbesondere mindestens ein Sterol, vorzugsweise ausgewählt aus der Gruppe von Zoosterolen, Phytosterolen und Mycosterolen sowie deren Kombinationen und Mischungen, bevorzugt Cholesterol und/oder Phytosterol, und/oder insbesondere mindestens ein Secosterol, vorzugsweise mindestens ein Calciferol;
wobei das Steroid und/oder Secosteroid, insbesondere das Sterol, einen HLB-Wert nach Griffin von höchstens 1,5 aufweist und/oder wobei das Steroid und/oder Secosteroid, insbesondere das Sterol, einen Log P-Wert von mindestens 5,40 aufweist;

(iv) ggf. mindestens ein kationisches Lipid, insbesondere aminbasiertes kationisches Lipid, vorzugsweise ein kationisches Lipid auf Basis eines tertiären Amins;
wobei das kationische Lipid einen HLB-Wert nach Griffin von höchstens 4 aufweist und/oder wobei das kationische Lipid einen Log P-Wert von mindestens 1,76 aufweist;

(v) ggf. mindestens einen Mono-, Di-, Tri- oder Polycarbonsäureester eines Polyols, insbesondere mindestens einen Mono-, Di- oder Tricarbonsäureester von Glycerin, vorzugsweise mindestens einen Mono-, Di- oder Trifettsäureester von Glycerin, bevorzugt mindestens einen $C_8$- bis $C_{16}$-Mono-, $C_8$- bis $C_{16}$-Di- oder $C_8$- bis $C_{16}$-Trifettsäureester von Glycerin, besonders bevorzugt mindestens einen $C_8$- bis $C_{14}$-Mono-, $C_8$- bis $C_{14}$-Di- oder $C_8$- bis $C_{14}$-Trifettsäureester von Glycerin;

wobei der Mono-, Di- oder Tricarbonsäureester von Glycerin einen HLB-Wert nach Griffin von höchstens 8 aufweist und/oder wobei der Mono-, Di- oder Tricarbonsäureester von Glycerin einen Log P-Wert von mindestens 0,80 aufweist;

wobei das Trägersystem bzw. das mit dem pharmazeutischen Wirkstoff beladene und/oder ausgerüstete Trägersystem in einem flüssigen Medium, insbesondere in einem bei einer Temperatur im Bereich von 20 °C bis 40 °C und Umgebungsdruck flüssigen Medium, vorzugsweise in Form eines Dispersionsmediums, enthalten ist und/oder vorliegt;

wobei das Trägersystem mit dem flüssigen Medium eine vorzugweise disperse Zusammensetzung, insbesondere eine Dispersion, welche das Trägersystem bzw. das mit dem pharmazeutischen Wirkstoff beladene und/oder ausgerüstete Trägersystem und das flüssige Medium umfasst, bildet und/oder wobei das Trägersystem mit dem flüssigen Medium eine Dispersion des Trägersystems bzw. des mit einem pharmazeutischen Wirkstoff beladenen und/oder ausgerüsteten Trägersystems in dem flüssigen Medium bildet;

wobei die Zusammenlagerung, insbesondere Aggregation, der Bestandteile zur Ausbildung des Trägersystems unter Eintrag von Energie, bevorzugt Eintrag von Misch- und/oder Rührenergie, in das zumindest einen Teil der Bestandteile und/oder Substanzen des Trägersystems aufweisende flüssige Medium und/oder in eine zumindest einen Teil der Bestandteile und/oder Substanzen des Trägersystems aufweisenden Mischung, erfolgt ist, wobei die eingetragene Energie eingestellt worden ist derart, dass eine zumindest im Wesentlichen laminare Strömung in dem zumindest einen Teil der Bestandteile und/oder Substanzen des Trägersystems aufweisenden flüssigen Medium und/oder in der zumindest einen Teil der Bestandteile und/oder Substanzen des Trägersystems aufweisenden Mischung, vorgelegen hat, insbesondere unter zumindest im Wesentlichen vollständiger Vermeidung der Ausbildung einer nichtlaminaren und/oder turbulenten Strömung und/oder insbesondere unter zumindest im Wesentlichen vollständiger Vermeidung der Ausbildung nichtlaminarer und/oder turbulenter Strömungsanteile; und

wobei eine kontinuierliche Verfahrens- und/oder Prozesskontrolle, insbesondere *online*- und/oder *inline*-Prozesskontrolle, zur Überwachung des Verfahrensstatus und/oder des Verfahrensfortschritts, vorzugsweise zur Überwachung und/oder Einstellung der Ausbildung der Partikel des Trägersystems, insbesondere Lipidnanopartikel, und zur Überwachung und/oder Einstellung von Verfahrensparametern, ausgewählt aus Eintrag von Energie, bevorzugt Eintrag von Misch- und/oder Rührenergie, Eintrag und/oder Abfuhr von Wärmenergie, Strömungsverhalten, Massendurchsatz, Volumenströmen oder dergleichen, erfolgt ist; wobei die Verfahrens- und/oder Prozesskontrolle über die Erfassung von mindestens einem Prozessparameter, insbesondere mindestens zwei Prozessparametern, erfolgt ist, wobei der Prozessparameter ausgewählt ist aus der Gruppe von Temperatur, Leitfähigkeit, Viskosität, pH-Wert jeweils des flüssigen Mediums mitsamt den darin eingebrachten Bestandteilen und/oder Substanzen, und/oder der Zusammensetzung;

wobei das Trägersystem und/oder das flüssige Medium und/oder die Zusammensetzung zumindest im Wesentlichen frei von Ethanol ist bzw. sind.

**5.** Trägersystem nach Anspruch 3 oder 4,

wobei die Substanzen (i) und (iii) <u>oder</u> die Substanzen (i) und (iv) <u>oder</u> die Substanzen (i) und (v) einen mittleren HLB-Wert nach Griffin im Bereich von 1 bis 10, insbesondere im Bereich von 2 bis 9, vorzugsweise im Bereich von 3 bis 8, aufweisen und/oder wobei die Substanzen (i) und (iii) oder die Substanzen (i) und (iv) oder die Substanzen (i) und (v) einen mittleren Log P-Wert im Bereich von 0,62 bis 8,58, insbesondere im Bereich von 0,70 bis 3,88, vorzugsweise im Bereich von 0,80 bis 2,44, aufweisen; und/oder

wobei die Substanzen (i), (iii) und (iv) oder die Substanzen (i), (iii) und (v) <u>oder</u> die Substanzen (i), (iv) und (v) einen mittleren HLB-Wert nach Griffin im Bereich von 1 bis 10, insbesondere im Bereich von 2 bis 9, vorzugsweise im Bereich von 3 bis 8, aufweisen und/oder wobei die Substanzen (i), (iii) und (iv) <u>oder</u> die Substanzen (i), (iii) und (v) <u>oder</u> die Substanzen (i), (iv) und (v) einen mittleren Log P-Wert im Bereich von 0,62 bis 8,58, insbesondere im Bereich von 0,70 bis 3,88, vorzugsweise im Bereich von 0,80 bis 2,44, aufweisen; und/oder

wobei die Substanzen (i), (iii), (iv) und (v) einen mittleren HLB-Wert nach Griffin im Bereich von 1 bis 10, insbesondere im Bereich von 2 bis 9, vorzugsweise im Bereich von 3 bis 8, aufweisen und/oder wobei die Substanzen (i), (iii), (iv) und (v) einen mittleren Log P-Wert im Bereich von 0,62 bis 8,58, insbesondere im Bereich von 0,70 bis 3,88, vorzugsweise im Bereich von 0,80 bis 2,44, aufweisen; und/oder

wobei die Substanz (i) einen kritischen Packungsparameter $P_{kr}$ von mindestens 0,5, insbesondere mindestens 0,7, insbesondere im Bereich von 0,7 bis 1,225, vorzugsweise im Bereich von 0,77 bis 1,12, bevorzugt im Bereich von 0,84 bis 1,05, aufweist; und/oder wobei die Substanz (ii) einen kritischen Packungsparameter $P_{kr}$ von höchstens 0,35,

insbesondere im Bereich von 0,007 bis 0,35, vorzugsweise im Bereich von 0,035 bis 0,315, bevorzugt im Bereich von 0,07 bis 0,28, aufweist; und/oder wobei die Substanz (iii) einen kritischen Packungsparameter $P_{kr}$ von mindestens 1,155,

insbesondere im Bereich von 1,155 bis 1,225, vorzugsweise im Bereich von 1,162 bis 1,218, bevorzugt im Bereich von 1,169 bis 1,211, aufweist; und/oder

wobei die Substanz (iv) einen kritischen Packungsparameter $P_{kr}$ von mindestens 0,98, insbesondere im Bereich von 0,98 bis 1,19, vorzugsweise im Bereich von 1,015 bis 1,155, bevorzugt im Bereich von 1,05 bis 1,12, aufweist; und/oder

wobei die Substanz (v) einen kritischen Packungsparameter $P_{kr}$ von mindestens 0,7, insbesondere im Bereich von 0,7 bis 1,19, vorzugsweise im Bereich von 0,77 bis 1,1725, bevorzugt im Bereich von 0,805 bis 1,155, aufweist.

6. Trägersystem nach einem der Ansprüche 3 bis 5,
wobei das Trägersystem durch mindestens eine(s) der nachfolgenden Merkmale und/oder Maßnahmen (1b) bis (5b), insbesondere durch eine Kombination von mindestens zwei, vorzugsweise mindestens drei, bevorzugt mindestens vier, besonders bevorzugt sämtlichen fünf, der nachfolgenden Merkmale und/oder Maßnahmen (1b) bis (5b), gekennzeichnet ist:

(1b) die Substanz (i) weist einen kritischen Packungsparameter $P_{kr}$ von mindestens 0,5, insbesondere mindestens 0,7, insbesondere im Bereich von 0,7 bis 1,225, vorzugsweise im Bereich von 0,77 bis 1,12, bevorzugt im Bereich von 0,84 bis 1,05, auf;
(2b) die Substanz (ii) weist einen kritischen Packungsparameter $P_{kr}$ von höchstens 0,35, insbesondere im Bereich von 0,007 bis 0,35, vorzugsweise im Bereich von 0,035 bis 0,315, bevorzugt im Bereich von 0,07 bis 0,28, auf;
(3b) wobei die Substanz (iii) weist einen kritischen Packungsparameter $P_{kr}$ von mindestens 1,155, insbesondere im Bereich von 1,155 bis 1,225, vorzugsweise im Bereich von 1,162 bis 1,218, bevorzugt im Bereich von 1,169 bis 1,211, auf;
(4b) die Substanz (iv) weist einen kritischen Packungsparameter $P_{kr}$ von mindestens 0,98, insbesondere im Bereich von 0,98 bis 1,19, vorzugsweise im Bereich von 1,015 bis 1,155, bevorzugt im Bereich von 1,05 bis 1,12, auf;
(5b) die Substanz (v) weist einen kritischen Packungsparameter $P_{kr}$ von mindestens 0,7, insbesondere im Bereich von 0,7 bis 1,19, vorzugsweise im Bereich von 0,77 bis 1,1725, bevorzugt im Bereich von 0,805 bis 1,155, auf.

7. Trägersystem nach einem der Ansprüche 3 bis 6,
wobei der Wirkstoff, insbesondere als Substanz (vi), Bestandteil des Trägersystems ist und/oder einen Bestandteil des Trägersystems ausbildet und/oder wobei das Trägersystem den Wirkstoff, insbesondere als Substanz (vi), aufweist.

8. Trägersystem nach einem der Ansprüche 3 bis 7,

wobei der Wirkstoff ein therapeutischer und/oder prophylaktischer Wirkstoff, insbesondere therapeutischer und/oder prophylaktischer pharmazeutischer Wirkstoff, ist;
insbesondere wobei der Wirkstoff ein zumindest im Wesentlichen wasserunlöslicher, vorzugsweise ein zumindest im Wesentlichen wasserunlöslicher amphiphiler Wirkstoff, ist; und/oder insbesondere wobei der Wirkstoff ein wasserlöslicher Wirkstoff ist; und/oder
insbesondere wobei der Wirkstoff ausgewählt ist aus der Gruppe von Vakzinwirkstoffen, antibiotischen Wirkstoffen, antimykotischen Wirkstoffen, antiviralen Wirkstoffen, antiphlogistischen Wirkstoffen, antipyretischen Wirkstoffen, antiallergischen Wirkstoffen, Antikrebswirkstoffen, Hormonwirkstoffen, antikoagulativen Wirkstoffen, Vitaminen, Mineralstoffen, UV-absorbierenden Stoffen und deren Mischungen sowie Kombinationen, vorzugsweise Vakzinwirkstoffen; und/oder
insbesondere wobei der Wirkstoff ausgewählt ist aus der Gruppe von RNA, insbesondere messenger-RNA; Retinol; Tocopherol; Kortikosteroiden, insbesondere Kortison; Antibiotika, insbesondere Tetracyklinen und Benzoylpenicillinen; Diclofenac; Estradiol, Estradiol-Hemihydrat, Salicylsäure, Acetylsalicylsäure; Indometacin; ätherischen Ölen; insbesondere amphiphilen UVA-Filtern und UVB-Filter; Nicotinamid; und deren Mischungen sowie Kombinationen; und/oder

insbesondere wobei der Wirkstoff ein Vakzinwirkstoff, insbesondere ein Nukleinsäurebasierter Vakzinwirkstoff und/oder genbasierter Vakzinwirkstoff, vorzugsweise ein Antigen eines Erregers und/oder eines Pathogens, insbesondere ein Antigen eines Virus, codierender Vakzinwirkstoff ist; und/oder

insbesondere wobei der Vakzinwirkstoff ausgewählt ist aus der Gruppe von DNA; RNA, insbesondere messenger-RNA; viralen Vektoren; Plasmiden, insbesondere Plasmid-DNA; vorzugsweise RNA; bevorzugt mRNA; und deren Mischungen sowie Kombinationen; und/oder

insbesondere wobei der Wirkstoff ein mRNA-basierter Vakzinwirkstoff und/oder mRNA ist, insbesondere wobei die mRNA einzelsträngig ausgebildet ist und/oder insbesondere wobei die mRNA eine 5'-Cap-Struktur aufweist; und/oder

insbesondere wobei der Wirkstoff ein mRNA-basierter Vakzinwirkstoff und/oder mRNA ist, insbesondere wobei die mRNA ein Protein, insbesondere Antigen, eines Coronavirus, vorzugsweise SARS-CoV-2, codiert, insbesondere wobei das Protein, insbesondere Antigen, das insbesondere membranverankerte Spike-Protein des Coronavirus, vorzugsweise SARS-CoV-2, zumindest teilweise, vorzugsweise zumindest im Wesentlichen vollständig, codiert; und/oder

insbesondere wobei der Wirkstoff ein Protein und/oder Antigen, insbesondere eines pathogenen Erregers, wie eines Bakteriums, Virus oder dergleichen, ist.

9. Trägersystem nach einem der Ansprüche 3 bis 8,

wobei die Zusammenlagerung, insbesondere Aggregation, der Bestandteile derart ist, dass das Trägersystem in partikulärer Form, insbesondere in Form von Lipidnanopartikeln, bevorzugt Lipidnanopartikeln in Form von Mizellen, Vesikeln und/oder diskförmigen Lipidnanopartikeln, bevorzugt diskförmigen Lipidnanopartikeln, resultiert; und/oder

wobei das Trägersystem in partikulärer Form, insbesondere in Form von Lipidnanopartikeln, bevorzugt Lipidnanopartikeln in Form von Mizellen, Vesikeln und/oder diskförmigen Lipidnanopartikeln, bevorzugt diskförmigen Lipidnanopartikeln, vorliegt; und/oder

wobei die Partikelgrößen, insbesondere die hydrodynamischen Durchmesser, der Partikel des Trägersystems, bevorzugt Lipidnanopartikel, zumindest im Wesentlichen eine Normalverteilung und/oder Gaußverteilung aufweisen, insbesondere wobei die Partikelgrößen, insbesondere die hydrodynamischen Durchmesser, mittels Lichtbeugung, insbesondere Laserdiffraktometrie, vorzugsweise Dynamischer Lichtstreuung, bevorzugt gemäß der ISO 22412:2017 und/oder DIN ISO 22412:2018-09, bestimmt werden; und/oder

wobei die Partikel des Trägersystems, bevorzugt die Lipidnanopartikel, eine Partikelgröße, insbesondere einen hydrodynamischen Durchmesser, im Bereich von 5 nm bis 10.000 nm, insbesondere im Bereich von 10 nm bis 5.000 nm, vorzugsweise im Bereich von 20 nm bis 3.000 nm, bevorzugt im Bereich von 50 nm bis 2.000 nm, besonders bevorzugt im Bereich von 75 nm bis 1.500 nm, ganz besonders bevorzugt im Bereich von 90 nm bis 1.250 nm, weiter bevorzugt im Bereich von 100 nm bis 1.000 nm, nochmals weiter bevorzugt im Bereich von 100 nm bis 900 nm, aufweisen, insbesondere bestimmt mittels Lichtbeugung, insbesondere Laserdiffraktometrie, vorzugsweise Dynamischer Lichtstreuung, bevorzugt gemäß der ISO 22412:2017 und/oder DIN ISO 22412:2018-09; und/oder

wobei die Partikel des Trägersystems, bevorzugt die Lipidnanopartikel, eine mittlere Partikelgröße, insbesondere einen mittleren hydrodynamischen Durchmesser, im Bereich von 5 nm bis 2.900 nm, insbesondere im Bereich von 10 nm bis 2.500 nm, vorzugsweise im Bereich von 20 nm bis 2.000 nm, bevorzugt im Bereich von 50 nm bis 1.500 nm, besonders bevorzugt im Bereich von 50 nm bis 1.000 nm, aufweisen, insbesondere bestimmt mittels Lichtbeugung, insbesondere Laserdiffraktometrie, vorzugsweise Dynamischer Lichtstreuung, bevorzugt gemäß der ISO 22412:2017 und/oder DIN ISO 22412:2018-09; und/oder

wobei die Varianz der Partikelgröße, insbesondere die Varianz des hydrodynamischen Durchmessers, der Partikel des Trägersystems, bevorzugt der Lipidnanopartikel, höchstens 45 %, insbesondere höchstens 35 %, vorzugsweise höchstens 25 %, bevorzugt höchstens 20 %, besonders bevorzugt höchstens 15 %, ganz besonders bevorzugt höchstens 10 %, beträgt, insbesondere bezogen auf die mittlere Partikelgöße bzw. den mittleren hydrodynamischen Durchmesser und/oder insbesondere bestimmt mittels Lichtbeugung, insbesondere Laserdiffraktometrie, vorzugsweise Dynamischer Lichtstreuung, bevorzugt gemäß der ISO 22412:2017 und/oder DIN ISO 22412:2018-09; und/oder

wobei die Varianz der Partikelgröße, insbesondere die Varianz des hydrodynamischen Durchmessers, der Partikel des Trägersystems, bevorzugt der Lipidnanopartikel, im Bereich von 5 % bis 45 %, insbesondere im Bereich von 10 % bis 40 %, vorzugsweise im Bereich von 15 % bis 35 %, bevorzugt im Bereich von 20 % bis 35 %, liegt, insbesondere bezogen auf die mittlere Partikelgöße bzw. den mittleren hydrodynamischen Durchmesser und/oder insbesondere bestimmt mittels Lichtbeugung, insbesondere Laserdiffraktometrie, vorzugsweise Dynamischer Lichtstreuung, bevorzugt gemäß der ISO 22412:2017 und/oder DIN ISO 22412:2018-09; und/oder wobei die Partikel des Trägersystems, bevorzugt die Lipidnanopartikel, eine mittlere Partikelgröße D50, insbesondere einen mittleren hydrodynamischen Durchmesser D50, im Bereich von 2,5 nm bis 950 nm, insbesondere im Bereich von 7,5 nm bis 850 nm, vorzugsweise im Bereich von 10 nm bis 800 nm, bevorzugt im Bereich von 15 nm bis 750 nm, aufweisen, insbesondere bestimmt mittels Lichtbeugung, insbesondere Laserdiffraktometrie, vorzugsweise Dynamischer Lichtstreuung, bevorzugt gemäß der ISO 22412:2017 und/oder DIN ISO 22412:2018-09; und/oder

wobei die Partikel des Trägersystems, bevorzugt die Lipidnanopartikel, eine mittlere Partikelgröße D90, insbesondere einen mittleren hydrodynamischen Durchmesser D90, im Bereich von 1 nm bis 1.000 nm, insbesondere im Bereich von 5 nm bis 900 nm, vorzugsweise im Bereich von 7,5 nm bis 850 nm, bevorzugt im Bereich von 10 nm bis 800 nm, aufweisen, insbesondere bestimmt mittels Lichtbeugung, insbesondere Laserdiffraktometrie, vorzugsweise Dynamischer Lichtstreuung, bevorzugt gemäß der ISO 22412:2017 und/oder DIN ISO 22412:2018-09.

10. Trägersystem nach einem der Ansprüche 3 bis 9,

wobei die Zusammenlagerung, insbesondere Aggregation, der Bestandteile derart ist, dass eine insbesondere lyotrope flüssig-kristalline Struktur auf Basis des Trägersystems und des flüssigen Mediums resultiert; und/oder wobei das Trägersystem zusammen mit dem flüssigen Medium als lyotrope flüssig-kristalline Struktur auf Basis des Trägersystems und des flüssigen Mediums vorliegt und/oder diese Struktur aufweist; und/oder wobei das Trägersystem in einem flüssigen Medium, insbesondere wie zuvor definiert, enthalten und/oder eingebracht ist, insbesondere wobei das Trägersystem mit dem flüssigen Medium eine Zusammensetzung, insbesondere pharmazeutische Zusammensetzung, bildet; und/oder wobei die Zusammensetzung eine vorzugweise disperse Zusammensetzung, insbesondere eine Dispersion, vorzugsweise wie zuvor definiert, ist, welche das Trägersystem bzw. das mit dem pharmazeutischen Wirkstoff beladene und/oder ausgerüstete Trägersystem und das flüssige Medium umfasst, und/oder wobei die Zusammensetzung, insbesondere wie zuvor definiert, in Form einer Dispersion des Trägersystems bzw. des mit einem pharmazeutischen Wirkstoff beladenen und/oder ausgerüsteten Trägersystems in dem flüssigen Medium vorliegt; und/oder wobei die Zusammensetzung das Trägersystem, vorzugsweise die Partikel, bevorzugt die Lipidnanopartikel, in einer Menge im Bereich von 5 Gew.-% bis 95 Gew.-%, insbesondere im Bereich von 10 Gew.-% bis 90 Gew.-%, vorzugsweise im Bereich von 15 Gew.-% bis 85 Gew.-%, bevorzugt im Bereich von 20 Gew.-% bis 80 Gew.-%, besonders bevorzugt im Bereich von 25 Gew.-% bis 75 Gew.-%, bezogen auf die Zusammensetzung, aufweist; und/oder wobei das Trägersystem in der Zusammensetzung in partikulärer Form, insbesondere in Form von Lipidnanopartikeln, bevorzugt Lipidnanopartikeln in Form von Mizellen, Vesikeln und/oder diskförmigen Lipidnanopartikeln, bevorzugt diskförmigen Lipidnanopartikeln, insbesondere wie zuvor definiert, vorliegt.

11. Trägersystem nach einem der Ansprüche 3 bis 10,

wobei die Zusammensetzung eine Wasseraktivität von weniger als 1, insbesondere höchstens 0,9, vorzugsweise höchstens 0,7, bevorzugt höchstens 0,5, besonders bevorzugt höchstens 0,3, aufweist; und/oder wobei die Zusammensetzung eine Wasseraktivität im Bereich von 0 bis 1, insbesondere im Bereich von 0 bis 0,9, vorzugsweise im Bereich von 0 bis 0,7, bevorzugt im Bereich von 0 bis 0,5, besonders bevorzugt im Bereich von 0 bis 0,3, aufweist; insbesondere bestimmt gemäß ISO 21807:2004 und/oder gemäß ISO 18787:2017 und/oder 21 CFR Part 11, insbesondere wie in der ISO 29621:2017 hinterlegt; und/oder wobei das Trägersystem und/oder die Zusammensetzung, insbesondere das Trägersystem, einen Log P-Wert, insbesondere Gesamt-Log P-Wert, von höchstens 5, insbesondere höchstens 4, bevorzugt höchstens 3, besonders bevorzugt höchstens 2, weiter bevorzugt höchstens 1, aufweist; und/oder wobei das Trägersystem und/oder die Zusammensetzung, insbesondere das Trägersystem, einen Log P-Wert, insbesondere Gesamt-Log P-Wert, im Bereich von 0,1 bis 5, insbesondere im Bereich von 0,2 bis 4, bevorzugt im Bereich von 0,3 bis 3, besonders bevorzugt im Bereich von 0,4 bis 2, aufweist; und/oder wobei die Bestandteile und/oder Substanzen des Trägersystems, insbesondere die Substanzen (i), (ii), (iii), (iv) und/oder (v), mit der Maßgabe ausgewählt sind, dass das resultierende Trägersystem einen Log P-Wert, insbesondere Gesamt-Log P-Wert, von höchstens 5, insbesondere höchstens 4, bevorzugt höchstens 3, besonders bevorzugt höchstens 2, weiter bevorzugt höchstens 1, aufweist; und/oder
wobei die Bestandteile und/oder Substanzen des Trägersystems, insbesondere die Substanzen (i), (ii), (iii), (iv) und/oder (v), mit der Maßgabe ausgewählt sind, dass das resultierende Trägersystem einen Log P-Wert, insbesondere Gesamt-Log P-Wert, im Bereich von 0,1 bis 5, insbesondere im Bereich von 0,2 bis 4, bevorzugt im Bereich von 0,3 bis 3, besonders bevorzugt im Bereich von 0,4 bis 2, aufweist.

12. Zusammensetzung, insbesondere pharmazeutische Zusammensetzung, wobei die Zusammensetzung mindestens ein lipidbasiertes Trägersystem in partikulärer Form, bevorzugt in Form von Lipidnanopartikeln, insbesondere für einen pharmazeutischen Wirkstoff; und/oder mindestens ein mit einem pharmazeutischen Wirkstoff beladenes und/oder ausgerüstetes lipidbasiertes Trägersystem in partikulärer Form, bevorzugt in Form von Lipidnanopartikeln, nach einem der Ansprüche 3 bis 11 in einem flüssigen Medium, insbesondere in einem bei einer Temperatur im Bereich von 20 °C bis 40 °C und Umgebungsdruck flüssigen Medium, vorzugsweise in Form eines Dispersionsmediums, enthält.

13. Arzneimittel oder Medikament, insbesondere zur Verwendung bei der prophylaktischen und/oder therapeutischen Behandlung von Erkrankungen des menschlichen oder tierischen Körpers,
wobei das Arzneimittel oder Medikament mindestens ein lipidbasiertes Trägersystem nach Anspruch 3 bis 11 oder eine Zusammensetzung nach Anspruch 12, insbesondere zusammen mit mindestens einem pharmazeutisch akzeptablen und/oder pharmazeutisch verträglichen Exzipienten, aufweist oder hieraus besteht.

14. Lipidbasiertes Trägersystem nach einem der Ansprüche 3 bis 11 oder Zusammensetzung nach Anspruch 12 zur Verwendung bei der prophylaktischen und/oder therapeutischen Behandlung von Erkrankungen des menschlichen oder tierischen Körpers.

15. Verwendung mindestens eines Prozessparameters zur Steuerung, insbesondere zur Verfahrens- und/oder Prozesskontrolle, eines Verfahrens nach Anspruch 1 oder 2, wobei der Prozessparameter ausgewählt wird aus der Gruppe von Temperatur, Leitfähigkeit, Viskosität, pH-Wert, elektrischem Widerstand und Oberflächenspannung, insbesondere dynamischer Oberflächenspannung, vorzugsweise aus der Gruppe von Temperatur, Leitfähigkeit, Viskosität und pH-Wert, insbesondere jeweils des flüssigen Mediums, vorzugsweise mitsamt den darin eingebrachten Bestandteilen und/oder Substanzen, und/oder der Zusammensetzung.

**Claims**

1. Method for producing a lipid-based carrier system in particulate form, preferably in the form of lipid nanoparticles, in particular for a pharmaceutically active substance; and/or for the preparation of a lipid-based carrier system loaded and/or equipped with a pharmaceutically active substance in particulate form, preferably in the form of lipid nanoparticles, wherein the components of the carrier system are brought into contact with one another and/or are brought into interaction with one another, in particular are brought into physical and/or chemical interaction, in such a way that an agglomeration, in particular aggregation, of the components to form the carrier system in particulate form, preferably in the form of lipid nanoparticles, is effected,

wherein the components of the carrier system comprise the following substances:

(i) at least one phospholipid, preferably phosphatidylcholine;
wherein the phospholipid comprises an HLB value according to Griffin of at most 11, in particular at most 8, and/or wherein the phospholipid comprises a Log P value of at least 0.55, in particular at least 0.80;
(ii) at least one non-ionic amphiphilic and/or surfactant substance, in particular selected from the group of polyglycerol esters, alkyl polyglycosides, amphiphilic and/or surfactant PEGylated substances and mixtures or combinations thereof; preferably selected from surfactant PEGylated substances, preferably selected from the group of PEGylated lipids, particularly preferably selected from the group of PEGylated fatty acids and their salts and esters, PEGylated triglycerides, PEGylated waxes, PEGylated phosholipids, PEGylated sphingolipids, PEGylated lipopolysaccharides and PEGylated isoprenoids and combinations and mixtures thereof, even more preferably selected from PEGylated fatty acids and salts and esters thereof;
wherein the non-ionic amphiphilic and/or surfactant substance comprises an HLB value according to Griffin of at least 13 and/or wherein the non-ionic amphiphilic and/or surfactant substance comprises a Log P value of at most 0.46;
(iii) optionally at least one steroid and/or secosteroid, in particular at least one sterol, preferably selected from the group of zoosterols, phytosterols and mycosterols and combinations and mixtures thereof, preferably cholesterol and/or phytosterol, and/or in particular at least one secosterol, preferably at least one calciferol; wherein the steroid and/or secosteroid, in particular the sterol, comprises an HLB value according to Griffin of at most 1.5 and/or in particular wherein the steroid and/or secosteroid, in particular the sterol, comprises a Log P value of at least 5.40;

(iv) optionally at least one cationic lipid, in particular an amine-based cationic lipid, preferably a cationic lipid based on a tertiary amine; wherein the cationic lipid comprises an HLB value according to Griffin of at most 4 and/or wherein the cationic lipid comprises a Log P value of at least 1.76;

(v) optionally at least one mono-, di-, tri- or polycarboxylic acid ester of a polyol, in particular at least one mono-, di- or tricarboxylic acid ester of glycerol, preferably at least one mono-, di- or trifatty acid ester of glycerol, preferably at least one $C_8$- to $C_{16}$-mono-, $C_8$- to $C_{16}$-di or $C_8$- to $C_{16}$-trifatty acid ester of glycerol, particularly preferred at least one $C_8$- to $C_{14}$-mono-, $C_8$- to $C_{14}$-di or $C_8$- to $C_{14}$-trifatty acid ester of glycerol; wherein the mono-, di- or tricarboxylic acid ester of glycerol comprises an HLB value according to Griffin of at most 8 and/or wherein the mono-, di- or tricarboxylic acid ester of glycerol comprises a Log P value of at least 0.80;

wherein the method, in particular producing the carrier system or the carrier system loaded and/or equipped with the pharmaceutical active ingredient, is carried out in a liquid medium, in particular in a liquid medium at a temperature in the range from 20°C to 40°C and at ambient pressure, preferably in the form of a dispersion medium, and after formation of the carrier system, a disperse composition, in particular a dispersion, which comprises the carrier system or the carrier system loaded and/or equipped with the pharmaceutical active ingredient and the liquid medium, results or, after formation of the carrier system, a dispersion of the carrier system or of the carrier system loaded and/or equipped with the active pharmaceutical ingredient and the liquid medium results;

wherein the agglomeration, in particular the aggregation, of the components to form the carrier system is carried out by introducing energy, preferably mixing and/or stirring energy, into the liquid medium comprising at least some of the components and/or substances of the carrier system and/or into a mixture comprising at least some of the components and/or substances of the carrier system, wherein the energy introduced is set in such a way that an at least substantially laminar flow is present in the liquid medium comprising at least a portion of the components and/or substances of the carrier system and/or in the mixture comprising at least some of the components and/or substances of the carrier system, in particular by at least substantially completely avoiding the formation of a non-laminar and/or turbulent flow and/or in particular by at least substantially completely avoiding the formation of non-laminar and/or turbulent flow components; and

wherein a continuous process and/or process control, in particular *online* and/or *inline* process control, is carried out for monitoring the process status and/or the process progress, preferably for monitoring and/or adjusting the formation of the particles of the carrier system, in particular lipid nanoparticles, and for monitoring and/or adjusting process parameters selected from input of energy, preferably input of mixing and/or stirring energy, input and/or removal of heat energy, flow behavior, mass throughput, volume flows or the like; wherein the process and/or process control is carried out by recording at least one process parameter, in particular at least two process parameters, wherein the process parameter is selected from the group of temperature, conductivity, viscosity and pH value of the liquid medium together with the components and/or substances introduced therein and/or composition;

wherein the process is carried out in the at least substantially complete absence of ethanol.

2. Method according to claim 1,

wherein the amount of energy introduced, in particular the amount of mixing and/or stirring energy introduced, is set such that a Reynolds number $R_e$, in particular a Reynolds number $R_e$ related to a stirrer flow, of at most 2,000, in particular at most 1,500, preferably at most 1,000, and preferably at most 500, is present; and/or wherein the active ingredient, in particular as substance (vi), is a constituent of the carrier system and/or forms a constituent of the carrier system and/or wherein the carrier system comprises the active ingredient, in particular as substance (vi).

3. Lipid-based carrier system in particulate form, preferably in the form of lipid nanoparticles, in particular for a pharmaceutical active ingredient, and/or lipid-based carrier system in particulate form, preferably in the form of lipid nanoparticles, loaded and/or equipped with a pharmaceutically active substance, obtainable by a method according to one of the preceding claims.

4. A lipid-based carrier system in particulate form, preferably in the form of lipid nanoparticles, in particular for a pharmaceutical active ingredient, and/or a lipid-based carrier system loaded and/or equipped with a pharmaceutical active ingredient, preferably in particulate form, preferably in the form of lipid nanoparticles, as defined in claim 3,

wherein the components of the carrier system are agglomerated, in particular aggregated, to form the carrier system and/or wherein the carrier system is preferably in particulate form, preferably in the form of lipid nanoparticles,
wherein the components of the carrier system comprise the following substances:

(i) at least one phospholipid, preferably phosphatidylcholine;
wherein the phospholipid comprises an HLB value according to Griffin of at most 11, in particular at most 8, and/or wherein the phospholipid comprises a Log P value of at least 0.55, in particular at least 0.80;
(ii) at least one non-ionic amphiphilic and/or surfactant substance, in particular selected from the group of polyglycerol esters, alkyl polyglycosides, amphiphilic and/or surfactant PEGylated substances and mixtures or combinations thereof; preferably selected from surfactant PEGylated substances, preferably selected from the group of PEGylated lipids, particularly preferably selected from the group of PEGylated fatty acids and their salts and esters, PEGylated triglycerides, PEGylated waxes, PEGylated phosholipids, PEGylated sphingolipids, PEGylated lipopolysaccharides and PEGylated isoprenoids and combinations and mixtures thereof, even more preferably selected from PEGylated fatty acids and salts and esters thereof;
wherein the non-ionic amphiphilic and/or surfactant substance comprises an HLB value according to Griffin of at least 13 and/or wherein the non-ionic amphiphilic and/or surfactant substance comprises a Log P value of at most 0.46;
(iii) optionally at least one steroid and/or secosteroid, in particular at least one sterol, preferably selected from the group of zoosterols, phytosterols and mycosterols and combinations and mixtures thereof, preferably cholesterol and/or phytosterol, and/or in particular at least one secosterol, preferably at least one calciferol;
wherein the steroid and/or secosteroid, in particular the sterol, comprises an HLB value according to Griffin of at most 1.5 and/or wherein the steroid and/or secosteroid, in particular the sterol, comprises a Log P value of at least 5.40;
(iv) optionally at least one cationic lipid, in particular an amine-based cationic lipid, preferably a cationic lipid based on a tertiary amine;
wherein the cationic lipid comprises an HLB value according to Griffin of at most 4 and/or wherein the cationic lipid comprises a Log P value of at least 1.76;
(v) optionally at least one mono-, di-, tri- or polycarboxylic acid ester of a polyol, in particular at least one mono-, di- or tricarboxylic acid ester of glycerol, preferably at least one mono-, di- or trifatty acid ester of glycerol, preferably at least one $C_8$- to $C_{(1)(6)}$-mono-, $C_8$- to $C_{16}$-di or $C_8$- to $C_{16}$-trifatty acid ester of glycerol, particularly preferred at least one $C_8$- to $C_{14}$-mono-, $C_8$- to $C_{14}$-di or $C_8$- to $C_{14}$-trifatty acid ester of glycerol; wherein the mono-, di- or tricarboxylic acid ester of glycerol comprises an HLB value according to Griffin of at most 8 and/or wherein the mono-, di- or tricarboxylic acid ester of glycerol comprises a Log P value of at least 0.80;

wherein the carrier system or the carrier system loaded and/or equipped with the pharmaceutical active ingredient is contained and/or present in a liquid medium, in particular in a liquid medium at a temperature in the range from 20°C to 40°C and at ambient pressure, preferably in the form of a dispersion medium;
wherein the carrier system with the liquid medium forms a preferably disperse composition, in particular a dispersion, which comprises the carrier system or the carrier system loaded and/or equipped with the pharmaceutical active substance and the liquid and/or wherein the carrier system with the liquid medium forms a dispersion of the carrier system or of the carrier system loaded and/or equipped with a pharmaceutical active substance in the liquid medium;
wherein the agglomeration, in particular the aggregation, of the constituents to form the carrier system is effected by introducing energy, preferably by mixing and/or stirring energy, into the liquid medium comprising at least some of the constituents and/or substances of the carrier system and/or into a mixture comprising at least some of the constituents and/or substances of the carrier system, wherein the energy introduced has been set in such a way that an at least substantially laminar flow in the liquid medium comprising at least some of the components and/or substances of the carrier system and/or in the mixture comprising at least some of the components and/or substances of the carrier system has existed, in particular with at least substantially complete avoidance of the formation of a non-laminar and/or turbulent flow and/or in particular with at least substantially complete avoidance of the formation of non-laminar and/or turbulent flow components; and
wherein a continuous method and/or process control, in particular *online* and/or *inline* process control, is carried out for monitoring the method status and/or method progress, preferably for monitoring and/or setting the formation of the particles of the carrier system, in particular lipid nanoparticles, and for monitoring and/or setting process parameters, selected from the group consisting of input of energy, preferably input of mixing and/or stirring energy, input and/or removal of thermal energy, flow behavior, mass throughput, volume flows or the like, is carried out; wherein the method and/or process control is effected by acquiring at least one process parameter, in particular at least two process parameters, wherein the process parameter is selected from the group of temperature, conductivity, viscosity, pH value of the liquid medium together with the components and/or substances introduced therein and/or the composition;

wherein the carrier system and/or the liquid medium and/or the composition is/are at least substantially free of ethanol.

5. Carrier system according to claim 3 or 4,

wherein the substances (i) and (iii) or the substances (i) and (iv) or the substances (i) and (v) comprise an average HLB value according to Griffin in the range from 1 to 10, in particular in the range from 2 to 9, preferably in the range from 3 to 8, and/or wherein the substances (i) and (iii) or the substances (i) and (iv) or the substances (i) and (v) comprise an average Log P value in the range from 0.62 to 8.58, in particular in the range from 0.70 to 3.88, preferably in the range from 0.80 to 2.44; and/or

wherein the substances (i), (iii) and (iv) or the substances (i), (iii) and (v) or the substances (i), (iv) and (v) comprise an average HLB value according to Griffin in the range from 1 to 10, in particular in the range from 2 to 9, preferably in the range from 3 to 8, and/or wherein the substances (i), (iii) and (iv) or the substances (i), (iii) and (v) or the substances (i), (iv) and (v) comprise an average Log P value in the range from 0.62 to 8.58, in particular in the range from 0.70 to 3.88, preferably in the range from 0.80 to 2.44; and/or

wherein the substances (i), (iii), (iv) and (v) comprise an average HLB value according to Griffin in the range from 1 to 10, in particular in the range from 2 to 9, preferably in the range from 3 to 8, and/or wherein the substances (i), (iii), (iv) and (v) comprise an average Log P value in the range from 0.62 to 8.58, in particular in the range from 0.70 to 3.88, preferably in the range from 0.80 to 2.44; and/or

wherein the substance (i) comprises a critical packing parameter $P_{kr}$ of at least 0.5, in particular at least 0.7, in particular in the range from 0.7 to 1.225, preferably in the range from 0.77 to 1.12, preferably in the range from 0.84 to 1.05; and/or

wherein the substance (ii) comprises a critical packing parameter $P_{kr}$ of at most 0.35, in particular in the range from 0.007 to 0.35, preferably in the range from 0.035 to 0.315, preferably in the range from 0.07 to 0.28; and/or

wherein the substance (iii) comprises a critical packing parameter $P_{kr}$ of at least 1.155, in particular in the range from 1.155 to 1.225, preferably in the range from 1.162 to 1.218, preferably in the range from 1.169 to 1.211; and/or

wherein the substance (iv) comprises a critical packing parameter $P_{kr}$ of at least 0.98, in particular in the range from 0.98 to 1.19, preferably in the range from 1.015 to 1.155, preferably in the range from 1.05 to 1.12; and/or

wherein the substance (v) comprises a critical packing parameter $P_{kr}$ of at least 0.7, in particular in the range from 0.7 to 1.19, preferably in the range from 0.77 to 1.1725, preferably in the range from 0.805 to 1.155.

6. Carrier system according to any one of claims 3 to 5,
wherein the carrier system is identified by at least one of the following features and/or measures (1b) to (5b), in particular by a combination of at least two, preferably at least three, preferably at least four, particularly preferably all five, of the following features and/or measures (1b) to (5b):

(1b) the substance (i) comprises a critical packing parameter $P_{kr}$ of at least 0.5, in particular at least 0.7, in particular in the range from 0.7 to 1.225, preferably in the range from 0.77 to 1.12, preferably in the range from 0.84 to 1.05;

(2b) the substance (ii) comprises a critical packing parameter $P_{kr}$ of at most 0.35, in particular in the range from 0.007 to 0.35, preferably in the range from 0.035 to 0.315, preferably in the range from 0.07 to 0.28;

(3b) wherein the substance (iii) comprises a critical packing parameter $P_{kr}$ of at least 1.155, in particular in the range from 1.155 to 1.225, preferably in the range from 1.162 to 1.218, preferably in the range from 1.169 to 1.211;

(4b) the substance (iv) comprises a critical packing parameter $P_{kr}$ of at least 0.98, in particular in the range from 0.98 to 1.19, preferably in the range from 1.015 to 1.155, preferably in the range from 1.05 to 1.12;

(5b) the substance (v) comprises a critical packing parameter $P_{kr}$ of at least 0.7, in particular in the range from 0.7 to 1.19, preferably in the range from 0.77 to 1.1725, preferably in the range from 0.805 to 1.155.

7. Carrier system according to any one of claims 3 to 6,

wherein the active ingredient, in particular as substance (vi), is a component of the carrier system and/or forms a component of the carrier system and/or wherein the carrier system comprises the active ingredient, in particular as substance (vi).

8. Carrier system according to any one of claims 3 to 7,

wherein the active ingredient is a therapeutic and/or prophylactic active ingredient, in particular a therapeutic and/or prophylactic pharmaceutical active ingredient;
in particular wherein the active ingredient is an at least substantially water-insoluble, preferably an at least substantially water-insoluble amphiphilic active ingredient; and/or in particular wherein the active ingredient is a water-soluble active ingredient; and/or
in particular wherein the active ingredient is selected from the group of vaccine active ingredients, antibiotic active ingredients, antifungal active ingredients, antiviral active ingredients, antiphlogistic active ingredients, antipyretic active ingredients, antiallergic active ingredients, anticancer active ingredients, hormone active ingredients, anticoagulant active ingredients, vitamins, minerals, UV-absorbing substances and mixtures thereof and combinations thereof, preferably vaccine active ingredients; and/or
in particular wherein the active ingredient is selected from the group of RNA, in particular messenger RNA; retinol; tocopherol; corticosteroids, in particular cortisone; antibiotics, in particular tetracyclines and benzoylpenicillins; diclofenac; estradiol, estradiol hemihydrate, salicylic acid, acetylsalicylic acid; indomethacin; essential oils; in particular amphiphilic UVA filters and UVB filters; nicotinamide; and mixtures and combinations thereof; and/or
in particular wherein the active substance is a vaccine active substance, in particular a nucleic acid-based vaccine active substance and/or gene-based vaccine active ingredient, preferably a vaccine active ingredient encoding an antigen of a pathogen and/or a pathogenic agent, in particular an antigen of a virus; and/or
wherein the vaccine active ingredient is selected from the group of DNA; RNA, in particular messenger RNA; viral vectors; plasmids, in particular plasmid DNA; preferably RNA; preferably mRNA; and mixtures and combinations thereof; and/or
in particular wherein the active ingredient is an mRNA-based vaccine active ingredient and/or mRNA, in particular wherein the mRNA is single-stranded and/or in particular wherein the mRNA comprises a 5'-cap structure; and/or
in particular wherein the active ingredient is an mRNA-based vaccine active ingredient and/or mRNA, in particular wherein the mRNA encodes a protein, in particular antigen, of a coronavirus, preferably SARS-CoV-2, in particular wherein the protein, in particular antigen, at least partially, preferably at least substantially completely, encodes the in particular membrane-anchored *spike protein* of the coronavirus, preferably SARS-CoV-2; and/or
in particular wherein the active ingredient is a protein and/or antigen, in particular of a pathogenic agent, such as a bacterium, virus or the like.

9. Carrier system according to any one of claims 3 to 8,

wherein the agglomeration, in particular aggregation, of the components is such that the carrier system results in particulate form, in particular in the form of lipid nanoparticles, preferably lipid nanoparticles in the form of micelles, vesicles and/or discoidal lipid nanoparticles, preferably discoidal lipid nanoparticles; and/or
wherein the carrier system is in particulate form, in particular in the form of lipid nanoparticles, preferably lipid nanoparticles in the form of micelles, vesicles and/or disc-shaped lipid nanoparticles, preferably disc-shaped lipid nanoparticles; and/or
wherein the particle sizes, in particular the hydrodynamic diameters, of the particles of the carrier system, preferably lipid nanoparticles, comprise at least essentially a normal distribution and/or Gaussian distribution, in particular wherein the particle sizes, in particular the hydrodynamic diameters, are determined using light diffraction, in particular laser diffraction, preferably dynamic light scattering, preferably according to ISO 22412:2017 and/or DIN ISO 22412:2018-09; and/or
wherein the particles of the carrier system, preferably the lipid nanoparticles, have a particle size, in particular a hydrodynamic diameter, in the range from 5 nm to 10,000 nm, in particular in the range from 10 nm to 5,000 nm, preferably in the range from 20 nm to 3,000 nm, more preferably in the range from 50 nm to 2,000 nm, particularly preferably in the range from 75 nm to 1.500 nm, particularly preferably in the range from 90 nm to 1,250 nm, further preferably in the range from 100 nm to 1,000 nm, again further preferably in the range from 100 nm to 900 nm, determined in particular using light diffraction, especially laser diffraction, preferably dynamic light scattering, preferably in accordance with ISO 22412:2017 and/or DIN ISO 22412:2018-09; and/or
wherein the particles of the carrier system, preferably the lipid nanoparticles, have an average particle size, in particular an average hydrodynamic diameter, in the range from 5 nm to 2,900 nm, in particular in the range from 10 nm to 2,500 nm, preferably in the range from 20 nm to 2.000 nm, more preferably in the range from 50 nm to 1,500 nm, particularly preferably in the range from 50 nm to 1,000 nm, in particular determined using light diffraction, in particular laser diffraction, preferably dynamic light scattering, preferably in accordance with ISO 22412:2017 and/or DIN ISO 22412:2018-09; and/or

wherein the variance of the particle size, in particular the variance of the hydrodynamic diameter, of the particles of the carrier system, preferably the lipid nanoparticles, is at most 45 %, in particular at most 35 %, preferably at most 25 %, more preferably at most 20 %, particularly preferably at most 15 %, even more preferably at most 10 %, in particular in relation to the mean particle size and/or the mean hydrodynamic diameter, and/or in particular determined using light diffraction, in particular laser diffraction, preferably dynamic light scattering, preferably in accordance with ISO 22412:2017 and/or DIN ISO 22412:2018-09; and/or

wherein the variance of the particle size, in particular the variance of the hydrodynamic diameter, of the particles of the carrier system, preferably of the lipid nanoparticles, is in the range from 5 % to 45 %, in particular in the range from 10 % to 40 %, preferably in the range from 15 % to 35 %, more preferably in the range from 20 % to 35 %, in particular with respect to the mean particle size or the mean hydrodynamic diameter and/or in particular determined by means of light diffraction, in particular laser diffraction, preferably dynamic light scattering, preferably in accordance with ISO 22412:2017 and/or DIN ISO 22412:2018-09; and/or

wherein the particles of the carrier system, preferably the lipid nanoparticles, comprise an average particle size D50, in particular an average hydrodynamic diameter D50, in the range from 2.5 nm to 950 nm, in particular in the range from 7.5 nm to 850 nm, preferably in the range from 10 nm to 800 nm, more preferably in the range from 15 nm to 750 nm, in particular determined using light diffraction, in particular laser diffraction, preferably dynamic light scattering, preferably according to ISO 22412:2017 and/or DIN ISO 22412:2018-09; and/or

wherein the particles of the carrier system, preferably the lipid nanoparticles, have an average particle size D90, in particular an average hydrodynamic diameter D90, in the range from 1 nm to 1.000 nm, in particular in the range from 5 nm to 900 nm, preferably in the range from 7.5 nm to 850 nm, more preferably in the range from 10 nm to 800 nm, in particular determined using light diffraction, in particular laser diffraction, preferably dynamic light scattering, preferably in accordance with ISO 22412:2017 and/or DIN ISO 22412:2018-09.

10. A carrier system according to any one of claims 3 to 9,

wherein the combination, in particular aggregation, of the components is such that in particular lyotropic liquid-crystalline structure on the basis of the carrier system and the liquid medium; and/or

wherein the carrier system together with the liquid medium is present as a lyotropic liquid-crystalline structure based on the carrier system and the liquid medium and/or comprises this structure; and/or

wherein the carrier system is contained and/or supplied in a liquid medium, in particular as defined above, in particular wherein the carrier system forms a composition, in particular a pharmaceutical composition, with the liquid medium; and/or

wherein the composition is a preferably disperse composition, in particular a dispersion, preferably as defined above, comprising the carrier system or the carrier system loaded and/or equipped with the pharmaceutically active agent and the liquid medium, and/or wherein the composition, in particular as defined above, is in the form of a dispersion of the carrier system or the carrier system loaded and/or equipped with a pharmaceutical active agent in the liquid medium; and/or

wherein the composition comprises the carrier system, preferably the particles, preferably the lipid nanoparticles, in an amount in the range from 5 wt.% to 95 wt.%, preferably in the range from 10 wt.% to 90 wt.%, more preferably in the range from 15 wt.% to 85 wt.%, more preferably in the range from 20 wt.% to 80 wt.%, particularly preferably in the range from 25 wt.% to 75 wt.%, based on the composition; and/or

wherein the carrier system is present in the composition in particulate form, in particular in the form of lipid nanoparticles, preferably lipid nanoparticles in the form of micelles, vesicles and/or disc-shaped lipid nanoparticles, preferably disc-shaped lipid nanoparticles, in particular as defined above.

11. A carrier system according to any one of claims 3 to 10,

wherein the composition comprises a water activity of less than 1, in particular at most 0.9, preferably at most 0.7, more preferably at most 0.5, particularly preferably at most 0.3; and/or

wherein the composition comprises a water activity in the range from 0 to 1, in particular in the range from 0 to 0.9, preferably in the range from 0 to 0.7, more preferably in the range from 0 to 0.5, particularly preferably in the range from 0 to 0.3;

in particular determined according to ISO 21807:2004 and/or according to ISO 18787:2017 and/or 21 CFR Part 11, in particular as defined in ISO 29621:2017; and/or

wherein the carrier system and/or the composition, in particular the carrier system, comprises a Log P value, in particular a total Log P value, of at most 5, in particular at most 4, preferably at most 3, particularly preferably at most 2, further preferred at most 1; and/or

wherein the carrier system and/or the composition, in particular the carrier system, comprises a Log P value, in particular a total Log P value, in the range from 0.1 to 5, in particular in the range from 0.2 to 4, preferably in the range from 0.3 to 3, particularly preferably in the range from 0.4 to 2; and/or

wherein the components and/or substances of the carrier system, in particular the substances (i), (ii), (iii), (iv) and/or (v), are selected with the proviso that the resulting carrier system comprises a Log P value, in particular a total Log P value, of at most 5, in particular at most 4, preferably at most 3, particularly preferably at most 2, further preferably at most 1; and/or

wherein the components and/or substances of the carrier system, in particular the substances (i), (ii), (iii), (iv) and/or (v), are selected with the proviso that the resulting carrier system comprises a Log P value, in particular a total Log P value, in the range from 0.1 to 5, in particular in the range from 0.2 to 4, preferably in the range from 0.3 to 3, particularly preferably in the range from 0.4 to 2.

12. Composition, in particular pharmaceutical composition, wherein the composition comprises at least one lipid-based carrier system in particulate form, preferably in the form of lipid nanoparticles, in particular for an active pharmaceutical ingredient; and/or at least one lipid-based carrier system loaded and/or equipped with a pharmaceutical active ingredient in particulate form, preferably in the form of lipid nanoparticles, according to one of claims 3 to 11 in a liquid medium, in particular in a medium which is liquid at a temperature in the range from 20°C to 40°C and ambient pressure, preferably in the form of a dispersion medium.

13. Drug or medicament, in particular for use in the prophylactic and/or therapeutic treatment of diseases of the human or animal body,

wherein the drug or medicament comprises or consists of at least one lipid-based carrier system according to claim 3 to 11 or a composition according to claim 12, in particular together with at least one pharmaceutically acceptable and/or pharmaceutically acceptable excipient.

14. Lipid-based carrier system according to any one of claims 3 to 11 or composition according to claim 12 for use in the prophylactic and/or therapeutic treatment of diseases of the human or animal body.

15. Use of at least one process parameter for controlling, in particular for method and/or process control, a method according to claim 1 or 2, wherein the process parameter is selected from the group of temperature, conductivity, viscosity, pH value, electrical resistance and surface tension, in particular dynamic surface tension, preferably from the group of temperature, conductivity, viscosity and pH value, in particular in each case of the liquid medium, preferably together with the components and/or substances introduced therein, and/or of the composition.

**Revendications**

1. Procédé de préparation d'un système de support à base de lipides sous forme particulaire, de préférence sous forme de nanoparticules lipidiques, en particulier pour une substance active pharmaceutique; et/ou de préparation d'un système de support à base de lipides chargé et/ou équipé d'une substance active pharmaceutique sous forme particulaire, de préférence sous forme de nanoparticules lipidiques,

les composants du système porteur étant mis en contact les uns avec les autres et/ou étant mis en interaction les uns avec les autres, en particulier étant mis en interaction physiquement et/ou chimiquement, de telle sorte qu'un assemblage, en particulier une agrégation, des composants soit provoqué pour former le système porteur sous forme particulaire, de préférence sous forme de nanoparticules lipidiques,

dans lequel les composants du système de support comprennent les substances suivantes:

(i) au moins un phospholipide, de préférence la phosphatidylcholine;
le phospholipide présentant une valeur HLB selon Griffin d'au plus 11, en particulier d'au plus 8, et/ou le phospholipide présentant une valeur Log P d'au moins 0,55, en particulier d'au moins 0,80;
(ii) au moins une substance amphiphile et/ou tensioactive non ionique, en particulier choisie dans le groupe des esters de polyglycérol, des alkylpolyglycosides, des substances PEGylées amphiphiles et/ou tensioactives et de leurs mélanges ou combinaisons; de préférence choisies parmi les substances PEGylées tensioactives, de préférence choisies dans le groupe des lipides PEGylés, de manière particulièrement préférée choisies dans le groupe des acides gras PEGylés et de leurs sels et esters, des triglycérides PEGylés, les cires PEGylées, les phospholipides PEGylés, les sphingolipides PEGylés, les lipopolysaccharides PEGylés et les isoprénoïdes PEGylés, ainsi que leurs combinaisons et mélanges, de manière particulièrement préférée choisis parmi les acides gras PEGylés et leurs sels et esters;

dans lequel la substance amphiphile et/ou tensioactive non ionique présente une valeur HLB selon Griffin d'au moins 13 et/ou dans lequel la substance amphiphile et/ou tensioactive non ionique présente une valeur Log P d'au plus 0,46;

(iii) le cas échéant, au moins un stéroïde et/ou un sécostéroïde, en particulier au moins un stérol, de préférence choisi dans le groupe des zostérols, des phytostérols et des mycostérols ainsi que leurs combinaisons et mélanges, de préférence le cholestérol et/ou le phytostérol, et/ou en particulier au moins un sécostérol, de préférence au moins un calciférol;

dans lequel le stéroïde et/ou le sécostéroïde, en particulier le stérol, présente une valeur HLB selon Griffin d'au plus 1,5 et/ou dans lequel le stéroïde et/ou le sécostéroïde, en particulier le stérol, présente une valeur Log P d'au moins 5,40;

(iv) éventuellement au moins un lipide cationique, en particulier un lipide cationique à base d'amine, de préférence un lipide cationique à base d'amine tertiaire;

dans lequel le lipide cationique présente une valeur HLB selon Griffin d'au plus 4 et/ou dans lequel le lipide cationique présente une valeur Log P d'au moins 1,76;

(v) le cas échéant, un au moins un ester d'acide mono-, di-, tri- ou polycarboxylique d'un polyol, en particulier au moins un ester d'acide mono-, di- ou tricarboxylique de glycérol, de préférence au moins un ester d'acide mono-, di- ou tri-gras de glycérol, de préférence au moins un ester d'acide mono-, di- ou tri-gras $C_8$- à $C_{16}$-, $C_8$- à $C_{16}$-Di- ou $C_8$- à $C_{16}$-triester d'acide gras de glycérol, de manière particulièrement préférée au moins un $C_8$- à $C_{14}$-mono-, $C_8$- à $C_{14}$-Di- ou $C_8$- à $C_{14}$-triester d'acide gras de glycérol;

l'ester d'acide mono-, di- ou tricarboxylique de glycérol présentant une valeur HLB selon Griffin de 8 au maximum et/ou l'ester d'acide mono-, di- ou tricarboxylique de glycérol présentant une valeur Log P d'au moins 0,80;

le procédé, en particulier la fabrication du système de support ou du système de support chargé et/ou équipé de la substance active pharmaceutique, étant réalisé dans un milieu liquide, en particulier dans un milieu liquide à une température dans la plage de 20°C à 40°C et à la pression ambiante, de préférence sous la forme d'un milieu de dispersion, et, après formation du système de support, une composition dispersée, en particulier une dispersion, qui comprend le système de support ou le système de support chargé et/ou équipé de la substance active pharmaceutique, étant obtenue. le système de support chargé et/ou équipé de la substance pharmaceutiquement active et le milieu liquide, ou après la formation du système de support, il en résulte une dispersion du système de support ou du système de support chargé et/ou équipé d'une substance pharmaceutiquement active dans le milieu liquide;

le regroupement, en particulier l'agrégation, des composants pour former le système porteur étant effectué avec apport d'énergie, de préférence un apport d'énergie de mélange et/ou d'agitation, dans le milieu liquide présentant au moins une partie des composants et/ou des substances du système porteur et/ou dans un mélange présentant au moins une partie des composants et/ou des substances du système porteur, l'énergie apportée étant réglée de telle sorte, de sorte qu'un écoulement au moins essentiellement laminaire est présent dans le milieu liquide présentant au moins une partie des composants et/ou des substances du système porteur et/ou dans le mélange présentant au moins une partie des composants et/ou des substances du système porteur, en particulier en évitant au moins essentiellement complètement la formation d'un écoulement non laminaire et/ou turbulent et/ou en particulier en évitant au moins essentiellement complètement la formation de parties d'écoulement non laminaires et/ou turbulentes; et

un contrôle continu du procédé et/ou du processus, en particulier *un contrôle du processus en ligne* et/ou *inline,* étant effectué pour surveiller l'état du procédé et/ou la progression du procédé, de préférence pour surveiller et/ou régler la formation des particules du système porteur, en particulier des nanoparticules lipidiques, et pour surveiller et/ou régler des paramètres du procédé, choisis parmi l'apport d'énergie, de préférence l'apport d'énergie de mélange et/ou d'agitation, l'apport et/ou l'évacuation d'énergie thermique, le comportement d'écoulement, le débit massique, les flux volumiques ou similaires ; le contrôle du procédé et/ou du processus s'effectuant par la saisie d'au moins un paramètre de processus, en particulier d'au moins deux paramètres de processus, le paramètre de processus étant choisi dans le groupe de la température, de la conductivité, de la viscosité et du pH respectivement du milieu liquide avec les composants et/ou les substances qui y sont introduits et/ou de la composition;

le procédé étant mis en œuvre en l'absence au moins sensiblement totale d'éthanol.

**2.** Procédé selon la revendication 1,

la quantité d'énergie introduite, en particulier la quantité introduite d'énergie de mélange et/ou d'agitation, étant réglée de telle sorte qu'il existe un nombre de Reynolds $R_e$, en particulier un nombre de Reynolds $R_e$ rapporté à un écoulement d'agitation, d'au maximum 2 000, en particulier d'au maximum 1 500, de préférence d'au maximum 1 000, de préférence d'au maximum 500; et/ou

la substance active, en particulier en tant que substance (vi), étant un composant du système de support et/ou formant un composant du système de support et/ou le système de support présentant la substance active, en particulier en tant que substance (vi).

3. Système de support à base de lipides sous forme particulaire, de préférence sous forme de nanoparticules lipidiques, en particulier pour un principe actif pharmaceutique, et/ou système de support à base de lipides chargé et/ou équipé d'un principe actif pharmaceutique sous forme particulaire, de préférence sous forme de nanoparticules lipidiques, pouvant être obtenu par un procédé selon l'une des revendications précédentes.

4. Système de support à base de lipides sous forme particulaire, de préférence sous forme de nanoparticules lipidiques, en particulier pour un principe actif pharmaceutique, et/ou système de support à base de lipides chargé et/ou équipé d'un principe actif pharmaceutique, de préférence sous forme particulaire, de préférence sous forme de nanoparticules lipidiques, tel que défini dans la revendication 3,

dans lequel les composants du système porteur sont stockés ensemble, en particulier agrégés, pour former le système porteur et/ou dans lequel le système porteur se présente de préférence sous forme particulaire, de préférence sous forme de nanoparticules lipidiques,
dans lequel les composants du système de support comprennent les substances suivantes:

(i) au moins un phospholipide, de préférence la phosphatidylcholine;
le phospholipide présentant une valeur HLB selon Griffin d'au plus 11, en particulier d'au plus 8, et/ou le phospholipide présentant une valeur Log P d'au moins 0,55, en particulier d'au moins 0,80;
(ii) au moins une substance amphiphile et/ou tensioactive non ionique, en particulier choisie dans le groupe des esters de polyglycérol, des alkylpolyglycosides, des substances PEGylées amphiphiles et/ou tensioactives et de leurs mélanges ou combinaisons; de préférence choisies parmi les substances PEGylées tensioactives, de préférence choisies dans le groupe des lipides PEGylés, de manière particulièrement préférée choisies dans le groupe des acides gras PEGylés et de leurs sels et esters, des triglycérides PEGylés, les cires PEGylées, les phospholipides PEGylés, les sphingolipides PEGylés, les lipopolysaccharides PEGylés et les isoprénoïdes PEGylés, ainsi que leurs combinaisons et mélanges, de préférence choisis parmi les acides gras PEGylés et leurs sels et esters;
dans lequel la substance amphiphile et/ou tensioactive non ionique présente une valeur HLB selon Griffin d'au moins 13 et/ou dans lequel la substance amphiphile et/ou tensioactive non ionique présente une valeur Log P d'au plus 0,46;
(iii) le cas échéant, au moins un stéroïde et/ou un sécostéroïde, en particulier au moins un stérol, de préférence choisi dans le groupe des zostérols, des phytostérols et des mycostérols ainsi que leurs combinaisons et mélanges, de préférence le cholestérol et/ou le phytostérol, et/ou en particulier au moins un sécostérol, de préférence au moins un calciférol;
dans lequel le stéroïde et/ou le sécostéroïde, en particulier le stérol, présente une valeur HLB selon Griffin d'au plus 1,5 et/ou dans lequel le stéroïde et/ou le sécostéroïde, en particulier le stérol, présente une valeur Log P d'au moins 5,40;
(iv) éventuellement au moins un lipide cationique, en particulier un lipide cationique à base d'amine, de préférence un lipide cationique à base d'amine tertiaire;
dans lequel le lipide cationique présente une valeur HLB selon Griffin d'au plus 4 et/ou dans lequel le lipide cationique présente une valeur Log P d'au moins 1,76;
(v) le cas échéant, un au moins un ester d'acide mono-, di-, tri- ou polycarboxylique d'un polyol, en particulier au moins un ester d'acide mono-, di- ou tricarboxylique de glycérol, de préférence au moins un ester d'acide mono-, di- ou tri-gras de glycérol, de préférence au moins un ester d'acide mono-, di- ou tri-gras $C_8$- à $C_{16}$-, $C_8$- à $C_{16}$-Di- ou $C_8$- à $C_{16}$-triester d'acide gras de glycérol, de manière particulièrement préférée au moins un $C_8$- à $C_{14}$-mono-, $C_8$- à $C_{14}$-Di- ou $C_8$- à $C_{14}$-triester d'acide gras de glycérol;
l'ester d'acide mono-, di- ou tricarboxylique de glycérol présentant une valeur HLB selon Griffin de 8 au maximum et/ou l'ester d'acide mono-, di- ou tricarboxylique de glycérol présentant une valeur Log P d'au moins 0,80;

le système de support ou le système de support chargé et/ou équipé de la substance active pharmaceutique étant contenu et/ou présent dans un milieu liquide, en particulier dans un milieu liquide à une température dans la plage de 20°C à 40°C et à la pression ambiante, de préférence sous la forme d'un milieu de dispersion;

dans lequel le système de support forme avec le milieu liquide une composition de préférence dispersée, en particulier une dispersion, qui comprend le système de support ou le système de support chargé et/ou équipé de la substance pharmaceutiquement active et le milieu liquide, et/ou dans lequel le système de support forme avec le milieu liquide une dispersion du système de support ou du système de support chargé et/ou équipé d'une substance pharmaceutiquement active dans le milieu liquide;

le regroupement, en particulier l'agrégation, des composants pour former le système porteur s'est effectué avec apport d'énergie, de préférence un apport d'énergie de mélange et/ou d'agitation, dans le milieu liquide présentant au moins une partie des composants et/ou des substances du système porteur et/ou dans un mélange présentant au moins une partie des composants et/ou des substances du système porteur, l'énergie apportée ayant été réglée de telle sorte, qu'un écoulement au moins essentiellement laminaire a eu lieu dans le milieu liquide présentant au moins une partie des composants et/ou des substances du système porteur et/ou dans le mélange présentant au moins une partie des composants et/ou des substances du système porteur, en particulier en évitant au moins essentiellement complètement la formation d'un écoulement non laminaire et/ou turbulent et/ou en particulier en évitant au moins essentiellement complètement la formation de parties d'écoulement non laminaires et/ou turbulentes; et

un contrôle continu du procédé et/ou du processus, en particulier *un contrôle du processus en ligne* et/ou *inline,* étant effectué pour surveiller l'état du procédé et/ou la progression du procédé, de préférence pour surveiller et/ou régler la formation des particules du système porteur, en particulier des nanoparticules lipidiques, et pour surveiller et/ou régler des paramètres du procédé, choisis parmi l'apport d'énergie, de préférence l'apport d'énergie de mélange et/ou d'agitation, l'apport et/ou l'évacuation d'énergie thermique, le comportement d'écoulement, le débit massique, les débits volumiques ou similaires; le contrôle du procédé et/ou du processus étant effectué par la saisie d'au moins un paramètre de processus, en particulier d'au moins deux paramètres de processus, le paramètre de processus étant choisi dans le groupe de la température, de la conductivité, de la viscosité, du pH du milieu liquide avec les composants et/ou les substances qui y sont introduits, et/ou de la composition;

dans lequel le système de support et/ou le milieu liquide et/ou la composition est/sont au moins sensiblement exempt(s) d'éthanol.

5. Système de support selon la revendication 3 ou 4,

les substances (i) et (iii) <u>ou</u> les substances (i) et (iv) <u>ou</u> les substances (i) et (v) ayant une valeur HLB moyenne selon Griffin dans la plage de 1 à 10, en particulier dans la plage de 2 à 9, de préférence dans la plage de 3 à 8, et/ou les substances (i) et (iii) <u>ou</u> les substances (i) et (iv) <u>ou</u> les substances (i) et (v) présentant une valeur log P moyenne dans la plage de 0,62 à 8,58, en particulier dans la plage de 0,70 à 3,88, de préférence dans la plage de 0,80 à 2,44; et/ou

les substances (i), (iii) et (iv) <u>ou</u> les substances (i), (iii) et (v) <u>ou</u> les substances (i), (iv) et (v) ayant une valeur HLB moyenne selon Griffin dans la plage de 1 à 10, en particulier dans la plage de 2 à 9, de préférence dans la plage de 3 à 8, et/ou dans lequel les substances (i), (iii) et (iv) <u>ou</u> les substances (i), (iii) et (v) <u>ou</u> les substances (i), (iv) et (v) présentent une valeur log P moyenne dans la plage de 0,62 à 8,58, en particulier dans la plage de 0,70 à 3,88, de préférence dans la plage de 0,80 à 2,44; et/ou

les substances (i), (iii), (iv) et (v) présentant une valeur HLB moyenne selon Griffin dans la plage de 1 à 10, en particulier dans la plage de 2 à 9, de préférence dans la plage de 3 à 8, et/ou les substances (i), (iii), (iv) et (v) présentant une valeur Log P moyenne dans la plage de 0,62 à 8,58, en particulier dans la plage de 0,70 à 3,88, de préférence dans la plage de 0,80 à 2,44; et/ou

la substance (i) présentant un paramètre de tassement critique $P_{kr}$ d'au moins 0,5, en particulier d'au moins 0,7, notamment dans la plage de 0,7 à 1,225, de préférence dans la plage de 0,77 à 1,12, de préférence dans la plage de 0,84 à 1,05; et/ou

la substance (ii) ayant un paramètre de tassement critique $P_{kr}$ d'au plus 0,35, en particulier dans la plage de 0,007 à 0,35, de préférence dans la plage de 0,035 à 0,315, de préférence dans la plage de 0,07 à 0,28; et/ou

ladite substance (iii) ayant un paramètre de tassement critique $P_{kr}$ d'au moins 1,155, en particulier dans la plage de 1,155 à 1,225, de préférence dans la plage de 1,162 à 1,218, de préférence dans la plage de 1,169 à 1,211; et/ou

la substance (iv) ayant un paramètre de tassement critique $P_{kr}$ d'au moins 0,98, en particulier dans la plage de 0,98 à 1,19, de préférence dans la plage de 1,015 à 1,155, de préférence dans la plage de 1,05 à 1,12; et/ou

ladite substance (v) ayant un paramètre de tassement critique $P_{kr}$ d'au moins 0,7, en particulier dans la plage de 0,7 à 1,19, de préférence dans la plage de 0,77 à 1,1725, de préférence dans la plage de 0,805 à 1,155.

**6.** Système de support selon l'une quelconque des revendications 3 à 5,
le système de support étant **caractérisé par** au moins l'une des caractéristiques et/ou mesures (1b) à (5b) suivantes,
en particulier par une combinaison d'au moins deux, de préférence d'au moins trois, de préférence d'au moins quatre,
de manière particulièrement préférée de toutes les cinq caractéristiques et/ou mesures (1b) à (5b) suivantes:

(1b) la substance (i) présente un paramètre de tassement critique $P_{kr}$ d'au moins 0,5, en particulier d'au moins 0,7, notamment dans la plage de 0,7 à 1,225, de préférence dans la plage de 0,77 à 1,12, de préférence dans la plage de 0,84 à 1,05;

(2b) la substance (ii) présente un paramètre de tassement critique $P_{kr}$ d'au plus 0,35, en particulier dans la plage de 0,007 à 0,35, de préférence dans la plage de 0,035 à 0,315, de préférence dans la plage de 0,07 à 0,28;

(3b) la substance (iii) présentant un paramètre de tassement critique $P_{kr}$ d'au moins 1,155, en particulier dans la plage de 1,155 à 1,225, de préférence dans la plage de 1,162 à 1,218, de préférence dans la plage de 1,169 à 1,211;

(4b) la substance (iv) présente un paramètre de tassement critique $P_{kr}$ d'au moins 0,98, en particulier dans la plage de 0,98 à 1,19, de préférence dans la plage de 1,015 à 1,155, de préférence dans la plage de 1,05 à 1,12;

(5b) la substance (v) présente un paramètre de tassement critique $P_{kr}$ d'au moins 0,7, en particulier dans la plage de 0,7 à 1,19, de préférence dans la plage de 0,77 à 1,1725, de préférence dans la plage de 0,805 à 1,155.

**7.** Système de support selon l'une quelconque des revendications 3 à 6,
dans lequel la substance active, en particulier en tant que substance (vi), fait partie du système de support et/ou forme un composant du système de support et/ou dans lequel le système de support présente la substance active, en particulier en tant que substance (vi).

**8.** Système de support selon l'une quelconque des revendications 3 à 7,

dans lequel le principe actif est un principe actif thérapeutique et/ou prophylactique, en particulier un principe actif pharmaceutique thérapeutique et/ou prophylactique;

en particulier dans lequel le principe actif est un principe actif au moins essentiellement insoluble dans l'eau, de préférence un principe actif amphiphile au moins essentiellement insoluble dans l'eau ; et/ou en particulier dans lequel le principe actif est un principe actif hydrosoluble; et/ou

en particulier dans lequel la substance active est choisie dans le groupe des substances actives vaccinales, des substances actives antibiotiques, des substances actives antimycosiques, des substances actives antivirales, des substances actives antiphlogistiques, des substances actives antipyrétiques, des substances actives antiallergiques, des substances actives anticancéreuses, des substances actives hormonales, des substances actives anticoagulantes, des vitamines, des substances minérales, des substances absorbant les UV et leurs mélanges ainsi que des combinaisons, de préférence des substances actives vaccinales; et/ou

en particulier dans lequel le principe actif est choisi dans le groupe des ARN, en particulier des ARN messagers; du rétinol; du tocophérol; des corticostéroïdes, en particulier de la cortisone; des antibiotiques, en particulier des tétracyclines et des benzoylpénicillines; diclofénac; estradiol, estradiol hémihydraté, acide salicylique, acide acétylsalicylique; indométacine; huiles essentielles; en particulier filtres UVA amphiphiles et filtres UVB; nicotinamide; et leurs mélanges et combinaisons; et/ou

en particulier dans lequel la substance active est une substance active vaccinale, en particulier une substance active vaccinale à base d'acide nucléique et/ou une substance active vaccinale à base de gènes, de préférence une substance active vaccinale codant pour un antigène d'un agent pathogène et/ou d'un pathogène, en particulier un antigène d'un virus; et/ou

en particulier où la substance active du vaccin est choisie dans le groupe de l'ADN; de l'ARN, en particulier de l'ARN messager; des vecteurs viraux; des plasmides, en particulier de l'ADN plasmidique; de préférence de l'ARN; de préférence de l'ARNm; et leurs mélanges ainsi que des combinaisons; et/ou

en particulier dans lequel le principe actif est un principe actif de vaccin à base d'ARNm et/ou d'ARNm, en particulier dans lequel l'ARNm est réalisé sous forme monocaténaire et/ou en particulier dans lequel l'ARNm présente une structure de coiffe en 5'; et/ou en particulier dans lequel le principe actif est un principe actif de vaccin à base d'ARNm et/ou d'ARNm, en particulier dans lequel l'ARNm code pour une protéine, en particulier un antigène, d'un coronavirus, de préférence le SRAS-CoV-2, en particulier dans lequel la protéine, en particulier l'antigène, code au moins partiellement, de préférence au moins essentiellement complètement, la *protéine spike,* en particulier ancrée dans la membrane, du coronavirus, de préférence le SRAS-CoV-2; et/ou

en particulier dans lequel la substance active est une protéine et/ou un antigène, en particulier d'un agent pathogène, tel qu'une bactérie, un virus ou similaire.

**9.** Système de support selon l'une quelconque des revendications 3 à 8,

l'assemblage, en particulier l'agrégation, des constituants étant tel que le système porteur résulte sous forme particulaire, en particulier sous forme de nanoparticules lipidiques, de préférence de nanoparticules lipidiques sous forme de micelles, de vésicules et/ou de nanoparticules lipidiques discrètes, de préférence de nanoparticules lipidiques discrètes; et/ou

le système porteur étant sous forme particulaire, en particulier sous forme de nanoparticules lipidiques, de préférence de nanoparticules lipidiques sous forme de micelles, de vésicules et/ou de nanoparticules lipidiques sous forme de disque, de préférence de nanoparticules lipidiques sous forme de disque; et/ou

dans lequel les tailles de particules, en particulier les diamètres hydrodynamiques, des particules du système porteur, de préférence des nanoparticules lipidiques, présentent au moins essentiellement une distribution normale et/ou une distribution gaussienne, en particulier dans lequel les tailles de particules, en particulier les diamètres hydrodynamiques, sont déterminées par diffraction de la lumière, en particulier par diffraction laser, de préférence par diffusion dynamique de la lumière, de préférence selon la norme ISO 22412:2017 et/ou DIN ISO 22412:2018-09; et/ou

les particules du système porteur, de préférence les nanoparticules lipidiques, ayant une taille de particule, en particulier un diamètre hydrodynamique, de l'ordre de 5 nm à 10 000 nm, en particulier de l'ordre de 10 nm à 5 000 nm, de préférence de l'ordre de 20 nm à 3 000 nm, de préférence de l'ordre de 50 nm à 2 000 nm, de manière particulièrement préférée de l'ordre de 75 nm à 1 000 nm.500 nm, tout particulièrement dans la plage de 90 nm à 1 250 nm, de préférence encore dans la plage de 100 nm à 1 000 nm, de préférence encore dans la plage de 100 nm à 900 nm, en particulier déterminée par diffraction de la lumière, en particulier par diffractométrie laser, de préférence par diffusion dynamique de la lumière, de préférence selon la norme ISO 22412:2017 et/ou DIN ISO 22412:2018-09; et/ou

les particules du système porteur, de préférence les nanoparticules lipidiques, ayant une taille moyenne de particule, en particulier un diamètre hydrodynamique moyen, dans la plage de 5 nm à 2 900 nm, en particulier dans la plage de 10 nm à 2 500 nm, de préférence dans la plage de 20 nm à 2 000 nm, de préférence dans la plage de 20 nm à 2 000 nm, de préférence dans la plage de 20 nm à 2 000 nm.000 nm, de préférence dans la plage de 50 nm à 1 500 nm, de manière particulièrement préférée dans la plage de 50 nm à 1 000 nm, en particulier déterminée par diffraction de la lumière, en particulier par diffractométrie laser, de préférence par diffusion dynamique de la lumière, de préférence selon la norme ISO 22412:2017 et/ou DIN ISO 22412:2018-09; et/ou

la variance de la taille des particules, en particulier la variance du diamètre hydrodynamique, des particules du système porteur, de préférence des nanoparticules lipidiques, étant d'au plus 45 %, en particulier d'au plus 35 %, de préférence d'au plus 25 %, de préférence d'au plus 20 %, de manière particulièrement préférée d'au plus 15 %, de manière tout particulièrement préférée d'au plus 10 %, en particulier par rapport à la taille moyenne des particules ou au diamètre hydrodynamique. le diamètre hydrodynamique moyen et/ou déterminé en particulier par diffraction de la lumière, en particulier par diffractométrie laser, de préférence par diffusion dynamique de la lumière, de préférence selon la norme ISO 22412:2017 et/ou DIN ISO 22412:2018-09; et/ou

la variance de la taille des particules, en particulier la variance du diamètre hydrodynamique, des particules du système porteur, de préférence des nanoparticules lipidiques, se situant dans la plage de 5 % à 45 %, en particulier dans la plage de 10 % à 40 %, de préférence dans la plage de 15 % à 35 %, de préférence dans la plage de 20 % à 35 %, en particulier par rapport à la taille moyenne des particules ou au diamètre hydrodynamique, le diamètre hydrodynamique moyen et/ou déterminé en particulier par diffraction de la lumière, en particulier par diffractométrie laser, de préférence par diffusion dynamique de la lumière, de préférence selon la norme ISO 22412:2017 et/ou DIN ISO 22412:2018-09; et/ou

les particules du système porteur, de préférence les nanoparticules lipidiques, présentant une taille moyenne de particules D50, en particulier un diamètre hydrodynamique moyen D50, dans la plage de 2,5 nm à 950 nm, en particulier dans la plage de 7,5 nm à 850 nm, de préférence dans la plage de 10 nm à 800 nm, de préférence dans la plage de 15 nm à 750 nm, déterminée en particulier par diffraction de la lumière, en particulier par diffractométrie laser, de préférence par diffusion dynamique de la lumière, de préférence selon la norme ISO 22412:2017 et/ou DIN ISO 22412:2018-09; et/ou

les particules du système porteur, de préférence les nanoparticules lipidiques, ayant une taille moyenne de particules D90, en particulier un diamètre hydrodynamique moyen D90, dans la plage de 1 nm à 1.000 nm, en particulier dans la plage de 5 nm à 900 nm, de préférence dans la plage de 7,5 nm à 850 nm, de préférence dans la plage de 10 nm à 800 nm, en particulier déterminée par diffraction de la lumière, en particulier par diffractométrie laser, de préférence par diffusion dynamique de la lumière, de préférence selon la norme ISO 22412:2017 et/ou DIN ISO 22412:2018-09.

**10.** Système de support selon l'une quelconque des revendications 3 à 9,

l'assemblage, en particulier l'agrégation, des composants étant tel qu'il en résulte une structure liquide-cristalline, en particulier lyotrope, sur la base du système de support et du milieu liquide; et/ou

le système de support étant présent avec le milieu liquide sous la forme d'une structure cristalline liquide lyotrope à base du système de support et du milieu liquide et/ou présentant cette structure; et/ou

dans lequel le système de support est contenu et/ou incorporé dans un milieu liquide, en particulier tel que défini précédemment, en particulier dans lequel le système de support forme avec le milieu liquide une composition, en particulier une composition pharmaceutique; et/ou

dans laquelle la composition est une composition de préférence dispersée, en particulier une dispersion, de préférence telle que définie précédemment, comprenant le système de support ou le système de support chargé et/ou équipé d'un agent pharmaceutiquement actif et le milieu liquide, et/ou dans laquelle la composition, en particulier telle que définie précédemment, est sous la forme d'une dispersion du système de support ou du système de support chargé et/ou équipé d'un agent pharmaceutiquement actif dans le milieu liquide; et/ou

la composition comprenant le système porteur, de préférence les particules, de préférence les nanoparticules lipidiques, en une quantité comprise dans la plage de 5 % à 95 % en poids, en particulier dans la plage de 10 % à 90 % en poids, de préférence dans la plage de 15 % à 85 % en poids, de préférence dans la plage de 20 % à 80 % en poids, de manière particulièrement préférée dans la plage de 25 % à 75 % en poids, par rapport à la composition; et/ou

le système porteur étant présent dans la composition sous forme particulaire, notamment sous forme de nanoparticules lipidiques, de préférence de nanoparticules lipidiques sous forme de micelles, de vésicules et/ou de nanoparticules lipidiques discoïdes, de préférence de nanoparticules lipidiques discoïdes, notamment telles que définies précédemment.

11. Système de support selon l'une quelconque des revendications 3 à 10,

la composition présentant une activité de l'eau inférieure à 1, en particulier au plus égale à 0,9, de préférence au plus égale à 0,7, de préférence au plus égale à 0,5, de manière particulièrement préférée au plus égale à 0,3; et/ou

ladite composition ayant une activité de l'eau dans la plage de 0 à 1, en particulier dans la plage de 0 à 0,9, de préférence dans la plage de 0 à 0,7, de préférence dans la plage de 0 à 0,5, de manière particulièrement préférée dans la plage de 0 à 0,3;

déterminée en particulier conformément à la norme ISO 21807:2004 et/ou à la norme ISO 18787:2017 et/ou à la norme 21 CFR Part 11, notamment telle que déposée dans la norme ISO 29621:2017; et/ou

le système de support et/ou la composition, en particulier le système de support, présentant une valeur Log P, en particulier une valeur Log P totale, d'au plus 5, en particulier d'au plus 4, de préférence d'au plus 3, de manière particulièrement préférée d'au plus 2, de manière encore plus préférée d'au plus 1; et/ou

le système de support et/ou la composition, en particulier le système de support, présentant une valeur Log P, en particulier une valeur Log P totale, dans la plage de 0,1 à 5, en particulier dans la plage de 0,2 à 4, de préférence dans la plage de 0,3 à 3, de manière particulièrement préférée dans la plage de 0,4 à 2; et/ou

les composants et/ou substances du système porteur, en particulier les substances (i), (ii), (iii), (iv) et/ou (v), étant sélectionnés avec la condition que le système porteur résultant présente une valeur Log P, en particulier une valeur Log P totale, d'au plus 5, en particulier d'au plus 4, de préférence d'au plus 3, de manière particulièrement préférée d'au plus 2, de manière encore plus préférée d'au plus 1; et/ou

les composants et/ou substances du système porteur, en particulier les substances (i), (ii), (iii), (iv) et/ou (v), étant sélectionnés avec la condition que le système porteur résultant présente une valeur Log P, en particulier une valeur Log P totale, dans la plage de 0,1 à 5, en particulier dans la plage de 0,2 à 4, de préférence dans la plage de 0,3 à 3, de manière particulièrement préférée dans la plage de 0,4 à 2.

12. Composition, notamment composition pharmaceutique, ladite composition comprenant au moins un système porteur à base de lipides sous forme particulaire, de préférence sous forme de nanoparticules lipidiques, notamment pour un principe actif pharmaceutique; et/ou au moins un système porteur à base de lipides sous forme particulaire, de préférence sous forme de nanoparticules lipidiques, chargé et/ou équipé d'un principe actif pharmaceutique, selon l'une quelconque des revendications 3 à 11, dans un milieu liquide, notamment dans un milieu liquide à une température comprise entre 20°C et 40°C et à la pression ambiante, de préférence sous forme d'un milieu de dispersion.

13. Médicament ou médicament, notamment destiné à être utilisé dans le traitement prophylactique et/ou thérapeutique de maladies du corps humain ou animal,

dans lequel le médicament ou le produit pharmaceutique comprend ou est constitué d'au moins un système de support à base de lipides selon les revendications 3 à 11 ou d'une composition selon la revendication 12, en particulier conjointement avec au moins un excipient pharmaceutiquement acceptable et/ou pharmaceutiquement tolérable.

14. Système de support à base de lipides selon l'une quelconque des revendications 3 à 11 ou composition selon la revendication 12, destiné à être utilisé dans le traitement prophylactique et/ou thérapeutique de maladies du corps humain ou animal.

15. Utilisation d'au moins un paramètre de procédé pour la commande, en particulier pour le contrôle du procédé et/ou du procédé, d'un procédé selon la revendication 1 ou 2, le paramètre de procédé étant choisi dans le groupe de la température, de la conductivité, de la viscosité, du pH, de la résistance électrique et de la tension superficielle, en particulier de la tension superficielle dynamique, de préférence dans le groupe de la température, de la conductivité, de la viscosité et du pH, en particulier respectivement du milieu liquide, de préférence avec les constituants et/ou substances qui y sont introduits, et/ou de la composition.

Fig. 1A

Fig. 1B

Fig. 1C

Fig. 2

Fig. 3

Fig. 4

Fig. 5

Fig. 6A

Fig. 6B

Fig. 7

Fig. 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2018078053 A1
- EP 3532094 A1
- US 20200163878 A1
- US 2021251898 A1
- WO 2018006052 A1
- WO 2019051289 A1
- WO 2017194454 A1
- WO 2021022173 A1
- WO 2014136086 A1

### In der Beschreibung aufgeführte Nicht-Patentliteratur

- **KIMURA, N. et al.** Development of a Microfluidic-Based Post-Treatment Process for Size-Controled Lipid Nanoparticles and Application to siRNA Delivery. *Applied Materials and Interfaces*, 15 July 2020, vol. 12 (30), 34011-34020
- **UMERSKA, A. et al.** Antibacterial action of lipid nanocapsules containing fatty acids or monoglycerides as co-surfactants. *European Journal of Pharmaceutics and Biopharmaceutics, Official Journal of Arbeitsgemeinschaft für Pharmazeutische Verfahrenstechnik*, October 2012, vol. 108
- **GRIFFIN**. W.C. Classification of Surface Active Agent by HLB. *J. SOC. Cosmet. Chem.*, 1949, vol. 1
- Heusch, eine experimentelle Methode zur Bestimmung des HLB-Werts von Tensiden. *Cholloid-Zeitschrift und Zeitschrift für Polymere*, vol. 236
- **CHENG, T. et al.** Computation of Octanol-Water Partition Coefficients by Guiding an Additive Model with Knowledge. *J. Chem. Inf. Model.*, 2007, vol. 47, 2140-2148